# FASCICULE DE BREVET EUROPEEN

(11) **EP 1 689 720 B1**
(45) Date de publication et mention de la délivrance du brevet: **15.04.2009**
(21) Numéro de dépôt: 04805499.3
(22) Date de dépôt: 22.11.2004
(51) Int. Cl.: C07D 231/20, C07D 453/02, C07D 401/06, C07D 487/08, C07D 453/06, C07D 403/06, C07D 231/14, C07D 231/12, C07D 401/04, C07D 409/04, C07D 403/04, A61K 31/41, A61K 31/435

(54) **DERIVES DE PYRAZOLE A TITRE DE MEDICAMENTS POUR LE TRAITEMENT DES DEGENERESCENCES NEURONALES AIGUES OU CHRONIQUES**
PYRAZOLYLDERIVATIVE IN FORM VON ARZNEIMITTELN ZUR BEHANDLUNG VON AKUTEN ODER CHRONISCHEN NEURONALEN REGRESSIONEN
PYRAZOLYL DERIVATIVES IN THE FORM OF DRUGS FOR TREATING ACUTE OR CHRONIC NEURONAL REGRESSIONS

(30) Priorité: 25.11.2003 FR 0313775
(43) Date de publication de la demande: 16.08.2006
(73) Titulaire: Aventis Pharma S.A., 92160 Antony (FR)
(72) Inventeur: GENEVOIS-BORELLA, Arielle, F-94320 THIAIS (FR); MALLERON, Jean-Luc, F-91460 MARCOUSSIS (FR); BOUQUEREL, Jean, F-93700 DRANCY (FR); DOERFLINGER, Gilles, F-91940 LES ULIS (FR); BOHME, Andrees, F-75020 PARIS (FR); TOUYER, Gaetan, F-77500 CHELLES (FR); SABUCO, Jean-François, F-75014 PARIS (FR); TERRIER, Corinne, F-93190 LIVRY GARGAN (FR); MIGNANI, Serge, F-92290 CHATENAY-MALABRY (FR); EVERS, Michel, F-94510 LA QUEUE-EN-BRIE (FR); EL-AHMAD, Youssef, F-94000 CRETEIL (FR)
(74) Mandataire: Morel-Pécheux, Muriel
(86) Numéro de dépôt international: PCT/FR2004/002968
(87) Numéro de publication internationale: WO 2005/051917

(56) Documents cités:
- EP-A- 0 427 390
- EP-A- 0 525 879
- EP-A- 1 004 592
- US-A- 5 409 946
- PLUEMPE ET AL.: "Einige neue in 4-Stellung substituierte Pyrazole" ARCHIV DER PHARMAZIE UND BERICHTE DER DEUTSCHEN PHARMAZEUTISCHEN GESELLSCHAFT, vol. 300, no. 8, 1967, pages 704-708, XP009028438

## Description

La présente invention concerne de nouveaux ligands des récepteurs à l'acétylcholine de type nicotiniques. Ces composés sont caractérisés plus particulièrement en ce qu'ils sont des ligands des récepteurs nicotiniques de type α7. Ces propriétés suggèrent que les composés de l'invention puissent être utilisés, chez les animaux y compris l'homme, comme traitement curatif et/ou symptomatique pour la prévention, le diagnostic et/ou le suivi de l'évolution des troubles ou maladies impliquant une perturbation du fonctionnement des récepteurs nicotiniques ou répondant favorablement à une modulation de ceux-ci. Plus particulièrement, les composés de l'invention pourraient être utiles dans des troubles ou maladies psychiatriques ou neurologiques du système nerveux central impliquant des altérations des fonctions cognitives, de l'attention, des facultés de concentration, des capacités d'apprentissage et de mémorisation, ou du traitement de l'information sensorielle. Ils peuvent également être utiles dans le traitement, la prévention, le diagnostic et/ou le suivi de l'évolution de maladies impliquant des processus neurodégénératifs spontanés ou consécutifs à des lésions, et de maladies impliquant des phénomènes inflammatoires. La présente invention se réfère également aux méthodes de traitement impliquant des récepteurs nicotiniques consistant en l'administration à des animaux y compris l'homme de doses thérapeutiquement efficaces d'un ou plusieurs composés de l'invention. La présente invention concerne également l'utilisation à des fins diagnostiques d'analogues de ces dérivés dans lesquels un ou plusieurs atomes ont été remplacés par un isotope de masse atomique ou de nombre de masse différent de la masse atomique ou du nombre de masse des atomes habituellement rencontrés dans la nature.

Certains dérivés, de pyrazoles sont déjà connus dans la littérature (EP0427390, EP0525879, US5409946). Le document EP0427390 décrit des composés azabicycliques pour le traitement de la démence comme agonistes muscariniques. Le document EP0525879 décrit des dérivés de pyrazole comme ayant une forte affinité pour les récepteurs muscariniques. Le document US5409946 décrit des dérivés d'isoxazole, isothiazole et pyrazole qui renforcent les fonctions cognitives.

La présente invention a donc pour objet l'utilisation de dérivés de pyrazole de formule (I) 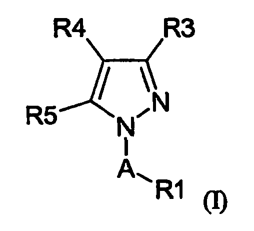 et leurs sels pharmaceutiquement acceptables pour la préparation de médicaments, compositions pharmaceutiques en tant que ligands des récepteurs nicotiniques α7.

De nombreux troubles ou maladies sont liées à un dysfonctionnement des récepteurs nicotiniques et peuvent ainsi bénéficier d'une modulation de ceux-ci par les composés de l'invention pour en corriger les symptômes et/ou en ralentir, arrêter ou inverser l'évolution. Les composés de l'invention sont à cet égard plus particulièrement intéressants dans le cas de troubles ou de maladies psychiatriques ou neurologiques du système nerveux central tels que par exemple les altérations des facultés d'apprentissage, de concentration et de mémorisation, les altérations cognitives légères, les démences séniles, les démences vasculaires, les démences à corps de Levy, la maladie d'Alzheimer, la maladie de Parkinson, la chorée de Huntington, le syndrome de Gilles de la Tournettes, les dégénérescences neuronales consécutives à un traumatisme, aux accidents vasculaires cérébraux, à l'ischémie ou à l'hypoxie cérébrale, l'atrophie multisystématisée, la paralysie supranucléaire progressive, la sclérose latérale amyotrophique, les neuropathies périphériques, les troubles moteurs tels que les dyskinésies, les dyskinésies tardives, les hyperkinésies, les dystonies et les épilepsies, les déficits de l'attention liés à l'hyperactivité, la schizophrénie, la dépression, la psychose maniaco-dépressive, l'anxieté, les phobies, les troubles obsessifs compulsifs, le syndrome de stress post-traumatique, les attaques de panique, les troubles de l'alimentation tels que l'anorexie, la boulimie et l'obésité, les troubles du sommeil y compris ceux associés aux décalages horaires. Les composés de l'invention peuvent être utiles pour instaurer une réduction de la consommation de substances addictives, pour aider au maintien de l'abstinence vis à vis de celles-ci ou pour en atténuer les symptômes du sevrage. Dans le cadre de la présente invention, le terme de « substance addictive » s'applique à des substances licites ou illicites dont la consommation peut donner lieu à abus et/ou dépendance telles que par exemple la nicotine et les produits du tabac, l'alcool, les dérivés du cannabis, les opiacés, la cocaine, les barbituriques, les benzodiazépines et les psychostimulants.

Les composés de l'invention pourraient également présenter un intérêt dans le traitement des douleurs aiguës ou chroniques telles que les douleurs post-chirurgicales, les douleurs consécutives à une amputation (douleur du membre fantôme), les douleurs associées aux lésions cancéreuses, aux migraines, aux neuropathies et les douleurs musculaires telles que les fibromyalgies. De plus, les composés de l'invention pourraient également être utilisés dans le cadre du traitement de troubles ou maladies impliquant des processus inflammatoires tels que par exemple, au niveau du tractus gastro-intestinal, la colite ulcéreuse, la maladie de Crohn, le syndrome du colon irritable, les diarrhées, et ailleurs dans l'organisme les arthrites (y-compris l'arthrite rhumatoide) et les inflammations cutanées telles que l'acnée. Enfin, les composés de l'invention pourraient être utiles dans les désordres endocriniens tels que le phéochromocytome et les troubles associés à la contraction des muscles lisses.

La présente invention couvre également l'utilisation des composés de l'invention à des fins diagnostiques ou d'imagerie médicale. Elle comprend les méthodes diagnostiques et d'imagerie consistant en l'analyse par des méthodes non-invasives de la distribution d'un composé-traceur à l'intérieur du corps intact d'un animal y compris l'homme à l'aide de moyens physiques tels que la tomographie par émission de positons, la tomographie à photon unique, la spectroscopie par résonance magnétique et l'imagerie par résonance magnétique nucléaire, la tomodensitométrie aux rayons X assistée par ordinateur (scanner) ou une combinaison de ces techniques. Dans le cadre de la présente invention, le terme « composé-traceur » désigne les composés de l'invention, leurs énantiomères ou leurs pro-drogues utilisés ou non sous une forme marquée permettant leur détection par des moyens physiques tels que décrits plus haut. Le marquage consiste en le remplacement d'un ou plusieurs atomes dans les composés de l'invention par un isotope de masse atomique ou de nombre de masse différent de la masse atomique ou du nombre de masse de ces atomes tels qu'ils sont habituellement rencontrés dans la nature. Il peut également consister en l'adjonction aux composés de l'invention de groupements chimiques porteurs de tels isotopes à l'aide par exemple de réactifs de méthylation. Les isotopes utilisés peuvent être par exemple des radionuclides isotopes de l'hydrogène, du carbone, de l'azote, de l'oxygène, du fluor, du phosphore, du soufre, du chlore, de l'iode ou du technétium tels que respectivement ²H, ³H, ¹¹C, ¹³C, ¹⁴C, ¹³N, ¹⁵O, ¹⁷O, ¹⁸F, ³⁵S, ³⁶Cl, ¹²³I, ¹²⁵I, ¹³¹I. Les composés marqués peuvent être synthétisés selon les méthodes décrites dans les modes opératoires de la présente invention en substituant un ou plusieurs réactifs dans le processus de synthèse par des réactifs identiques contenant le ou les isotopes marqueurs.

La présente invention concerne des dérivés de formule (I) dans laquelle :

A est, si il est présent, un radical alkyle (C1-C6), un radical alkényle (C3-C6), un radical alkynyle (C3-C6), un radical cycloalkyle (C3-C7), un radical cycloalkényle (C5-C7), ces radicaux sont éventuellement substitués par 1 ou plusieurs substituants choisis parmi alkyle (C1-C5), alkényle (C2-C5), alkynyle (C2-C5), cycloalkyle (C3-C7), cycloalkényle (C5-C7), arylalkyle, hétéroarylalkyle, aryle, hétéroaryle, halogène,

R1 est un groupement NR6R7, azacycloalkyle (C4-C7), azacycloalkényle (C5-C7) azabicycloalkyle (C5-C9), azabicycloallcényle (C5-C9), ces groupements sont éventuellement substitués par 1 ou plusieurs substituants choisis parmi alkyle (C1-C5), cycloalkyle (C3-C5), halogène,

A-R1 est tel que l'azote de R1 et l'azote 1 du pyrazole sont obligatoirement séparés par au moins deux atomes de carbone,

R3 est un radical H, halogène, OH, SH, NH2, ORc, SRc, SORa, SO2Ra, NHCHO, NRaRb, NHC(O)Ra, NHC(S)Ra, NHSO2Ra ,

R4 est un radical aryle ou hétéroaryle étant éventuellement substitué par 1 ou plusieurs substituants choisis parmi halogène, CN, NO₂, NH₂, OH, SH, COOH, CHO, C(O)NH2, C(S)NH2, SO2H, SO2NH2, NHCHO, C(O)Ra, C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra aryle, hétéroaryle, hétérocycloalkyle (C4-C7), polyfluoroalkyle, trifluorométhylsulfanyle, trifluorométhoxy, (1-6C) alkyle linéaire ou ramifié, alkényle (C2-C6), alkynyle (C2-C6), ces substituants étant éventuellement substitués par un ou plusieurs alkyle, halogène, OH, méthoxy,

R5 est un radical H, halogène, CF3, CHF2, CH2F, alkyle (Cl -C6) linéaire ou ramifié, cycloalkyl (C3-C7),

Ra est (1-6C) alkyle linéaire ou ramifié, alkényle, alkynyle , cycloalkyle (C3-C7), cycloalkényle (C5-C7), hétérocycloalkyle (C4-C7)hétérocycloalkyle (C4-C7), arylalkyle, hétéroarylalkyle, aryle, hétéroaryle, polyfluoroalkyle,

Rb est indépendamment de Ra un hydrogène, (1-6C) alkyle linéaire ou ramifié, alkényle, alkynyle, cycloalkyle (C3-C7), cycloalkényle (C5-C7), hétérocycloalkyle (C4-C7)hétérocycloalkyle (C4-C7), arylalkyle, hétérogrylalkyle, aryle, hétéroaryle, polyfluoroalkyle,

Ra et Rb peuvent former un cycle saturé ou insaturé à 5, 6 ou 7 chaînons ayant ou non un hétéroatome tel que O, S, N, ce cycle étant éventuellement substitué par un ou plusieurs radicaux alkyle, halogène,

Rc est un radical, alkyle linéaire ou ramifié (C1-C6), alkényle (C3-C6), alkynyle (C3-C6), cycloalkyle (C3-C7), cycloalkényle (C5-C7), hétérocycloalkyle (C4-C7)hétérocycloalkyle (C4-C7), (hétéro)arylalkyle, (hétéro)aryle, (poly)fluoroalkyle, C(O)R8, C(S)R8, SO2R8,

R6 et R7 sont indépendamment l'un de l'autre un hydrogène, alkyle (C1-C6), alkényle (C3-C6), alkynyle (C3-C6), cycloalkyle (C3-C7), cycloalkényle (C5-C7), hétérocycloalkyle (C4-C7), un arylalkyle, hétéroarylalkyle,

R6 et R7 peuvent former un cycle saturé ou insaturé à 5, 6 ou 7 chaînons ayant ou non un hétéroatome tel que O, S, N et qui est éventuellement substitué par un ou plusieurs alkyles, halogènes,

R8 est un radical Ra ou NRaRb,
leurs racémiques, énantiomères, diastéréoisomères et leurs mélanges, leurs tautomères et leurs sels pharmaceutiquement acceptables.

De manière préférée, la présente invention concerne des dérivés de formule (I) dans laquelle :
A est, si il est présent, un radical alkyle (C1-C6), un radical alkényle (C3-C6), un radical alkynyle (C3-C6), un radical cycloalkyle (C3-C7), un radical cycloalkényle (C5-C7), ces radicaux sont éventuellement substitués par 1 ou plusieurs substituants choisis parmi alkyle (C1-C5), alkényle (C2-C5), alkynyle (C2-C5), cycloalkyle (C3-C7), cycloalkényle (C5-C7), arylalkyle, hétéroarylalkyle, aryle, hétéroaryle, halogène,
R1 est un groupement NR6R7, azacycloalkyle (C4-C7), azacycloalkényle (C5-C7) azabicycloalkyle (C5-C9), azabicycloallcényle (C5-C9), ces groupements sont éventuellement substitués par 1 ou plusieurs substituants choisis parmi alkyle (C1-C5), cycloalkyle (C3-C5), halogène,
A-R1 est tel que l'azote de R1 et l'azote 1 du pyrazole sont obligatoirement séparés par au moins deux atomes de carbone,
R3 est un radical OH, NH2, OMe, H
R4 est un radical aryle ou hétéroaryle étant éventuellement substitué par 1 ou plusieurs substituants choisis parmi halogène, CN, NO₂, NH₂, OH, SH, COOH, CHO, C(O)NH2, C(S)NH2, SO2H, SO2NH2, NHCHO, C(O)Ra, C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra aryle, hétéroaryle, hétérocycloalkyle (C4-C7), polyfluoroalkyle, trifluorométhylsulfanyle, trifluorométhoxy, (1-6C) alkyle linéaire ou ramifié, alkényle (C2-C6), alkynyle (C2-C6), ces substituants étant éventuellement substitués par un ou plusieurs alkyle, halogène, OH, méthoxy,
R5 est un radical hydrogène ou Me,
Ra est (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Rb est indépendamment de Ra un hydrogène, (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Ra et Rb peuvent former un cycle saturé ou insaturé à 5, 6 ou 7 chaînons ayant ou non un hétéroatome tel que O, S, N, ce cycle étant éventuellement substitué par un ou plusieurs radicaux alkyle, halogène,
Rc est un radical, alkyle linéaire ou ramifié (C1-C6), alkényle (C3-C6), alkynyle (C3-C6), cycloalkyle (C3-C7), cycloalkényle (C5-C7), hétérocycloalkyle (C4-C7), (hétéro)arylalkyle, (hétéro)aryle, (poly)fluoroalkyle, C(O)R8, C(S)R8, SO2R8,
R6 et R7 sont indépendamment l'un de l'autre un hydrogène, alkyle (C1-C6), alkényle (C3-C6), alkynyle (C3-C6), cycloalkyle (C3-C7), cycloalkényle (C5-C7), hétérocycloalkyle (C4-C7), un arylalkyle, hétéroarylalkyle,
R6 et R7 peuvent former un cycle saturé ou insaturé à 5, 6 ou 7 chaînons ayant ou non un hétéroatome tel que O, S, N et qui est éventuellement substitué par un ou plusieurs alkyles, halogènes,
R8 est un radical Ra ou NRaRb,
leurs racémiques, énantiomères, diastéréoisomères et leurs mélanges, leurs tautomères et leurs sels pharmaceutiquement acceptables.

Plus particulièrement la présente invention concerne des dérivés de formule (I) dans laquelle :
A est, si il est présent, un radical alkyle (C1-C6), un radical alkényle (C3-C6), un radical alkynyle (C3-C6), un radical cycloalkyle (C3-C7), un radical cycloalkényle (C5-C7), ces radicaux sont éventuellement substitués par 1 ou plusieurs substituants choisis parmi alkyle (C1-C5), alkényle (C2-C5), alkynyle (C2-C5), cycloalkyle (C3-C7), cycloalkényle (C5-C7), arylalkyle, hétéroarylalkyle, aryle, hétéroaryle, halogène,
R1 est un groupement NR6R7, azacycloalkyle (C4-C7), azacycloalkényle (C5-C7) azabicycloalkyle (C5-C9), azabicycloalkényle (C5-C9), ces groupements sont éventuellement substitués par 1 ou plusieurs substituants choisis parmi alkyle (C1-C5), cycloalkyle (C3-C5), halogène,
A-R1 est tel que l'azote de R1 et l'azote 1 du pyrazole sont obligatoirement séparés par au moins deux atomes de carbone,
R3 est un radical OH, NH2, OMe, H
R4 est un radical aryle ou hétéroaryle étant éventuellement substitué par 1 ou plusieurs substituants choisis parmi halogène, CN, NO₂, NH₂, OH, SH, COOH, CHO, C(O)NH2, C(S)NH2, SO2H, SO2NH2, NHCHO, C(O)Ra, C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra aryle, hétéroaryle, hétérocycloalkyle (C4-C7), polyfluoroalkyle, trifluorométhylsulfanyle, trifluorométhoxy, (1-6C) alkyle linéaire ou ramifié, alkényle (C2-C6), alkynyle (C2-C6), ces substituants étant éventuellement substitués par un ou plusieurs alkyle, halogène, OH, méthoxy,
R5 est un hydrogène,
Ra est (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Rb est indépendamment de Ra un hydrogène, (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Ra et Rb peuvent former un cycle saturé ou insaturé à 5, 6 ou 7 chaînons ayant ou non un hétéroatome tel que O, S, N, ce cycle étant éventuellement substitué par un ou plusieurs radicaux alkyle, halogène,
Rc est un radical, alkyle linéaire ou ramifié (C1-C6), alkényle (C3-C6), alkynyle (C3-C6), cycloalkyle (C3-C7), cycloalkényle (C5-C7), hétérocycloalkyle (C4-C7), (hétéro)arylalkyle, (hétéro)aryle, (poly)fluoroalkyle, C(O)R8, C(S)R8, SO2R8,
R6 et R7 sont indépendamment l'un de l'autre un hydrogène, alkyle (C1-C6), alkényle (C3-C6), alkynyle (C3-C6), cycloalkyle (C3-C7), cycloalkényle (C5-C7), hétérocycloalkyle (C4-C7), un arylalkyle, hétéroarylalkyle,
R6 et R7 peuvent former un cycle saturé ou insaturé à 5, 6 ou 7 chaînons ayant ou non un hétéroatome tel que O, S, N et qui est éventuellement substitué par un ou plusieurs alkyle, halogènes,
R8 est un radical Ra ou NRaRb,
leurs racémiques, énantiomères, diastéréoisomères et leurs mélanges, leurs tautomères et leurs sels pharmaceutiquement acceptables.

Dans les définitions précédentes et celles qui suivent, les radicaux alkyles (C1-C6) contiennent 1 à 6 atomes de carbone en chaîne droite ou ramifiée; les radicaux cycloalkyles (C3-C7) contiennent 3 à 7 atomes de carbone ; les radicaux alkényles contiennent 2 à 6 atomes de carbone et une à 2 doubles liaisons conjuguées ou non en chaîne droite ou ramifiée, la double liaison n'étant pas en alpha d'un hétéroatome ; les radicaux alkynyles contiennent 2 à 6 atomes de carbone et 1 à 2 triples liaisons conjuguées ou non en chaîne droite ou ramifiée, la triple liaison n'étant pas en alpha d'un hétéroatome; les radicaux aryles sont choisis parmi phényle, naphtyle ou indényle; les radicaux hétéroaryles contiennent 3 à 10 chaînons, contenant éventuellement un ou plusieurs hétéroatomes choisis parmi oxygène, soufre et azote en particulier, thiazolyle, thiényle, pyrrolyle, pyrazolyle, pyridinyle, furyle, imidazolyle, oxazolyle, pyrazinyle, pyrimidyle, tétrazolyle, oxadiazolyle, thiadiazolyle, isoxadiazolyle, isothiadiazolyle, isothiazolyle, isoxazolyle, triazolyle, indolyle, benzofuranyle, benzothiényle, azaindolyle, pyrazolyle, indolyle ; le radical halogène est soit, chlore, iode, fluor, brome ; les radicaux azacycloalkyles (C4-C7) contiennent un azote et 4 à 7 atomes de carbone et en particulier azétidinyle, pyrrolidinyle, pipéridinyle ; les radicaux azacycloalkényles contiennent un azote et 5 à 7 atomes de carbone; les radicaux azabicycloalkyles (C5-C9) contiennent 5 à 9 atomes de carbone et sont illustrés de manière non limitative dans la liste (A) ; les radicaux azabicycloalkényles (C5-C9) contiennent 5 à 9 atomes de carbone et sont illustrés de manière non limitative dans la liste (B); les radicaux hétérocycloalkyles (C4-C7) contiennent 5 à 7 atomes de carbone et 1 à plusieurs hétéroatomes choisis parmi oxygène, soufre et azote ; les radicaux polyfluoroalkyles contiennent 1 à 6 atomes de carbone en chaîne droite ou ramifiée qui sont substitués par 1 ou plusieurs atomes de fluor et en particulier trifluorométhyl, difluorométhyl ;

A titre d'illustration voici des structures de 1a Liste (A), ces structures peuvent être reliées au noyau principal par n'importe laquelle de leur position :

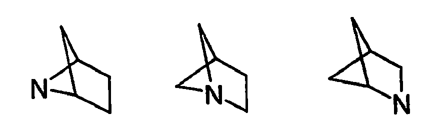

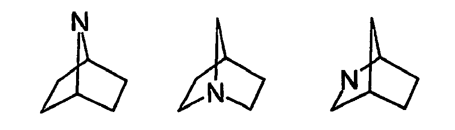

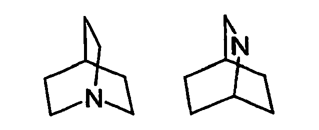

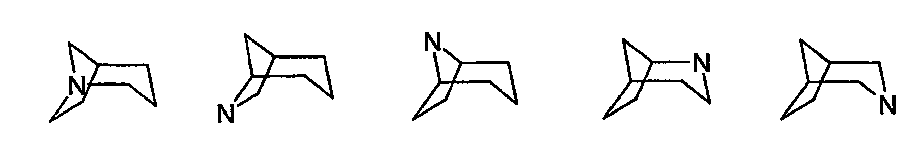

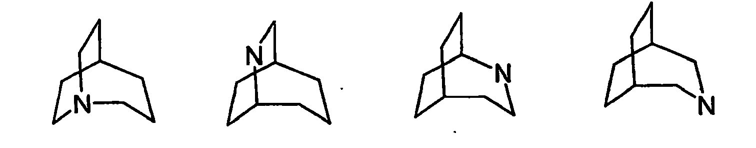

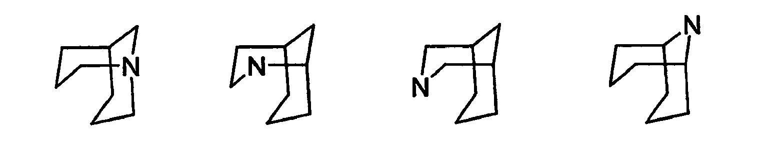

A titre d'illustration voici des structures de la Liste (B) ces structures peuvent être reliées au noyau principal par n'importe laquelle de leur position : 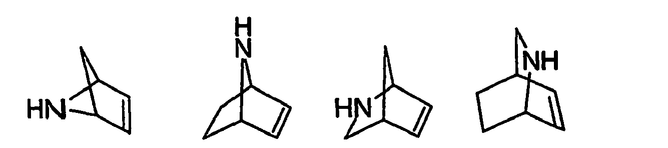 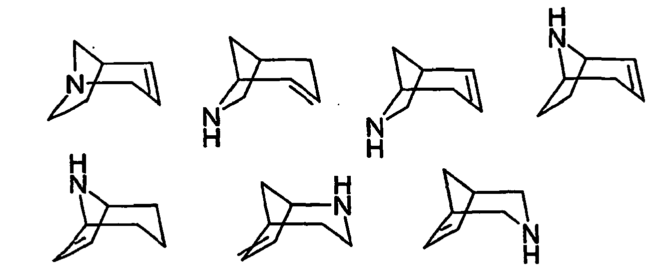 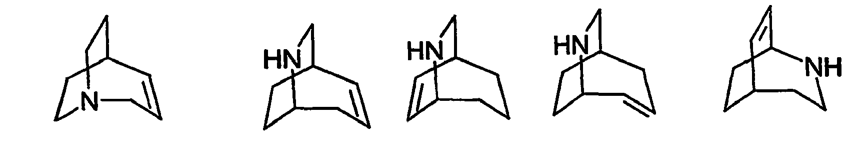 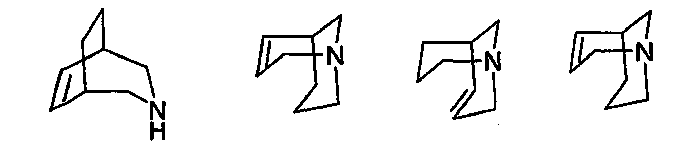 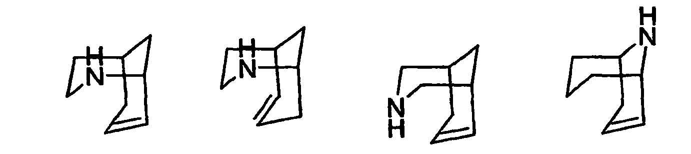 choisis parmi alkyle (C1-C5), alkényle (C2-C5), alkynyle (C2-C5), cycloalkyl (C3-C7), cycloalkényle (C5-C7), arylalkyle, hétéroarylalkyle, aryle, hétéroaryle, halogène,
R1 est un groupement NR6R7, azacycloalkyle (C4-C7), azacycloalkényle (C5-C7) azabicycloalkyle (C5-C9), azabicycloalkényle (C5-C9), ces groupements sont éventuellement substitués par 1 ou plusieurs substituants choisis parmi alkyle (C1-C5), cycloalkyle (C3-C5), halogène,
A-R1 est tel que l'azote de R1 et l'azote 1 du pyrazole sont obligatoirement séparés par au moins deux atomes de carbone,
R3 est un radical H, halogène, OH, SH, NH2, ORc, SRc, SORa, SO2Ra, NHCHO, NRaRb, NHC(O)Ra, NHC(S)Ra, NHSO2Ra ,
R4 est un radical aryle ou hétéroaryle étant éventuellement substitué par 1 ou plusieurs substituants choisis parmi halogène, CN, NO₂, NH₂, OH, SH, COOH, CHO, C(O)NH2, C(S)NH2, SO2H, SO2NH2, NHCHO, C(O)Ra, C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra, aryle, hétéroaryle, hétérocycloalkyle (C4-C7), polyfluoroalkyle, triffuorométhylsulfanyle, trifluorométhoxy, (1-6C) alkyle linéaire ou ramifié, alkényle (C2-C6), alkynyle (C2-C6), ces substituants étant éventuellement substitués par un ou plusieurs alkyle, halogène, OH, méthoxy,
R5 est un radical H, halogène, CF3, CHF2, CH2F, alkyle (C1-C6) linéaire ou ramifié, cycloalkyl (C3-C7),
Ra est (1-6C) alkyle linéaire ou ramifié, alkényle, alkynyle, cycloalkyle (C3-C7), cycloalkényle (C5-C7), hétérocycloalkyle (C4-C7), arylalkyle, hétéroarylalkyle, aryle, hétéroaryle, polyfluoroalkyle,
Rb est indépendamment de Ra un hydrogène, (1-6C) alkyle linéaire ou ramifié, alkényle, alkynyle, cycloalkyle (C3-C7), cycloalkényle (C5-C7), hétérocycloalkyle (C4-C7), arylalkyle, hétéroarylalkyle, aryle, hétéroaryle, polyfluoroalkyle,
Ra et Rb peuvent former un cycle saturé ou insaturé à 5, 6 ou 7 chaînons ayant ou non un hétéroatome tel que O, S, N, ce cycle étant éventuellement substitué par un ou plusieurs radicaux alkyle, halogène,
Rc est un radical, alkyle linéaire ou ramifié (C1-C6), alkényle (C3-C6), alkynyle (C3-C6), cycloalkyle (C3-C7), cycloalkényle (C5-C7), hétérocycloalkyle (C4-C7), (hétéro)arylalkyle, (hétéro)aryle, (poly)fluoroalkyle, C(O)R8, C(S)R8; SO2R8,
R6 et R7 sont indépendamment l'un de l'autre un hydrogène, alkyle (C1-C6), alkényle (C3-C6), alkynyle (C3-C6), cycloalkyle (C3-C7), cycloalkényle (C5-C7), hétérocycloalkyle (C4-C7), un arylalkyl, hétéroarylalkyle,
R6 et R7 peuvent former un cycle saturé ou insaturé à 5, 6 ou 7 chaînons ayant ou non un hétéroatome tel que O, S, N et qui est éventuellement substitué par un ou plusieurs alkyles, halogènes,
R8 est un radical Ra ou NRaRb,
leurs racémiques, énantiomères, diastéréoisomères et leurs mélanges, leurs tautomères et leurs sels pharmaceutiquement acceptables.

De manière préférée, la présente invention concerne également les compostions pharmaceutiques contenant en tant que principe actif un dérivé de formule (I) dans laquelle :
A est, si il est présent, un radical alkyle (C1-C6), un radical alkényle (C3-C6), un radical alkynyle (C3-C6), un radical cycloalkyle (C3-C7), un radical cycloalkényle (C5-C7), ces radicaux sont éventuellement substitués par 1 ou plusieurs substituants choisis parmi alkyle (C1-C5), alkényle (C2-C5), alkynyle (C2-C5), cycloalkyle (C3-C7), cycloalkényle (C5-C7), arylalkyle, hétéroarylalkyle, aryle, hétéroaryle, halogène,
R1 est un groupement NR6R7, azacycloalkyle (C4-C7), azacycloalkényle (C5-C7) azabicycloalkyle (C5-C9), azabicycloalkényle (C5-C9), ces groupements sont éventuellement substitués par 1 ou plusieurs substituants choisis parmi alkyle (C1-C5), cycloalkyle (C3-C5), halogène,
A-R1 est tel que l'azote de R1 et l'azote 1 du pyrazole sont obligatoirement séparés par au moins deux atomes de carbone,
R3 est un radical OH, NH2, OMe, H
R4 est un radical aryle ou hétéroaryle étant éventuellement substitué par 1 ou plusieurs substituants choisis parmi halogène, CN, NO₂, NH₂, OH, SH, COOH, CHO, C(O)NH2, C(S)NH2, SO2H, SO2NH2, NHCHO, C(O)Ra, C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra, aryle, hétéroaryle, hétérocycloalkyle (C4-C7), polyfluoroalkyle, trifluorométhylsulfanyle, trifluorométhoxy, (1-6C) alkyle linéaire ou ramifié, alkényle (C2-C6), alkynyle (C2-C6), ces substituants étant éventuellement substitués par un ou plusieurs alkyle, halogène, OH, méthoxy,
R5 est un radical hydrogène ou Me,
Ra est (1-6C) alkyle linéaire ou ramifié, alkényle, alkynyle, cycloalkyle (C3-C7), cycloalkényle (C5-C7), hétérocycloalkyle (C4-C7), arylalkyle, hétéroarylalkyle, aryle, hétéroaryle, polyfluoroalkyle,
Rb est indépendamment de Ra un hydrogène, (1-6C) alkyle linéaire ou ramifié, alkényle, alkynyle, cycloalkyle (C3-C7), cycloalkényle (C5-C7), hétérocycloalkyle (C4-C7), arylalkyle, hétéroarylalkyle, aryle, hétéroaryle, polyfluoroalkyle,
Ra et Rb peuvent former un cycle saturé ou insaturé à 5, 6 ou 7 chaînons ayant ou non un hétéroatome tel que O, S, N, ce cycle étant éventuellement substitué par un ou plusieurs radicaux alkyle, halogène,
Rc est un radical, alkyle linéaire ou ramifié (C1-C6), alkényle (C3-C6), alkynyle (C3-C6), cycloalkyle (C3-C7), cycloalkényle (C5-C7), hétérocycloalkyle (C4-C7), (hétéro)arylalkyle, (hétéro)aryle, (poly)fluoroalkyle, C(O)R8, C(S)R8, SO2R8,
R6 et R7 sont indépendamment l'un de l'autre un hydrogène, alkyle (C1-C6), alkényle (C3-C6), alkynyle (C3-C6), cycloalkyle (C3-C7), cycloalkényle (C5-C7), hétérocycloalkyle (C4-C7), un arylalkyle, hétéroarylalkyle,
R6 et R7 peuvent former un cycle saturé ou insaturé à 5, 6 ou 7 chaînons ayant ou non un hétéroatome tel que O, S, N et qui est éventuellement substitué par un ou plusieurs alkyles, halogènes,
R8 est un radical Ra ou NRaRb,
leurs racémiques, énantiomères, diastéréoisomères et leurs mélanges, leurs tautomères et leurs sels pharmaceutiquement acceptables.

Plus particulièrement, la présente invention concerne également les compostions pharmaceutiques contenant en tant que principe actif un dérivé de formule (I) dans laquelle :
A est, si il est présent, un radical alkyle (C1-C6), un radical alkényle (C3-C6), un radical alkynyle (C3-C6), un radical cycloallcyle (C3-C7), un radical cycloalkényle (C5-C7), ces radicaux sont éventuellement substitués par 1 ou plusieurs substituants choisis parmi alkyle (C1-C5), alkényle (C2-C5), alkynyle (C2-C5), cycloalkyle (C3-C7), cycloalkényle (C5-C7), arylalkyle, hétéroarylalkyle, aryle, hétéroaryle, halogène,
R1 est un groupement NR6R7, azacycloalkyle (C4-C7), azacycloalkényle (C5-C7) azabicycloalkyle (C5-C9), azabicycloalkényle (C5-C9), ces groupements sont éventuellement substitués par 1 ou plusieurs substituants choisis parmi alkyle (C1-C5), cycloalkyle (C3-C5), halogène,
A-R1 est tel que l'azote de R1 et l'azote 1 du pyrazole sont obligatoirement séparés par au moins deux atomes de carbone,
R3 est un radical OH, NH2, OMe, H
R4 est un radical aryle ou hétéroaryle étant éventuellement substitué par 1 ou plusieurs substituants choisis parmi halogène, CN, NO₂, NH₂, OH, SH, COOH, CHO, C(O)NH2, C(S)NH2, SO2H, SO2NH2, NHCHO, C(O)Ra, C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra, aryle, hétéroaryle, hétérocycloalkyle (C4-C7), polyfluoroalkyle, trifluorométhylsulfanyle, trifluorométhoxy, (1-6C) alkyle linéaire ou ramifié, alkényle (C2-C6), alkynyle (C2-C6), ces substituants étant éventuellement substitués par un ou plusieurs alkyle, halogène, OH, méthoxy,
R5 est un hydrogène,
Ra est (1-6C) alkyle linéaire ou ramifié, alkényle, alkynyle, cycloalkyle (C3-C7), cycloalkényle (C5-C7), hétérocycloalkyle (C4-C7), arylalkyle, hétéroarylalkyle, aryle, hétéroaryle, polyfluoroalkyle,
Rb est indépendamment de Ra un hydrogène, (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Ra et Rb peuvent former un cycle saturé ou insaturé à 5, 6 ou 7 chaînons ayant ou non un hétéroatome tel que O, S, N, ce cycle étant éventuellement substitué par un ou plusieurs radicaux alkyle, halogène,
Rc est un radical, alkyle linéaire ou ramifié (C1-C6), alkényle (C3-C6), alkynyle (C3-C6), cycloalkyle (C3-C7), cycloalkényle (C5-C7), hétérocycloalkyle (C4-C7), (hétéro)arylalkyle, (hétéro)aryle, (poly)fluoroalkyle, C(O)R8, C(S)R8, SO2R8,
R6 et R7 sont indépendamment l'un de l'autre un hydrogène; alkyle (C1-C6), alkényle (C3-C6), alkynyle (C3-C6), cycloalkyle (C3-C7), cycloalkényle (C5-C7), hétérocycloalkyle (C4-C7), un arylalkyle, hétéroarylalkyle,
R6 et R7 peuvent former un cycle saturé ou insaturé à 5, 6 ou 7 chaînons ayant ou non un hétéroatome tel que O, S, N et qui est éventuellement substitué par un ou plusieurs alkyles, halogènes,
R8 est un radical Ra ou NRaRb,
leurs racémiques, énantiomères, diastéréoisomères et leurs mélanges, leurs tautomères et leurs sels pharmaceutiquement acceptables.

Les dérivés de pyrazoles de formule (I) dans laquelle:
A est, si il est présent, un radical alkyle (C1-C6), un radical alkényle (C3-C6), un radical alkynyle (C3-C6), un radical cycloalkyle (C3-C7), un radical cycloalkényle (C5-C7), ces radicaux sont éventuellement substitués par 1 ou plusieurs substituants choisis parmi alkyle (C1-C5), alkényle (C2-C5), alkynyle (C2-C5), cycloalkyle (C3-C7), cycloalkényle (C5-C7), arylalkyle, hétéroarylalkyle, aryle, hétéroaryle, halogène,
R1 est un groupement NR6R7, azacycloalkyle (C4-C7), azacycloalkényle (C5-C7) azabicycloalkyle (C5-C9), azabicycloalkényle (C5-C9), ces groupements sont éventuellement substitués par 1 ou plusieurs substituants choisis parmi alkyle (C1-C5), cycloalkyle (C3-C5), halogène,
A-R1 est tel que l'azote de R1 et l'azote 1 du pyrazole sont obligatoirement séparés par au moins deux atomes de carbone,
R3 est un radical H, halogène, OH, SH, NH2, ORc, SRc, SORa, SO2Ra NHCHO, NRaRb, NHC(O)Ra, NHC(S)Ra, NHSO2Ra ,
R4 est un radical aryle ou hétéroaryle étant éventuellement substitué par 1 ou plusieurs substituants choisis parmi halogène, CN, NO₂, NH₂, OH, SH, COOH, CHO, C(O)NH2, C(S)NH2, SO2H, SO2NH2, NHCHO, C(O)Ra, C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra, aryle, hétéroaryle, hétérocycloalkyle (C4-C7), polyfluoroalkyle, trifluorométhylsulfanyle, trifluorométhoxy, (1-6C) alkyle linéaire ou ramifié, alkényle (C2-C6), alkynyle (C2-C6), ces substituants étant éventuellement substitués par un ou plusieurs alkyle, halogène, OH, méthoxy,
R5 est un radical H, halogène, CF3, CHF2, CH2F, alkyle (C1-C6) linéaire ou ramifié, cycloalkyl (C3-C7),
Ra est (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Rb est indépendamment de Ra un hydrogène, (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Ra et Rb peuvent former un cycle saturé ou insaturé à 5, 6 ou 7 chaînons ayant ou non un hétéroatome tel que O, S, N, ce cycle étant éventuellement substitué par un ou plusieurs radicaux alkyle, halogène,
Rc est un radical, alkyle linéaire ou ramifié (C1-C6), alkényle (C3-C6), alkynyl (C3-C6), cycloalkyle (C3-C7), cycloalkényle (C5-C7), hétérocycloalkyle (C4-C7), (hétéro)arylallcyle, (hétéro)aryle, (poly)fluoroallcyle, C(O)R8, C(S)R8, SO2R8,
R6 et R7 sont indépendamment l'un de l'autre un hydrogène, alkyle (C1-C6), alkényle (C3-C6), alkynyle (C3-C6), cycloalkyle (C3-C7), cycloalkényle (C5-C7), hétérocycloalkyle (C4-C7), un arylalkyle, hétéroarylalkyle,
R6 et R7 peuvent former un cycle saturé ou insaturé à 5, 6 ou 7 chaînons ayant ou non un hétéroatome tel que O, S, N et qui est éventuellement substitué par un ou plusieurs alkyles, halogènes,
R5 est un radical hydrogène ou Me,
Ra est (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Rb est indépendamment de Ra un hydrogène, (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyflucroalkyle,
Ra et Rb peuvent former un cycle saturé ou insaturé à 5, 6 ou 7 chaînons ayant ou non un hétéroatome tel que O, S, N, ce cycle étant éventuellement substitué par un ou plusieurs radicaux alkyle, halogène,
Rc est un radical, alkyle linéaire ou ramifié (C1-C6), alkényle (C3-C6), alkynyle (C3-C6), cycloalkyle (C3-C7), cycloalkényle (C5-C7), hétérocycloalkyle (C4-C7), (hétéro)arylalkyle, (hétéro)aryle, (poly)fluoroalkyle, C(O)R8, C(S)R8, SO2R8,
R6 et R7 sont indépendamment l'un de l'autre un hydrogène, alkyle (C1-C6), alkényle (C3-C6), alkynyle (C3-C6), cycloalkyle (C3-C7), cycloalkényle (C5-C7), hétérocycloalkyle (C4-C7), un arylalkyle, hétéroarylalkyle,
R6 et R7 peuvent former un cycle saturé ou insaturé à 5, 6 ou 7 chaînons ayant ou non un hétéroatome tel que O, S, N et qui est éventuellement substitué par un ou plusieurs alkyles, halogènes,
R8 est un radical Ra ou NRaRb,
leurs racémiques, énantiomères, diastéréoisomères et leurs mélanges, leurs tautomères et leurs sels pharmaceutiquement acceptables, peuvent être utilisés

comme médicaments.

Plus particulièrement des dérivés de pyrazoles de formule (I) dans laquelle:
R8 est un radical Ra ou NRaRb,
leurs racémiques, énantiomères, diastéréoisomères et leurs mélanges, leurs tautomères et leurs sels pharmaceutiquement acceptables, peuvent être utilisés comme médicaments.

De manière préférée les dérivés de pyrazoles de formule (I) dans laquelle:
A est, si il est présent, un radical alkyle (C1-C6), un radical alkényle (C3-C6), un radical alkynyle (C3-C6), un radical cycloalkyle (C3-C7), un radical cycloalkényle (C5-C7), ces radicaux sont éventuellement substitués par 1 ou plusieurs substituants choisis parmi alkyle (C1-C5), alkényle (C2-C5), alkynyle (C2-C5), cycloalkyle (C3-C7), cycloalkényle (C5-C7), arylalkyle, hétéroarylalkyle, aryle, hétéroaryle, halogène,
R1 est un groupement NR6R7, azacycloalkyle (C4-C7), azacycloalkényle (C5-C7) azabicycloalkyle (C5-C9), azabicycloalkényle (C5-C9), ces groupements sont éventuellement substitués par 1 ou plusieurs substituants choisis parmi alkyle (C1-C5), cycloalkyle (C3-C5), halogène,
A-R1 est tel que l'azote de R1 et l'azote 1 du pyrazole sont obligatoirement séparés par au moins deux atomes de carbone,
R3 est un radical OH, NH2, OMe, H
R4 est un radical aryle ou hétéroaryle étant éventuellement substitué par 1 ou plusieurs substituants choisis parmi halogène, CN, NO₂, NH₂, OH, SH, COOH, CHO, C(O)NH2, C(S)NH2, SO2H, SO2NH2, NHCHO, C(O)Ra, -C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra, aryle, hétéroaryle, hétérocycloalkyle (C4-C7), polyfluoroalkyle, trifluorométhylsulfanyle, trifluorométhoxy, (1-6C) alkyle linéaire ou ramifié, alkényle (C2-C6), alkynyle (C2-C6), ces substituants étant éventuellement substitués par un ou plusieurs alkyle, halogène, OH, méthoxy,
A est, si il est présent, un radical alkyle (C1-C6), un radical alkényle (C3-C6), un radical alkynyle (C3-C6), un radical cycloalkyle (C3-C7), un radical cycloalkényle (C5-C7), ces radicaux sont éventuellement substitués par 1 ou plusieurs substituants choisis parmi alkyle (C1-C5), alkényle (C2-C5), alkynyle (C2-C5), cycloalkyle (C3-C7), cycloalkényle (C5-C7), arylalkyle, hétéroarylalkyle, aryle, hétéroaryle, halogène,
R1 est un groupement NR6R7, azacycloalkyle (C4-C7), azacycloalkényle (C5-C7) azabicycloalkyle (C5-C9), azabicycloalkényle (C5-C9), ces groupements sont éventuellement substitués par 1 ou plusieurs substituants choisis parmi alkyle (C1-C5), cycloalkyle (C3-C5), halogène,
A-R1 est tel que l'azote de R1 et l'azote 1 du pyrazole sont obligatoirement séparés par au moins deux atomes de carbone,
R3 est un radical OH, NH2, OMe, H
R4 est un radical aryle ou hétéroaryle étant éventuellement substitué par 1 ou plusieurs substituants choisis parmi halogène, CN, NO₂, NH₂, OH, SH, COOH, CHO, C(O)NH2, C(S)NH2, SO2H, SO2NH2, NHCHO, C(O)Ra, C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra, aryle, hétéroaryle, hétérocycloalkyle (C4-C7), polyfluoroalkyle, trifluorométhylsulfanyle, trifluorométhoxy, (1-6C) alkyle linéaire ou ramifié, alkényle (C2-C6), alkynyle (C2-C6), ces substituants étant éventuellement substitués par un ou plusieurs alkyle, halogène, OH, méthoxy,
R5 est un hydrogène,
Ra est (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Rb est indépendamment de Ra un hydrogène, (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Ra et Rb peuvent former un cycle saturé ou insaturé à 5, 6 ou 7 chaînons ayant ou non un hétéroatome tel que O, S, N, ce cycle étant éventuellement substitué par un ou plusieurs radicaux alkyle, halogène,
Rc est un radical, alkyle linéaire ou ramifié (C1-C6), alkényle (C3-C6), alkynyl (C3-C6), cycloalkyle (C3-C7), cycloalkényle (C5-C7), hétérocycloallcyle (C4-C7), (hétéro)arylalkyle, (hétéro)aryle, (poly)fluoroalkyle, C(O)R8, C(S)R8, SO2R8,
R6 et R7 sont indépendamment l'un de l'autre un hydrogène, alkyle (C1-C6), alkényle (C3-C6), alkynyle (C3-C6), cycloalkyle (C3-C7), cycloalkényle (C5-C7), hétérocycloalkyle (C4-C7), un arylalkyle, hétéroarylalkyle,
R6 et R7 peuvent former un cycle saturé ou insaturé à 5, 6 ou 7 chaînons ayant ou non un hétéroatome tel que O, S, N et qui est éventuellement substitué par un ou plusieurs alkyles, halogènes,
R8 est un radical Ra ou NRaRb,
   leurs racémiques, énantiomères, diastéréoisomères et leurs mélanges, leurs tautomères et leurs sels pharmaceutiquement acceptables, peuvent êtres utilisés comme médicaments.

Parmi les composés de formule (I) utiles selon l'invention on peut citer les composés suivants:
1-[2-(3-méthoxy 4-phényl-pyrazol-1-yl)-éthyl]-pipéridine
1-(1-aza-bicyclo[2.2.2]oct-3-ylméthyl)-4-phényl-1H-pyrazol-1-ol
3-(3-benzyloxy-4-phényl-pyrazol-1-ylméthyl)-1-aza-bicyclo[2.2.2]octane
3-(3-méthoxy-4-phényl-pyrazol-1-ylméthyl)-1-aza-bicyclo[2.2.2]octane
1-(1-aza bicyclo[2.2.2]oct-3-yl-4-phényl-1H-pyrazol-3-ol
1-(2-perhydro-azépin-1-yl-éthyl)-4-phényl-1H-pyrazol-3-ol
1-[2-(2-méthyl-pipéridin-1-yl)-éthyl]-4-phényl-1H-pyrazol-3-ol
1-[2-(4-fluoro-pipéridin-1-yl)-éthyl]-4-phényl-1H-pyrazol-3-ol
1-[2-(3-méthyl-pipéridin-1-yl)-éthyl]-4-phényl-1H-pyrazol-3-ol
1-[2-(3,6-dihydro-2H-ppidin-1-yl)-éthyl]-4-phényl-1H-pyrazol-3-ol
1-[2-(7-aza-bicyclo[2.2.1]hept-7-yl)-éthyl]-4-phényl-1H-pyrazol-3-ol
1-[2-(2-aza-bicyclo[2.2.2]oct-2-yl)-éthyl]-4-phényl-1H-pyrazol-3-ol
1-[2-(2-aza-bicyclo[2.2.1]hept-2-yl)-éthyl]-4-phényl-1H-pyrazol-3-ol
1-[2-diméthylamino-éthyl]-4-phényl-1H-pyrazol-3-ol
1-[3-diméthylamino-propyl]-4-phényl-1H-pyrazol-3-ol
1-[2-((2S,6R)-2,6-diméthyl-pipéridin-1-yl)-éthyl]-4-phényl-1H-pyrazol-3-ol
1-[2-diéthylamino-éthyl]-4-phényl-1H-pyrazol-3-ol
1-(2-diisopropylamino-éthyl)-4-phényl-1H-pyrazol-3-ol
4-phényl-1-(2-pyrrolidin-1-yl-éthyl)-1H-pyrazol-3-ol
3-(3-méthoxy-4-phényl-pyrazol-1-yl)-1-aza bicyclo[2.2.2]octane
1-[2-(3-difluorométhoxy-4-phényl-pyrazol-1-yl)éthyl]-pipéridine
4-phényl-1-(2-pipéridin-1-yl-éthyl)-1H-pyrazol-3-ylamine
4-phényl-1-(2-pipéridin-1-yl-éthyl)-1H-pyrazol-3-ylamine
N-[4-phényl-1-(2-pipéridin-1-yl-éthyl)-1H-pyrazol-3-yl]-acétamide
N-[4-phényl-1-(2-pipéridin-1-yl-éthyl)-1H-pyrazol-3-yl]-méthanesulfonamide
1-(2-diméthylamino-propyl)4-phényl-1H-pyrazol-3-ol
1-(1-méthyl-pipéridin-3-ylméthyl)-4-phényl-1H-pyrazol-3-ol
5-méthyl-4-phényl-1-(2-pipéridin-1-yl-éthyl-1H-pyrazol-3-ol
4-(3-amino-phényl)-1-(2-diméthylamino-éthyl)-1H-pyrazol-3-ol
N-(3-[3-hydroxy-1-(2-diméthylamino-éthyl)-1H-pyrazol-4-yl]-phényl)-acétamide
4-(4-amino-phényl)-1-(2-diméthylamino-éthyl)-1H-pyrazol-3-ol
1-(2-diméthylamino-éthyl)-4-(4'-fluoro-biphényl-3-yl)-1H-pyrazol-3-ol
4-biphényl-3-yl-1-(2-diméthylamino-éthyl)-1H-pyrazol-3-ol
1-(2-diméthylamino-éthyl)-4-(4'-fluoro-biphényl-4-yl)-1H-pyrazol-3-ol
1-(2-pipéridin-1-yl-éthyl)-4-pyridin-2-yl-1H-pyrazol-3-ol
1-(2-pipéridin-1-yl-éthyl)-4-pyridin-4-yl-1H-pyrazol-3-ol
4-(4-fluoro-phényl)-1-(2-pipéridin-1-yl-éthyl)-1H-pyrazol-3-ol
4-(4-trifluorométhoxy-phényl)-1-(2-pipéridin-1-yl-éthyl)-1H-pyrazol-3-ol
4-phényl-1-(2-pipéridin-1-yl-propyl)-1H-pyrazol-3-ol
3-(4-phényl-pyrazol-1-yl-méthyl)-1-aza-bicyclo[2.2.2]octane
4-(5-chloro-thiophèn-2-yl)-1-(2-pipéridin-1-yl-éthyl)-1H-pyrazol-3-ol
4-(3-méthoxy-phényl)-1-(2-pipéridin-1-yl-éthyl)-1H-pyrazol-3-ol
4-(2-méthoxy-phényl)-1-(2-pipéridin-1-yl-éthyl)-1H-pyrazol-3-ol
4-(3-hydroxy-phényl)-1-(2-pipéridin-1-yl-éthyl)-1H-pyrazol-3-ol
4-(4-hydroxy-phényl)-1-(2-pipéridin-1-yl-éthyl)-1H-pyrazol-3-ol
4-(4-méthoxy-phényl)-1-(2-pipéridin-1-yl-éthyl)-1H-pyrazol-3-ol
4-(3-fluoro-phényl)-1-(2-pipéridin-1-yl-éthyl)-1H-pyrazol-3-ol
1-(2-pipéridin-1-yl-éthyl)-4-(3-trifluorométhyl-phényl)-1H-pyrazol-3-ol
1-(2-pipéridin-1-yl-éthyl)-4-pyridin-3-yl-1H-pyrazol-3-ol
4-(4-chloro-phényl)-1-(2-pipéridin-1-yl-éthyl)-1H-pyrazol-3-ol
4-(3-chloro-phényl)-1-(2-pipéridin-1-yl-éthyl)-1H-pyrazol-3-ol
4-(2-fluoro-phényl)-1-(2-pipéridin-1-yl-éthyl)-1H-pyrazol-3-ol :
4-(2-chloro-phényl)-1-(2-pipéridin-1-yl-éthyl)-1H-pyrazol-3-ol
1-(1-aza-bicyclo[2.2.2]oct-3-yl)-4-(4-chloro-phényl)-1H-pyrazol-3-ol
1-(1-aza-bicyclo[2.2.2]oct-3-yl)-4-(3-chloro-phényl)-1H-pyrazol-3-ol
1-(1-aza-bicyclo[2.2.2]oct-3-yl)-4-(3-fluoro-phényl)-1H-pyrazol-3-ol
1-(1-méthyl-pyrrolidin-3-yl)-4-phényl-1H-pyrazol-3-ol
1-[2-(1-méthyl-pyrrolidin-2-yl)-éthyl]-4-phényl-1H-pyrazol-3-ol
1-(pyrrolidin-3-yl)-4-phényl-1H-pyrazol-3-ol
1-[(1-méthyl-pyrrolidin-2-(S)-yl)-méthyl]-4-phényl-1H-pyrazol-3-ol
4-Phényl-1-(2-pipéridin-1-yl-éthyl)-1H-pyrazol-3-ol
1-[2-(4-Phényl-pyrazol-1-yl)-éthyl]-pipéridine
1-[2-(4-Méthyl-pipéridin-1-yl)-éthyl]-4-phényl-1H-pyrazol-3-ol
3-(3-Difluorométhoxy-4-phényl-pyrazol-1-ylméthyl)-1-aza-bicyclo[2.2.2]octane
3-(4-Phényl-pyrazol-1-yl)-1-aza-bicyclo[2.2.2]octane
4-Benzo[b]thiophèn-2-yl-1-(2-pipéridin-1-yl-éthyl)-1H-pyrazol-3-ol
4-Phényl-1-pipéridin-3-yl-1H-pyrazol-3-ol
1-(2-Pipéridin-1-yl-éthyl)-4-thiophèn-3-yl-1H-pyrazol-3-ol
1-(2-Pipéridin-1-yl-éthyl)-4-p-tolyl-1H-pyrazol-3-ol
1-(1-Aza-bicyclo[2.2.2]oct-2-ylméthyl)-4-phényl-1H-pyrazol-3-ol
(S)-1-(1-Aza-bicyclo[2.2.2]oct-3-yl)-4-phényl-1H-pyrazol-3-ol
(R)-1-(1-Aza-bicyclo[2.2.2]oct-3-yl)-4-phényl-1H-pyrazol-3-ol
4-Phényl-1-pyrrolidin-3-ylméthyl-1H-pyrazol-3-ol
1-(2-Pipéridin-1-yl-éthyl)-4-thiophèn-2-yl-1H-pyrazol-3-ol
4-[3-Hydroxy-1-(2-pipéridin-1-yl-éthyl)-1H-pyrazol-4-yl]-benzamide
3-[3-Hydroxy-1-(2-pipéridin-1-yl-éthyl)-1H-pyrazol-4-yl]-benzamide
1-[(S)-1-(1-Aza-bicyclo[2.2.2]oct-3-yl)méthyl]-4-phényl-1H-pyrazol-3-ol
1-[(R)-1-(1-Aza-bicyclo[2.2.2]oct-3-yl)méthyl]-4-phényl-1H-pyrazol-3-ol
1-[(1S,4R)-2-(2-Aza-bicyclo[2.2.1]hept-2-yl)-éthyl]-4-phényl-1H-pyrazol-3-ol
1-[(1R,4S)-2-(2-Aza-bicyclo[2.2,1]hept-2-yl)-éthyl]-4-phényl-1H-pyrazol-3-ol
1-((R)-1-Méthyl-pyrrolidin 2-ylméthyl)-4-phényl-1H-pyrazol-3-ol
1-((S)-1-Méthyl-pyrrolidin-3-yl)-4-phényl-1H-pyrazol-3-ol
1-((R)-1-Méthyl-pyrrolidin 3-yl)-4-phényl-1H-pyrazol-3-ol
1-[1-(7-Aza-bicyclo[2.2.1]hept-2-yl)-éthyl]-4-phényl-1H-pyrazol-3-ol
1-(1-Aza-bicyclo[2.2.1]hept-3-yl)-4-phényl-1H-pyrazol-3-ol
4-Phényl-1-pipéridin-2-ylméthyl-1H-pyrazol-3-ol
1-(1-Méthyl-pipéridin-2-ylméthyl)-4-phényl-1H-pyrazol-3-ol
1-(1-Ethyl-pyrrolidin-2-ylméthyl)-4-phényl-1H-pyrazol-3-ol
1-(1-Méthyl-2-pipéridin-1-yl-éthyl)-4-phényl-1H-pyrazol-3-ol
1-[2-(2-Aza-bicyclo[2.2.1]hept-2-yl)-1-méthyl-éthyl]-4-phényl-1H-pyrazol-3-ol
1-(2-Diméthylamino-cyclopentyl)-4-phényl-1H-pyrazol-3-ol
1-(R)-1-Aza-bicyclo[2.2.2]oct-3-yl-4-(4-chloro-phényl)-1H-pyrazol-3-ol
1-(S)-1-Aza-bicyclo[2.2.2]oct-3-yl-4-(4-chloro-phényl)-1H-pyrazol-3-ol
1-(R)-1-Aza-bicyclo[2.2.2]oct-3-yl-4-(3-chloro-phényl)-1H-pyrazol-3-ol
1-(S)-1-Aza-bicyclo[2.2.2]oct-3-yl-4-(3-chloro-phényl)-1H-pyrazol-3-ol
1-(R)-1-Aza-bicyclo[2.2.2]oct-3-yl-4-(3-fluoro-phényl)-1H-pyrazol-3-ol
1-(S)-1-Aza-bicyclo[2.2.2]oct-3-yl-4-(3-fluoro-phényl)-1H-pyrazol-3-ol
4-(5-Bromo-thiophèn-2-yl)-1-(2-pipéridin-1-yl-éthyl)-1H-pyrazol-3-ol
4-(5-Phenyl-thiophèn-2-yl)-1-(2-pipéridin-1-yl-éthyl)-1H-pyrazol-3-ol
1-(2-Piperidin-1-yl-éthyl)-4-(5-pyridin-2-yl-thiophèn-2-yl)-1H-pyrazol-3-ol
4-(4-Chloro-thiophén-2-yl)-1-(2-pipéridin-1-yl-éthyl)-1H-pyrazol-3-ol
4-(4-Bromo-phényl)-1-(2-pipéridin-1-yl-éthyl)-1H-pyrazol-3-ol
1-(2-Pipéridin-1-yl-éthyl)-4-(3-trifluorométhoxy-phényl)-1H-pyrazol-3-ol
4-(3,4-Dichloro-phényl)-1-(2-pipéridin-1-yl-éthyl)-1H-pyrazol-3-ol
4-(3,5-Dichloro-phényl)-1-(2-pipéridin-1-yl-éthyl)-1H-pyrazol-3-ol
4-(6-Chloro-pyridin-2-yl)-1-(2-pipéridin-1-yl-éthyl)-1H-pyrazol-3-ol
4-(1H-Indol-6-yl)-1-(2-pipéridin-1-yl-éthyl)-1H-pyrazol-3-ol
4-(1H-Indol-5-yl)-1-(2-pipéridin-1-yl-éthyl)-1H-pyrazol-3-ol
4-(1H-Indol-3-yl)-1-(2-pipéridin-1-yl-éthyl)-1H-pyrazol-3-ol
4-(1-Méthyl-1H-indol-3-yl)-1-(2-pipéridin-1-yl-éthyl)-1H-pyrazol-3-ol
N-{4-[3-Hydroxy-1-(2-pipéridin-1-yl-éthyl)-1H-pyrazol-4-yl]-phényl}-méthanesulfonamide
N-{3-[3-Hydroxy-1-(2-pipéridin-1-yl-éthyl)-1H pyrazol-4-yl]-phényl}-méthanesulfonamide
4-[3-(1H-Imidazol-2-yl)-phényl]-1-(2-pipéridin-1-yl-éthyl)-1H-pyrazol-3-ol
4-[4-(1H-Imidazol-2-yl)-phényl]-1-(2-pipéridin-1-yl-éthyl)-1H-pyrazol-3-ol
4-(3-Chloro-4-hydroxy-phényl)-1-(2-pipéridin-1-yl-éthyl)-1H-pyrazol-3-ol
4-(4-Hydroxy-3-méthyl-phényl)-1-(2-pipéridin-1-yl-éthyl)-1H-pyrazol-3-ol
4-(4-Amino-3-chloro-phényl)-1-(2-pipéridin-1-yl-éthyl)-1H-pyrazol-3-ol
1-(1-Aza-bicyclo[2.2.2]oct-3-yl)-4-(5-chloro-thiophèn-2-yl)-1H-pyrazol-3-ol
1-(1-Aza-bicyclo[2.2.2]oct-3-ylméthyl)-4-(5-chloro-thiophèn-2-yl)-1H-pyrazol-3-ol
1-(1-Aza-bicyclo[2.2.2]oct-3-ylméthyl)-4-(4-chloro-phényl)-1H-pyrazol-3-ol
1-(1-Aza-bicyclo[2.2.2]oct-3-ylméthyl)-4-(3-chloro-phényl)-1H-pyrazol-3-ol
1-(1-Aza-bicyclo[2.2.2]oct-3-ylméthyl)-4-(3-fluoro-phényl)-1H-pyrazol-3-ol
1-(1-Aza-bicyclo[2.2.2]oct-3-ylméthyl)-4-(3-hydroxy-phényl)-1H-pyrazol-3-ol
1-(1-Aza-bicyclo[2.2.2]oct-3-ylméthyl)-4-(4-hydroxy-phényl)-1H-pyrazol-3-ol
1-[2-(2-Aza-bicyclo[2.2.1]hept-2-yl)-éthyl]-4-(5-chloro-thiophèn-2-yl)-1H-pyrazol-3-ol
1-[2-(2-Aza-bicyclo[2.2.1]hept-2-yl)-éthyl]-4-(4-chloro-phényl)-1H-pyrazol-3-ol
1-[2-(2-Aza-bicydo[2.2.1]hept-2-yl)-éthyl]-4-(3-chloro-phényl)-1H-pyrazol-3-ol
1-[2-(2-Aza-bicyclo[2.2.1]hept-2-yl)-éthyl]-4-(3-fluoro-phényl)-1H-pyrazol-3-ol
1-[2-(2-Aza-bicyclo[2.2.1]hept-2-yl)-éthyl]4-(3-hydroxy-phényl)-1H-pyrazol-3-ol
1-[2-(2-Aza-bicyclo[2.2.1]hept-2-yl)-éthyl]-4-(4-hydroxy-phényl)-1H-pyrazol-3-ol
1-[2-(2-Aza-bicyclo[2.2.1]hept-2-yl)-éthyl]-4-(3-chloro-4-hydroxy-phényl)-1H-pyrazol-3-ol
1-(1-Aza-bicydo[2.2.2]oct-3-ylméthyl)-4-(3-chloro-4-hydroxy-phényl)-1H-pyrazol-3-ol
1-(1-Aza-bicyclo[2.2.2]oct-3-yl)-4-(3-hydroxy-phényl)-1H-pyrazol-3-ol
1-(1-Aza-bicyclo[2.2.2]oct-3-yl)-4-(4-hydroxy-phényl)-1H-pyrazol-3-ol
1-(1-Aza-bicyclo[2.2.2]oct-3-yl)-4-(3-chloro-4-hydroxy-phényl)-1H-pyrazol-3-ol
2-[1-(2-Pipéridin-1-yl-éthyl)-1H-pyrazol-4-yl]-benzamide
N-Méthyl-2-[1-(2-pipéridin-1-yl-éthyl)-1H-pyrazol-4-yl]-benzamide
2-[1-(2-Pipéridin-1-yl-éthyl)-1H-pyrazol-4-yl]-benzènesulfonamide
N-Méthyl-2-[1-(2-pipéridin-1-yl-éthyl)-1H-pyrazol-4-yl]-benzènesulfonamide
{2-[1-(2-Pipéridin-1-yl-éthyl)-1H-pyrazol-4-yl]-phényl}-méthanol
4-Phényl-1-(2-pipéridin-1-yl-éthyl)-1H-pyrazole-3-thiol
1-(1-Aza-bicyclo[2.2.2]oct-3-yl)-4-phényl-1H-pyrazol-3-ylamine
N-[1-(1-Aza-bicyclo[2.2.2]oct-3-yl)-4-phényl-1H-pyrazol-3-yl]-méthanesulfonamide
1-(1-Aza-bicyclo[2.2.2]oct-3-yl)-4-phényl-1H-pyrazole-3-thiol
4-[1-(1-Aza-bicydo[2.2.2]oct-3-yl)-1H-pyrrol-4-yl]-2-chloro-phénol
4-[1-(1-Aza-bicyclo[2.2.2]oct-3-yl)-1H-pyrazol-4-yl]-phénol
3-[4-(4-Chloro-phényl)-pyrazol-1-ylméthyl]-1-aza-bicyclo[2.2.2]octane
4-[1-(1-Aza-bicyclo[2.2.2]oct-3-ylméthyl)-1H-pyrazol-4-yl]-phénol
4-[1-(1-Aza-bicyclo[2.2.2]oct-3-ylméthyl)-1H-pyrazol-4-yl]-2-chloro-phénol
3-(3-Cyclopropylméthoxy-4-phényl-pyrazol-1-ylméthyl)-1-aza-bicyclo[2.2.2]octane
3-[4-(4-Chloro-phényl)-3-cyclopropylméthoxy-pyrazol-1-ylméthyl]-1-aza-bicyclo[2.2.2]octane
4-[1-(1-Aza-bicyclo[2.2.2]oct-3-ylméthyl)-3-cyclopropylméthoxy-1H-pyrazol-4-yl]-phénol
4-[1-(1-Aza-bicyclo[2.2.2]oct-3-ylméthyl)-3-cyclopropylméthoxy-1H-pyrazol-4-yl]-2-chloro-phénol
3-[4-Phényl-3-(2,2,2-trifluoro-éthoxy)-pyrazol-1-ylméthyl]-1-aza-bicyclo[2.2.2]octane
3-[4-(4-Chloro-phényl)-3-(2,2,2-trifluoro-éthoxy)-pyrazol-1-ylméthyl]-1-aza-bicyclo[2.2.2]octane
4-[1-(1-Aza-bicyclo[2.2.2]oct-3-ylméthyl)-3-(2,2,2-trifluoro-éthoxy)-1H-pyrazol-4-yl]-phénol
4-[1-(1-Aza-bicyclo[2.2.2]oct-3-ylméthyl)-3-(2,2,2-trifluoro-éthoxy)-1H-pyrazol-4-yl]-2-chloro-phénol
N-[1-(1-Aza-bicyclo[2.2.2]oct-3-ylméthyl)-4-phényl-1H-pyrazol-3-yl]-méthanesulfonamide
1-(1-Aza-bicyclo[2.2.2]oct-3-ylméthyl)-4-phényl-1H-pyrazole-3-thiol
1-(1-Aza-bicyclo[2.2.2]oct-3-ylméthyl)-4-phényl-1H-pyrazol-3-ylamine
2-[2-(4-Phényl-pyrazol-1-yl)-éthyl]-2-aza-bicyclo[2.2.1]heptane
2-{2-[4-(4-Chloro-phényl)-pyrazol-1-yl]-éthyl}-2-aza-bicyclo[2.2.1]heptane
4-{1-[2-(2-Aza-bicyclo[2.2.1]hept-2-yl)-éthyl]-1H-pyrazol-4-yl}-phénol
4-{1-[2-(2-Aza-bicyclo[2.2.1]hept-2-yl)-éthyl]-1H-pyrazol-4-yl}-2-chloro-phénol
1-[2-(2-Ethyl-4-méthyl-pyrrolidin-1-yl)-éthyl]-4-phényl-1H-pyrazole-3-thiol
N-{1-[2-(2-Aza-bicyclo[2.2.1]hept-2-yl)-éthyl]-4-phényl-1H-pyrazol-3-yl}-méthanesulfonamide
1-[2-(2-Aza-bicyclo[2.2.1]hept-2-yl)-éthyl]-4-phényl-1H-pyrazol-3-ylamine
4-(4-Chloro-phényl)-1-[2-(2-éthyl-4-méthyl-pyrrolidin-1-yl)-éthyl]-1H-pyrazole-3-thiol
N-[1-[2-(2-Aza-bicyclo[2.2.1]hept-2-yl)-éthyl]-4-(4-chloro-phényl)-1H-pyrazol-3-yl]-méthanesulfonamide
1-[2-(2-Aza-bicyclo[2.2,1]hept-2-yl)-éthyl]-4-(4-chloro-phényl)-1H-pyrrol-3-ylamine
1-(1-Aza-bicyclo[2.2.2]oct-3-yl)-4-(4-chloro-phényl)-1H-pyrazole-3-thiol
N-[1-(1-Aza-bicyclo[2.2.2]oct-3-ylméthyl)-4-(4-chloro-phényl)-1H-pyrazol-3-yl]-méthanesulfonamide
1-(1-Aza-bicyclo[2.2.2]oct-3-ylméthyl)-4-(4-chloro-phényl)-1H-pyrazol-3-ylamine
1-(1-Aza bicyclo[2.2.2]oct-3-ylméthyl)-4-(4-chloro-phényl)-1H-pyrazole-3-thiol
1-(1-Méthyl-perhydro-azépin-3-yl)-4-phényl-1H-pyrazol-3-ol
1-(2-Méthylamino-cyclopentyl)-4-phényl-1H-pyrazol-3-ol
1-(3-Diméthylamino-cyclopentyl)-4-phényl-1H-pyrazol-3-ol
1-(3-Méthylamino-cyclopentyl)-4-phényl-1H-pyrazol-3-ol
1-(2-Diméthylamino-cyclohexyl)-4-phényl-1H-pyrazol-3-ol
1-(2-Méthylamino-cyclohexyl)-4-phényl-1H-pyrazol-3-ol
1-(3-Diméthylamino-cyclohexyl)-4-phényl-1H-pyrazol-3-ol
1-(3-Méthylamino-cyclohexyl)-4-phényl-1H-pyrrol-3-ol
1-(Octahydro-indolizin-3-ylméthyl)-4-phényl-1H-pyrrol-3-ol
1-((S)-1-Ethyl-pyrrolidin-2-ylméthyl)-4-phényl-1H-pyrazol-3-ol
4-phényl-1-pyrrolidin-3-yl-méthyl-1H-pyrazol-3-ol
1-((2R) 1-méthyl-pyrrolidin-2-ylméthyl)-4-phényl-1H-pyrazol-3-ol
4-phényl-1-(piperidin-3-yl)-1H-pyrazol-3-ol
1-(1-méthyl-pipéridin-2-ylméthyl)-4-phényl-1H-pyrazol-3-ol
1-(1-méthyl-azepan-3-yl)-4-phényl-1H-pyrazol-3-ol
4-phényl-1-(2-pipéridin-1-yl-éthyl)-1H-pyrazol-3-ol
4-(thiophèn-2-yl)-1-(2-pipéridin-1-yl-éthyl)-1H-pyrazol-3-ol
4-(3,4-dichloro-phényl)-1-(2-pipéridin-1-yl-éthyl)-1H-pyrazol-3-ol
4-(4-Bromo-phényl)-1-(2-pipéridin-1-yl-éthyl)-1H-pyrazol-3-ol
4-(1H-Indol-5-yl)-1-(2-pipéridin-1-yl-éthyl)-1H-pyrazol-3-ol
4-(5-Bromo-thiophèn-2-yl)-1-(2-pipéridin-1-yl-éthyl)-1H-pyrazol-3-ol
2-[1-(2-Pipéridin-1-yl-éthyl)-1H-pyrazol-4-yl] benzamide
4-(2-hydroxy-phényl)-1-(2-pipéridin-1-yl-éthyl)-1H-pyrazole
4-(1H-Indol-5-yl)-1-(2-pipéridin-1-yl-éthyl)-1H-pyrazole
4-(4-méthyl-phényl)-1-(2-pipéridin-1-yl-éthyl)-1H-pyrazol-3-ol
1-(1-Aza-bicyclo[2.2.2]oct-3-yl)-4-(1H-indol-5-yl)-1H-pyrazole
(+) 1-(1-Aza-bicyclo[2.2.2]oct-3-yl)-4-(1H-indol-5-yl)-1H-pyrazole
(-) 1-(1-Aza-bicyclo[2.2.2]oct-3-yl)-4-(1H-indol-5-yl)-1H-pyrazole
1-(1-aza-bicyclo[2.2.2]oct-3-yl)-4-(5-chloro-thiophén-2-yl)-1H-pyrazol-3-ol,
1-(1-aza-bicyclo[2.2.2]oct-3-yl)-4-(5-chloro-thiophèn-2-yl)-1H-pyrazol-3-ol,
1-(1-aza-bicyclo[2.2.2]oct-2-ylméthyl)-4-phényl-1H-pyrazol-3-ol
3-[4-(3,5-difluoro-phényl)-pyrazol-1-yl]-1-aza bicyclo[2.2.2]octane :
4-benzo[b]thiophèn-2-yl-1-(2-pipéridin-1-yl-éthyl)-1H-pyrazol-3-ol
1-(2-pipéridin-1-yl-éthyl)-4-thiophén-3-yl-1H-pyrazol-3-ol
4-[3-hydroxy-1-(2-pipéridin-1-yl-éthyl)-1H-pyrazol-4-yl]-benzamide
3-[3-hydroxy-1-(2-pipéridin-1-yl-éthyl)-1H-pyrazol-4-yl]-benzamide
(-)-1-(1-aza-bicyclo[2.2.2]oct-3-yl)-4-phényl-1H-pyrazol-3-ol
(+)-1-(1-aza-bicyclo[2.2.2]oct-3-yl)-4-phényl-1H-pyrazol-3-ol
(-)-1-(1-aza-bicyclo[2.2.2]oct-3-ylméthyl)-4-phényl-1H-pyrazol-1-ol
(+)-1-(1-aza-bicyclo[2.2.2]oct-3-ylméthyl)-4-phényl-1H-pyrazol-1-ol
1-(1-aza-bicylo[2.2.2]oct-3-ylméthyl)-4-(4-chloro-phényl)-1H-pyrazol-3-ol
(-)-1-(1-aza-bicyclo[2.2.2]oct-3-yl)-4-(4-chloro-phényl)-1H-pyrazol-3-ol
(+)-1-(1-aza-bicyclo[2.2.2]oct-3-yl)-4-(4-chloro-phényl)-1H-pyrazol-3-ol
(-)-1-(1-aza-bicyclo[2.2.2]oct-3-yl)-4-(4-fluoro-phényl)-1H-pyrazol-3-ol
(+)-1-(1-aza bicyclo[2.2.2]oct-3-yl)-4-(4-fluoro-phényl)-1H-pyrazol-3-ol
3-[4-(4-chloro-phényl)-pyrazol-1-yl]-1-aza-bicyclo[2.2.2]octane
3-[4-(4-chloro-phényl)-pyrazol-1-ylméthyl]-1-aza-bicyclo[2.2.2]octane
3-[4-(3-Chloro-4-méthoxy-phényl)-pyrazol-1-ylméthyl]-1-aza-bicyclo[2.2.2]octane
4-[1-(1-aza-bicyclo[2.2.2]oct-3-ylméthyl)-1H-pyrazol-4-yl]-2-chloro-phénol
4-[1-(1-Aza bicyclo[2.2.2]oct-3-yl-1H-pyrazol-4-yl]-2-chloro-phénol
(-)-4-[1-(1-Aza-bicyclo[2.2.2]oct-3-yl)-1H-pyrazol-4-yl]-2-chloro-phénol
(+)-4-[1-(1-Aza-bicyclo[2.2.2]oct-3-yl)-1H-pyrazol-4-yl]-2-chloro-phénol
(+)-1-(1-Aza-bicyclo[2.2.2]oct-3-yl)-4-pyridin-2-yl-1H-pyrazol-3-ol
(-)-1-(1-aza-bicyclo[2.2.2]oct-3-yl)-4-pyridin-2-yl-1H-pyrazol-3-ol
(+)-1-(1-Aza-bicyclo[2.2.2]oct-3-yl)-4-phenyl-1H-pyrazol-3-ylamine
(-)-1-(1-aza-bicyclo[2.2.2]oct-3-yl)-4-phenyl-1H-pyrazol-3-ylamine
(+)-1-(1-aza-bicyclo[2.2.2]oct-3-yl)-4-(1H-indol-4-yl)-1H-pyrazole,
(-)-1-(1-aza-bicyclo[2.2.2]oct-3-yl)-4-(1H-indol-4-yl)-1H-pyrazole,
leurs racémiques, énantiomères, diastéréoisomères et leurs mélanges, leurs tautomères et leurs sels pharmaceutiquement acceptables.

Les modes d'obtention des dérivés de la présente invention sont illustrés ci dessous et pour une meilleure lisibilité des procédés, les composés de formule (I) sont divisés en huit sous-familles (Ia) pour R3 = OH, (Ib) pour R3 = ORc, (Ic) pour R3 = H, (Id) pour R3 = NH2, (Ie) pour R3 = NHCHO, NRaRb, NHC(O)Ra, NHC(S)Ra, NHS02Ra, (If) pour R3 = SH, (Ig) pour R3 = SRc, (Ih) pour R3 = S(O)Ra, S02Ra. Les définitions de différents substituants sont les mêmes que celles de la formule générale (I) sauf indication contraire.

Pour des facilités de lecture, des groupements GP, GP', GP", GP"', GP^{iv} et GP^{v} sont des groupements protecteurs des fonctions sensibles aux conditions de réaction et sont introduits tel que défini dans T. W. Greene et coll., Protective Groups in Organic Synthesis, Wiley-Interscience, third édition (1999), susceptibles de ne pas être affectés par les étapes suivantes de la synthèse et d'être déprotégés dans des conditions ne touchant pas le reste de la molécule.

Les dérivés de formule (I) pour lesquels R3 est un hydroxy (Ia) peuvent être obtenus à partir de dérivés de formule (II) (I avec R3 = OGP) pour lesquels GP est un groupement protecteur de la fonction hydroxyle.

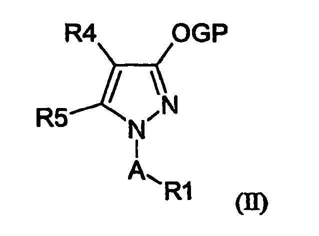

On entend par GP un groupement protecteur de fonction hydroxyle, tel que défini dans T. W. Greene et coll., Protective Groups in Organic Synthesis, Wiley-Interscience, third edition (1999), susceptible de ne pas être affecté par les étapes suivantes de la synthèse et d'être déprotégé dans des conditions ne touchant pas le reste de la molécule. Par exemple, le groupement GP pourra être un groupe silylé tel que tert-butyl-diméthyl-silyle, triisopropyl-silyle ou diphényl-méthyl-silyle, un résidu alkyle, aralkyle, alkylidène, cycloalkylidène, hétéroalkyle ou hétérocycloalkyle tel que méthyle, allyle, cyclohex-2-ènyle, benzyle ou tétrahydropyran-2-yle. Le groupe GP est de préférence un benzyle ou un cyclohex-2-ènyle. La déprotection du groupe GP est réalisée selon les méthodes décrites dans T. W. Greene et coll., Protective Groups in Organic Synthesis, Wiley-Interscience, third edition (1999).

Par exemple, lorsque le groupe GP est un benzyle, la déprotection s'effectue par hydrolyse en présence d'acide chlorhydrique concentré dans un alcool tel que l'éthanol, le méthanol ou l'isopropanol à une température comprise entre 20°C et la température d'ébullition du milieu réactionnel, de préférence dans l'éthanol, à la température d'ébullition du milieu réactionnel.

Alternativement, la débenzylation peut être réalisée par les opérations successives suivantes :
a) Formation du chlorhydrate du composé à déprotéger en présence d'acide chlorhydrique en solution aqueuse ou en solution dans un solvant organique tel que l'éthanol, le méthanol, le dioxane ou l'éther diéthylique à une température voisine de 20°C
b) Hydrogénation en présence d'un catalyseur comme le palladium sur charbon, dans un alcool tel que l'éthanol, le méthanol ou l'isopropanol, à une pression d'hydrogène comprise entre 1 bar et 20 bars et à une température comprise entre 20°C et la température d'ébullition du milieu réactionnel.

L'hydrogénolyse du groupement benzyle peut également être effectuée en présence d'un catalyseur comme le palladium sur charbon, en présence d'acide chlorhydrique concentré, dans un alcool tel que l'éthanol, le méthanol ou l'isopropanol, à une pression d'hydrogène comprise entre 1 bar et 30 bars et à une température comprise entre 20°C et la température d'ébullition du milieu réactionnel. La réaction peut aussi être réalisée avec du formiate d'ammonium, en présence d'un catalyseur comme le palladium sur charbon, dans un alcool tel que l'éthanol, le méthanol ou l'isopropanol, à une température comprise entre 20°C et la température d'ébullition du milieu réactionnel, de préférence dans le méthanol à la température d'ébullition du milieu réactionnel.

Lorsque le groupe GP est un cyclohexènyle, la déprotection est réalisée par hydrolyse en milieu acide, par exemple en présence d'une solution d'acide chlorhydrique dans un éther ou un alcool, dans un solvant tel que le méthanol ou l'éthanol à une température comprise entre 20°C et la température d'ébullition du milieu réactionnel.

Les dérivés de formule 1 pour lesquels R3 est ORc (Rc étant différent de C(O)R8, C(S)R8, SO2R8), H, NH2 ou OGP (Ib), (Ic), (Id) ou (II) peuvent être obtenus selon trois voies de synthèse différentes.

La première voie de synthèse consiste à utiliser des composés de formule (III) :

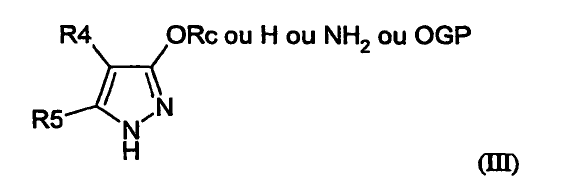

Les composés de formule (Ib), (Ic), (Id) ou (II) peuvent être obtenus à partir d'un pyrazole de formule (III) et d'un composé de formule (IV) R1-A-X pour lequel X = une fonction telle que Cl, Br, I, OTs, OMs, OTf. L'alkylation est réalisée sous atmosphère inerte, par exemple sous argon ou sous azote, en milieu basique dans un solvant aprotique, par exemple en présence d'hydrure de sodium, au sein d'un solvant aprotique tel que le diméthylformamide, à une température comprise entre 20°C et la température d'ébullition du milieu réactionnel ou en présence de tertiobutylate de potassium, au sein d'un solvant tel que le diméthylformamide, à une température comprise entre 20°C et la température d'ébullition du milieu réactionnel. La réaction peut également être effectuée en présence de carbonate de potassium et éventuellement d'iodure de potassium, dans un solvant tel que l'acétone, la méthyléthylcétone, l'acétonitrile ou le diméthylformamide, de préférence dans la méthyléthylcétone, à la température d'ébullition du milieu réactionnel.

Les composés (IV) sont commerciaux ou peuvent être obtenus à partir des alcools correspondants de formule R1-A-OH par des méthodes connues de l'homme de l'art telles que décrites dans J. March, Advanced Organic Chemistry, Wiley-Interscience, fourth edition (1992) ou R.C. Larock, Comprehensive Organic Transformations, VCH Publishers (1989). Les alcools de formule R1-A-OH sont commerciaux ou peuvent être obtenus par adaptation de méthodes décrites dans la littérature en utilisant les connaissances normales de l'homme du métier.

La seconde voie de synthèse peut être utilisée pour les composés de formule (I) pour lesquels R3 est ORc (Rc étant différent de C(O)R8, C(S)R8, S02R8), H ou OGP, et R1-A est groupe où le radical A est relié à R1 par un atome d'azote.

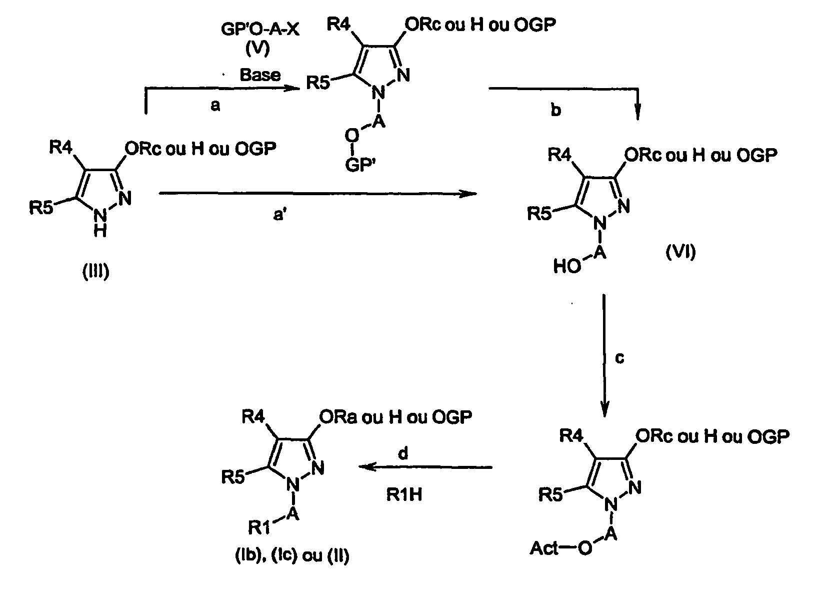

Les composés de formule (Ib), (Ic) ou (II) peuvent être obtenus en trois ou quatre étapes à partir des pyrazoles de formule (III) selon le protocole suivant :
a) Alkylation du pyrazole (III) par un composé de formule (V) GP'O-A-X dans lequel GP' est un groupement protecteur de fonction hydroxyle, tel que défini dans T. W. Greene et coll., Protective Groups in Organic Synthesis, Wiley-Interscience, third édition (1999), susceptible de ne pas être altéré au cours de l'étape d'alkylation et d'être déprotégé dans des conditions ne touchant pas le reste de la molécule (par exemple, le groupement GP' pourra être un groupe silylé tel que tert-butyl-diméthyl-silyle triisopropyl-silyle ou diphényl-méthyl-silyle, un résidu aralkyle, alkylidène, cycloalkylidène, hétéroalkyle, ou hétérocycloalkyle tel que allyle, cyclohex-2-ènyle, benzyle ou tétrahydropyran-2-yle) ; le groupe GP' est de préférence un groupe tétrahydropyran-2-yle ou tert-butyl-diméthyl-silyle; le radical X est une fonction telle que Cl, Br, I, OTs, OMs, OTf. L'alkylation est effectuée sous atmosphère inerte, par exemple sous argon ou sous azote, en milieu basique dans un solvant aprotique, par exemple en présence d'hydrure de sodium, au sein d'un solvant aprotique tel que le diméthylformamide, à une température comprise entre 20°C et la température d'ébullition du milieu réactionnel ou en présence de tertiobutylate de potassium, au sein d'un solvant tel que le diméthylformamide, à une température comprise entre 20°C et la température d'ébullition du milieu réactionnel. La réaction peut également être effectuée en présence de carbonate de potassium et éventuellement d'iodure de potassium, dans un solvant tel que l'acétone, la méthyléthylcétone, l'acétonitrile ou le diméthylformamide, de préférence dans la méthyléthylcétone, à la température d'ébullition du milieu réactionnel.
b) Obtention des intermédiaires de formule (VI) après clivage du groupe protecteur GP' selon les méthodes décrites dans T. W. Greene et coll., Protective Groups in Organic Synthesis, Wiley-Interscience, third édition (1999), et n'affectant pas les autres fonctions portées par la molécule. Par exemple, lorsque le groupe GP' est un tétrahydropyran-2-yle, la déprotection de l'alcool peut être effectuée en milieu acide, par exemple en présence d'acide chlorhydrique aqueux, dans un solvant tel que l'éthanol ou le méthanol, à une température comprise entre 20°C et la température d'ébullition du milieu réactionnel, de préférence en présence d'acide chlorhydrique aqueux, dans l'éthanol, à une température voisine de 20°C.
a') Alternativement, lorsque A est radical éthyle ou cycloalkyle (C5-C7), ces radicaux étant éventuellement substitués par 1 ou plusieurs substituants choisis parmi alkyle (C1-C5), cycloalkyle (C3-C7), arylalkyle, hétéroarylalkyle, aryle, hétéroaryle, les intermédiaires de formule (VI) peuvent être obtenus par réaction entre un composé de formule (III) et un époxyde approprié en présence d'une base telle que le tertiobutylate de potassium, dans un solvant aprotique comme le diméthylformamide, à une température comprise entre 20°C et la température d'ébullition du milieu réactionnel, selon Villalgordo J.M., Synthesis 1999, 1613.
c) Activation du résidu alcool des composés de formule (VI) par exemple par formation d'un tosylate ou d'un mésylate dénommé dans le schéma de synthèse comme Act. La réaction s'effectue alors avec du chlorure de tosyle ou du chlorure de mésyle en milieu basique, par exemple en présence de pyridine, dans un solvant comme le dichlorométhane, à une température comprise entre -20°C et la température d'ébullition du milieu réactionnel, de préférence à une température comprise entre -10°C et une température voisine de 20°C.d) Substitution du résidu alcool activé par une amine primaire ou secondaire de formule R1H. La réaction est réalisée en milieu basique, par exemple en présence de carbonate de potassium, dans un solvant polaire, tel que le diméthylformamide ou l'acétonitrile, à une température comprise entre 20°C et la température d'ébullition du milieu réactionnel, de préférence dans le diméthylformamide, à une température voisine de 80°C.

Les composés (V) sont commerciaux ou peuvent être obtenus à partir des alcools correspondants de formule GP'O-A-OH par des méthodes connues de l'homme de l'art telles que décrites dans J. March, Advanced Organic Chemistry, Wiley-Interscience, fourth edition (1992) ou R.C. Larock, Comprehensive Organic Transformations, VCH Publishers (1989). Les alcools de formule GP'O-A-OH sont commerciaux ou peuvent être obtenus par exemple par mono-protection d'un dialcool de formule HO-A-OH selon des méthodes connues de l'homme de l'art telles que décrites dans J. March, Advanced Organic Chemistry, Wiley-Interscience, fourth edition (1992) ou R.C. Larock, Comprehensive Organic Transformations, VCH Publishers (1989). Les composés de formule HO-A-OH sont commerciaux ou accessibles par l'homme de l'art par utilisation ou adaptation de méthodes décrites dans la littérature.

Une troisième voie de synthèse consiste lorsque R3 est ORc (Rc étant différent de C(O)R8, C(S)R8, SO2R8), H ou OGP à réaliser la synthèse à partir d'un dérivé de formule (VU) selon le schéma de synthèse décrit ci-dessous.

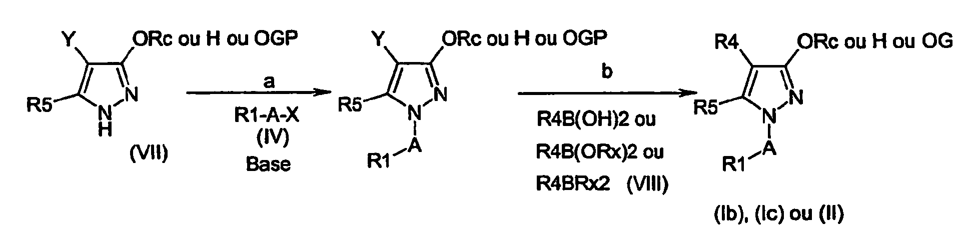

Les composés de formule (Ib), (Ic), et (II) peuvent être obtenus en deux étapes à partir des composés de formule (VII) pour lesquels Y = Br, I ou Cl (de préférence Br ou I) :
a) Alkylation du 4-halogéno-pyrazole de formule (VII) par un composé de formule (IV) tel que précédemment défini. La réaction est effectuée sous atmosphère inerte, par exemple sous argon ou sous azote, en milieu basique dans un solvant aprotique, par exemple en présence d'hydrure de sodium, au sein d'un solvant aprotique tel que le diméthylformamide, à une température comprise entre 20°C et la température d'ébullition du milieu réactionnel ou en présence de tertiobutylate de potassium, au sein d'un solvant tel que le diméthylformamide, à une température comprise entre 20°C et la température d'ébullition du milieu réactionnel. La réaction peut également être effectuée en présence de carbonate de potassium et éventuellement d'iodure de potassium, dans un solvant tel que l'acétone, la méthyléthylcétone, l'acétonitrile ou le diméthylformamide, de préférence dans la méthyléthylcétone, à la température d'ébullition du milieu réactionnel
b) Couplage de Suzuki de l'intermédaire obtenu et d'un acide boronique, d'un boronate d'alkyle, de cycloalkyle ou d'un (hétéro)aryldialkyle bore de formule (VIII) pour lequel Rx est un radical alkyle ou cycloalkyle. La réaction est effectuée sous atmosphère inerte en présence d'une base minérale comme K3PO4, Na2CO3 ou Ba(OH)2 , d'un sel de palladium (catalyseur) comme le bistriphénylphosphino dichloro palladium (PdCl2(PPh3)2), le tétrakistriphénylphosphine palladium (Pd(PPh3)4) ou le diphénylphosphinoférrocènyl palladium (PdCl2dppf), dans un solvant comme le diméthylformamide, diméthoxyéthane, tétrahydrofurane, dioxane, toluène, xylène ou éthanol, en présence éventuellement d'eau, à une température comprise entre 20°C et la température d'ébullition du milieu réactionnel (Kotha S. et coll, Tetrahedron 2002, 58, 9633).

Les acides boroniques, boronates d'alkyle, cycloalkyle ou (hétéro)aryldialkyl bore de formule (VIII) sont commerciaux ou sont obtenus par utilisation ou adaptation de méthodes décrites dans la littérature, par exemple dans Kabalka G.W. et coll., Tetrahedron Letters 1986, 27, 3843, Nicoud J.F. et coll., Tetrahedron Letters 1993, 34, 8237, Tour J.M. et coll., J. Amer. Chem. Soc. 1994, 116, 11723, ou Mueller T.J.J. et coll., Synthesis 2002, 9, 1163.

Les intermédiaires de formule (III) dans le cas où l'on a un radical ORc (Rc étant différent de C(O)R8, C(S)R8, SO2R8 et OGP en position 3 du pyrazole sont obtenus selon le schéma réactionnel représenté ci dessous :

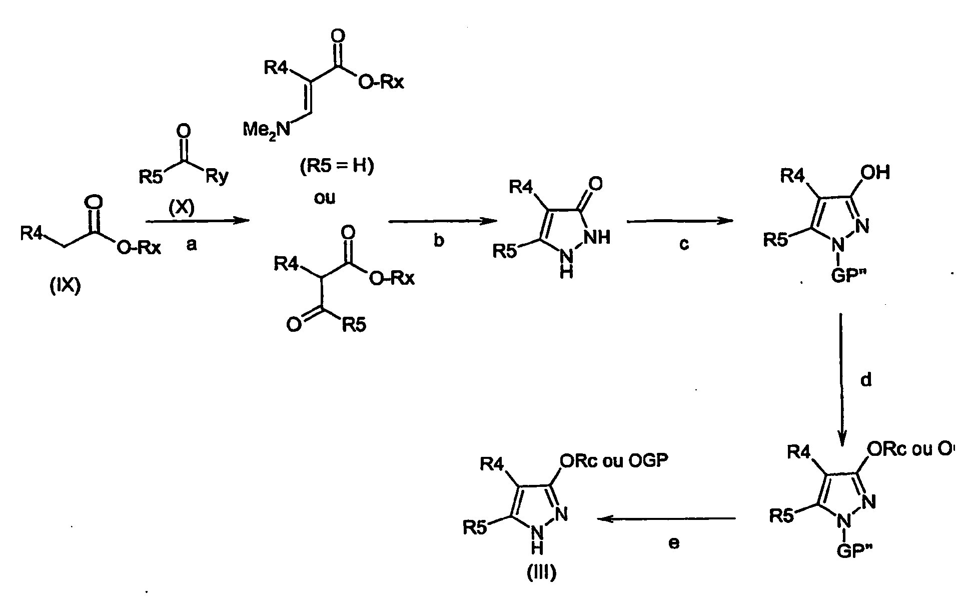

Les pyrazoles de formule (III) peuvent être obtenus en quatre étapes à partir des composés de formule (IX) selon le protocole suivant :
a) Condensation d'un (hétéro)aryl-acétate d'(aryl)alkyle pour lequel Rx = alkyle, aralkyle de formule (IX) avec un agent d'amino-méthylènation ou un agent de carbonylation de formule (X) pour le lequel Ry est un radical Cl, O-alkyle, O-aralkyle ou O-CO-alkyle, de préférence pour lequel Ry est un radical O-alkyle. La réaction d'amino-méthylènation peut être réalisée en présence d'un réactif tel que la N,N,N',N',N",N"-hexaméthyl-méthanetriamine, la C-méthoxy N,N,N',N'-tetraméthyl-méthanediamine ou la C-tert-butoxy-N,N,N',N'-tetraméthyl-méthanediamine, en l'absence de solvant ou dans un solvant comme le tétrahydrofurane ou le dioxane à une température comprise entre 20°C et la température d'ébullition du milieu réactionnel, de préférence en présence de C-tert butoxy-N,N,N',N'-tetraméthyl-méthanediamine dans le tétrahydrofurane à une température comprise entre 20°C et la température d'ébullition du milieu réactionnel. La réaction de carbonylation entre un (hétéro)aryl-acétate d'(aryl)alkyle et un agent de carbonylation de formule (X) s'effectue sous atmosphère inerte, par exemple sous argon ou sous azote, en milieu basique, par exemple en présence d'hydrure de sodium, dans un solvant aprotique tel que le diméthylformamide, à une température comprise entre -20°C et la température d'ébullition du solvant, de préférence à une température voisine de 20°C.
b) Formation du cycle 1H-pyrazol-3-ol par réaction de l'intermédiaire obtenu à l'étape précédente avec de l'hydrazine, généralement sous forme monohydratée, dans un alcool tel que l'éthanol, le propanol ou l'isopropanol, à une température comprise entre 20°C et la température d'ébullition du milieu réactionnel, de préférence dans l'éthanol, à la température d'ébullition du milieu réactionnel.
c) Protection de l'azote 1 du 1H-pyrazol-3-ol par un groupe protecteur tel qu'un acétyle, alkyloxycarbonyle ou tosyle, de préférence par un groupe acétyle . La réaction est effectuée avec un agent d'acétylation, d'alkyloxycarbonylation ou de tosylation, de préférence avec l'anhydride acétique sans solvant ou en présence d'un solvant tel que la pyridine, à une température comprise entre 20°C et la température d'ébullition du milieu réactionnel, de préférence à une température voisine de 100°C.d) Protection du groupement hydroxyle du pyrazole ou introduction du résidu -Rc sur l'hydroxyle du pyrazole, suivie de la déprotection de l'azote 1 du pyrazole. La protection du groupement hydroxyle du pyrazole et l'introduction du résidu -Rc sur l'hydroxyle du pyrazole peuvent être réalisées par exemple par alkylation du groupement hydroxyle du pyrazole avec les composés de formule GP-X ou Rc-X pour lesquels X est une fonction telle que Cl, Br, I, OTs, OMs , OTf. Dans le cas où Rc = Me ou Et, le diméthylsulfate ou le diéthylesulfate peuvent également être utilisés comme agents d'alkylation et seront choisis de préférence. La réaction s'effectue en milieu basique, par exemple en présence d'une base telle que le carbonate de potassium, dans un solvant tel que l'acétone, la méthyléthylcétone, l'acétonitrile ou le diméthylformamide, à une température comprise entre 20° et la température d'ébullition du milieu réactionnel, de préférence dans la méthyléthylcétone, à la température d'ébullition du milieu réactionnel. Lorsque Rc = -CHF2, l'alkylation peut être réalisée avec du chloro-difluoro-acétate de méthyle, en milieu basique, par exemple en présence d'une base telle que le carbonate de potassium, dans un solvant tel que le diméthylformamide, à une température comprise entre 20° et la température d'ébullition du milieu réactionnel, de préférence à une température voisine de 65°C. La déprotection de l'azote 1 du pyrazole est réalisée selon les méthodes décrites dans T. W. Greene et coll., Protective Groups in Organic Synthesis, Wiley-Interscience, third edition (1999). Par exemple, lorsque le groupe protecteur est un acétyle, la déprotection peut être réalisée en présence d'une base telle que la soude ou le carbonate de potassium, dans un alcool tel que l'éthanol ou le méthanol, éventuellement additionné d'un solvant tel que le tétrahydrofurane ou le dioxane, à une température comprise entre 20°C et la température d'ébullition du milieu réactionnel, de préférence en présence de soude dans un mélange d'éthanol et de tétrahydrofurane, à une température voisine de 20°C.

Les composés de formule (IX) sont commerciaux ou peuvent être obtenus par utilisation ou adaptation de méthodes décrites dans la littérature.

Les composés de formule (X) sont commerciaux ou peuvent être obtenus par utilisation ou adaptation de méthodes décrites dans la littérature.

Les composés de formule GP-X sont commerciaux. Les composés de formule ou Rc-X sont commerciaux ou peuvent être obtenus à partir des alcools correspondants de formule Rc-OH par des méthodes connues de l'homme de l'art telles que décrites dans J. March, Advanced Organic Chemistry, Wiley-Interscience, fourth edition (1992) ou R.C. Larock, Comprehensive Organic Transformations, VCH Publishers (1989). Les alcools de formule Rc-OH sont commerciaux ou peuvent être obtenus par utilisation ou adaptation de méthodes décrites dans la littérature.

Les intermédiaires de formule (III) dans le cas ou l'on a un hydrogène en positon 3 du pyrazole sont obtenus selon le schéma réactionnel représenté ci dessous :

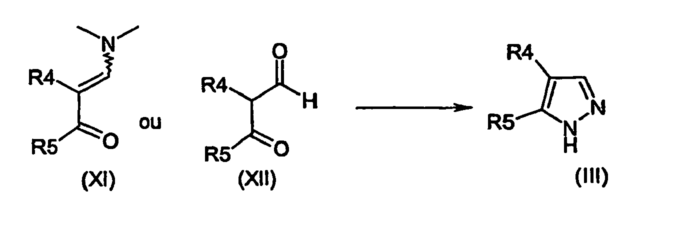

Les composés de formule (III) peuvent être obtenus à partir des composés de formule (XI) ou (XII) et d'hydrazine, généralement sous forme monohydratée. La réaction s'effectue par exemple dans un alcool tel que l'éthanol, le propanol ou l'isopropanol, à une température comprise entre 20°C et la température d'ébullition du milieu réactionnel, de préférence dans l'éthanol, à la température d'ébullition du milieu réactionnel.

Les composés de formule (XI) ou (XII) sont commerciaux ou peuvent être obtenus par utilisation ou adaptation de méthodes décrites dans la littérature.

Les intermédiaires de formule (III) dans le cas où l'on a un radical ORc (Rc étant différent de C(O)R8, C(S)R8, SO2R8 ou OGP ou H en position 3 du pyrazole peuvent également être obtenus selon le schéma réactionnel représenté ci dessous :

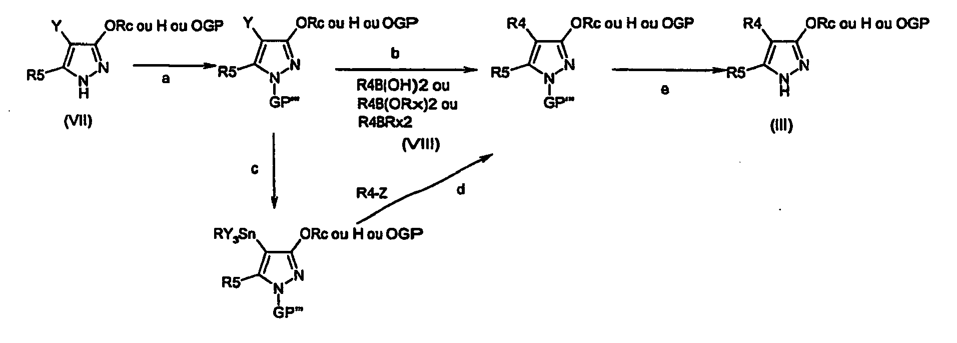

Les composés de formule (III) peuvent être préparés en trois ou quatre étapes à partir de composés de formule (VII):
a) Protection des composés de formule (VII) par exemple par un groupement tosyle, mésyle ou acétyle, de préférence par un groupement tosyle. Cette réaction est réalisée selon les procédés connus de l'homme de l'art et décrits dans T. W. Greene et coll., Protective Groups in Organic Synthesis, Wiley-Interscience, third edition (1999). Par exemple, lorsque le groupements protecteur est un tosyle, la réaction s'effectue avec du chlorure de tosyle en milieu basique, par exemple en présence d'hydrure de sodium ou de tertiobutylate de potassium dans un solvant aprotique tel que le diméthylformamide à une température comprise entre -10°C et la température d'ébullition du milieu réactionnel.
b) Introduction du groupe R4 par un couplage de Suzuki ou par deux réactions de Stille consécutives. L'introduction du groupe R4 par un couplage de Suzuki s'effectue à partir du 4-halogéno-pyrazole protégé obtenu dans l'étape précédente et d'un acide boronique, d'un boronate d'alkyle, cycoalkyle ou d'un (hétéro)aryldialkyle bore de formule (VII) pour lequel Rx est un radical alkyle ou cycloalkyle, sous atmosphère inerte, en présence d'une base minérale comme K3PO4 , Na2CO3 ou Ba(OH)2 , d'un sel de palladium (catalyseur) comme le bistriphénylphosphino dichloro palladium (PdCl2(PPh3)2), le tétrakistriphénylphosphine palladium (Pd(PPh3)4) ou le diphénylphosphinoférrocènyl palladium (PdCl2dppf), dans un solvant comme le diméthylformamide, diméthoxyéthane, tétrahydrofurane, dioxane, toluène, xylène ou éthanol, en présence éventuellement d'eau à une température comprise entre 20°C et la température d'ébullition du milieu réactionnel. Alternativement l'introduction du groupe R4 peut être effectuée par deux réactions de Stille consécutives. La première réaction de Stille est effectuée sous atmosphère inerte à partir du 4-halogéno-pyrazole protégé obtenu dans l'étape précédente et de bis(tributylétain) en présence de iodure cuivreux, d'un sel de palladium (catalyseur) comme le diacétate de palladium (Pd(OAc)2) et de triphénylphosphine, dans un solvant comme le tétrahydrofurane à une température comprise entre 20°C et la température d'ébullition du milieu réactionnel, selon Scott A.L et coll., Tetrahedron Lett. 1996, 37, 3247. La seconde réaction de Stille se fait à partir de l'organoétain précédent et d'un dérivé aromatique halogéné de formule R4-Z pour lequel Z est un radical Br, I ou Cl (de préférence Br ou I), avec un sel de palladium (catalyseur) comme le tris(dibenzylidène)dipalladium (Pd2dba3) et de tristrifurylphosphine, dans un solvant comme le dioxane à une température comprise entre 20°C et la température d'ébullition du milieu réactionnel, selon U. Hacksell et coll., Bioorg. & Med. Chem. Lett., 1994,2837.
c) Clivage du groupement protecteur introduit à la première étape. Cette réaction est réalisée selon les procédés connus de l'homme de l'art et décrits dans T. W. Greene et coll., Protective Groups in Organic Synthesis, Wiley-Interscience, third edition (1999). Par exemple, lorsque le groupement protecteur est un tosyle, la réaction peut être réalisée avec du fluorure de téhabutylammonium dans un solvant comme le tétrahydrofurane ou le dioxane à une température comprise entre 20°C et la température d'ébullition du milieu réactionnel selon Sakamoto T. et coll., Tetrahedron Lett. 1998, 39, 595.

Les intermédiaires (III) dans le cas où l'on a en position 3 du pyrazole un radical NH2 peuvent être obtenus selon le schéma suivant :

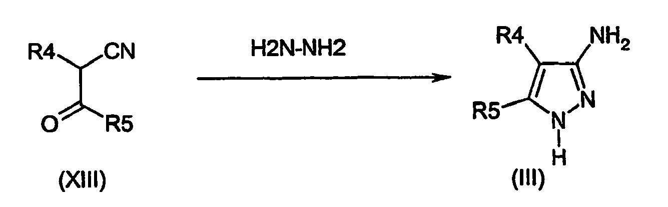

Les composés de formule (III) peuvent être obtenus par condensation d'hydrazine, généralement sous forme monohydratée, sur un 2-(hétéro)aryl-3-oxo-propionitrile de formule (XIII) en milieu acide, par exemple en présence d'acide acétique, dans un alcool tel que l'éthanol, le propanol ou l'isopropanol, à une température comprise entre 20°C et la température d'ébullition du milieu réactionnel, de préférence dans l'éthanol, à la température d'ébullition du milieu réactionnel.

Les composés de formule (XIII) peuvent être obtenus par utilisation ou adaptation de méthodes décrites dans la littérature.

Les intermédiaires (VII) pour lesquels en position trois du pyrazole on a un H, OGP, ORc (Rc étant différent de C(O)R8, C(S)R8, S02R8) sont obtenus à partir du dérivé de formule (XIV)

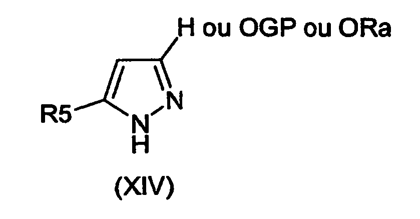

Les intermédiaires (VII) pour lesquels Y= Br, I ou Cl (de préférence Br ou I) sont commerciaux ou peuvent être obtenus à partir d'intermédiaires de formule (XIV). La réaction s'effectue avec un agent d'halogénation tel que le brome ou le chlorure d'iode dans un solvant comme le dichlorométhane ou le chloroforme, en présence d'une base telle que le carbonate de potassium, à une température comprise entre - 10°C et la température d'ébullition du milieu réactionnel, de préférence avec le brome, dans le dichlorométhane, à une température voisine de 20°C.

Les intermédiaires (XIV) pour lesquels en position 3 du pyrazole on a un hydrogène sont commerciaux ou sont obtenus par utilisation ou adaptation des méthodes décrites dans la littérature.

Les intermédiaires (XIV) pour lesquels en position 3 du pyrazole on a un radical OGP ou un radical ORc (Rc étant différent de C(O)R8, C(S)R8, S02R8) peuvent être obtenus en deux étapes à partir de composés de formule (XV) selon le protocole suivant : 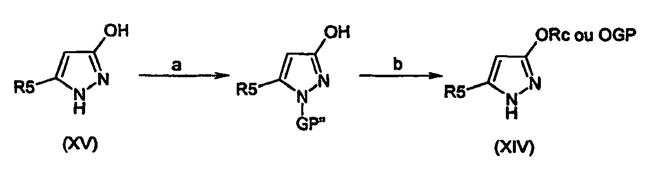
a) Protection de l'azote 1 du 1H-pyrazol-3-ol par un groupe protecteur tel qu'un acétyle, alkyloxycarbonyle ou tosyle, de préférence par un groupe acétyle. La réaction est effectuée avec un agent d'acétylation, d'alkyloxycarbonylation ou de tosylation, de préférence avec l'anhydride acétique sans solvant ou en présence d'un solvant tel que la pyridine, à une température comprise entre 20°C et la température d'ébullition du milieu réactionnel, de préférence à une température voisine de 100°C.
b) Protection du groupement hydroxyle du pyrazole ou introduction du résidu -Rc sur l'hydroxyle du pyrazole, suivie de la déprotection de l'azote 1 du pyrazole. La protection du groupement hydroxyle du pyrazole et l'introduction du résidu -Rc sur l'hydroxyle du pyrazole peuvent être réalisées par alkylation du groupement hydroxyle du pyrazole avec les composés de formule GP-X ou Rc-X pour lesquels X est une fonction telle que Cl, Br, I, OTs, OMs , OTf. Dans le cas où Rc est un groupement méthyle ou éthyle, le diméthylsulfate ou le diéthylesulfate peuvent également être utilisés comme agents d'alkylation et seront choisis de préférence. La réaction s'effectue en milieu basique, par exemple en présence d'une base telle que le carbonate de potassium, dans un solvant tel que l'acétone, la méthyléthylcétone, l'acétonitrile ou le diméthylformamide, à une température comprise entre 20° et la température d'ébullition du milieu réactionnel, de préférence dans la méthyléthylcétone, à la température d'ébullition du milieu réactionnel. Lorsque Rc est un groupement CHF2, l'alkylation peut être réalisée avec du chloro-difluoro-acétate de méthyle, en milieu basique, par exemple en présence d'une base telle que le carbonate de potassium, dans un solvant tel que le diméthylformamide, à une température comprise entre 20° et la température d'ébullition du milieu réactionnel, de préférence à une température voisine de 65°C. La déprotection de l'azote du pyrazole est réalisée selon les méthodes décrites dans T. W. Greene et coll. dans Protective Groups in Organic Synthesis, Wiley-Interscience, third édition (1999). Par exemple, lorsque le groupe protecteur est un acétyle, la déprotection peut être réalisée en présence d'une base telle que la soude ou le carbonate de potassium, dans un alcool tel que l'éthanol ou le méthanol, éventuellement additionné d'un solvant tel que le tétrahydrofurane ou le dioxane, à une température comprise entre 20°C et la température d'ébullition du milieu réactionnel, de préférence en présence de soude dans un mélange d'éthanol et de tétrahydrofurane, à une température voisine de 20°C.

Les composés de formule (XV) sont obtenus par utilisation ou adaptation des méthodes décrites dans la littérature.

Les composés de formule (Id) peuvent également être obtenus en sept ou huit étapes à partir des composés de formule (XVI) pour lesquels Y= Br, I ou Cl (de préférence Br ou I) selon le protocole suivant : 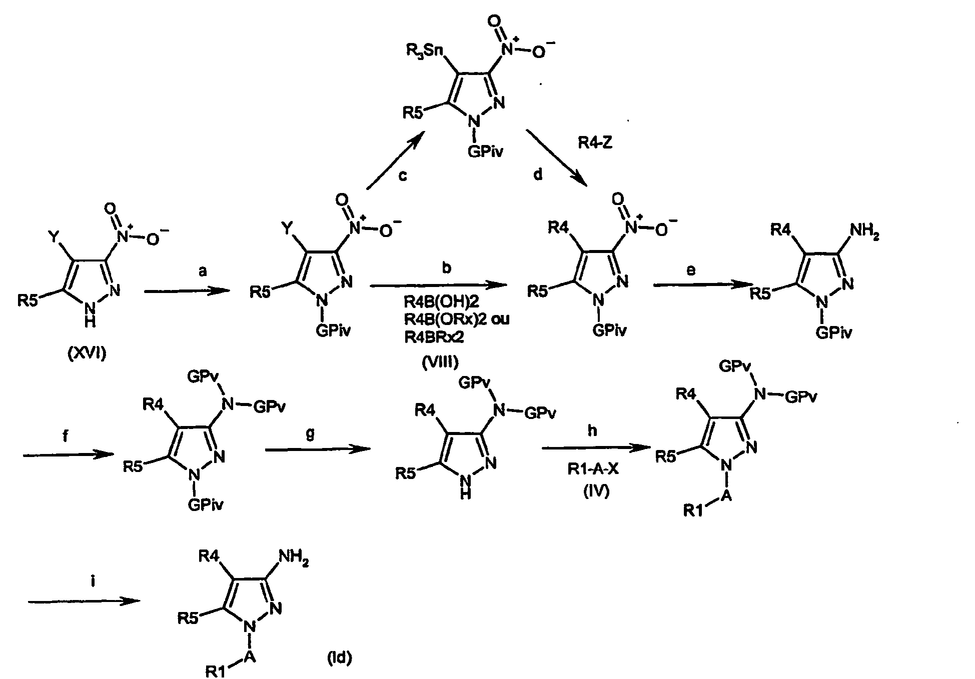
a) Protection des 4-halogéno-3-nitro-pyrazoles de formule (XVI) par exemple par un groupement 2-triméthylsilanyl-éthoxyméthyle. Cette réaction est réalisée selon les procédés connus de l'homme de l'art et décrits dans T. W. Greene et coll., Protective Groups in Organic Synthesis, Wiley-Interscience, third edition (1999). Par exemple, la réaction s'effectue avec du chlorure de 2-triméthylsilanyl-éthoxyméthyle en milieu basique, par exemple en présence d'hydrure de sodium dans un solvant aprotique tel que le diméthylformamide à une température comprise entre -10°C et la température d'ébullition du milieu réactionnel.
b) Introduction du groupe R4 par un couplage de Suzuki ou par deux réactions de Stille consécutives. L'introduction du groupe R4 par un couplage de Suzuki s'effectue à partir du 4-halogéno-3-nitro-pyrazole protégé obtenu dans l'étape précédente et d'un acide boronique, d'un boronate d'alkyle, cycoalkyle ou d'un (hétéro)aryldialkyle bore de formule (VII) pour lequel Rx est un radical alkyle ou cycloalkyle, sous atmosphère inerte, en présence d'une base minérale comme K3P04 Na2CO3 ou Ba(OH)2 , d'un sel de palladium (catalyseur) comme le bistriphénylphosphino dichloro palladium (PdCl2(PPh3)2), le tétrakistriphénylphosphine palladium (Pd(PPh3)4) ou le diphénylphosphinoférrocènyl palladium (PdCl2dppf), dans un solvant comme le diméthylformamide, diméthoxyéthane, tétrahydrofurane, dioxane, toluène, xylène ou éthanol, en présence éventuellement d'eau à une température comprise entre 20°C et la température d'ébullition du milieu réactionnel. Alternativement l'introduction du groupe R4 peut être effectuée par deux réactions de Stille consécutives. La première réaction de Stille est effectuée sous atmosphère inerte à partir du 4-halogéno-3-nitro-pyrazole protégé obtenu dans l'étape précédente et de bis(tributylétain) en présence de iodure cuivreux, d'un sel de palladium (catalyseur) comme le diacétate de palladium (Pd(OAc)2) et de triphénylphosphine, dans un solvant comme le tétrahydrofurane à une température comprise entre 20°C et la température d'ébullition du milieu réactionnel, selon Scott A.I. et coll., Tetrahedron Lett. 1996, 37, 3247. La seconde réaction de Stille se fait à partir de l'organoétain précédent et d'un dérivé aromatique halogéné de formule R4-Z pour lequel Z est un radical Br, I ou Cl (de préférence Br ou I), avec un sel de palladium (catalyseur) comme le tris(dibenzylidène)dipalladium (Pd2dba3) et de tristrifurylphosphine, dans un solvant comme le dioxane à une température comprise entre 20°C et la température d'ébullition du milieu réactionnel, selon U. Hacksell et coll., Bioorg. & Med. Chem. Lett., 1994, 2837.
e) Réduction de la fonction nitro selon un protocole tel que décrit dans J. March, Advanced Organic Chemistry, Wiley-Interscience, fourth edition (1992) ou R.C. Larock, Comprehensive Organic Transformations, VCH Publishers (1989). Par exemple, cette réaction peut être réalisée à l'aide de fer en présence de chlorure d'ammonium dans un mélange d'un alcool tel que l'éthanol et d'eau à une température comprise entre 20°C et la température d'ébullition du milieu réactionnel.
f) Double protection du résidu amino obtenu dans l'étape précédente par un groupe protecteur GP^{v}. Le groupe GP^{v} est un groupe protecteur d'amine tel que défini dans T. W. Greene et coll., Protective Groups in Organic Synthesis, Wiley-Interscience, third edition (1999) et résistant aux conditions de déprotection du groupe GP^{iv}. Par exemple le groupe GP^{v} peut être un allyle, un benzyle ou un para-méthoxy-benyle. Le groupe GP^{v} est introduit selon les procédés connus de l'homme de l'art et décrits dans T. W. Greene et coll., Protective Groups in Organic Synthesis, Wiley-Interscience, third edition (1999). Par exemple, lorsque le groupe protecteur GP^{v} est un allyle, la réaction s'effectue avec du bromure d'allyle en présence d'une base telle que le carbonate de césium, dans un solvant aprotique comme l'acétonitrile ou le diméthylformamide, à une température comprise entre 20° C et la température d'ébullition du milieu réactionnel.
g) Clivage du groupe protecteur GP^{iv} introduit à la première étape selon les procédés connus de l'homme de l'art et décrits dans T. W. Greene et coll., Protective Groups in Organic Synthesis, Wiley-Interscience, third edition (1999). Par exemple, lorsque le groupe protecteur GP^{iv} est un 2-triméthylsilanyl-éthoxyméthyle, la réaction peut être réalisée avec du fluorure de tétrabutylammonium dans un solvant comme le tétrahydrofurane ou le dioxane à une température comprise entre 20°C et la température d'ébullition du milieu réactionnel.
h) Alkylation du composé obtenu à l'étape précédente par un composé de formule (IV) R1-A-X tel que défini précédemment. La réaction est réalisée sous atmosphère inerte, par exemple sous argon ou sous azote, en milieu basique dans un solvant aprotique, par exemple en présence d'hydrure de sodium, au sein d'un solvant aprotique tel que le diméthylformamide, à une température comprise entre 20°C et la température d'ébullition du milieu réactionnel ou en présence de tertiobutylate de potassium, au sein d'un solvant tel que le diméthylformamide, à une température comprise entre 20°C et la température d'ébullition du milieu réactionnel. La réaction peut également être effectuée en présence de carbonate de potassium et éventuellement d'iodure de potassium, dans un solvant tel que l'acétone, la méthyléthylcétone, l'acétonitrile ou le diméthylformamide, de préférence dans la méthyléthylcétone, à la température d'ébullition du milieu réactionnel.
i) Clivage du groupe protecteur GP^{v} introduit à l'étape f) selon les procédés connus de l'homme de l'art et décrits dans T. W. Greene et coll., Protective Groups in Organic Synthesis, Wiley-Interscience, third edition (1999). Par exemple, lorsque le groupe protecteur GP^{v} est un allyle, la réaction peut être réalisée avec un sel de palladium tel que le tétrakistriphénylphosphine palladium (Pd(PPh3)4) en présence d'un acide tel que l'acide N,N-diméthylbarbiturique dans un solvant aproptique comme le dichlorométhane à une température comprise entre 20°C et la température d'ébullition du milieu réactionnel.

Les composés de formule (XVI) sont commerciaux ou sont obtenus par analogie avec des méthodes décrites dans la littérature.

Les composés (Ib) pour lesquels Rc est un radical C(O)R8, C(S)R8 ou SO2R8 peuvent être obtenus à partir des composés (Ia) suivant des protocoles connus de l'homme de l'art et décrits par exemple dans J. March, Advanced Organic Chemistry, Wiley-Interscience, fourth edition (1992), R.C. Larock, Comprehensive Organic Transformations, VCH Publishers (1989) ou Bradford P. Mundy et Michael G. Ellerd, Name Reactions and Reagents in Organic Synthesis, A. Wiley-Interscience Publication (1988).

Les composés (Ie) peuvent être obtenus à partir des composés (Id) suivant des protocoles connus de l'homme de l'art et décrits par exemple dans J. March, Advanced Organic Chemistry, Wiley-Interscience, fourth edition (1992), R.C. Larock, Comprehensive Organic Transformations, VCH Publishers (1989) ou Bradford P. Mundy et Michael G. Ellerd, Name Reactions and Reagents in Organic Synthesis, A. Wiley-Interscience Publication (1988).

Les composés (If) peuvent être obtenus à partir des composés (Ia) par réaction avec un agent de thionation, tel que par exemple le réactif de Lawesson, et suivant des protocoles décrits par exemple dans J. March, Advanced Organic Chemistry, Wiley-Interscience, fourth edition (1992).

Les composés (Ig) peuvent être obtenus à partir des composés (If) suivant des protocoles connus de l'homme de l'art et décrits par exemple dans J. March, Advanced Organic Chemistry, Wiley-Interscience, fourth edition (1992), R.C. Larock, Comprehensive Organic Transformations, VCH Publishers (1989) ou Bradford P. Mundy et Michael G. Ellerd, Name Reactions and Reagents in Organic Synthesis, A. Wiley-Interscience Publication (1988).

Les composés (Ih) peuvent être obtenus par oxydation des composés (Ig) pour lesquels Rc=Ra à l'aide de réactifs tels que par exemple l'eau oxygénée, le permanganate de potassium ou l'oxone , et suivant des protocoles décrits par exemple dans J. March, Advanced Organic Chemistry, Wiley-Interscience, fourth edition (1992).

Les composés de formule (I) pour lesquels le groupe R4 est substitué par un ou plusieurs radicaux OH peuvent être obtenus par déméthylation des composés méthoxylés correspondants selon un protocole n'affectant pas le reste de la molécule tel que décrit dans Protective Groups in Organic Synthesis, T.W. Greene, Ed. by Wiley, third edition (1999). Cette réaction peut être par exemple réalisée avec du tribromure de bore dans un solvant comme le .dichlorométhane à une température comprise entre -5°C et la température d'ébullition du milieu réactionnel.

Les composés de formule (I) pour lesquels le groupe R4 est substitué par un ou plusieurs radicaux NH2 peuvent être obtenus par réduction des composés nitrés correspondants selon un protocole tel que décrit dans J. March, Advanced Organic Chemistry, Wiley-Interscience, fourth edition (1992) ou R.C. Larock, Comprehensive Organic Transformations, VCH Publishers (1989). Par exemple, cette réaction peut être réalisée par hydrogénation en présence d'un catalyseur comme le palladium sur charbon, et éventuellement d'un acide tel que l'acide chlorhydrique, dans un alcool tel que l'éthanol, le méthanol ou l'isopropanol, à une pression d'hydrogène comprise entre 1 bar et 20 bars et à une température comprise entre 20°C et la température d'ébullition du milieu réactionnel.

Les composés de formule (Ib), (Ic) ou (II) pour lesquels le groupe R4 est substitué par un ou plusieurs radicaux NRaRb, NHC(O)Ra, C(O)NRaRb, NHS02Ra ou NHC(S)Ra peuvent être obtenus par réduction des composés nitrés correspondants , suivie d'une fonctionalisation appropriée des dérivés aminés obtenus. La réduction des composés nitrés s'effectue selon un protocole n'affectant pas le reste de la molécule, tel que décrit dans J. March, Advanced Organic Chemistry, Wiley-Interscience, fourth edition (1992) ou R.C. Larock, Comprehensive Organic Transformations, VCH Publishers (1989). Par exemple, cette réaction peut être réalisée avec un agent réducteur comme la poudre de fer, en présence de chlorure d'ammonium dans un mélange d'eau et d'un alcool tel que le méthanol ou l'éthanol, à une température comprise entre 20°C et la température d'ébullition du milieu réactionnel, de préférence dans l'éthanol à la température d'ébullition du milieu réactionnel. La fonctionalisation des dérivés aminés résultants se fait suivant des méthodes n'affectant pas le reste de la molécule, connues de l'homme de l'art et décrites, par exemple, dans J. March, Advanced Organic Chemistry, Wiley-Interscience, fourth edition (1992), R.C. Larock, Comprehensive Organic Transformations, VCH Publishers (1989), Bradford P. Mundy et Michael G. Ellerd, Name Reactions and Reagents in Organic Synthesis, A. Wiley - Interscience Publication (1988) ou Hartwig J.F., Angew. Chem. Int. Ed. Engl. 1998,2047.

Les composés de formule (Ib), (Ic) ou (II) pour lesquels le groupe R4 est substitué par un ou plusieurs radicaux aryle ou hétéroaryle peuvent être obtenus à partir des composés halogénés correspondants (de préférence bromés ou iodés) et des acides boroniques, des boronates d'alkyle, de cycloalkyle ou des (hétéro)aryldialkyle bore appropriés par couplage de Suzuki. Cette réaction est réalisée sous atmosphère inerte en présence d'une base minérale comme K3PO4, Na2CO3 ou Ba(OH)2 , d'un sel de palladium (catalyseur) comme le bistriphénylphosphino dichloro palladium (PdCI2(PPh3)2), le tétrakistriphénylphosphine palladium (Pd(PPh3)4) ou le diphénylphosphinoférrocènyl palladium (PdCl2dppf), dans un solvant comme le diméthylformamide, diméthoxyéthane, tétrahydrofurane, dioxane, toluène, xylène ou éthanol, en présence éventuellement d'eau, à une température comprise entre 20°C et la température d'ébullition du milieu réactionnel.

Les composés de formule (I) sont isolés et peuvent être purifiés par les méthodes connues habituelles, par exemple par cristallisation, chromatographie ou extraction.

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Comme exemples de sels pharmaceutiquement acceptables, peuvent être cités les sels suivants : benzènesulfonate, bromhydrate, chlorhydrate, citrate, éthanesulfonate, fumarate, gluconate, iodate, maléate, iséthionate, méthanesulfonate, , nitrate, oxalate, palmoate, phosphate, salicylate, succinate, sulfate, tartrate, théophyllinacétate et p-toluènesulfonate.

Les composés de l'invention ont été testés quant à leur capacité à lier les récepteurs nicotiniques contenant la sous-unité α7 à l'aide d'un test de liaison sur préparations de membranes de cerveau de rat selon les méthodes décrites ci-dessous:

### Préparations des membranes :

Des échantillons congelés d'hippocampe de cerveau de rats femelles Sprague-Dawley ont été conservés à -20°C jusqu'à utilisation. Les hippocampes de 10 rats ont été regroupés et homogénéisés à l'aide d'un broyeur de type Polytron dans 10 volumes d'un tampon refroidis dans la glace de composition suivante: KCl (11 mM); KH₂PO₄ (6mM); NaCl (137 mM); Na₂HPO₄ (8 mM); HEPES (20 mM); iodoacetamide (5 mM); EDTA (1.5 mM); PMSF (0.1 mM). Le pH a été ajusté à 7.4 à l'aide de NaOH. le mélange obtenu a été centifugé à 24000 g pendant 20 minutes à 4°C et le culot remis en suspension dans 20 volumes d'eau glacée. Après 60 minutes d'incubation à 4°C, un nouveau culot a été obtenu par centrifugation à 24000 g pendant 20 minutes à 4°C. Ce dernier a été remis en suspension dans du tampon de composition ci-dessus et congelé à -20°C. Le jour de l'essai, les membranes ont été décongelées, centrifugées à 24000 g pendant 20 minutes, puis remises en suspension à une concentration finale de 2-5 mg de protéines/mL dans du tampon phosphate de Dulbecco à pH 7,4 contenant 0.05 % d'albumine sérique de boeuf.

### Mesure de l'affinité pour les récepteurs contenant la sous-unité α7:

La liaison des composés de l'invention pour les récepteurs contenant la sous-unité α7 a été mesurée par compétition vis-à-vis de [³H]-methyllycaconitine ([³H]-MLA), un traceur radiomarqué reconnaissant les récepteurs α7 (Davies et al., Neuropharmacology 1999, 38, 679-690) selon des méthodes classiques adaptées au format de plaques 96-puits. La capacité des composés de l'invention à déplacer la liaison de [³H]-MLA sur des membranes d'hippocampe de rat a été déterminée en duplicate après 2 heures d'incubation à température ambiante. Chaque puits contenait un échantillon d'environ 150 µg de protéines membranaires, 5 nM de [³H]-MLA et l'un des composés de l'invention dilué à une concentration déterminée dans du tampon phosphate de Dulbecco à pH 7,4 contenant 0.05% d'albumine sérique de boeuf pour un volume final de 150 µL. La liaison non-spécifique a été déterminée dans des puits spécifiques contenant 10 µM de MLA non-radiomarquée. L'incubation a été stoppée en filtrant le contenu de chaque puits à travers des filtres en fibres de verre (Whatman GF/B) préalablement trempés dans une solution de polyéthylènimine à 0,33 % dans du tampon phosphate de Dulbecco pour diminuer la liaison non spécifique. Les filtres ont ensuite été lavés 3 fois avec du tampon phosphate de Dulbecco, puis séchés à 50°C pendant environ 2 heures. La radioactivité retenue sur les filtres a été mesurée par application de scintillant (MeltiLex A, Perkin Elmer) suivi d'un comptage en luminométrie (Trilux 1450 microbeta, Perkin-Elmer).

### Analyse des données.

Pour chaque composé testé, la radioactivité résiduelle sur les filtres a été exprimée en coups par minutes. Les déterminations en duplicate ont été moyennées et la concentration de composé qui inhibe de moitié la liaison spécifique du traceur radioactif (CI₅₀) a été calculée par régression curviligne à l'aide d'un logiciel spécifique (GraphPad Prism). Les constantes d'affinité apparente Ki des composés de l'invention ont été calculées à l'aide de l'équation de Cheng et Prusoff (Cheng et Prusoff, Biochem. Pharmacol. 1973, 22, 3099-3108).

Les composés de l'invention qui ont été étudiés dans ce test présentent une valeur de Ki inférieure à 10 µM.

Les exemples suivants illustrent l'invention de manière non limitative.

### Exemple 1

Dichlorhydrate de 1-[2-(3-méthoxy-4-phényl-pyrazol-1-yl)-éthyl]-pipéridine

A une solution de 0,25 g de 3-méthoxy-4-phényl-pyrazole dans 20 cm³ de diméthylformamide anhydre on ajoute progressivement, sous atmosphère d'argon et à température ambiante, 0,303 g d'hydrure de sodium (à 75 % en masse dans l'huile de vaseline). Après 0,75 heure d'agitation à une température voisine de 50°C on ajoute par petites portions 0,793 g de chlorhydrate de 1-(2-chloro-éthyl)-pipéridine, puis chauffe pendant 8 heures à une température voisine de 50°C. Le mélange est refroidi à température ambiante puis est additionné de 10 cm³ d'eau et concentré à sec sous pression réduite (3 kPa). Le résidu d'évaporation est repris dans 25 cm³ d'eau et extrait avec 250 cm³ d'acétate d'éthyle. La phase organique est lavée avec 3 fois 25 cm³ d'eau, puis est séchée sur sulfate de magnésium anhydre, filtrée et concentrée à sec sous pression réduite (3 kPa), pour donner un résidu huileux qui est purifié par chromatographie sur gel de silice (granulométrie 15-35 µm), en éluant par un mélange d'acétate d'éthyle et de cyclohexane (67 / 33 en volumes). Après concentration des fractions sous pression réduite, on obtient 0,3 g d'une huile incolore, qui est dissoute dans 15 cm³ d'acétone et additionnée de 30 cm³ d'une solution environ 3 M d'éther chlorhydrique. Le précipité blanc formé est filtré puis séché sous vide (70 Pa) à une température de 60°C. On obtient ainsi 0,325 g de dichlorhydrate de 1-[2-(3-méthoxy-4-phényl-pyrazol-1-yl)-éthyl]-pipéridine sous forme d'un solide blanc fondant à 208°C (avec décomposition).

Spectre de R.M.N. ¹H (300 MHz, (CD₃)₂SO d6, δ en ppm) : 1,40 (mt : 1H) ; de 1,60 à 1,90 (mt : 5H) ; 2,94 (mt : 2H) ; de 3,40 à 3,60 (mt : 4H) ; 3,95 (s : 3H) ; 4,47 (t, J = 6,5 Hz : 2H) ; 7,29 (t large, J = 7,5 Hz : 1H) ; 7,36 (t large, J = 7,5 Hz : 2H) ; 7,62 (d large, J = 7,5 Hz : 2H) ; 8,14 (s : 1H) ; 10,03 (mf : 1H). Spectre IR (KBr) : 3031; 2945; 2632; 2540; 1606; 1579; 1518; 1456; 1411; 1047; 1030; 764 et 697 cm-¹.

Le 3-méthoxy-4-phényl-pyrazole peut être préparé de la manière suivante :

Une suspension de 2 g de 1-(3-hydroxy-4-phényl-pyrazol-1-yl)-éthanone, 1,37 g de carbonate de potassium et 1,13 cm³ (1,5 g) de sulfate de diéthyle dans 70 cm³ de 2-butanone est agitée à une température de 70°C pendant 4 heures. Le mélange est additionné de 24 cm³ d'une solution d'hydroxyde de sodium 1,66 N et agité 4 heures à température ambiante, puis concentré partiellement sous pression réduite (3 kPa) pour chasser la 2-butanone. Le résidu est repris par 10 cm³ d'eau et extrait avec 250 cm³ d'acétate d'éthyle. La phase organique est lavée avec 3 fois 25 cm³ d'eau, puis est séchée sur sulfate de magnésium anhydre, filtrée et concentrée à sec sous pression réduite (3 kPa), pour donner un résidu solide brun clair, qui est purifié par chromatographie sur gel de silice (granulométrie 15-35 µm), en éluant par un mélange de dichlorométhane et de méthanol (98,5 / 1,5 en volumes). Après concentration des fractions sous pression réduite, on obtient 0,3 g de 3-méthoxy-4-phényl-pyrazole sous forme d'une poudre jaune pâle fondant à 150°C.

La 1-(3-hydroxy-4-phényl-pyrazol-1-yl)-éthanone peut être préparée de la manière suivante :

A une solution de 6,4 g de 4-phényl-1H-pyrazol-3-ol dans 64 cm³ de pyridine préchauffée à 100°C on ajoute, en 10 minutes, 3,4 cm³ d'anhydride acétique. Après 30 minutes supplémentaires à 100 °C, le mélange est refroidi et versé dans 600 cm³ de mélange eau-glace. Le précipité apparu est filtré, lavé avec 4 fois 100 cm³ d'eau glacée puis avec 4 fois 100 cm³ d'heptane, puis séché sous vide (70 Pa) à une température de 60°C. On obtient ainsi 5,09 g de 1-(3-hydroxy-4-phényl-pyrazol-1-yl)-éthanone sous forme d'une poudre beige fondant à 215°C.

Le 4-phényl-1H-pyrazol-3-ol peut être obtenu selon la méthode décrite par D. L. Selwood et col., J. Med. Chem. 2001, 44, 78-93.

### Exemple 2

### Dichlorhydrate de 1-(1-aza-bicyclo[2.2.2]oct-3-ylméthyl)-4-phényl-1H-pyrazol-1-ol

Une suspension de 0,67 g de dichlorhydrate de 3-(3 benzyloxy-4-phényl-pyrazol-1-ylméthyl)-1-aza-bicyclo[2.2.2]octane et 0,08 g de palladium sur charbon (à 10%) dans 20 cm³ d'éthanol est agitée en autoclave sous une pression d'hydrogène de 500 kPa, à une température de 20°C pendant 20 heures. Le milieu réactionnel est ensuite filtré sur Célite® et concentré à sec sous pression réduite (3 kPa), pour donner un résidu pâteux qui est recouvert par 50 cm³ d'acétone et trituré pendant une nuit. Après filtration du solide apparu et séchage sous vide (70 Pa) à une température de 60°C on obtient 0,265 g de dichlorhydrate de 1-(1-aza-bicyclo[2.2.2]oct-3-ylméthyl)-4-phényl-1H-pyrazol-1-ol sous forme de cristaux beiges hygroscopiques fondant vers 240°C (avec décomposition). Spectre de R.M.N. ¹H (300 MHz, (CD₃)₂SO d6, δ en ppm) : de 1,65 à 1,95 (mt : 4H); de 1,95 à 2,15 (mt : 1H) ; de 2,40 à 2,60 (mt : 1H) ; 2,96 (dd large, J = 12,5 et 7,5 Hz : 1H) ; de 3,10 à 3,40 (mt : 5H) ; 4,03 (dd, J = 13,5 et 7,5 Hz : 1H) ; 4,10 (dd, J = 13,5 et 7,5 Hz : 1H) ; 7,15 (t large, J = 7,5 Hz : 1H) ; 7,34 (t large, J = 7,5 Hz : 2H) ; 7,66 (d large, J = 7,5 Hz : 2H) ; 8,00 (s : 1H) ; 10,51 1 (mf: 1H). Spectre IR (KBr) : 3417; 2940; 2546; 1601; 1474; 1388; 1189; 768; 702 et 611 cm⁻¹.

### Exemple 3

### Dichlorhydrate de 3-(3-benzyloxy-4-phényl-pyrazol-1-ylméthyl)-1-aza-bicyclo[2.2.2]octane

A une suspension de 3,84 g d'hydrure de sodium (à 75% en masse dans l'huile de vaseline) dans 20 cm³ de diméthylformamide anhydre on ajoute progressivement, sous atmosphère d'argon et à température ambiante, une solution de 5 g de 3-benzyloxy-4-phényl-pyrazole dans 30 cm³ de diméthylformamide anhydre. Après 0,75 heure d'agitation à une température voisine de 50°C on ajoute par petites portions 11,78 g de chlorhydrate de 3-chlorométhyl-1-aza-bicyclo[2.2.2]octane, puis chauffe pendant 18 heures à une température voisine de 50°C. Le mélange est refroidi à température ambiante puis est additionné lentement de 25 cm³ d'eau, puis versé dans 300 cm³ d'eau et extrait avec 2 fois 300 cm³ d'acétate d'éthyle. Les phases organiques réunies sont lavées avec 3 fois 100 cm³ d'eau, puis séchées sur sulfate de magnésium anhydre, filtrées et concentrées à sec sous pression réduite (3 kPa). Le résidu huileux obtenu est purifié par chromatographie sur alumine, en éluant par un mélange d'acétate d'éthyle et de méthanol (90 / 10 en volumes). Après concentration des fractions sous pression réduite, on obtient 2,81 g d'une huile brune, qui est dissoute dans 200 cm³ d'éthanol et additionnée de 6,25 cm³ d'une solution aqueuse environ 6 M d'acide chlorhydrique. La solution est concentrée à sec sous pression réduite (3 kPa). Par 2 fois, le résidu est repris par 200 cm³ d'éthanol et ramené à sec. On obtient ainsi 3,01 g de dichlorhydrate de 3-(3-benzyloxy-4-phényl-pyrazol-1-ylméthyl)-1-aza-bicyclo[2.2.2]octane sous forme d'une meringue beige. Spectre de R.M.N. ¹H (400 MHz, (CD₃)₂SO d6, δ en ppm) : de 1,65 à 1,90 (mt : 4H) ; 2,06 (mt : 1H) ; de 2,50 à 2,65 (mt : 1H) ; 2,94 (dd, J = 10 et 5 Hz : 1H) ; de 3,10 à 3,40 (mt : 5H) ; 4,11 (dd, J = 10,5 et 6 Hz : 1H) ; 4,16 (dd J = 10,5 et 6 Hz : 1H) ; 5,33 (s : 2H) ; 7,07 (t large, J = 7,5 Hz : 1H) ; 7,36 (t large, J = 7,5 Hz : 3H) ; 7,43 (t large, J = 7,5 Hz : 2H) ; 7,50 (d large, J = 7,5 Hz : 2H) ; 7,65 (d large, J = 7,5 Hz : 2H) ; 8,14 (s : 1H) ; 10,51 (mf : 1H). Spectre IR (KBr) : 3031; 2936; 2803; 2564; 1606; 1578; 1569; 1510; 1454; 1435; 1360; 1046; 1024; 764; 697; 615 et 511 cm⁻¹.

Le 3-benzyloxy-4-phényl-pyrazole peut être préparé de la manière suivante :

Une suspension de 5,7 g de 1-(3-hydroxy-4-phényl-pyrazol-1-yl)-éthanone, 3,9 g de carbonate de potassium et 3,7 cm³ (5,3 g) de bromure de benzyle dans 250 cm³ de 2-butanone est agitée à la température d'ébullition du milieu réactionnel pendant 2,25 heures. L'insoluble minéral est éliminé par filtration et le filtrat est concentré à sec sous pression réduite (3 kPa). Le résidu est dissous dans 50 cm³ de tétrahydrofurane, additionné de 50 cm³ de méthanol et de 1 cm³ d'une solution d'hydroxyde de sodium 10 N et agité 0,25 heure à température ambiante, puis concentré à sec sous pression réduite (3 kPa). Le résidu est repris par 5 cm³ d'eau et extrait avec 250 cm³ d'acétate d'éthyle. La phase organique est séchée sur sulfate de magnésium anhydre, filtrée et concentrée à sec sous pression réduite (3 kPa), pour donner un résidu solide blanc, qui est trituré dans un mélange d'éther isopropylique et d'éther de pétrole. Après filtration et séchage à l'air on obtient 4,43 g de 3-benzyloxy-4.-phényl-pyrazole sous forme d'un solide blanc fondant à 163°C.

### Exemple 4

### Chlorhydrate monohydrate de 3-(3-méthoxy-4-phényl-pyrazol-1-ylméthyl)-1-aza-bicyclo[2.2.2]octane

A une solution de 0,9 g de 3-méthoxy-4-phényl-pyrazole dans 15 cm³ de diméthylformamide anhydre on ajoute progressivement, sous atmosphère d'argon et à température ambiante, 0,99 g d'hydrure de sodium (à 75 % en masse dans l'huile de vaseline). Après 0,75 heure d'agitation à une température voisine de 50°C on ajoute par petites portions 3,04 g de chlorhydrate de 3-chlorométhyl-1-aza-bicyclo[2.2.2]octane, puis chauffe pendant 16 heures à une température voisine de 50°C. Le mélange est refroidi à température ambiante puis est additionné lentement de 10 cm³ d'eau et concentré sous pression réduite (3 kPa). Le résidu est repris par 25 cm³ d'eau et extrait avec 3 fois 100 cm³ d'acétate d'éthyle. Les phases organiques réunies sont lavées avec 3 fois 25 cm³ d'eau, puis séchées, filtrées et concentrées à sec sous pression réduite (3 kPa). Le résidu huileux obtenu est purifié par chromatographie sur alumine, en éluant par un mélange d'acétate d'éthyle et de méthanol (90 / 10 en volumes). Après concentration des fractions sous pression réduite on obtient 0,3 g d'une huile brune, qui est dissoute dans 40 cm³ d'acétone et additionnée de 35 cm³ d'éther chlorhydrique environ 3 M. La solution est concentrée à sec sous pression réduite (3 kPa) et le résidu pâteux est lavé par 2 fois 50 cm³ d'éther éthylique puis trituré dans 50 cm³ d'éther éthylique pendant une nuit. Après filtration du solide obtenu et séchage sous vide (70 Pa) à une température de 60°C on obtient 0,25 g de chlorhydrate monohydrate de 3-(3-méthoxy-4-phényl-pyrazol-1-ylméthyl)-1-aza-bicyclo[2.2.2]octane sous forme d'une poudre blanche fondant vers 125°C (avec décomposition). Spectre de R.M.N. ¹H (300 MHz, (CD₃)₂SO d6, δ en ppm) : de 1,60 à 1,90 (mt : 4H) ; 2,05 (mf : 1H) ; de 2,45 à 2,60 (mt : 1H) ; 2,94 (dd large, J = 13 et 7 Hz : 1H) ; de 3,05 à 3,40 (mt : 5H) ; 3,93 (s : 3H) ; 4,12 (mt : 2H) ; 7,17 (t large, J = 7,5 Hz : 1H) ; 7,35 (t large, J = 7,5 Hz : 2H) ; 7,62 (d large, J = 7,5 Hz : 2H) ; 8,10 (s : 1H) ; de 9,40 à 9,90 (mf très étalé : 1H). Spectre IR (KBr) : 2942; 2562; 1609; 1579; 1517; 1458; 1406; 1048; 1030; 759; 698; 601 et 508 cm⁻¹.

### Exemple 5

### Dichlorhydrate de 1-(1-aza-bicyclo[2.2.2]oct-3-yl)-4-phényl-1H-pyrazol-3-ol

Une suspension de 0,163 g de 3-(3-benzyloxy-4-phényl-pyrazol-1-yl)-1-aza-bicyclo[2.2.2]octane, 0,38 cm³ d'acide chlorhydrique 6 M et 0,024 g de palladium sur charbon (à 10%) dans 20 cm³ d'éthanol est agitée en autoclave sous une pression d'hydrogène de 1000 kPa , à une température de 20°C pendant 8 heures. Le milieu réactionnel est ensuite filtré sur Célite® et concentré à sec sous pression réduite (3 kPa), pour donner un résidu huileux hygroscopique, qui est dissous dans 10 cm³ d'eau et lyophilisé. On obtient ainsi 0,083 g de dichlorhydrate de 1-(1-aza-bicyclo[2.2.2]oct-3-yl)-4-phényl-1H-pyrazol-1-ol sous forme de solide brun amorphe. Spectre de R.M.N. ¹H (300 MHz, (CD₃)₂SO d6, δ en ppm) : de 1,65 à 2,05 (mt : 4H) ; 2,41 (mt : 1H) ; 3,26 (mt : 3H) ; 3,40 (mt : 1H) ; 3,77 (mt : 2H) ; 4,68 (mt : 1H) ; 7,15 (t large, J = 7,5 Hz : 1H) ; 7,34 (t large, J = 7,5 Hz : 2H) ; 7,69 (d large, J = 7,5 Hz : 2H) ; 8,22 (s : 1H) ; de 10,15 à 10,75 (mf étalé : 1H) ; 11,07 (mf: 1H). Spectre IR (KBr) : 3417; 2956; 2806; 2666; 1607; 1580; 1522; 1450; 1168; 995; 762; 697; 671 et 513 cm⁻¹_{.}

Le 3-(3-benzyloxy-4-phényl-pyrazol-1-yl)-1-aza-bicyclo[2.2.2]octane peut être préparé de la manière suivante :

A une solution de 0,5 g de 3-benzyloxy-4-phényl-pyrazole dans 30 cm³ de diméthylformamide anhydre on ajoute progressivement, sous atmosphère d'argon et à température ambiante, 0,96 g d'hydrure de sodium (à 75% en masse dans l'huile de vaseline). Après 0,75 heure d'agitation à une température voisine de 50°C, on ajoute goutte à goutte la solution de 0,725 g de 3-[(méthanesulfonyl)oxy]-1-aza-bicyclo[2.2.2]octane dans 5 cm³ de diméthylformamide anhydre, puis chauffe pendant 20 heures à une température voisine de 110°C. Le mélange est refroidi à température ambiante puis est additionné lentement de 5 cm³ d'eau et concentré sous pression réduite (3 kPa). Le résidu est repris par 10 cm³ d'eau et extrait avec 50 cm³ d'acétate d'éthyle. La phase organique est lavée avec 3 fois 10 cm³ d'eau, puis séchée, filtrée et concentrée à sec sous pression réduite (3 kPa). Le résidu huileux obtenu est purifié par CLHP préparative sur Kromasil C8 10µ en éluant avec un mélange d'acétonitrile et d'eau (50 / 50 en volumes) puis d'acétonitrile et de méthanol ammoniacal (7 M) (98 / 2 en volumes). Après concentration des fractions sous pression réduite on obtient 0,163 g de 3-(3-benzyloxy-4-phényl-pyrazol-1-yl)-1-aza-bicyclo[2.2.2]octane sous forme d'une huile jaune engagée telle quelle dans l'étape suivante.

Le 3-[(méthanesulfonyl)oxy]-1-aza-bicyclo[2.2.2]octane peut être obtenu selon la méthode décrite par S. M. Jenkins et col., J. Med. Chem. 1992, 35, 2392-2406.

### Exemple 6

### Dichlorhydrate de 1-(2-perhydro-azépin-1-yl-éthyl)-4-phényl-1H-pyrazol-3-ol

Une suspension de 0,65 g de 1-[2-(3-benzyloxy-4-phényl-pyrazol-1-yl)-éthyl]-perhydro-azépine, 1,44 cm³ d'acide chlorhydrique 6 M et 0,092 g de palladium sur charbon (à 10%) dans 20 cm³ d'éthanol est agitée en autoclave sous une pression d'hydrogène de 1000 kPa , à une température de 20°C pendant 8 heures. Le milieu réactionnel est ensuite filtré sur Célite® et concentré à sec sous pression réduite (3 kPa). Le résidu est trituré dans 40 cm³ d'acétone et isolé par filtration. On obtient ainsi 0,541 g de dichlorhydrate de 1-(2-perhydro-azépin-1-yl-éthyl)-4-phényl-1H-pyrazol-3-ol sous forme d'une poudre beige fondant à 228°C (avec décomposition). Spectre de R.M.N. ¹H (300 MHz, (CD₃)₂SO d6, δ en ppm) 1,64 (mt : 4H) ; 1,84 (mt : 4H) ; 3,17 (mt : 2H) ; de 3,25 à 3,55 (mt : 4H) ; 4,41 (t, J = 6,5 Hz : 2H) ; 7,15 (t large, J = 7,5 Hz : 1H) ; 7,34 (t large, J = 7,5 Hz : 2H) ; 7,65 (d large, J = 7,5 Hz : 2H) ; 8,05 (s : 1H) ; 10,35 (mf : 1H) ; 10,49 (mf : 1H). Spectre IR (KBr) : 3431; 2934; 2638; 2422; 1608; 1582; 1572; 1528; 1452; 1210; 1179; 1013; 760; 692; 673 et 511 cm⁻¹.

La 1-[2-(3-benzyloxy-4-phényl-pyrazol-1-yl)-éthyl]-perhydro-azépine peut être préparée de la manière suivante :

Une suspension de 1 g de 1-[(toluène-4-sulfonyl)oxy]-2-(3-benzyloxy-4-phényl-pyrazol-1-yl)-éthyle, 0,29 cm³ de perhydro-azépine et 0,88 g de carbonate de potassium dans 25 cm³ d'acétonitrile est agitée pendant 3 heures à une température voisine de 80°C, puis 0,15 cm³ de perhydro-azépine est ajouté et le chauffage est poursuivi pendant 2 heures. Le mélange est concentré à sec sous pression réduite (3 kPa). Le résidu est repris par 50 cm³ d'eau et extrait avec 200 cm³ d'acétate d'éthyle. La phase organique est lavée avec 3 fois 25 cm³ d'eau, puis séchée, filtrée et concentrée à sec sous pression réduite (3 kPa). Le résidu huileux obtenu est purifié par chromatographie sur gel de silice (granulométrie 15-35 µm), en éluant par un mélange de dichlorométhane et de méthanol (96 / 4 en volumes). Après concentration des fractions sous pression réduite, on obtient 0,72 g de 1-[2-(3-benzyloxy-4-phényl-pyrazol-1-yl)-éthyl]-perhydro-azépine sous forme d'une huile visqueuse incolore, qui est utilisée telle quelle dans l'étape suivante. Spectre de masse (IE) : m/z 375 (M^{+.}), m/z 112 (pic de base).

Le 1-[(toluène-4-sulfonyl)oxy]-2-(3-benzyloxy-4-phényl-pyrazol-1-yl)-éthyle peut être préparé de la manière suivante :

A une suspension de 13,7 g de chlorhydrate de 2-(3-benzyloxy-4-phényl-pyrazol-1-yl)-éthanol dans 400 cm³ de dichlorométhane on ajoute, goutte à goutte à température ambiante, 59 cm³ de triéthylamine. Le mélange réactionnel est refroidi vers 5°C et est additionné, en 0,5 heure, de la solution de 22,5 g de chlorure de toluène-4-sulfonyle dans 200 cm³ de dichlorométhane. Après 16 heures d'agitation à température ambiante le mélange est concentré à sec sous pression réduite (3 kPa). Le résidu est repris par 100 cm³ d'eau et extrait avec (500 + 250) cm³ d'acétate d'éthyle. Les phases organiques réunies sont lavées avec 3 fois 100 cm³ d'eau, puis séchées sur sulfate de magnésium anhydre, filtrées et concentrées à sec sous pression réduite (3 kPa). Le résidu huileux obtenu est purifié par chromatographie sur gel de silice (granulométrie 15-35 µm), en éluant par du dichlorométhane puis un mélange de dichlorométhane et de méthanol (95 / 5 en volumes). Après concentration des fractions sous pression réduite, on obtient 19 g de 1-[(toluène-4-sulfonyl)oxy]-2-(3-benzyloxy-4-phényl-pyrazol-1-yl)-éthyle sous forme d'une huile visqueuse incolore, qui est utilisée telle quelle dans l'étape suivante. Spectre de masse (IE) : m/z 448 (M^{+.}), m/z 91 (pic de base).

Le 2-(3-benzyloxy-4-phényl-pyrazol-1-yl)-éthanol peut être préparé de la manière suivante :

A une solution de 17 g de 3-benzyloxy-4-phényl-1-[2-(tétrahydro-pyran 2-yloxy)-éthyl]-1H-pyrazole dans 750 cm³ d'éthanol on ajoute à température ambiante 750 cm³ d'acide chlorhydrique à 37%. Après 2 heures d'agitation à température ambiante le mélange est concentré à sec sous pression réduite (3 kPa). Par 3 fois le résidu est repris par 1 dm³ d'éthanol et concentré à sec pour donner 13,8 g de 2-(3-benzyloxy-4-phényl-pyrazol-1-yl)-éthanol sous forme d'un solide fondant à 115°C, qui est utilisé tel quel dans l'étape suivante.

Le 3-benzyloxy-4-phényl-1-[2-(tétrahydro-pyran-2-yloxy)-éthyl]-1H-pyrazole peut être préparé de la manière suivante :

A une solution de 16 g de 3-benzyloxy-4-phényl-pyrazole dans 110 cm³ de diméthylformamide anhydre on ajoute progressivement, sous atmosphère d'argon et à température ambiante, 3,07 g d'hydrure de sodium (à 75% en masse dans l'huile de vaseline). Après 0,75 heure d'agitation à une température voisine de 50°C on ajoute goutte à goutte la solution de 11,06 cm³ (15,31 g) de 2-(2-bromo-éthoxy)-tétrahydro-pyrane dans 40 cm³ de diméthylformamide anhydre, puis chauffe pendant 0,75 heure à une température voisine de 50°C. Le mélange est ensuite additionné lentement de 25 cm³ d'eau puis est versé dans 90 cm³ d'eau et extrait avec 3 fois 300 cm³ d'acétate d'éthyle. Les phases organiques réunies sont lavées avec 3 fois 100 cm³ d'eau, puis séchées, filtrées et concentrées à sec sous pression réduite (3 kPa). Le résidu huileux obtenu est purifié par chromatographie sur gel de silice (granulométrie 20-45 µm), en éluant par un mélange de dichlorométhane et d'acétate d'éthyle (90 / 10 en volumes). Après concentration des fractions sous pression réduite, on obtient 17,35 g de 3-benzyloxy-4-phényl-1-[2-(tétrahydro-pyran-2-yloxy)-éthyl]-1H-pyrazole sous forme d'un solide pâteux incolore, qui est utilisé tel quel dans l'étape suivante. Spectre de R.M.N. ¹H (300 MHz, (CD₃)₂SO d6, 8 en ppm) : de 1,25 à 1,75 (mt : 6H) ; de 3,25 à 3,45 (mt : 1H) ; 3,60 (ddd, J = 11,5 - 8,5 et 3 Hz : 1H) ; 3,72 (mt : 1H) ; 3,94 (ddd, J = 10,5 - 6 et 4,5 Hz : 1H) ; 4,16 (mt : 2H) ; 4,55 (mt : 1H) ; 5,32 (s : 2H) ; 7,14 (tt, J = 7,5 et 1,5 Hz : 1H) ; de 7,25 à 7,45 (mt : 3H) ; 7,33 (t large, J = 7,5 Hz : 2H) ; 7,50 (d large, J = 7,5 Hz : 2H) ; 7,63 (d large, J = 7,5 Hz : 2H) ; 8,05 (s : 1H).

### Exemple 7

### Dichlorhydrate de 1-[2-(2-méthyl-pipéridin-1-yl)-éthyl]-4-phényl-1H-pyrazol-3-ol

Une suspension de 0,58 g de 1-[2-(3-benzyloxy-4-phényl-pyrazol-1-yl)-éthyl]-2-méthyl-pipéridine, 1,29 cm³ d'acide chlorhydrique 6 M et 0,082 g de palladium sur charbon (à 10%) dans 20 cm³ d'éthanol est agitée en autoclave sous une pression d'hydrogène de 1000 kPa , à une température de 20°C pendant 8 heures. Le milieu réactionnel est ensuite filtré sur Célite^{®} et concentré à sec sous pression réduite (3 kPa). Le résidu est trituré dans 40 cm³ d'acétone et isolé par filtration. On obtient ainsi 0,54 g de dichlorhydrate 1-[2-(2-méthyl-pipéridin-1-yl)-éthyl]-4-phényl-1H-pyrazol-3-ol sous forme d'une poudre beige fondant à 118°C (avec décomposition). Spectre de R.M.N. ¹H (300 MHz, (CD₃)₂SO d6 avec ajout de quelques gouttes de CD₃COOD d4, 8 en ppm) : 1,29 (d, J = 6,5 Hz : 3H); de 1,35 à 1,95 (mt : 6H) ; 3,04 (ddd, J = 12 - 9 et 3, 5 Hz : 1H) ; de 3,30 à 3,50 (mt : 3H) ; de 3,50 à 3,65 (mt : 1H) ; 4,37 (t, J = 6,5 Hz : 2H) ; 7,14 (t large, J = 7,5 Hz : 1H) ; 7,33 (t large, J = 7,5 Hz : 2H) ; 7,64 (d large, J = 7,5 Hz : 2H) ; 8;02 (s : 1H). Spectre IR (KBr) : 3051; 2949; 2653; 2565; 1606; 1581; 1522; 1441; 1228; 1171; 995; 768; 700; 671 et 587 cm⁻¹.

La 1-[2-(3-benzyloxy-4-phényl-pyrazol-1-yl)-éthyl]-2-méthyl-pipéridine peut être préparée de la manière suivante :

Une suspension de 1 g de 1-[(toluène-4-sulfonyl)oxy]-2-(3-benzyloxy-4-phényl-pyrazol-1-yl)-éthyle, 0,46 cm³ de 2-méthyl-pipéridine et 0,88 g de carbonate de potassium dans 25 cm³ d'acétonitrile est agitée pendant 6 heures à une température voisine de 80°C. Le mélange est concentré à sec sous pression réduite (3 kPa). Le résidu est repris par 50 cm³ d'eau et extrait avec 200 cm³ d'acétate d'éthyle. La phase organique est lavée avec 3 fois 25 cm³ d'eau, puis séchée, filtrée et concentrée à sec sous pression réduite (3 kPa). Le résidu huileux obtenu est purifié par chromatographie sur gel de silice (granulométrie 15-35 µm), en éluant par un mélange de dichlorométhane et de méthanol (97 / 3 en volumes). Après concentration des fractions sous pression réduite, om obtient 0,65 g de 1-[2-(3-benzyloxy-4-phényl-pyrazol-1-yl)-éthyl]-2-méthyl-pipéridine sous forme d'une huile visqueuse incolore, qui est utilisée telle quelle dans l'étape suivante. Spectre de masse (IE) : m/z 375 (M^{+.}), m/z 112 (pic de base).

### Exemple 8

### Dichlorhydrate de 1-[2-(4-fluoro-pipéridin-1-yl)-éthyl]-4-phényl-1H-pyrazol-3-ol

Une suspension de 0,5 g de 1-[2-(3-benzyloxy-4-phényl-pyrazol-1-yl)-éthyl]-4-fluoro-pipéridine , 1,1 cm³ d'acide chlorhydrique 6 M et 0,071 g de palladium sur charbon (à 10%) dans 20 cm³ d'éthanol est agitée en autoclave sous une pression d'hydrogène de 1000 kPa , à une température de 20°C pendant 8 heures. Le milieu réactionnel est ensuite filtré sur Célite^{®} et concentré à sec sous pression réduite (3 kPa). Le résidu est trituré dans 40 cm³ d'acétone et isolé par filtration. On obtient ainsi 0,54 g de dichlorhydrate 1-[2-(4-fluoro-pipéridin-1-yl)-éthyl]-4-phényl-1H-pyrazol-3-ol sous forme d'une poudre beige fondant à 228°C (avec décomposition). Spectre de R.M.N. ¹H (400 MHz, (CD₃)₂SO d6 avec ajout de quelques gouttes de CD₃COOD d4, 8 en ppm) : de 2,00 à 2,25 (mt : 4H) ; 3,29 (mf : 4H) ; 3,52 (t, J = 6 Hz : 2H) ; 4,40 (t, J = 6 Hz : 2H) ; 4,94 (d large, J = 48 Hz : 1H) ; 7,14 (t large, J = 7,5 Hz : 1H) ; 7,33 (t large, J = 7,5 Hz : 2H) ; 7,65 (d large, J = 7,5 Hz : 2H) ; 7,99 (s : 1H). Spectre IR (KBr) : 3054; 2963; 2633; 2531; 1608; 1582; 1528; 1452; 1177; 1031; 1015; 764; 698 et 509 cm⁻¹.

La 1-[2-(3-benzyloxy-4-phényl-pyrazol-1-yl)-éthyl]-4-fluoro-pipéridine peut être préparée de la manière suivante :

Une suspension de 1 g de 1-[(toluène-4-sulfonyl)oxy]-2-(3-benzyloxy-4-phényl-pyrazol-1-yl)-éthyle, 0,702 g de bromhydrate de 4-fluoro-pipéridine et 1,18 g de carbonate de potassium dans 25 cm³ d'acétonitrile est agitée pendant 6 heures à une température voisine de 80°C. Le mélange est concentré à sec sous pression réduite (3 kPa). Le résidu est repris par 50 cm³ d'eau et extrait avec 200 cm³ d'acétate d'éthyle. La phase organique est lavée avec 3 fois 25 cm³ d'eau, puis séchée, filtrée et concentrée à sec sous pression réduite (3 kPa). Le résidu huileux obtenu est purifié par chromatographie sur gel de silice (granulométrie 15-35 µm), en éluant par un mélange de dichlorométhane et de méthanol (98,5 / 1,5 en volumes). Après concentration des fractions sous pression réduite, on obtient 0,61 g de 1-[2-(3-benzyloxy-4-phényl-pyrazol-1-yl)-éthyl]-4-fluoro-pipéridine sous forme d'une huile visqueuse incolore, qui est utilisée telle quelle dans l'étape suivante. Spectre de masse (IE) : m/z 379 (M^{+.}), m/z 250 et m/z 116 (pic de base).

### Exemple 9

### Dichlorhydrate de 1-[2-(3-méthyl-pipéridin-1-yl)-éthyl]-4-phényl-1H-pyrazol-3-ol

Une suspension de 0,57 g de 1-[2-(3-benzyloxy-4-phényl-pyrazol-1-yl)-éthyl]-3-méthyl-pipéridine , 1,27 cm³ d'acide chlorhydrique 6 M et 0,081 g de palladium sur charbon (à 10%) dans 20 cm³ d'éthanol est agitée en autoclave sous une pression d'hydrogène de 1000 kPa , à une température de 20°C pendant, 8 heures. Le milieu réactionnel est ensuite filtré sur Célite^{®} et concentré à sec sous pression réduite (3 kPa). Le résidu est trituré dans 75 cm³ d'acétone et isolé par filtration. On obtient ainsi 0,198 g de dichlorhydrate de 1-[2-(3-méthyl-pipéridin-1-yl)-éthyl]-4-phényl-1H-pyrazol-3-ol sous forme d'une poudre jaune pâle fondant à 220°C (avec décomposition). Spectre de R.M.N. ¹H (300 MHz, (CD₃)₂SO d6 avec ajout de quelques gouttes de CD₃COOD, δ en ppm) : 0,89 (d, J = 7 Hz : 3H) ; 1,05 (mt : 1H) ; de 1,60 à 2,00 (mt : 4H) ; 2,58 (mt : 1H) ; 2,83 (t très large, J = 12 Hz : 1H) ; de 3,30 à 3,55 (mt : 4H) ; 4,39 (t, J = 6,5 Hz : 2H) ; 7,13 (t large, J = 7,5 Hz : 1H) ; 7,32 (t large, J = 7,5 Hz : 2H) ; 7,63 (d large, J = 7,5 Hz : 2H) ; 7,99 (s : 1H). Spectre IR (KBr) : 3057; 2960; 2651; 2550; 1607; 1581; 1523; 454; 1179; 761; 697; 614 et 513 cm⁻¹.

La 1-[2-(3-benzyloxy-4-phényl-pyrazol-1-yl)-éthyl]-3-méthyl-pipéridine peut être préparée de la manière suivante :

Une suspension de 1 g de 1-[(toluène-4-sulfonyl)oxy]-2-(3-benzyloxy-4-phényl-pyrazol-1-yl)-éthyle, 0,46 cm³ de 3-méthyl-pipéridine et 0,88 g de carbonate de potassium dans 25 cm³ d'acétonitrile est agitée pendant 6 heures à une température voisine de 80°C. Le mélange est concentré à sec sous pression réduite (3 kPa). Le résidu est repris par 50 cm³ d'eau et extrait avec 200 cm³ d'acétate d'éthyle. La phase organique est lavée avec 3 fois 25 cm³ d'eau, puis séchée, filtrée et concentrée à sec sous pression réduite (3 kPa). Le résidu huileux obtenu est purifié par chromatographie sur gel de silice (granulométrie 15-35 µm), en éluant par un mélange de dichlorométhane et de méthanol (97 / 3 en volumes). Après concentration des fractions sous pression réduite, on obtient 0,58 g de 1-[2-(3-benzyloxy-4-phényl-pyrazol-1-yl)-éthyl]-3-méthyl-pipéridine sous forme d'une huile visqueuse incolore, qui est utilisée telle quelle dans l'étape suivante. Spectre de masse (IE) : m/z 375 (M^{+.}), m/z 112 (pic de base).

### Exemple 10

### Dichlorhydrate de 1-[2-(3,6-dihydro-2H-pyridin-1-yl)-éthyl]-4-phényl-1H-pyrazol-3-ol

Une solution de 0,6 g de 1-[2-(3-benzyloxy-4-phényl-pyrrol-1-yl)-éthyl]-1,2,3,6-tétrahydro-pyridine dans un mélange de 5 cm³ d'acide chlorhydrique à 37% et de 5 cm³ d'éthanol est chauffée à 80°C pendant 6 heures, puis concentrée à sec sous pression réduite (3 kPa). Par 4 fois le résidu est repris par 100 cm³ d'éthanol et concentré à sec. Le résidu est trituré dans 40 cm³ d'acétone et isolé par filtration. On obtient ainsi 0,403 g de dichlorhydrate de 1-[2-(3,6-dihydro-2H-pyridin-1-yl)-éthyl]-4-phényl-1H-pyrazol-3-ol sous forme d'une poudre brune fondant à 192°C.

Spectre de R.M.N. ¹H (300 MHz, (CD₃)₂SO d6, δ en ppm) : 2,29 (d très large, J = 18 Hz : 1H) ; de 2,40 à 2,60 (mt : 1H) ; 3,07 (mt : 1H) ; 3,47 (mt : 1H) ; 3,55 (mt : 2H) ; 3,62 (d très large, J = 16,5 Hz : 1H) ; 3,84 (d large, J = 16,5 Hz : 1H) ; 4,47 (t, J = 6,5 Hz : 2H) ; 5,72 (d large, J = 10,5 Hz : 1H) ; 5,93 (d très large, J = 10,5 Hz : 1H) ; 7,15 (t large, J = 7,5 Hz : 1H) ; 7,34 (t large, J = 7,5 Hz : 2H) ; 7,66 (d large, J = 7,5 Hz : 2H) ; 8,06 (s : 1H) ; de 10,20 à 10,80 (mf étalé : 1H) ; 10,88 (mf : 1H). Spectre IR (KBr) : 3422; 2948; 2688; 2579; 1607; 1526; 1452; 1184; 1023; 768; 699; 667; 670 et 511 cm⁻¹.

La 1-[2-(3-benzyloxy-4-phényl-pyrrol-1-yl)-éthyl]-1,2,3,6-tétrahydro-pyridine peut être préparée de la manière suivante :

Une suspension de 1 g de 1-[(toluène-4-sulfonyl)oxy]-2-(3-benzyloxy-4-phényl-pyrazol-1-yl)-éthyle, 0,36 cm³ de 1,2,3,6-tétrahydro-pyridine et 0,88 g de carbonate de potassium dans 25 cm³ d'acétonitrile est agitée pendant 6 heures à une température voisine de 80°C. Le mélange est concentré à sec sous pression réduite (3 kPa). Le résidu est repris par 50 cm³ d'eau et extrait avec 200 cm³ d'acétate d'éthyle. La phase organique est lavée avec 3 fois 25 cm³ d'eau, puis séchée, filtrée et concentrée à sec sous pression réduite (3 kPa). Le résidu huileux obtenu est purifié par chromatographie sur gel de silice (granulométrie 15-35 µm), en éluant par un mélange de dichlorométhane et de méthanol (97 / 3 en volumes). Après concentration des fractions sous pression réduite, on obtient 0,6 g de 1-[2-(3-benzyloxy-4-phényl-pyrrol-1-yl)-éthyl]-1,2,3,6-tétrahydro-pyridine sous forme d'une huile visqueuse incolore, qui est utilisée telle quelle dans l'étape suivante. Spectre de masse (IC): m/z 360 ([M+H]⁺) (pic de base).

### Exemple 11

### Dichlorhydrate de 1-[2-(7-aza-bicyclo[2.2.1]hept-7-yl)-éthyl]-4-phényl-1H-pyrazol-3-ol

Une solution de 0,6 g de 7-[2-(3-benzyloxy-4-phényl-pyrazol-1-yl)-éthyl]-7-aza-bicyclo[2.2.1]heptane dans 40 cm³ d'éthanol est additionnée de 3 cm³ d'acide chlorhydrique 1 M, agitée 0,25 heure à température ambiante, puis concentrée à sec sous pression réduite (3 kPa). Le résidu obtenu et 0,078 g de palladium sur charbon (à 10%) sont mis en suspension dans 20 cm³ d'éthanol et agités en autoclave sous une pression d'hydrogène de 1000 kPa , à une température de 20°C pendant 8 heures. Le milieu réactionnel est ensuite filtré sur Célite^{®} et concentré à sec sous pression réduite (3 kPa). Le résidu est trituré dans 25 cm³ d'acétone et isolé par filtration. On obtient ainsi 0,466 g de dichlorhydrate de 1-[2-(7-aza-bicyclo[2.2.1]hept-7-yl)-éthyl]-4-phényl-1H-pyrazol-3-ol sous forme d'une poudre blanche fondant à 228°C (avec décomposition). Spectre de R.M.N. ¹H (300 MHz, (CD₃)₂SO d6,8 en ppm) : 1,66 (mf : 4H) ; 2,00 (mf : 4H) ; 3,43 (mt : 2H) ; 3,93 (s large : 2H) ; 4,43 (t large, J = 6,5 Hz : 2H) ; 7,15 (t large, J = 7,5 Hz : 1H) ; 7,35 (t large, J = 7,5 Hz : 2H) ; 7,65 (d large, J = 7,5 Hz : 2H) ; 8,08 (s : 1H) ; de 10,35 à 10,55 (mf étalé : 1H) ; 10,47 (mf : 1H). Spectre IR (KBr) : 2988; 2789; 2661; 2537; 1608; 1533; 1449; 1279; 1179; 875; 761; 698; 674 et 510 cm⁻¹.

Le 7-[2-(3-benzyloxy-4-phényl-pyrazol-1-yl)-éthyl]-7-aza-bicyclo[2.2.1]heptane peut être préparé de la manière suivante :

Une suspension de 1 g de 1-[(toluène-4-sulfonyl)oxy]-2-(3-benzyloxy-4-phényl-pyrazol-1-yl)-éthyle, 0,616 g de chlorhydrate de 7-aza-bicyclo[2.2.1]heptane et 0,88 g de carbonate de potassium dans 25 cm³ d'acétonitrile est agitée pendant 5 heures à une température voisine de 80°C. Le mélange est concentré à sec sous pression réduite (3 kPa). Le résidu est repris par 30 cm³ d'eau et extrait avec 250 cm³ d'acétate d'éthyle. La phase organique est lavée avec 3 fois 30 cm³ d'eau, puis séchée, filtrée et concentrée à sec sous pression réduite (3 kPa). Le résidu huileux obtenu est purifié par chromatographie sur gel de silice (granulométrie 15-35 µm), en éluant par un mélange de dichlorométhane et de méthanol (98 / 2 en volumes). Après concentration des fractions sous pression réduite, on obtient 0,6 g de 7-[2-(3-benzyloxy-4-phényl-pyrazol-1-yl)-éthyl]-7-aza-bicyclo[2.2.1]heptane sous forme d'une huile visqueuse incolore, qui est utilisée telle quelle dans l'étape suivante. Spectre de masse (IC) : m/z 374 ([M+H]⁺) (pic de base).

### Exemple 12

### Dichlorhydrate de 1-[2-(2-aza-bicyclo[2.2.2]oct-2-yl)-éthyl]-4-phényl-1H-pyrazol-3-ol

Une solution de 0,9 g de 2-[2-(3-benzyloxy-4-phényl-pyrazol-1-yl)-éthyl]-2-aza-bicyclo[2.2.2]octane dans 50 cm³ d'éthanol est additionnée de 2 cm³ d'acide chlorhydrique 6 M, agitée 0,25 heure à température ambiante, puis concentrée à sec sous pression réduite (3 kPa). Le résidu obtenu et 0,124 g de palladium sur charbon (à 10%) sont mis en suspension dans 20 cm³ d'éthanol et agités en autoclave sous une pression d'hydrogène de 1000 kPa , à une température de 20°C pendant 8 heures. Le milieu réactionnel est ensuite filtré sur Célite^{®} et concentré à sec sous pression réduite (3 kPa). Le résidu est trituré dans 25 cm³ d'acétone et isolé par filtration. On obtient ainsi 0,56 g de dichlorhydrate de 1-[2-(2-aza-bicyclo[2.2.2]oct-2-yl)-éthyl]-4-phényl-1H-pyrazol-3-ol sous forme d'une poudre beige fondant à 171°C (avec décomposition). Spectre de R.M.N. ¹H (300 MHz, (CD₃)₂SO d6, δ en ppm) : de 1,50 à 1,75 (mt : 6H) ; 1,90 (s très large : 1H) ; de 2,00 à 2,15 (mt : 1H) ; 2,28 (mt : 1H) ; 2,86 (dd très large, J = 12 et 4,5 Hz : 1H) ; de 3,35 à 3,55 (mt : 1H) ; 3,38 (s très large : 1H) ; 3,55 (t large, J = 6,5 Hz : 2H) ; 4,46 (t large, J = 6,5 Hz : 2H) ; 7,14 (t large, J = 7,5 Hz : 1H) ; 7,34 (t large, J = 7,5 Hz : 2H) ; 7,65 (d large, J = 7,5 Hz : 2H) ; 8,04 (s : 1H) ; 10,84 (mf : 1H). Spectre IR (KBr) : 2949; 2870; 2629; 2184; 1608; 1579; 1510; 1455; 1198; 870; 761; 692; 670 et 510 cm⁻¹.

Le 2-[2-(3-benzyloxy-4-phényl-pyrazol-1-yl)-éthyl]-2-aza-bicyclo[2.2.2]octane peut être préparé de la manière suivante :

Une suspension de 1 g de 1-[(toluène-4-sulfonyl)oxy]-2-(3-benzyloxy-4-phényl-pyrazol-1-yl)éthyle, 0,563 g de chlorhydrate de 2-aza-bicyclo[2.2.2]octane et 0,88 g de carbonate de potassium dans 25 cm³ d'acétonitrile est agitée pendant 8 heures à une température voisine de 80°C. Le mélange est concentré à sec sous pression réduite (3 kPa). Le résidu est repris par 30 cm³ d'eau et extrait avec 200 cm³ d'acétate d'éthyle. La phase organique est lavée avec 2 fois 30 cm³ d'eau, puis séchée, filtrée et concentrée à sec sous pression réduite (3 kPa). Le résidu huileux obtenu est purifié par chromatographie sur gel de silice (granulométrie 15-35 µm), en éluant par un mélange de dichlorométhane et de méthanol (95 / 5 en volumes). Après concentration des fractions sous pression réduite, on obtient 0,92 g de 2-[2-(3-benzyloxy-4-phényl-pyrazol-1-yl)-éthyl]-2-aza-bicyclo[2.2.2]octane sous forme d'une huile visqueuse incolore, qui est utilisée telle quelle dans l'étape suivante. Spectre de masse (IC) : m/z 388 ([M+H]⁺) (pic de base).

Le chlorhydrate de 2-aza-bicyclo[2.2.2]octane peut être obtenu selon la méthode décrite par Yokota, M. et col., Eur.J.Med.Chem.Chim.Ther., 1997, 32 (5), 377-384.

### Exemple 13

### Dichlorhydrate de 1-[2-(2-aza-bicyclo[2.2.1]hept-2-yl)-éthyl]-4-phényl-1H-pyrazol-3-ol

Une solution de 0,7 g de 2-[2-(3-benzyloxy-4-phényl-pyrazol-1-yl)-éthyl]-2-aza-bicyclo[2.2.1]heptane dans 50 cm³ d'éthanol est additionnée de 1,6 cm³ d'acide chlorhydrique 6 M, agitée 0,25 heure à température ambiante, puis concentrée à sec sous pression réduite (3 kPa). Le résidu obtenu et 0,10 g de palladium sur charbon (à 10%) sont mis en suspension dans 20 cm³ d'éthanol et agités en autoclave sous une pression d'hydrogène de 1000 kPa , à une température de 20°C pendant 8 heures. Le milieu réactionnel est ensuite filtré sur Célite^{®} et concentré à sec sous pression réduite (3 kPa). Le résidu est trituré dans 40 cm³ d'acétone et isolé par filtration. On obtient ainsi 0,565 g de dichlorhydrate de 1-[2-(2-aza-bicyclo[2.2.1]hept-2-yl)-éthyl]-4-phényl-1H-pyrazol-3-ol sous forme d'une poudre beige fondant à 173°C (avec décomposition). Spectre de R.M.N. ¹H (400 MHz, (CD₃)₂SO d6 avec ajout de quelques gouttes de CD₃COOD d4, à une température de 363 K, δ en ppm) : de 1,45 à 1,55 (mt : 1H) ; de 1,65 à 1,80 (mt : 3H) ; de 1,95 à 2,05 (mt : 2H) ; 2,65 (mt : 1H) ; de 3,10 à 3,25 (mf étalé : 2H) ; 3,48 (mt : 1H) ; 3,59 (mt : 1H) ; 4,04 (mt : 1H) ; 4,34 (t large, J = 6,5 Hz : 2H) ; 7,15 (t large, J = 7,5 Hz : 1H) ; 7,32 (t large, J = 7,5 Hz : 2H) ; 7,62 (d large, J = 7,5 Hz : 2H) ; 7,90 (s : 1H). Spectre IR (KBr) : 2955; 2827; 2601; 2554; 1607; 1528; 1454; 1177; 1010; 767; 699; 672 et 515 cm⁻¹.

Le 2-[2-(3-benzyloxy-4-phényl-pyrazol-1-yl)-éthyl]-2-aza-bicyclo[2.2.1]heptane peut être préparé de la manière suivante :

Une suspension de 1 g de 1-[(toluène-4-sulfonyl)oxy]-2-(3-benzyloxy-4-phényl-pyrazol-1-yl)-éthyle, 0,51 g de chlorhydrate de 2-aza-bicyclo[2.2.1]heptane et 0,88 g de carbonate de potassium dans 25 cm³ d'acétonitrile est agitée pendant 8 heures à une température voisine de 80°C. Le mélange est concentré à sec sous pression réduite (3 kPa). Le résidu est repris par 30 cm³ d'eau et extrait avec 200 cm³ d'acétate d'éthyle. La phase organique est lavée avec 2 fois 30 cm³ d'eau, puis séchée, filtrée et concentrée à sec sous pression réduite (3 kPa). Le résidu huileux obtenu est purifié par chromatographie sur gel de silice (granulométrie 15-35 µm), en éluant par un mélange de dichlorométhane et de méthanol (95 / 5 en volumes). Après concentration des fractions sous pression réduite, on obtient 0,75 g de 2-[2-(3-benzyloxy-4-phényl-pyrazol-1-yl)-éthyl]-2-aza-bicyclo[2.2.1]heptane sous forme d'une huile visqueuse incolore, qui est utilisée telle quelle dans l'étape suivante. Spectre de masse (IE) : m/z 373 (M^{+.}), m/z 110 (pic de base).

Le chlorhydrate de 2-aza-bicyclo[2.2.1]heptane peut être obtenu selon la méthode décrite par J. R. Malpass et col., J. C. S., Perkin Trans. 1 1977, 8, 874-884.

### Exemple 14

### Dichlorhydrate de 1-[2-diméthylamino-éthyl]-4-phényl-1H-pyrazol-3-ol

On opère comme à l'exemple 2 mais avec 0,10 g de dichlorhydrate de [2-(3-benzyloxy-4-phényl-pyrazol-1-yl)-éthyl]-diméthyl-amine et 0,012 g de palladium sur charbon (à 10%). On obtient ainsi 0,049 g de dichlorhydrate de 1-[2-diméthylamino-éthyl]-4-phényl-1H-pyrazol-3-ol sous forme d'une poudre beige fondant vers 135°C (avec décomposition). Spectre de R.M.N. ¹H (300 MHz, (CD₃)₂SO d6, δ en ppm) : 2,81 (d, J = 5 Hz : 6H) ; 3,51 (mt : 2H) ; 4,38 (t, J = 6,5 Hz : 2H) ; 7,16 (t large, J = 7,5 Hz : 1H) ; 7,35 (t large, J = 7,5 Hz : 2H) ; 7,65 (d large, J = 7,5 Hz : 2H) ; 8,05 (s : 1H) ; 10,27 (mf : 1H) ; de 10,30 à 10,70 (mf très étalé : 1H). Spectre IR (KBr) : 3311; 2985; 2558; 2463; 1629; 1582; 1508; 1467; 1409; 1190; 985; 760; 687 et 673 cm⁻¹.

Le dichlorhydrate de [2-(3-benzyloxy-4-phényl-pyrazol-1-yl)-éthyl]-diméthyl-amine peut être préparé de la manière suivante :

A une solution de 0,25 g de 3-benzyloxy-4.-phényl-pyrazole dans 3 cm³ de diméthylformamide anhydre on ajoute progressivement, sous atmosphère d'argon et à température ambiante, 0,154 g d'hydrure de sodium (à 75% en masse dans l'huile de vaseline), puis, après disparition des mousses, 0,5 g de bromhydrate de (2-bromo-éthyl)-diméthyl-amine. Après 2 heures d'agitation à température ambiante le mélange est additionné lentement d'eau et extrait à l'acétate d'éthyle. La phase organique est séchée sur sulfate de magnésium, filtrée et concentrée à sec sous pression réduite (3 kPa). Le résidu huileux obtenu est purifié par chromatographie sur gel de silice, en éluant par un mélange de dichlorométhane, de méthanol et d'ammoniaque à 28% (90 / 8/2 en volumes). Après concentration des fractions sous pression réduite, on obtient 0,21 g d'une huile qui est dissoute dans l'éther éthylique , additionnée de 1 cm³ d'une solution environ 3 M d'éther chlorhydrique, puis ramenée à sec. Le résidu est trituré dans l'acétone puis isolé par filtration. On obtient ainsi 0,1 g de dichlorhydrate de [2-(3 benzyloxy-4-phényl-pyrazol-1-yl)-éthyl]-diméthyl-amine sous forme de cristaux blancs fondant à 105°C. Spectre de R.M.N. ¹H (300 MHz, (CD₃)₂SO d6, δ en ppm) : 2,82 (d, J = 5 Hz : 6H) ; 3,57 (mt : 2H) ; 4,34 (t, J = 6,5 Hz : 2H) ; 5,36 (s : 2H) ; 7,18 (t large, J = 7,5 Hz : 1H) ; de 7,30 à 7,50 (mt : 5H) ; 7,52 (d large, J = 7,5 Hz : 2H) ; 7,65 (d large, J = 7,5 Hz : 2H) ; 8,17 (s : 1H) ; 9,68 (mf : 1H).

Le bromhydrate de (2-bromo-éthyl)-diméthyl-amine peut être obtenu selon la méthode décrite par L. H. Amundsen et coll., J. Am. Chem. Soc. 1941, 63, 305-307.

### Exemple 15

### Dichlorhydrate de 1-[3-diméthylamino-propyl]-4-phényl-1H-pyrazol-3-ol

On opère comme à l'exemple 2 mais avec 0,274 g de dichlorhydrate de [3-(3-benzyloxy-4-phényl-pyrazol-1-yl)-propyl]-diméthyl-amine et 0,04 g de palladium sur charbon (à 10%). On obtient ainsi 0,209 g de dichlorhydrate de 1-[3-diméthylamino-propyl]-4-phényl-1H-pyrazol-3-ol sous forme d'une poudre beige fondant vers 208°C (avec décomposition). Spectre de R.M.N. ¹H (300 MHz, (CD₃)₂SO d6, δ en ppm) : 2,20 (mt : 2H) ; 3,5 (d, J = 5 Hz : 6H) ; 3,07 (mt : 2H) ; 4,04 (t, J = 6,5 Hz : 2H) ; 7,13 (t large, J = 7,5 Hz : 1H) ; 7,33 (t large, J = 7,5 Hz : 2H) ; 7,66 (d large, J = 7,5 Hz : 2H) ; 7,99 (s : 1H) ; 10,82 (mf: 1H). Spectre IR (KBr) : 3078; 2954; 2591; 2470; 1603; 1476; 1369; 1268; 1188; 881; 763; 700; 570 et 494 cm⁻¹.

Le dichlorhydrate de [3-(3-benzyloxy-4-phényl-pyrazol-1-yl)-propyl]-diméthyl-amine peut être préparé de la manière suivante :

Une suspension de 0,38 g d'oxalate de [3-(3-benzyloxy-4-phényl-pyrazol-1-yl)-propyl]-diméthyl-amine dans 10 cm³ d'eau est additionnée de 3,6 cm³ de solution d'hydroxyde de sodium 1 N et agitée pendant 0,25 heure, puis extraite par 3 fois 25 cm³ d'acétate d'éthyle. Les phases organiques réunies sont séchées sur sulfate de magnésium, puis concentrées à sec sous pression réduite (3 kPa). Le résidu obtenu est dissous dans 25 cm³ d'éthanol, additionné d'un excès d'une solution environ 3 M d'éther chlorhydrique, puis ramené à sec. On obtient ainsi 0,274 g de dichlorhydrate de [3-(3-benzyloxy-4-phényl-pyrazol-1-yl)-propyl]-diméthyl-amine sous forme d'un solide blanc pâteux utilisé tel quel dans l'étape suivante.

L'oxalate de [3-(3-benzyloxy-4-phényl-pyrazol-1-yl)-propyl]-diméthyl-amine peut être préparé de la manière suivante :

A une solution de 0,25 g de 3-benzyloxy-4-phényl-pyrazole dans 15 cm³ de diméthylformamide anhydre on ajoute progressivement, sous atmosphère d'argon et à température ambiante, 0,106 g d'hydrure de sodium (à 75% en masse dans l'huile de vaseline). Après 0,75 heure d'agitation à une température voisine de 50°C on ajoute par petites portions 0,316 g de chlorhydrate de (3-chloro-propyl)-diméthyl-amine, puis agite pendant 16 heures à température ambiante. Le mélange est versé dans 150 cm³ d'eau et extrait avec 3 fois 150 cm³ d'acétate d'éthyle. Les phases organiques réunies sont lavées avec 50 cm³ d'eau, puis sont séchées sur sulfate de magnésium anhydre, filtrées et concentrées à sec sous pression réduite (3 kPa). Le résidu huileux obtenu (0,8 g) est dissous dans 10 cm³ d'éther éthylique et additionné d'une solution de 0,09 g d'acide oxalique dans 5 cm³ d'éther éthylique. Le précipité blanc formé est filtré puis séché sous vide (70 Pa) à température ambiante. On obtient ainsi 0,395 g d'oxalate de [3-(3-benzyloxy-4-phényl-pyrazol-1-yl)-propyl]-diméthyl-amine sous forme d'un solide blanc utilisé tel quel dans l'étape suivante. Spectre de R.M.N. ¹H (300 MHz, (CD₃)₂SO d6, δ en ppm) : 2,15 (mt : 2H) ; 30 (s : 6H) ; 2,98 (mt : 2H) ; 4,08 (t, J = 6,5 Hz : 2H) ; 5,33 (s : 2H) ; 7,16 (t large, J = 7,5 Hz : 1H) ; de 7,30 à 7,45 (mt : 3H) ; 7,43 (t large, J = 7,5 Hz : 2H) ; 7,51 (d large, J = 7,5 Hz : 2H) ; 7,65 (d large, J = 7,5 Hz : 2H) ; 8,09 (s : 1H).

### Exemple 16

### Dichlorhydrate de 1-[2-((2S,6R)-2,6-diméthyl-pipéridin-1-yl)-éthyl]-4-phényl-1H-pyrazol-3-ol

On opère comme à l'exemple 2 mais avec 0,123 g de dichlorhydrate de (2S,6R)-1-[2-(3-benzyloxy-4-phényl-pyrazol-1-yl)-éthyl]-2,6-diméthyl-pipéridine et 0,014 g de palladium sur charbon (à 10%). On obtient ainsi 0,075 g de dichlorhydrate de 1-[2-((2S,6R)-2,6-diméthyl-pipéridin-1-yl)-éthyl]-4-phényl-1H-pyrazol-3-ol sous forme d'une poudre beige fondant vers 206°C (avec décomposition). Spectre de R.M.N. ¹H (400 MHz, (CD₃)₂SO d6, à une température de 403K, δ en ppm) : 1,36 (d, J = 6,5 Hz : 6H) ; 1,57 (mt : 1H) ; de 1,65 à 1,90 (mt : 5H) ; 3,28 (mf : 2H) ; 3,49 (mf : 2H) ; 4,36 (t large, J = 6,5 Hz : 2H) ; 7,15 (tt, J = 7,5 et 1,5 Hz : 1H) ; 7,33 (t large, J = 7,5 Hz : 2H) ; 7,66 (d large, J = 7,5 Hz : 2H) ; 8,00 (s : 1H). Spectre IR (KBr) : 3428; 3058; 2978; 2942; 2657; 2571; 1606; 1580; 1521; 1452; 1388; 1173; 997; 914; 766; 699; 671 et 511 cm⁻¹.

Le dichlorhydrate de (2S,6R)-1-[2-(3-benzyloxy-4-phényl-pyrazol-1-yl)-éthyl]-2,6-diméthyl-pipéridine peut être préparé de la manière suivante :

Une solution de 0,117 g de (2S,6R)-1-[2-(3-benzyloxy-4-phényl-pyrazol-1-yl)-éthyl]-2,6-diméthyl-pipéridine dans 25 cm³ d'éthanol est additionnée de 0,5 cm3 d'une solution environ 3 M d'éther chlorhydrique, puis ramenée à sec. On obtient ainsi 0,123 g de dichlorhydrate de (2S,6R)-1-[2-(3-benzyloxy-4-phényl-pyrazol-1-yl)-éthyl]-2,6-diméthyl-pipéridine sous forme d'une pâte incolore utilisée telle quelle dans l'étape suivante.

La (25,6R)-1-[2-(3-benzyloxy-4-phényl-pyrazol-1-yl)-éthyl]-2,6-diméthyl-pipéridine peut être préparée de la manière suivante :

A une solution de 0,25 g de 3-benzyloxy-4-phényl-pyrazole dans 20 cm3 de diméthylformamide anhydre on ajoute progressivement, sous atmosphère d'argon et à température ambiante, 0,211 g d'hydrure de sodium (à 75% en masse dans l'huile de vaseline). Après 0,75 heure d'agitation à une température voisine de 50°C on ajoute par petites portions 0,636 g de chlorhydrate de (2S,6R)-1-(2-chloro-éthyl)-2,6-diméthyl-pipéridine, puis agite pendant 16 heures à température ambiante. Le mélange est versé dans 150 cm3 d'eau et extrait avec 2 fois 150 cm3 d'acétate d'éthyle. Les phases organiques réunies sont lavées avec 50 cm3 d'eau, puis sont séchées sur sulfate de magnésium anhydre, filtrées et concentrées à sec sous pression réduite (3 kPa). Le résidu huileux obtenu est dissous dans 25 cm3 d'éther éthylique et additionné d'une solution de 0,09 g d'acide oxalique dans 25 cm3 d'éther éthylique. Le produit pâteux formé est lavé avec 3 fois 25 cm3 d'éther éthylique, puis est repris par 25 cm3 d'eau et additionné de 4 cm3 de solution d'hydroxyde de sodium 1 N et agité pendant 0,25 heure, puis extrait par 2 fois 25 cm3 d'acétate d'éthyle. Les phases organiques réunies sont séchées sur sulfate de magnésium, puis concentrées à sec sous pression réduite (3 kPa). Le résidu huileux obtenu est purifié par chromatographie sur gel de silice, en éluant par un mélange d'acétate d'éthyle et de méthanol (80 / 20 en volumes). Après concentration des fractions sous pression réduite, on obtient 0,117 g de (2S,6R)-1-[2-(3-benzyloxy-4-phényl-pyrazol-1-yl)-éthyl]-2,6-diméthyl-pipéridine sous forme d'une huile incolore utilisée telle quelle dans l'étape suivante. Spectre de R.M.N. ¹H (300 MHz, (CD3)2SO d6, 8 en ppm) : 1,08 (d, J = 6,5 Hz : 6H) ; 1,17 (t dédoublé large, J = 12 et 3 Hz : 2H) ; 1,28 (mt : 1H) ; 1,53 (d large, J = 12 Hz : 2H) ; 1,62 (mt : 1H) ; 2,48 (mt : 2H) ; 2,95 (t, J = 6,5 Hz : 2H) ; 3,98 (t, J = 6,5 Hz : 2H) ; 5,32 (s : 2H) ; 7,15 (tt, J = 7,5 et 1,5 Hz : 1H) ; de 7,30 à 7,45 (mt : 1H) ; 7,34 (t large, J = 7,5 Hz : 2H) ; 7,42 (t large, J = 7,5 Hz : 2H); 7,52 (d large, J = 7,5 Hz : 2H) ; 7,66 (d large, J = 7,5 Hz : 2H) ; 8,10 (s :1H).

Le chlorhydrate de (2S,6R)-1-(2-chloro-éthyl)-2,6-diméthyl-pipéridine amine peut être obtenu selon la méthode décrite par R. Dahlbom et coll., Acta Phannaceutica Suecica 1969, 6 (3), 413-418.

### Exemple 17

### Dichlorhydrate de 1-[2-diéthylamino-éthyl]-4-phényl-1H-pyrazol-3-ol

On opère comme à l'exemple 2 mais avec 0,31 g de dichlorhydrate de [2-(3-benzyloxy-4-phényl-pyrazol-1-yl)-éthyl]-diéthyl-amine et 0,04 g de palladium sur charbon (à 10%). On obtient ainsi 0,139 g de dichlorhydrate de 1-[2-diéthylamino-éthyl]-4-phényl-1H-pyrazol-3-ol sous forme d'une poudre blanche fondant vers 174°C (avec décomposition). Spectre de R.M.N. ¹H (300 MHz, (CD₃)₂O d6, δ en ppm) : 1,23 (t, J = 7 Hz : 6H) ; 3,12 (q très large, J = 7 Hz : 4H) ; 3,46 (t très large, J = 6,5 Hz : 2H) ; 4,40 (t large, J = 6,5 Hz : 2H) ; 7,14 (t large, J = 7,5 Hz : 1H) ; 7,33 (t large, J = 7,5 Hz : 2H) ; 7,65 (d large, J = 7,5 Hz : 2H) ; 8,07 (s : 1H) ; 10,46 (mf : 1H) ; de 10,60 à 10,85 (mf étalé : 1H). Spectre IR (KBr) : 3065; 2974; 2589; 2484; 1609; 1530; 1454; 1179; 1012; 765; 693; 677 et 508 cm⁻¹.

Le dichlorhydrate de [2-(3-benzyloxy-4-phényl-pyrazol-1-yl)-éthyl]-diéthyl-amine peut être obtenu de la manière suivante :

On opère comme à l'exemple 15 mais avec 0,31 g d' oxalate de [2-(3-benzyloxy-4-phényl-pyrazol-1-yl)-éthyl]-diéthyl-amine. On obtient ainsi 0,31 g de dichlorhydrate de [2-(3-benzyloxy-4-phényl-pyrazol-1-yl)-éthyl]-diéthyl-amine sous la forme d'une gomme incolore utilisée telle quelle dans l'étape suivante.

L'oxalate de [2-(3-benzyloxy-4-phényl-pyrazol-1-yl)-éthyl]-diéthyl-amine peut être obtenu de la manière suivante :

On opère comme à l'exemple 15 mais avec 0,211 g d'hydrure de sodium (à 75 % en masse dans l'huile de vaseline) et 0,516 g de chlorhydrate de (2-chloro-éthyl)-diéthyl-amine. On obtient ainsi 0,376 g d'oxalate de [2-(3-benzyloxy-4-phényl-pyrazol-1-yl)-éthyl]-diéthyl-amine sous forme d'une poudre blanche fondant à 133°C. Spectre de R.M.N. ¹H (300 MHz, (CD₃)₂SO d6, δ en ppm) : 1,12 (t, J = 7 Hz : 6H) ; 2,98 (q large, J = 7 Hz : 4H) ; 3,35 (t très large, J = 6,5 Hz : 2H) ; 4,31 (t large, J = 6,5 Hz : 2H) ; 5,35 (s : 2H) ; 7,17 (t large, J = 7,5 Hz : 1H) ; 7,36 (mt : 3H) ; 7,43 (t large, J = 7,5 Hz : 2H) ; 7,51 (d large, J = 7,5 Hz : 2H) ; 7,65 (d large, J = 7,5 Hz : 2H) ; 8,14 (s : 1H).

### Exemple 18

### Dichlorhydrate de 1(2-diisopropylamino-éthyl)-4-phényl-1-pyrazol-3-ol

On opère comme à l'exemple 2 mais avec 0,21 g de dichlorhydrate de [2-(3-benzyloxy-4-phényl-pyrazol-1-yl)-éthyl]-diisopropyl-amine et 0,025 g de palladium sur charbon (à 10%). On obtient ainsi 0,122 g de dichlorhydrate de 1-[2-diisopropylamino-éthyl]-4-phényl-1-pyrazol-3-ol sous forme d'une poudre beige fondant vers 220°C (avec décomposition). Spectre de R.M.N. ¹H (300 MHz, (CD₃)₂SO d6, δ en ppm) : 1,32 (d large, J = 7 Hz : 6H) ; 1,34 (d large, J = 7 Hz : 6H) ; 3,47 (mf: 2H); 3,71 (mt: 2H); 4,40 (t large, J = 6,5 Hz: 2H); 7,14 (t large, J = 7,5 Hz : 1H) ; 7,33 (t large, J = 7,5 Hz : 2H) ; 7,65 (d large, J = 7,5 Hz : 2H) ; 8,12 (s : 1H) ; 9,96 (mf : 1H) ; 10,49 (s large : 1H). Spectre IR (KBr) : 2984; 2654; 2507; 2469; 1607; 1580;1531; 1453; 1193; 759; 693; 673 et 511 cm⁻¹.

Le dichlorhydrate de [2-(3-benzyloxy-4-phényl-pyrazol-1-yl)-éthyl]-diisopropyl-amine peut être obtenu de la manière suivante :

On opère comme à l'exemple 15 mais avec 0,31 g d' oxalate de [2-(3-benzyloxy-4-phényl-pyrazol-1-yl)-éthyl]-diisopropyl-amine. On obtient ainsi 0,21 g de dichlorhydrate de [2-(3-benzyloxy-4-phényl-pyrazol-1-yl)-éthyl]-diisopropyl-amine sous la forme d'un semi-solide beige utilisé tel quel dans l'étape suivante.

L'oxalate de [2-(3-benzyloxy-4-phényl-pyrazol-1-yl)-éthyl]-diisopropyl-amine peut être obtenu de la manière suivante :

On opère comme à l'exemple 15 mais avec 0,211 g d'hydrure de sodium (à 75 % en masse dans l'huile de vaseline) et 0,6 g de chlorhydrate de (2-chloro-éthyl)-diisopropyl-amine. On obtient ainsi 0,312 g d'oxalate de [2-(3-benzyloxy-4-phényl-pyrazol-1-yl)-éthyl]-diéthyl-amine sous forme d'une poudre blanche fondant à 134°C. Spectre de R.M.N. ¹H (300 MHz, (CD₃)₂SO d6 avec ajout de quelques gouttes de CD₃COOD d4, δ en ppm) : 1,21 (d, J = 6 Hz : 12H) ; 3,39 (t large, J = 6,5 Hz : 2H) ; 3,57 (mt : 2H) ; 4,30 (t, J = 6,5 Hz : 2H) ; 5,35 (s : 2H) ; 7,17 (t large, J = 7,5 Hz : 1H) ; 7,35 (mt : 3H) ; 7,41 (t large, J = 7,5 Hz : 2H) ; 7,49 (d large, J = 7,5 Hz : 2H) ; 7,65 (d large, J = 7,5 Hz : 2H) ; 8,10 (s : 1H).

### Exemple 19

### Dichlorhydrate de 4-phényl-1-(2-pyrrolidin-1-yl-éthyl)-1H-pyrazol-3-ol

On opère comme à l'exemple 2 mais avec 0,285 g de dichlorhydrate de 3-benzyloxy-4-phényl-1-(2-pyrrolidin-1-yl-éthyl)-1H-pyrazole et 0,037 g de palladium sur charbon (à 10%). On obtient ainsi 0,101 g de dichlorhydrate de 4-phényl-1-(2-pyrrolidin-1-yl-éthyl)-1H-pyrazol-3-ol sous forme d'une poudre beige fondant vers 173°C (avec décomposition). Spectre de R.M.N. ¹H (300 MHz, (CD₃)₂SO d6, δ en ppm) : de 1,80 à 2,10 (mt : 4H) ; de 2,90 à 3,10 (mf : 2H) ; de 3,45 à 3,65 (mf : 4H) ; 4,34 (t large, J = 6,5 Hz : 2H) ; 7,14 (t large, J = 7,5 Hz : 1H) ; 7,33 (t large, J = 7,5 Hz : 2H) ; 7,65 (d large, J = 7,5 Hz : 2H) ; 8,02 (s : 1H) ; de 10,30 à 10,60 (mf étalé : 1H) ; 10,43 (mf : 1H). Spectre IR (KBr) : 3416; 3054; 2973; 2670; 2585; 2476; 2405; 1608; 1581; 1527; 1453; 1247; 1175; 1011; 768; 702; 673 et 514 cm⁻¹.

Le dichlorhydrate de 3-benzyloxy-4-phényl-1-(2-pyrrolidin-1-yl-éthyl)-1H-pyrazole peut être obtenu de la manière suivante :

On opère comme à l'exemple 15 mais avec 0,34 g d'oxalate de 3-benzyloxy-4-phényl-1-(2-pyrrolidin-1-yl-éthyl)-1H-pyrazole. On obtient ainsi 0,285 g de dichlorhydrate de 3-benzyloxy-4-phényl-1-(2-pyrrolidin-1-yl-éthyl)-1H-pyrazole sous la forme d'une gomme beige utilisée telle quelle dans l'étape suivante.

L'oxalate de 3-benzyloxy-4-phényl-1-(2-pyrrolidin-1-yl-éthyl)-1H-pyrazole peut être obtenu de la manière suivante :

On opère comme à l'exemple 15 mais avec 0,211 g d'hydrure de sodium (à 75% en masse dans l'huile de vaseline) et 0,51 g de chlorhydrate de 1-(2-chloro-éthyl)-pyrrolidine. On obtient ainsi 0,354 g d'oxalate de 3-benzyloxy-4-phényl-1-(2-pyrrolidin-1-yl-éthyl)-1H-pyrazole sous forme d'une poudre blanche fondant à 144°C. Spectre de R.M.N. ¹H (300 MHz, (CD₃)₂SO d6, δ en ppm) : 1,87 (mt : 4H) ; 3,10 (mt : 4H) ; 3,46 (mt : 2H) ; 4,33 (t, J = 6,5 Hz : 2H) ; 5,35 (s : 2H) ; 7,18 (t large, J = 7,5 Hz : 1H) ; 7,36 (mt : 3H) ; 7,43 (t large, J = 7,5 Hz : 2H) ; 7,52 (d large, J = 7,5 Hz : 2H) ; 7,66 (d large, J = 7,5 Hz : 2H) ; 8,13 (s : 1H).

### Exemple 20

### Chlorhydrate de 3-(3-méthoxy-4-phényl-pyrazol-1-yl)-1-aza-bicyclo[2.2.2]octane

A une solution de 1,2 g de 3-méthoxy-4-phényl-pyrazole dans 20 cm³ de diméthylformamide anhydre on ajoute progressivement, sous atmosphère d'argon et à température ambiante, 1,94 g de *tert-*butylate de potassium. Après 1,5 heure d'agitation à température ambiante on ajoute goutte à goutte la solution de 2,8 g de 3-[(méthanesulfonyl)oxy]-1-aza-bicyclo[2.2.2]octane dans 20 cm³ de diméthylformamide anhydre, puis chauffe pendant 16 heures à une température voisine de 100°C. Le mélange est refroidi à température ambiante puis est concentré sous pression réduite (3 kPa). Le résidu est repris par 30 cm³ d'eau et extrait avec 250 cm³ d'acétate d'éthyle. La phase organique est lavée avec 3 fois 30 cm³ d'eau, puis séchée, filtrée et concentrée à sec sous pression réduite (3 kPa). Le résidu huileux obtenu est purifié par chromatographie sur gel de silice en éluant avec un mélange d'acétate d'éthyle et de méthanol (90 / 10, puis 75 /25 en volumes). Après concentration des fractions sous pression réduite on obtient 0,36 g d'une huile, qui est dissoute dans 15 cm³ d'acétone et additionnée de 5 cm³ d'une solution environ 1 M d'éther chlorhydrique. Le précipité apparu est trituré pendant une nuit puis isolé par filtration. On obtient ainsi 0,308 g de chlorhydrate de 3-(3-méthoxy-4-phényl-pyrazol-1-yl)-1-aza-bicyclo[2.2.2]octane sous forme d'une poudre beige hygroscopique fondant vers 207°C (avec décomposition). Spectre de R.M.N. ¹H (300 MHz, (CD₃)₂SO d6, δ en ppm) : de 1,65 à 2,05 (mt : 4H) ; 2,42 (mt : 1H) ; 3,28 (mt : 3H) ; de 3,35 à 3,55 (mt : 1H) ; 3,80 (mt : 2H) ; 3,97 (s : 3H) ; 4,75 (mt : 1H) ; 7,18 (t large, J = 7,5 Hz : 1H) ; 7,36 (t large, J = 7,5 Hz : 2H) ; 7,65 (d large, J = 7,5 Hz : 2H) ; 8,30 (s : 1H) ; 10,76 (mf : 1H). Spectre IR (KBr) : 3430; 2939; 2907; 2666; 2584; 1607; 1580; 1570; 1518; 1454; 1409; 1049; 1028; 764; 698; 623 et 513 cm⁻¹.

### Exemple 21

### Chlorhydrate de 1-[2-(3-difluorométhoxy-4-phényl-pyrazol-1-yl)-éthyl]-pipéridine

On opère comme à l'exemple 1 mais avec 0,25 g de 3-difluorométhoxy-4-phényl-1H-pyrazole, 0,303 g d'hydrure de sodium (à 75% en masse dans l'huile de vaseline) et 0,6 g de chlorhydrate de 1-(2-chloro-éthyl)-pipéridine, puis en éluant par un mélange de dichlorométhane et de méthanol (95 / 5 en volumes). On obtient ainsi 0,175 g de chlorhydrate de 1-[2-(3-difluorométhoxy-4-phényl-pyrazol-1-yl)-éthyl]-pipéridine sous forme d'un solide blanc fondant vers 174°C (avec décomposition). Spectre de RM.N. ¹H (300 MHz, (CO₃)₂SO d6, δ en ppm) : 1,40 (mf: 1H) ; de 1,60 à 1,90 (mt : 5H) ; 2,94 (mf : 2H) ; 3,47 (d très large, J = 12 Hz : 2H) ; 3,54 (mf: 2H); 4,55 (t large, J = 6,5 Hz : 2H) ; 7,29 (tt, J = 7,5 et 2,5 Hz : 1H) ; 7,41 (t, J = 72 Hz : 1H); 7,44 (t large, J = 7,5 Hz : 2H) ; 7,59 (d large, J = 7,5 Hz : 2H) ; 8,30 (s : 1H) ; de 10,00 à 10,20 (mf: 1H). Spectre IR (KBr) : 3100; 2931; 2644; 2543; 1609; 1581; 1507; 1482; 1456; 1364; 1181; 1125; 1100; 1076; 761; 694 et 513 cm⁻¹.

Le 3-difluorométhoxy-4-phényl-1H-pyrazole peut être préparé de la manière suivante :

Une suspension de 2,55 g de 1-(3-hydroxy-4-phényl-pyrazol-1-yl)-éthanone, 1,75 g de carbonate de potassium et 1,82 g de 2-chloro-2,2-difluoroacétate de méthyle dans 40 cm³ de diméthylformamide est agitée, sous atmosphère d'argon, à température ambiante pendant 16 heures, puis à une température de 65°C pendant 8 heures. Après refroidissement le mélange est additionné de 10 cm³ d'une solution d'hydroxyde de sodium 10 N et agité 1 heure à température ambiante, puis concentré sous pression réduite (3 kPa). Le résidu est extrait avec 200 cm³ d'acétate d'éthyle. La phase organique est lavée avec 3 fois 25 cm³ d'eau, séchée et concentrée sous pression réduite (3 kPa). Le résidu huileux obtenu est purifié par chromatographie sur gel de silice en éluant avec un mélange de dichlorométhane et de méthanol (99 / 1 en volumes). Après concentration des fractions sous pression réduite on obtient 0,8 g de 3-difluorométhoxy-4-phényl-1H-pyrazole sous la forme d'un solide jaune fondant à 125°C. Spectre de masse (IE) : m/z 210 (M^{+.}) (pic de base), m/z 160 [M - CF₂]⁺.

### Exemple 22

### Dichlorhydrate de 4-phényl-1-(2-pipéridin-1-yl-éthyl)-1H-pyrazol-3-ylamine

Une solution de 0,2 g de 4-phényl-1-(2-pipéridin-1-yl-éthyl)-1H-pyrazol-3-ylamine dans 10 cm³ de méthanol est additionnée de 10 cm³ d'une solution 1N d'éther chlorhydrique en excès, puis concentrée à sec sous pression réduite (3 kPa). Le résidu fournit, après trituration dans l'éther éthylique, 0,244 g de dichlorhydrate de 4-phényl-1-(2-pipéridin-1-yl-éthyl)-1H-pyrazol-3-ylamine sous forme de cristaux blancs fondant vers 120°C. Spectre de RM.N. ¹H (300 MHz, (CD₃)₂SO d6, δ en ppm) : de 1,25 à 1,55 (mt : 1H) ; de 1,60 à 1,90 (mt : 5H) ; 2,95 (mf : 2H) ; 3,44 (mf : 2H) ; 3,51 (mt : 2H) ; 4,53 (t, J = 6,5 Hz : 2H) ; 7,27 (t large, J = 7,5 Hz : 1H) ; 7,42 (t large, J = 7,5 Hz : 2H) ; 7,56 (d large, J = 7,5 Hz : 2H) ; 8,07 (s : 1H) ; 10,46 (mf : 1H). Spectre IR (KBr) : 3277; 2945; 2630; 2545; 1612; 1540; 1451; 1099; 1005; 768; 707; 572 et 559 cm⁻¹.

La 4-phényl-1-(2-pipéridin-1-yl-éthyl)-1H-pyrazol-3-ylamine peut être préparée de la manière suivante :

Une suspension de 1,59 g de 4-phényl-1H-pyrazol-3-ylamine, 2,2 g de chlorhydrate de 1-(2-chloro-éthyl)-pipéridine, 4 g de carbonate de potassium et 1,66 g d'iodure de potassium dans 50 cm³ de 2-butanone est agitée à la température d'ébullition du milieu réactionnel pendant 22 heures. Après refroidissement le mélange est amené à sec sous pression réduite (3 kPa). Le résidu est repris par 40 cm³ de solution 0,5 N d'hydroxyde de sodium et extrait avec 50 cm³ d'acétate d'éthyle. La phase organique est séchée sur sulfate de magnésium, filtrée et concentrée à sec sous pression réduite (3 kPa). Le résidu huileux jaune est purifié par chromatographie sur alumine basique en éluant successivement avec un mélange d'acétate d'éthyle et de dichlorométhane (50 / 50 en volumes) puis avec de l'acétate d'éthyle pur. On obtient ainsi 0,2 g de 4-phényl-1-(2-pipéridin-1-yl-éthyl)-1H-pyrazol-3-ylamine sous forme d'un solide blanc fondant à 96°C et ayant un Rf de 0,4 (acétate d'éthyle, plaque d'oxyde d'aluminium référence 105731, Merck). Spectre de R.M.N. ¹H (300 MHz, (CD₃)₂SO d6, δ en ppm) : 1,40 (mt : 2H) ; 1,49 (mt : 4H) ; 2,39 (t large, J = 5 Hz : 4H) ; 2,64 (t, J = 6,5 Hz: 2H); 3,98 (t,J = 6,5 Hz : 2H) ; 4,63 (s : 2H) ; 7,15 (tt, J = 7,5 et 1,5 Hz : 1H) ; 7,34 (t large, J = 7,5 Hz : 2H) ; 7,49 (d large, J = 7,5 Hz : 2H) ; 7,74 (s : 1H).

La 4-phényl-1H-pyrazol-3-ylamine peut être préparée selon la méthode décrite par S. A. Lang, Jr. et coll., J. Heterocyclic Chem. 1977, 14, 65-69.

### Exemple 23

### Dichlorhydrate de 4-phényl-1-(2-pipéridin-1-yl-éthyl)-1H-pyrazol-3-ylamine

Une solution de 0,25 g de N-[4-phényl-1-(2-pipéridin-1-yl-éthyl)-1H-pyrazol-3-yl]-formamide dans 10 cm³ de tétrahydrofurane anhydre est additionnée progressivement, à température ambiante et sous atmosphère d'argon, de 2 cm³ d'une solution 1 M d'hydrure de lithium et d'aluminium. Après 66 heures d'agitation à température ambiante le mélange est additionné progressivement de 1 cm³ de solution 1 N d'hydroxyde de sodium et extrait avec 20 cm³ d'acétate d'éthyle. Après élimination du gel par filtration la phase organique est concentrée à sec sous pression réduite (3 kPa). Le résidu huileux obtenu est purifié par chromatographie sur alumine basique en éluant avec un mélange de dichlorométhane et d'acétate d'éthyle (80 / 20 en volumes). Après concentration des fractions sous pression réduite on obtient une huile incolore, qui est dissoute dans 10 cm³ d'éther éthylique et additionnée de 1 cm³ d'une solution environ 6 N de dioxane chlorhydrique, puis concentrée à sec sous pression réduite (3 kPa). Le résidu est trituré dans l'acétone et isolé par filtration. On obtient ainsi 0,045 g de dichlorhydrate de 4-phényl-1-(2-pipéridin-1-yl-éthyl)-1H-pyrazol-3-ylamine sous forme d'un solide blanc fondant vers 165°C (avec décomposition). Spectre de R.M.N. ¹H (300 MHz, (CD₃)₂SO d6, δ en ppm) : de 1,25 à 1,50 (mf : 1H) ; de 1,60 à 1,90 (mt : 5H) 1,6 (s : 3H) ; 2,94 (mf : 2H) ; de 3,40 à 3,65 (mt : 4H) ; 4,42 (t, J = 6,5 Hz : 2H) ; 7,20 (t large, J = 7,5 Hz : 1H) ; 7,37 (t large, J = 7,5 Hz : 2H) ; 7,47 (d large, J = 7,5 Hz : 2H) ; 7,89 (s : 1H) ;9,93 (mf : 1H). Spectre IR (KBr) : 3289; 2943; 2600; 2534; 2481; 1627; 1530; 1446; 1342; 1189; 850; 770; 706 et 499 cm⁻¹.

Le N-[4-phényl-1-(2-pipéridin-1-yl-éthyl)-1H-pyrazol-3-yl]-formamide peut être préparé de la manière suivante:

Une solution de 0,24 g de 4-phényl-1-(2-pipéridin-1-yl-éthyl)-1H-pyrazol-3-ylamine dans 10 cm³ de formiate d'éthyle est agitée à la température d'ébullition du milieu réactionnel pendant 23 heures. Après refroidissement, le mélange est concentré à sec sous pression réduite (3 kPa). On obtient ainsi 0,31 g de N-[4-phényl-1-(2-pipéridin-1-yl-éthyl)-1H-pyrazol-3-yl]-formamide sous forme d'une laque incolore, qui est utilisée telle quelle dans l'étape suivante. Spectre de R.M.N. ¹H (400 MHz, (CD₃)₂SO d6, à une température de 373 K, δ en ppm) : 1,42 (mt : 2H) ; 1,52 (mt : 4H) ; 2,45 (t, J = 5 Hz : 4H) ; 3,6 (t, J = 6,5 Hz : 2H) ; 4,17 (t, J = 6,5 Hz : 2H) ; 7,24 (t large, J = 7,5 Hz : 1H) ; 7,38 (t large, J = 7,5 Hz : 2H) ; 7,49 (d large, J = 7,5 Hz : 2H) ; 7,96 (s : 1H) 8,30 (d large, J = 5 Hz : 1H) ; 9,49 (mf : 1H). Spectre IR (KBr) : 34434; 3218; 2955; 2799; 1683; 1631; 1607; 1325; 1289; 765; 698 et 592 cm⁻¹.

### Exemple 24

### Oxalate de N-[4-phényl-1-(2-pipéridin-1-yl-éthyl)-1H-pyrazol-3-yl]-acétamide

Une solution de 0,27 g de 4-phényl-1-(2-pipéridin-1-yl-éthyl)-1H-pyrazol-3-ylamine dans 5 cm³ de chloroforme est additionnée de 0,1 cm³ d'anhydride acétique, puis est agitée à température ambiante pendant 100 heures. Après concentration à sec du mélange sous pression réduite (3 kPa), le résidu est additionné de 15 cm³ d'une solution saturée d'hydrogénocarbonate de sodium et extrait avec 20 cm³ d'acétate d'éthyle. La phase organique est séchée sur sulfate de magnésium, filtrée et concentrée à sec sous pression réduite (3 kPa). Le résidu huileux obtenu est dissous dans 10 cm³ d'acétone et additionné de 0,1 g d'acide oxalique. La solution obtenue est concentrée à sec sous pression réduite (3 kPa) et le résidu est trituré dans l'éther éthylique et isolé par filtration. On obtient ainsi 0,05 g d'oxalate de N-[4-phényl-1-(2-pipéridin-1-yl-éthyl)-1H-pyrazol-3-yl]-acétamide sous la forme d'un solide blanc hygroscopique. Spectre de R.M.N. ¹H (300 MHz, (CD₃)₂SO d6, δ en ppm) : 1,52 (mt : 2H) ; 1,70 (mt : 4H) ; 2,01 (s large : 3H) ; 3,01 (mf : 4H) ; 3,36 (t large, J = 6,5 Hz : 2H) : 4,44 (t large, J = 6,5 Hz : 2H) ; 7,25 (t large, J = 7,5 Hz : 1H) ; 7,38 (t large, J = 7,5 Hz : 2H) ; 7,47 (d large, J = 7,5 Hz : 2H) ; 8,11 (s large : 1H) ; 9,66 (mf: 1H). Spectre IR (KBr) : 3258; 3026; 2952; 2683; 2540; 1725; 1640; 1525; 1447; 1373; 1202; 1008; 765 et 700 cm⁻¹.

### Exemple 25

### N-[4-phényl-1-(2-pipéridin-1-yl-éthyl)-1H-pyrazol-3-yl]-méthanesulfonamide

Une solution de 0,18 g de 4-phényl-1-(2-pipéridin-1-yl-éthyl)-1H-pyrazol-3-ylamine dans 5 cm³ de chloroforme est additionnée de 0,06 cm³ de chlorure de méthanesulfonyle, puis est agitée à température ambiante pendant 22 heures. Le mélange est additionné de 0,04 cm³ de chlorure de méthanesulfonyle et l'agitation est poursuivie à température ambiante pendant 3 heures. Le mélange est additionné de 15 cm³ d'une solution saturée d'hydrogénocarbonate de sodium et extrait avec 25 cm³ d'acétate d'éthyle. La phase organique est séchée sur sulfate de magnésium, filtrée et concentrée à sec sous pression réduite (3 kPa). Le résidu huileux obtenu est purifié par chromatographie sur alumine basique en éluant successivement avec de l'acétate d'éthyle pur, puis avec un mélange d'acétate d'éthyle et de méthanol (30 / 1 en volumes). Après concentration des fractions sous pression réduite on obtient un résidu incolore, que l'on fait cristalliser par trituration dans l'éther éthylique et isole par filtration. On obtient ainsi 0,05 g de N-[4-phényl-1-(2-pipéridin-1-yl-éthyl)-1H-pyrazol-3-yl]-méthanesulfonamide sous forme d'un solide blanc fondant à 121°C. Spectre de R.M.N. ¹H (300 MHz, (CD₃)₂SO d6, δ en ppm) : de 1,40 à 1,55 (mt : 6H) ; 2,41 (t large, J = 5 Hz : 4H) ; 3,1 (t, J = 6,5 Hz : 2H) ; 3,11 (s : 3H) ; 4,18 (t, J = 6,5 Hz : 2H) ; 7,23 (t large, J = 7,5 Hz : 1H) ; 7,38 (t large, J = 7,5 Hz : 2H) ; 7,69 (d large, J = 7,5 Hz : 2H) ; 8,06 (s : 1H) ; de 9,00 à 9,70 (mf très étalé : 1H). Spectre IR (KBr) : 3105; 2928; 1610; 1440; 1321; 1149; 976; 765; 699; 524 et 518 cm⁻¹.

### Exemple 26

### Dichlorhydrate de 1-(2-diméthylamino-propyl)-4-phényl-1H-pyrazol-3-ol

A une solution sous agitation de 0,42 g de {2-[3-(cyclohex-2-ènyloxy)-4-phényl-pyrazol-1-yl]-1-méthyl-éthyl}-diméthyl-amine dans 5 cm³ de méthanol à une température voisine de 20°C, on ajoute progressivement 1 cm³ d'une solution chlorhydrique 4 N dans le dioxane. Après 15 heures d'agitation à une température voisine de 20°C, le mélange réactionnel est concentré à sec sous pression réduite (2,7 kPa) et séché à 40°C sous pression réduite (2,7 kPa) pour donner 0,37 g de dichlorhydrate de 1-(2-diméthylamino-propyl)-4-phényl-1H-pyrazol-3-ol sous forme d'un solide blanc fondant à 189°C. Spectre de masse (IC) : m/z 246 (MH⁺) pic de base.

La {2-[3-(cyclohex-2-ènyloxy)-4-phényl-pyrazol-1-yl]-1-méthyl-éthyl}-diméthyl-amine peut-être préparée de la manière suivante :

A une suspension agitée sous atmosphère d'argon de 1,3 g d'hydrure de sodium (à 75% dans l'huile de vaseline) dans 5 cm³ de diméthylformamide, on ajoute une solution de 1 g de 3-(cyclohex-2-ènyloxy)-4-phényl-1H-pyrazole dans 5 cm³ de diméthylformamide. Après 15 minutes d'agitation à une température voisine de 20°C, puis 30 minutes à 50°C, le milieu réactionnel est refroidi à une température voisine de 20°C, et on ajoute sous agitation 1,3 g de chlorhydrate de (2-chloro-1-méthyl-éthyl)-diméthyl-amine, puis le mélange est porté 15 heures à 50°C. Après addition de 0,14 g d'hydrure de sodium à 75 % dans l'huile de vaseline et 0,7 g de chlorhydrate de (2-chloro-1-méthyl-éthyl)-diméthyl-amine supplémentaires, la réaction est poursuivie 15 heures à 50°C, puis le milieu réactionnel est refroidi à une température voisine de 20°C et concentré à sec sous pression réduite (2,7 kPa). Le résidu obtenu est repris dans 100 cm³ d'eau ; la phase aqueuse résultante est extraite par 3 fois 30 cm³ de dichlorométhane, et la phase organique est séchée sur sulfate de magnésium, concentrée à sec sous pression réduite (2,7 kPa). On obtient 1,2 g d'une huile marron qui est purifiée par chromatographie-flash sur silice sous pression d'argon (50 kPa) [éluant : dichlorométhane / méthanol (95 / 5 en volumes)]. Après concentration à sec des fractions sous pression réduite (2,7 kPa), on obtient 0,42 g de {2-[3-(cyclohex-2-ènyloxy)-4-phényl-pyrazol-1-yl]-1-méthyl-éthyl}-diméthyl-amine sous forme d'une huile [CCM : éluant : dichlorométhane / méthanol (95 / 5 en volumes), Rf = 0,13]. Spectre de masse (IE) : m/z 325 (M^{+.}), m/z 72 (C4H10N⁺).

Le 3-(cyclohex-2-ènyloxy)-4-phényl-1H-pyrazole peut-être préparé de la manière suivante :

A 20 cm³ de méthyléthylcétone sous agitation à une température voisine de 20°C, on ajoute 2,02 g de 1-(3-hydroxy-4-phényl-pyrazol-1-yl)-éthanone, 1,27 cm³ de 3-bromo-cyclohexène et 1,52 g de carbonate de potassium. Après 3 heures de chauffage au reflux du solvant, le milieu réactionnel est refroidi à une température voisine de 20°C et concentré à sec sous pression réduite (2,7 kPa). Le résidu obtenu est repris dans un mélange de 20 cm³ de tétrahydrofurane et de 20 cm³ de méthanol, puis il est additionné sous agitation de 2 cm³ de soude 5 N. Après 30 minutes d'agitation à une température voisine de 20°C, le milieu réactionnel est concentré à sec sous pression réduite (2,7 kPa) conduisant à un résidu qui est solubilisé dans 100 cm³ d'acétate d'éthyle. La solution organique est lavée par 2 fois 20 cm³ d'eau et par 20 cm³ d'eau saturée en chlorure de sodium, séchée sur sulfate de magnésium, filtrée, et évaporée à sec sous pression réduite (2,7 kPa). Le solide obtenu est repris par 5 cm³ d'acétate d'éthyle à chaud sous agitation ; la solution est additionnée de 40 cm³ d'éther diisopropylique, portée au reflux du solvant pendant 15 minutes, puis refroidie à une température voisine de 20°C. Un premier jet de cristallisation est filtré, lavé par 10 cm³ d'éther diisopropylique et 10 cm³ de pentane, et séché sous pression réduite (2,7 kPa) pour donner 1,07 g de 3-(cyclohex-2-ènyloxy)-4-phényl-1H-pyrazole sous forme d'une poudre blanche. Le filtrat de cristallisation est évaporé à sec sous pression réduite (2,7 kPa), repris par 20 cm³ d'éther diisopropylique et additionné de 20 cm³ de pentane ; un second jet de cristallisation est filtré et séché sous pression réduite (2,7 kPa), pour donner 0,33 g d'un lot identique au précédent [CCM : éluant : cyclohexane / acétate d'éthyle (70 / 30 en volumes), Rf = 0,23]. Spectre de masse (IE) : m/z 240 (M^{+.}), m/z 160 [(M-C6H8)⁺] .

### Exemple 27

### 1-(1-Méthyl-pipéridin-3-ylméthyl)-4-phényl-1H-pyrazol-3-ol

A 20 cm³ d'éthanol à une température voisine de 20°C, on ajoute 0,86 g de 3-(3-benzyloxy-4-phényl-pyrazol-1-ylméthyl)-1-méthyl-pipéridine, 0,1 g de palladium sur charbon à 10 %, 10 cm³ de cyclohexène et 20 cm³ d'éthanol. Après 15 heures à 50°C, on ajoute au milieu réactionnel 0,1 g de palladium sur charbon à 10 % et 10 cm³ de cyclohexène ; on porte le mélange au reflux du solvant 1 heure, puis on additionne encore 15 cm³ de cyclohexène et la réaction est poursuivie au reflux du solvant pendant 5 heures. Le catalyseur est filtré sur supercel, la solution est évaporée à sec sous pression réduite (2,7 kPa) pour donner 0,48 g d'un solide qu'on triture dans 10 cm³ d'un mélange d'éther diisopropylique et de pentane. On obtient après filtration 0,3 g d'un solide qui est remis en réaction avec 10 cm³ d'éthanol, 10 cm³ de cyclohexène et 0,1 g de de palladium sur charbon à 10 % sous agitation et au reflux du solvant pendant 15 heures. Le catalyseur est filtré sur supercel, et le filtrat est évaporé à sec sous pression réduite (2,7 kPa) pour donner 0,3 g d'un solide qui est purifié par chromatographie-flash sur silice sous pression d'argon (50 kPa) [éluant : dichlorométhane / méthanol / solution aqueuse d'ammoniac à 38 % (88 / 10 / 2 en volumes)]. Après concentration des fractions sous pression réduite (2,7 kPa), on obtient 0,15 g d'un solide jaunâtre qu'on reprend dans 70 cm³ méthanol à une température voisine de 20°C. La solution est additionnée de 1 cm³ d'acide chlorhydrique 4 N dans le dioxane, agitée 15 minutes à une température voisine de 20°C, puis elle est évaporée à sec sous pression réduite (2,7 kPa) pour donner 0,19 g d'une meringue qui est triturée dans de l'éther diisopropylique et filtrée. Le solide déliquescent est repris par 1 cm³ de soude 1 N, et la phase aqueuse est lavée par du dichlorométhane, évaporée partiellement sous pression réduite (2,7 kPa), ajustée à pH 8 par addition d'acide chlorhydrique 0,1 N, et extraite par du dichlorométhane. La phase organique est séchée sur sulfate de magnésium, filtrée et évaporée sous pression réduite (2,7 kPa) pour donner 0,15 g de 1-(1-méthyl-pipéridin-3-ylméthyl)-4-phényl-1H-pyrazol-3-ol sous forme d'une meringue crème fondant à 132°C. Spectre de masse (ES) : m/z 272 (MH⁺).

La 3-(3-benzyloxy-4-phényl-pyrazol-1-ylméthyl)-1-méthyl-pipéridine peut-être préparée de la manière suivante :

A une solution de 1 g de 3-benzyloxy-4-phényl-1H-pyrazole dans 10 cm³ de diméthylformamide sous atmosphère d'argon et sous agitation, on ajoute progressivement, à une température voisine de 20°C, 0,4 g d'hydrure de sodium à 75 % dans l'huile de vaseline, puis le mélange est porté 10 minutes à 50°C. Après addition de 1,5 g de chlorhydrate de 3-chlorométhyl-1-méthylpipéridine, le milieu réactionnel est chauffé 15 heures à 80°C, puis il est refroidi à une température voisine de 20°C et jeté dans 100 cm³ d'eau. Le mélange est extrait par du dichlorométhane; la phase organique est séchée sur sulfate de magnésium, filtrée et évaporée à sec sous pression réduite (2,7 kPa) pour donner 1,6 g d'une huile marron qui est purifiée par chromatographie-flash sur silice sous pression d'argon (50 kPa) [éluant : dichlorométhane, méthanol (95 / 5 en volumes)]. Après concentration des fractions sous pression réduite à sec (2,7 kPa), on obtient 0,86 g de 3-(3-benzyloxy-4-phényl-pyrazol-1-ylméthyl)-1-méthyl-pipéridine sous forme d'une huile jaune [CCM : éluant : dichlorométhane / méthanol / solution aqueuse d'ammoniac à 38 % (88 / 10 / 2 en volumes), Rf = 0,41]. Spectre de masse (IE) : m/z 361 (M^{+.}), m/z 270 [(M-C7H7)⁺].

### Exemple 28

### Dichlorhydrate de 5-méthyl-4-phényl-1-(2-pipéridin-1-yl-éthyl)-1H-pyrazol-3-ol

A une solution de 0,3 g de 1-{2-[3-(cyclohex-2-ènyloxy)-5-méthyl-4-phényl-pyrazol-1-yl]-éthyl}-pipéridine dans 10 cm³ de méthanol, on ajoute progressivement sous agitation, à une température voisine de 20°C, 1,5 cm³ d'une solution chlorhydrique 4 N dans le dioxane. Après 15 heures d'agitation à une température voisine de 20°C, le mélange réactionnel est concentré à sec sous pression réduite (2,7 kPa). Le résidu est trituré dans de l'éther diisopropylique, filtré, et séché sous pression réduite (2,7 kPa) à 40°C pendant 2 heures pour donner 0,19 g de dichlorhydrate 5-méthyl-4-phényl-1-(2-pipéridin-1-yl-ethyl)-1H-pyrazol-3-ol sous forme d'un solide crème fondant à 222°C. Spectre de masse (IC) : m/z 286 (MH⁺).

La 1-{2-[3-(cyclohex-2-ènyloxy)-5-méthyl-4-phényl-pyrazol-1-yl]-éthyl}-pipéridine peut-être préparée de la manière suivante :

A une solution de 0,46 g de 3-(cyclohex-2-ènyloxy)-5-méthyl-4-phényl-1H-pyrazole dans 15 cm³ de diméthylformamide sous agitation et sous atmosphère d'argon, on ajoute progressivement, à une température voisine de 20°C, 0,2 g d'hydrure de sodium à 75% dans l'huile de vaseline. Après 5 minutes de chauffage à 50°C, le mélange réactionnel est additionné de 0,67 g de chlorhydrate de 1-(2-chloro-éthyl)-pipéridine, puis la solution est chauffée à 80°C pendant 15 heures. Le milieu réactionnel est jeté dans 100 cm³ d'eau ; la phase aqueuse est extraite par du dichlorométhane qui est séché sur sulfate de magnésium et concentré à sec sous pression réduite (2,7 kPa). L'huile marron (0,8 g) résultante est purifiée par chromatographie-flash sur silice sous pression d'argon (50 kPa) [éluant : dichlorométhane / méthanol (95 / 5 en volumes)]. Après concentration à sec des fractions sous pression réduite (2,7 kPa), on obtient 0,3 g de 3-(cyclohex-2-en 1-yloxy)-5-méthyl-4-phényl-1-(2-pipéridin-1-yl-éthyl)-1H-pyrazole sous forme d'une huile jaune [CCM : éluant dichlorométhane / méthanol (90 /10 en volumes), Rf = 0,27]. Spectre de masse (IC) : m/z 366 (MH⁺).

Le 3-(cyclohex-2-ènyloxy)-5-méthyl-4-phényl-1H-pyrazole peut-être préparé de la manière suivante :

A une solution sous agitation de 0,8 g de 1-[3-(cyclohex-2-ènyloxy)-5-méthyl-4-phényl-pyrazol-1-yl]-éthanone dans un mélange de 20 cm³ de méthanol et 20 cm³ de tétrahydrofurane, on ajoute progressivement, à une température voisine de 20°C, 0,54 cm³ de soude 5 N. Après 6 heures d'agitation à une température voisine de 20°C, le mélange réactionnel est concentré à sec sous pression réduite (2,7 kPa), et le résidu est repris par 100 cm³ de dichlorométhane et 10 cm³ d'eau ; la phase organique est décantée, séchée sur sulfate de magnésium, filtrée et concentrée à sec sous pression réduite (2,7 kPa) pour donner 0,46 g de 3-(cyclohex-2-ènyloxy)-5-méthyl-4-phényl-1H-pyrazole sous forme d'une gomme jaune [CCM : éluant : cyclohexane / acétate d'éthyle (70 / 30 en volumes)), Rf = 0,19]. Spectre de masse (IE) : m/z (254 M^{+.}), m/z 174 [(M-C6H8)⁺].

La 1-[3-(cyclohex-2-ènyloxy)-5-méthyl-4-phényl-pyrazol-1-yl]-éthanone peut-être préparée de la manière suivante :

A 100 cm³ de méthyléthylcétone à 20°C sous agitation, on ajoute 2 g de 1-(3-hydroxy-5-méthyl-4-phényl-pyrazol-1-yl)-émanone, 1,3 g de potassium carbonate et 1,06 cm³ de 3 bromo-cyclohexène. Après 5 heures de chauffage au reflux du solvant, le milieu réactionnel est refroidi à une température voisine de 20°C, puis concentré à sec sous pression réduite (2,7 kPa). Le résidu est repris par 100 cm³ d'eau et 100 cm³ de dichlorométhane ; la phase organique est décantée, séchée sur sulfate de magnésium, filtrée et concentrée à sec sous pression réduite (2,7 kPa). L'huile marron résultante (2,7 g) est purifiée par chromatographie-flash sur silice [éluant : cyclohexane / acétate d'éthyle (70 / 30 en volumes)]. Après concentration à sec des fractions sous pression réduite (2,7 kPa), on obtient 0,8 g de 1-[3-(cyclohex-2-ènyloxy)-5-méthyl-4-phényl-pyrazol-1-yl]-émanone sous forme d'une huile jaune [CCM : éluant : cyclohexane / acétate d'éthyle (70 / 30 en volumes), Rf = 0,74]. Spectre de masse (IE) : m/z 296 (M^{+.}), m/z 174 [(216-C2H2O)⁺.].

La 1-(3-hydroxy-5-méthyl-4-phényl-pyrazol-1-yl)-éthanone peut-être préparée de la manière suivante :

A une solution de 1,74 g de 5-méthyl-4-phényl-1H-pyrazol-3-ol (N° CAS : 64754-67-2) dans 17 cm³ de pyridine sous agitation et portée à 100°C, on ajoute 0,85 cm³ d'anhydride acétique. Après 30 minutes de chauffage à cette température, le milieu réactionnel est refroidi à une température voisine de 20°C, puis il est jeté dans 100 cm³ d'un mélange de glace et d'eau. La solution est extraite par 2 fois 50 cm³ d'acétate d'éthyle ; les phases organiques sont rassemblées, lavées par 2 fois 100 cm³ d'eau, séchées sur sulfate de magnésium, filtrées et évaporées à sec sous pression réduite (2,7 kPa) ; on obtient 2 g de 1-(3-hydroxy-5-méthyl-4 phényl-pyrazol-1-yl)-éthanone sous forme d'une huile jaune orangée. Spectre de masse (IE) : m/z 216 (M^{+.}), m/z 174 [(M-C2H2O)⁺].

### Exemple 29

### Trichlorhydrate de 4-(3-amino-phényl)-1-(2-diméthylamino-éthyl)-1H-pyrazol-3-ol

Un mélange de 860 mg de formiate d'ammonium et de 50 mg d'hydroxyde de palladium à 10 % dans 15 cm³ de méthanol est additionné d'une solution de 500 mg de {2-[3-benzyloxy-4-(3-nitro-phényl)-pyrazol-1-yl]-éthyl}-diméthyl-amine dans 15 cm³ de méthanol, puis il est chauffé 3 h au reflux du solvant sous agitation. Le milieu réactionnel est alors filtré sur supercel, et le filtrat est évaporé sous pression réduite (2,7 kPa). Le brut de réaction est repris par du dichlorométhane et le mélange résultant est lavé par successivement une solution aqueuse saturée d'hydrogénocarbonate, de l'eau et une solution aqueuse saturée de chlorure de sodium. Les phases aqueuses sont rassemblées et évaporées sous pression réduite (2,7 kPa). Le résidu obtenu est repris par du méthanol et la suspension est filtrée. Après évaporation du filtrat sous pression réduite (2,7 kPa), le solide résiduel est trituré dans de l'éthanol chlorhydrique 3 N. Le précipité formé est filtré et séché sous vide (2,7 kPa) pour donner 110 mg de trichlorhydrate de 4-(3-amino-phényl)-1-(2-diméthylamino-éthyl)-1H-pyrazol-3-ol sous forme d'un solide beige. Spectre IR (KBr) : 3432; 2839; 2689; 2586; 1627; 1603; 1523; 1462; 1178; 786 et 696 cm⁻¹. Spectre de R.M.N. ¹H (300 MHz, (CD₃)₂SO d6, δ en ppm) : 2,80 (s : 6H) ; 3,45 (t, J = 6,5 Hz : 2H) ; 4,41 (t, J = 6,5 Hz : 2H) ; 7,10 (d large, J = 8 Hz : 1H) ; 7,42 (t, J = 8 Hz : 1H) ; 7,58 (d large, J = 8 Hz : 1H) ; 7,67 (s large : 1H) ; 8,10 (s : 1H) ; de 9,50 à 10,40 (mf très étalé : 1H) ; 10,50 (mf : 1H) ; 10,73 (mf : 1H).

La{2-[3-benzyloxy-4-(3-nitro-phényl)-pyrazol-1-yl]-éthyl}-diméthyl-amine peut être préparée de la manière suivante :

A une suspension de 1,13 g d'hydrure de sodium (à 75% dans l'huile de vaseline) dans 50 cm³ de diméthylformamide sous atmosphère d'argon et sous agitation, est ajoutée une solution de 3,45 g de 3-benzyloxy-4-(3-nitro-phényl)-1H-pyrazole dans 50 cm³ de diméthylformamide. Après 30 min de chauffage à 50°C, le mélange est agité 1 h à une température voisine de 20°C, puis il est refroidi dans un bain de glace et additionné d'une solution de 4,5 g de bromhydrate de (2-bromo-éthyl)-diméthyl-amine dans 50 cm³ de diméthylformamide. Le milieu réactionnel est agité 15 h à une température voisine de 20°C, et 1,5 h à 50°C , puis il est refroidi à une température voisine de 20°C et jeté dans 400 cm³ d'eau. La phase aqueuse est extraite deux fois par de l'acétate d'éthyle. Les phases organiques sont réunies, lavées successivement par de l'eau et une solution aqueuse saturée de chlorure de sodium, séchées sur du sulfate de magnésium, filtrées et évaporées sous pression réduite (2,7 kPa) pour donner une huile orangée qui est purifiée par chromatographie-flash sur silice sous pression d'argon (50 kPa) [éluant : acétate d'éthyle, puis acétate d'éthyle / méthanol (90 / 10 en volumes)]. Après concentration des fractions sous pression réduite, on obtient 1,7 g de {2-[3-benzyloxy-4-(3-nitro-phényl)-pyrazol-1-yl]-éthyl}-diméthyl-amine sous forme d'une huile orangée. Spectre de masse (IE) : m/z 366 (M^{+.}), m/z 91 (C₇H₇⁺), m/z 71 (C₄H₉N⁺), m/z 58 (C₃H₈N⁺).

Le 3-benzyloxy-4-(3-nitro-phényl)-1H-pyrazole peut être préparé de la manière suivante :

A une suspension de 4,1 g de 1-[3-hydroxy-4-(3-nitro-phényl)-pyrazol-1-yl]-éthanone dans 50 cm³ de méthyléthylcétone sous agitation, sont ajoutés 2,75 g de carbonate de potassium et 2,2 cm³ de bromure de benzyle. Le mélange est chauffé au reflux du solvant pendant 2,5 h, refroidi à une température voisine de 20°C, et il est filtré. Le filtrat est évaporé sous pression réduite (2,7 kPa) et le résidu est repris par 25 cm³ de tétrahydrofurane et 25 cm³ de méthanol, puis il est additionné de 1 cm³ d'hydroxyde de sodium 10 N. Après 30 min d'agitation à une température voisine de 20°C, le milieu réactionnel est évaporé sous pression réduite (2,7 kPa). Le brut de réaction est repris dans du dichlorométhane. La phase organique est lavée successivement par de l'eau et une solution aqueuse saturée de chlorure de sodium, séchée sur du sulfate de magnésium, filtrée et évaporée sous pression réduite (2,7 kPa) pour donner une huile qui est triturée dans de l'oxyde de diisopropyle. Le précipité formé est filtré et séché sous vide (2,7 kPa) pour donner 3,47 g de 3-benzyloxy-4-(3-nitro-phényl)-1H-pyrazole sous forme d'un solide jaune. Spectre de R.M.N. ¹H (300 MHz, (CD₃)₂SO d6, δ en ppm) : 5,40 (s : 2H) ; 7,36 (t large, J = 7,5 Hz : 1H) ; 7,44 (t large, J = 7,5 Hz : 2H) ; 7,55 (d large, J = 7,5 Hz : 2H) ; 7,64 (t, J = 8 Hz : 1H) ; 8,00 (dd, J = 8 et 2 Hz : 1H) ; 8,13 (d large, J = 8 Hz : 1H) ; 8,33 (s : 1H) ; 8,62 (t, J = 2 Hz : 1H) ; de 12,00 à 12,80 (mf très étalé : 1H).

La 1-[3-hydroxy-4-(3-nitro-phényl)-pyrazol-1-yl]-éthanone peut être préparée de la manière suivante :

Une solution de 3,8 g de 4-(3-nitro-phényl)-1H-pyrazol-3-olate de diméthyl-ammonium dans 40 cm³ de pyridine sous atmosphère d'argon et sous agitation est chauffée à 90°C, puis elle est additionnée goutte à goutte de 1,5 cm³ d'anhydride acétique. Après 1 h de chauffage à 90°C, le milieu réactionnel est refroidi à une température voisine de 20°C, et il est jeté dans 100 cm³ d'eau glacée. Le précipité formé est filtré, lavé trois fois avec de l'eau et il est séché sous vide (2,7 kPa) pour donner 4,33 g d'un solide qui est remis en réaction avec 40 cm³ de pyridine et 0,39 cm³ d'anhydride acétique suivant le protocole énoncé ci-dessus. On obtient 4,1 g de 1-[3-hydroxy-4-(3-nitro-phényl)-pyrazol-1-yl]-éthanone sous la forme d'un solide jaune pâle. Spectre IR (KBr) : 3118; 3082; 2669; 1730; 1604; 1520; 1390; 1349; 1256; 1223; 1101; 748 et 719 cm⁻¹.

Le 4-(3-nitro-phényl)-1H-pyrazol-3-olate de diméthyl-ammonium peut être préparé de la manière suivante :

Une solution sous agitation de 9,3 g d'ester benzylique de l'acide 3-diméthylamino-2-(3-nitro-phényl)-acrylique et de 1,4 cm³ de monohydrate d'hydrazine dans 100 cm³ d'éthanol est chauffée 3 h au reflux du solvant, puis refroidie dans un bain de glace. Le solide formé est filtré, lavé avec de l'eau, et il est séché sous vide (2,7 kPa) pour donner 4,44 g de 4-(3-nitro-phényl)-1H-pyrazol-3-olate de dimémyl-ammonium sous forme d'un solide orangé. Spectre IR (KBr) : 3346; 3199; 3071; 2855; 2685; 2386; 1583;1538; 1469; 1350; 934; 766; 747 et 681 cm⁻¹.

L'ester benzylique de l'acide 3-diméthylamino-2-(3-nitro-phényl)-acrylique peut être préparé de la manière suivante :

Une solution de 10 g d'ester benzylique d'acide 2-(3-nitro-phényl)-acrylique dans 100 cm³ de tétrahydrofurane est additionnée de 11,5 cm³ de C-tert-butoxy-N,N,N',N'-tétraméthyl-méthanediamine, et chauffée 15 h au reflux du solvant. Après refroidissement à une température voisine de 20°C, le mélange réactionnel est évaporé à sec sous pression réduite (2,7 kPa). Le résidu est repris par de l'acétate d'éthyle, et la solution organique est lavée par 3 fois de l'eau, puis une solution aqueuse saturée de chlorure de sodium, séchée sur sulfate de magnésium, filtrée et évaporée sous pression réduite (2,7 kPa). L'huile brune obtenue est purifiée par chromatographie-flash sur silice sous pression d'argon (50 kPa) [éluant : cyclohexane / acétate d'éthyle (70 / 30 en volumes)]. Après concentration des fractions sous pression réduite, on obtient 9,3 g d'ester benzylique de l'acide 3-diméthylamino-2-(3-nitro-phényl)-acrylique sous forme d'une huile orangée. Spectre de masse (IE) : m/z 326 (M^{+.}), m/z 235 [(M - C₇H₇)⁺], m/z 91 (C₇H₇⁺).

### Exemple 30

### Dichlorhydrate de N-{3-[3-hydroxy-1-(2-diméthylamino-éthyl)-1H-pyrazol-4-yl]-phényl}-acétamide

Une solution de 400 mg de N-{3-[3-benzyloxy-1-(2-diméthylamino-éthyl)-1H-pyrazol-4-yl]-phényl}-acétamide dans 20 cm³ d'éthanol est additionnée de 1,8 cm³ d'éther diéthylique chlorhydrique 3 N. Après 15 min d'agitation à une température voisine de 20°C, la solution est évaporée à sec sous pression réduite (2,7 kPa). Le résidu est repris par 20 cm³ d'éthanol . La solution obtenue est introduite dans un autoclave, et elle est additionnée de 50 mg de palladium sur charbon à 10 %, puis elle est placée sous hydrogène (5 bars). Après 2 h d'agitation à une température voisine de 20°C, le milieu réactionnel est filtré sur supercel et le filtrat est évaporé. L'huile jaune obtenue (440 mg) est dissoute dans 20 cm³ d'éthanol et remise en réaction avec 50 mg de palladium sur charbon à 10 %, sous hydrogène (5 bars), à 40°C et sous agitation pendant 4 h. Le milieu réactionnel est ensuite filtré sur supercel, le filtrat est évaporé, et le résidu est trituré dans de l'éther diisopropylique. Le précipité formé est filtré, séché sous vide (2,7 kPa) pour donner 289 mg de dichlorhydrate de N-{3-[3-hydroxy-1-(2-diméthylamino-éthyl)-1H-pyrazol-4-yl]-phényl}-acétamide sous forme d'un solide jaune pâle. Spectre IR (KBr): 3242; 3130; 2967; 2573; 2464; 1678; 1614; 1588; 1525; 1462; 1258; 1187; 787 et 690 cm⁻¹. Spectre de R.M.N. ¹H (300 MHz, (CD₃)₂SO d6, δ en ppm) : 2,05 (s : 3H) ; 2,80 (s large : 6H) ; 3,48 (t large, J = 6,5 Hz : 2H) ; 4,35 (t large, J = 6,5 Hz : 2H) ; 7,23 (t, J = 7,5 Hz : 1H) ; 7,31 (d large, J = 7,5 Hz : 1H) ; 7,38 (d large, J = 7,5 Hz : 1H) ; 7,89 (s large : 1H) ; 7,95 (s : 1H) ; de 9,60 à 9,85 (mf étalé : 1H) ; 9,91 (s large : 1H) ; 10,45 (s large : 1H).

Le N-{3-[3-benzyloxy-1-(2-diméthylamino-éthyl)-1H-pyrazol-4-yl]-phényl}-acétamide peut être préparé de la manière suivante : A une solution de 500 mg de 3-[3-benzyloxy-1-(2-diméthylamino-éthyl)-1H pyrazol-4-yl]-phénylamine et de 0,418 cm³ de triéthylamine dans 20 cm³ de dichlorométhane sous atmosphère d'argon et sous agitation, on ajoute 0,116 cm³ de chlorure d'acétyle en maintenant la température du milieu à 5°C. Après 15 h d'agitation à une température voisine de 20°C, 0,1 cm³ de triéthylamine et 0,1 cm³ de chlorure d'acétyle supplémentaires sont additionnés au mélange réactionnel et la réaction est poursuivie pendant 2 h. Le milieu réactionnel est alors lavé successivement par deux fois de l'eau et une solution aqueuse saturée de chlorure de sodium, séché sur du sulfate de magnésium, filtré et évaporé sous pression réduite (2,7 kPa) pour donner 540 mg de N-{3-[3-benzyloxy-1-(2-diméthylamino-éthyl)-1H-pyrazol-4-yl]-phényl}-acétamide sous forme d'huile jaune. Spectre IR (CCl₄) : 3444; 3305; 2945; 2822; 2773; 1670; 1614; 1588; 1549; 1502; 1452; 1423; 1357; 1177; 1018; 695 et 537 cm⁻¹.

La 3-[3-benzyloxy-1-(2-diméthylamino-éthyl)-1H-pyrazol-4-yl]-phénylamine peut être préparée de la manière suivante :

A un mélange sous agitation de 840 mg de poudre fer, 200 mg de chlorure d'ammonium dans 15 cm³ d'éthanol et 15 cm³ d'eau, chauffé au reflux du solvant, est additionnée une solution de 1,1 g de {2-[3-benzyloxy-4-(3-nitro-phényl)-pyrazol-1-yl]-éthyl}-diméthyl-amine dans 15 cm³ d'éthanol. L'agitation est poursuivie 3 h au reflux du solvant, puis 15 h à une température voisine de 20°C. Le mélange réactionnel est filtré et le filtrat est évaporé. Le résidu est repris par un mélange d'acétate d'éthyle, d'eau et de soude 1 N. La phase organique est décantée, lavée successivement par de l'eau et une solution aqueuse saturée de chlorure de sodium, séchée sur du sulfate de magnésium, filtrée et évaporée sous pression réduite (2,7 kPa) pour donner 1 g de 3-[3-benzyloxy-1-(2-diméthylamino-éthyl)-1H-pyrazol-4-yl]-phénylamine sous forme d'huile orangée. Spectre de masse (IE) : m/z 336 (M^{+.}), m/z 265 [(M - C₇H₇)⁺], m/z 91(C₇H₇⁺), m/z 71(C₄H₉N⁺), m/z 58 (C₃H₈N⁺).

### Exemple 31

### Dichlorhydrate de 4-(4-amino-phényl)-1-(2-diméthylamino-éthyl)-1H-pyrazol-3-ol

Une solution de 250 mg de {2-[3-benzyloxy-4-(4-nitro-phényl)-pyrazol-1-yl]-éthyl}-diméthyl-amine dans 20 cm³ d'éthanol est additionnée de 1,2 cm³ d'éther diéthylique chlorhydrique 3 N. Après 20 min d'agitation à une température voisine de 20°C, la solution est évaporée à sec sous pression réduite (2,7 kPa) Le résidu est repris par 20 cm³ d'éthanol. La solution obtenue est introduite dans un autoclave, et elle est additionnée de 36 mg de palladium sur charbon à 10 %, puis elle est placée sous hydrogène (7 bars). Après 5 h d'agitation à 40°C, le milieu réactionnel est filtré sur supercel, le filtrat est évaporé et le résidu est trituré dans de l'éther diisopropylique. Le précipité formé est filtré, séché sous vide (2,7 kPa) pour donner 169 mg de dichlorhydrate de 4-(4-ambo-phényl)-1-(2-diméthylamino-éthyl)-1H-pyrazol-3-ol sous forme d'un solide jaune. Spectre IR (KBr) : 3372; 3296; 3205; 3025; 1627; 1592, 1522; 1514; 1451; 1280; 1177; 828; 612 et 525 cm⁻¹. Spectre de R.M.N. 1H (300 MHz, (CD₃)₂SO d6, δ en ppm) : 2,75 (s : 6H) ; 3,42 (mt : 2H) ; 4,27 (t, J = 6 Hz : 2H) ; de 4,70 à 5,30 (mf étalé : 2H) ; 6,55 (d, J = 8,5 Hz : 2H) ; 7,31 (d, J = 8,5 Hz : 2H) ; 7,75 (s : 1H) ; 10,08 (mf : 1H).

La {2-[3-benzyloxy-4-(4-nitro-phényl)-pyrazol-1-yl]-éthyl}-diméthyl-amine peut être préparée de la manière suivante :

A une suspension de 980 mg d'hydrure de sodium (à 75% dans l'huile de vaseline) dans 50 cm³ de diméthylformamide sous atmosphère d'argon et sous agitation, est ajoutée une solution de 3 g de 3-benzyloxy-4-(4-nitro-phényl)-1H-pyrazole dans 50 cm³ de diméthylformamide. Après 30 min de chauffage à 50°C, le mélange est agité 1 h à une température voisine de 20°C, puis il est refroidi dans un bain de glace et additionné d'une solution de 4,7 g de bromhydrate de (2-bromo-éthyl)-diméthyl-amine dans 50 cm³ de diméthylformamide. Le milieu réactionnel est agité 15 h à une température voisine de 20°C, puis il est jeté dans 400 cm³ d'eau. La phase aqueuse est extraite deux fois par de l'acétate d'éthyle. Les phases organiques sont réunies, lavées successivement par deux fois de l'eau et une solution aqueuse saturée de chlorure de sodium, séchées sur du sulfate de magnésium, filtrées et évaporées sous pression réduite (2,7 kPa) pour donner une huile orangée qui est purifiée par chromatographie-flash sur silice sous pression d'argon (50 kPa) [éluant : acétate d'éthyle, puis acétate d'éthyle / méthanol (90 / 10 en volumes)]. Après concentration des fractions sous pression réduite, on obtient 1,2 g de {2-[3-benzyloxy-4-(4-nitro-phényl)-pyrazol-1-yl]-éthyl}-diméthyl-amine sous forme d'une huile brune. Spectre de masse (IE) : m/z 366 (M^{+.}), m/z 91 (C₇H₇⁺), m/z 71 (C₄H₉N⁺), m/z 58 (C₃H₈N⁺).

Le 3-benzyloxy-4-(4-nitro-phényl)-1H-pyrazole peut être préparé de la manière suivante :

A une suspension de 4,5 g de 1-[3-hydroxy-4-(4-nitro-phényl)-pyrazol-1-yl]-éthanone dans 50 cm³ de méthyléthylcétone sous agitation, sont ajoutés 3 g de carbonate de potassium, et 2,2 cm³ de bromure de benzyle. Le mélange est chauffé au reflux du solvant pendant 2,5 h, refroidi à une température voisine de 20°C, et il est filtré. Le filtrat est évaporé sous pression réduite (2,7 kPa) et le résidu est repris par 25 cm³ de tétrahydrofurane et 25 cm³ de méthanol, puis il est additionné de 2 cm³ d'hydroxyde de sodium 10 N. Après 30 min d'agitation à une température voisine de 20°C, le milieu réactionnel est évaporé sous pression réduite (2,7 kPa). Le brut de réaction est repris dans du dichlorométhane. La phase organique est lavée successivement par de l'eau et une solution aqueuse saturée de chlorure de sodium, séchée sur du sulfate de magnésium, filtrée et évaporée sous pression réduite (2,7 kPa) pour donner une huile qui est triturée dans de l'oxyde de diisopropyle. Le précipité formé est filtré et séché sous vide (2,7 kPa) pour donner 3 g de 3-benzyloxy-4-(4-nitro-phényl)-1H-pyrazole sous forme d'un solide ocre. Spectre de R.M.N. ¹H (300 MHz, (CD₃)₂SO d6, δ en ppm) : 5,41 (s : 2H) ; de 7,30 à 7,60 (mt : 5H) ; 7,97 (d, J = 9 Hz : 2H) ; 8,23 (d, J = 9 Hz : 2H) ; 8,36 (s : 1H) ; 12,49 (mf : 1H).

La 1-[3-hydroxy-4-(4-nitro-phényl)-pyrazol-1-yl]-éthanone peut être préparée de la manière suivante :

Une solution de 4,85 g de 4-(4-nitro-phényl)-1H-pyrazol-3-olate de diméthyl-ammonium dans 40 cm³ de pyridine sous atmosphère d'argon et sous agitation est chauffée à 90°C, puis elle est additionnée goutte à goutte de 2 cm³ d'anhydride acétique. Après 1 h de chauffage à 90°C, le milieu réactionnel est refroidi à une température voisine de 20°C, et il est jeté dans 100 cm³ d'eau glacée. Le précipité formé est filtré, lavé trois fois avec de l'eau et il est séché sous vide (2,7 kPa) pour donner 4,5 g de 1-[3-hydroxy-4-(4-nitro-phényl)-pyrazol-1-yl]-éthanone sous la forme d'un solide jaune. Spectre IR (KBr) : 3370; 3128; 2980; 2587; 1721; 1615; 1600; 1509; 1341; 1224; 1111; 855; 757 et 643 cm⁻¹.

Le 4-(4-nitro-phényl)-1H-pyrazol-3-olate de diméthyl-ammonium peut être préparé de la manière suivante :

Une solution sous agitation de 10,7 g d'ester méthylique de l'acide 3-diméthylamino-2-(4-nitro-phényl)-acrylique et de 2,1 cm³ de monohydrate d'hydrazine dans 120 cm³ d'éthanol est chauffée 3 h au reflux du solvant, et refroidie dans un bain de glace. Le solide formé est filtré, rinçé avec de l'éther diisopropylique, et il est séché sous vide (2,7 kPa) pour donner 5 g de 4-(4-nitro-phényl)-1H-pyrazol-3-olate de diméthyl-ammonium sous forme d'un solide orangé. Spectre IR (KBr) : 3188; 3089; 2909; 2728; 2423; 1603; 1589; 1567; 1538; 1501; 1345; 1330; 1212; 1112; 923; 880; 761 et 581 cm⁻¹.

L'ester méthylique de l'acide 3-diméthylamino-2-(4-nitro-phényl)-acrylique peut être préparé de la manière suivante :

Une solution de 10,5 g d'ester méthylique de l'acide 2-(4-nitro-phényl)-acrylique dans 100 cm³ de tétrahydrofurane est additionnée de 16,6 cm³ de C-tert-butoxy-N,N,N',N'-tétraméthyl-méthanediamine, et chauffée 2,5 h au reflux du solvant. Après 15 h d'agitation à une température voisine de 20°C, le mélange réactionnel est évaporé à sec sous pression réduite (2,7 kPa). Le résidu est repris par de l'acétate d'éthyle, et la solution organique est lavée par 3 fois de l'eau, puis séchée sur sulfate de magnésium, filtrée et évaporée sous pression réduite (2,7 kPa) pour donner 10,7 g d'ester méthylique de l'acide 3-diméthylamino-2-(4-nitro-phényl)-acrylique sous forme d'une huile brune. Spectre IR (CCl₄) : 2949; 1693; 1603; 1519; 1433; 1344; 1219; 1095; 1048 et 855 cm⁻¹.

### Exemple 32

### Dichlorhydrate de 1-(2-diméthylamino-éthyl)-4-(4'-fluoro-biphényl-3-yl)-1H-pyrazol-3-ol

Une solution de 300 mg de {2-[3-benzyloxy-4-(4'-fluoro-biphényl-3-yl)-pyrazol-1-yl]-éthyl}-diméthyl-amine dans 20 cm³ d'éthanol est additionnée de 1,2 cm³ d'éther diéthylique chlorhydrique 3 N. Après 30 min d'agitation à une température voisine de 20°C, la solution est évaporée à sec sous pression réduite (2,7 kPa). Le résidu est repris par 20 cm³ d'éthanol. La solution obtenue est introduite dans un autoclave, et elle est additionnée de 14 mg de palladium sur charbon à 10 %, puis elle est placée sous hydrogène (7 bars). Après 5 h d'agitation à 30°C, le milieu réactionnel est filtré sur supercel, et le filtrat est évaporé. Le résidu est additionné d'éther diisopropylique conduisant à une suspension qui est chauffée au reflux du solvant et filtrée à chaud. Le solide résultant est séché sous vide (2,7 kPa) pour donner 84 mg de dichlorhydrate de 1-(2-diméthylamino-éthyl)-4-(4'-fluoro-biphényl-3-yl)-1H-pyrazol-3-ol sous forme d'une poudre blanche. Spectre IR (KBr) : 3049; 2962; 2682; 2355; 1608; 1514; 1460; 1221; 1184; 1162; 843; 804; 703 et 560 cm⁻¹. Spectre de R.M.N. ¹H (300 MHz, (CD3)2SO d6, δ en ppm) : 2,76 (s large: 6H) ; 3,45 (mt : 2H) ; 4,32 (t large, J = 6 Hz : 2H) ; 7,32 (t, J = 8,5 Hz : 2H) ; de 7,35 à 7,50 (mt : 2H) ; 7,66 (mt : 1H) ; 7,70 (dd, J = 9 et 6 Hz : 2H) ; 7,90 (s large : 1H) ; 8,14 (s : 1H) ; 9,76 (mf : 1H) ; 10,50 (s large : 1H).

La {2-[3-benzyloxy-4-(4'-fluoro-biphényl-3-yl)-pyrazol-1-yl]-éthyl}-diméthyl-amine peut être préparée de la manière suivante :

A une solution agitée de 620 mg de {2-[3-benzyloxy-4-(3-bromo-phényl)-pyrazol-1-yl]-éthyl}-diméthyl-amine dans 25 cm³ de toluène sous atmosphère d'argon, on ajoute 860 mg d'acide 4-fluoro-phényl boronique, 1,3 g de phosphate de potassium et 330 mg de chlorure de bis(triphénylphosphine)palladium. Après 15 h de chauffage au reflux du solvant, le milieu réactionnel est refroidi à une température voisine de 20°C, et additionné d'acétate d'éthyle et d'eau, puis il est filtré sur supercel. Le filtrat est décanté, puis la phase organique est lavée successivement par de l'hydroxyde de sodium 0,5 N, de l'eau et une solution aqueuse saturée de chlorure de sodium ; elle est séchée sur sulfate de magnésium, filtrée et évaporée sous pression réduite (2,7 kPa). L'huile brune obtenue (1,3 g) est purifiée par chromatographie-flash sur alumine CTB1 sous pression d'argon (50 kPa) [éluant : cyclohexane / acétate d'éthyle (80 / 20 en volumes)]. Après concentration des fractions sous pression réduite, on obtient 300 mg de {2-[3-benzyloxy-4-(4'-fluoro-biphényl-3-yl)-pyrazol-1-yl]-éthyl}-diméthyl-amine sous forme d'une huile jaune. Spectre IR (CCl4) : 2823; 2773; 1610; 1571; 1515; 1462; 1358; 1235; 1158; 1014; 837; 696 et 559 cm⁻¹.

La {2-[3-benzyloxy-4-(3-bromo-phényl)-pyrazol-1-yl]-éthyl}-diméthyl-amine peut être préparée de la manière suivante :

A une suspension de 2,25 g d'hydrure de sodium (à 75% dans l'huile de vaseline) dans 70 cm³ de diméthylformamide sous atmosphère d'argon et sous agitation, est ajoutée une solution de 7,67 g de 3-benzyloxy-4-(3-bromo-phényl)-1H-pyrazole dans 70 cm³ de diméthylformamide. Après 30 min de chauffage à 50°C, le mélange est agité 1 h à une température voisine de 20°C, puis il est refroidi dans un bain de glace et additionné d'une solution de 10,85 g de bromhydrate de (2-bromo-éthyl)-diméthyl-amine dans 100 cm³ de diméthylformamide. Le milieu réactionnel est agité 15 h à une température voisine de 20°C, et 3 h à 50°C , puis il est refroidi à une température voisine de 20°C et jeté dans 500 cm³ d'eau. La phase aqueuse est extraite deux fois par de l'acétate d'éthyle. Les phases organiques sont réunies, lavées successivement par deux fois de l'eau et une solution aqueuse saturée de chlorure de sodium, séchées sur du sulfate de magnésium, filtrées et évaporées sous pression réduite (2,7 kPa) pour donner une huile orangée qui est purifiée par chromatographie-flash sur silice sous pression d'argon (50 kPa) [éluant : acétate d'éthyle, acétate d'éthyle / méthanol (90 / 10 en volumes)]. Après concentration des fractions sous pression réduite, on obtient 2,4 g de {2-[3-benzyloxy-4-(3-bromo-phényl)-pyrazol-1-yl]-éthyl}-diméthyl-amine sous forme d'une huile orangée. Spectre de masse (IC) : m/z 400 (MH⁺), m/z 322 [(M - Br + 2H)⁺].

Le 3-benzyloxy-4-(3-bromo-phényl)-1H-pyrazole peut être préparé de la manière suivante :

A une suspension de 4,4 g de 1-[3-benzyloxy-4-(3-bromo-phényl)-pyrazol-1-yl]-éthanone dans 50 cm³ de méthyléthylcétone sous agitation, sont ajoutés 2,6 g de carbonate de potassium et 2,05 cm³ de bromure de benzyle. Le mélange est chauffé au reflux du solvant pendant 2,5 h, refroidi à une température voisine de 20°C, et il est filtré. Le filtrat est évaporé sous pression réduite (2,7 kPa), et le résidu est repris par 25 cm³ tétrahydrofurane et 25 cm³ de méthanol, puis il est additionné de 1 cm³ d'hydroxyde de sodium 10 N. Après 30 min d'agitation à une température voisine de 20°C, le milieu réactionnel est évaporé sous pression réduite (2,7 kPa). Le brut de réaction est repris dans du dichlorométhane. La phase organique est lavée successivement par deux fois de l'eau et une solution aqueuse saturée de chlorure de sodium, séchée sur sulfate de magnésium, filtrée et évaporée sous pression réduite (2,7 kPa) pour donner une huile qui est purifiée par chromatographie-flash sur silice sous pression d'argon (50 kPa) [éluant : cyclohexane / acétate d'éthyle (80 / 20 en volumes)]. Après concentration des fractions sous pression réduite, on obtient 3,3 g de 3-benzyloxy-4-(3-bromo-phényl)-1H-pyrazole sous forme d'un solide crème. Spectre de R.M.N. ¹H (300 MHz, (CD₃)₂SO d6, δ en ppm) : 5,35 (s : 2H) ; de 7,25 à 7,40 (mt : 3H) ; 7,42 (t large, J = 7,5 Hz : 2H) ; 7,51 (d large, J = 7,5 Hz : 2H) ; 7,71 (dt, J = 7,5 et 2 Hz : 1H) ; 7,93 (s large : 1H) ; 8,18 (s : 1H) ; 12,25 (mf : 1H).

La 1-[4-(3-bromo-phényl)-3-hydroxy-pyrazol-1-yl]-éthanone peut être préparée de la manière suivante :

Une solution de 4,3 g de 4-(3-bromo-phényl)-1H-pyrazol-3-ol dans 40 cm³ de pyridine sous atmosphère d'argon et sous agitation est chauffée à 90°C, puis elle est additionnée goutte à goutte de 1,6 cm³ d'anhydride acétique. Après 1 h de chauffage à 90°C, le milieu réactionnel est refroidi à une température voisine de 20°C et il est jeté dans 100 cm³ d'eau glacée. Le précipité formé est filtré, lavé trois fois avec de l'eau et il est séché sous vide (2,7 kPa) pour donner 4,42 g de 1-[4-(3-bromo-phényl)-3-hydroxy-pyrazol-1-yl]-éthanone sous forme d'un solide crème. Spectre IR (KBr) : 3125; 2687; 2577; 1729; 1616; 1529; 1391; 1318; 1256; 1219; 945; 791; 715 et 629 cm⁻¹.

Le 4-(3-bromo-phényl)-1H-pyrazol-3-ol peut être préparé de la manière suivante :

Une solution sous agitation de 12,22 g d'ester méthylique de l'acide 2-(3-bromo-phényl)-3-diméthylamino-acrylique et de 2,1 cm³ de monohydrate d'hydrazine dans 100 cm³ d'éthanol est chauffée 3 h au reflux du solvant. Le mélange réactionnel est évaporé à sec sous pression réduite (2,7 kPa) et le résidu est trituré dans de l'éther diisopropylique. Le solide formé est filtré et il est séché sous vide (2,7 kPa) pour donner 5,1 g de 4-(3-bromo-phényl)-1H-pyrazol-3-olate de diméthyl-ammonium sous forme d'un solide crème. Le filtrat est évaporé sous pression réduite (2,7 kPa), le résidu est trituré dans de l'éther diisopropylique, et le solide formé est filtré, et il est séché sous vide (2,7 kPa) conduisant à 4,3 g de 4-(3-bromo-phényl)-1H-pyrazol-3-ol sous forme d'un solide crème. Spectre IR (KBr) : 3099; 2768; 2668; 1620; 1590; 1410; 1241; 1081; 787; 712 et 689 cm⁻¹.

L'ester méthylique de l'acide 2-(3-bromo-phényl)-3-diméthylamino-acrylique peut être préparé de la manière suivante :

Une solution de 10,5 g d'ester méthylique de l'acide 2-(3-bromo-phényl)-acrylique dans 100 cm³ de tétrahydrofurane est additionnée de 14,4 cm³ de C-tert-butoxy-N,N,N' ,N'-tétraméthyl-méthanediamine et chauffée 2,5 h au reflux du solvant-Après 15 h d'agitation à une température voisine de 20°C, le mélange réactionnel est évaporé à sec sous pression réduite (2,7 kPa). Le résidu est repris par de l'acétate d'éthyle, et la solution organique est lavée par 3 fois de l'eau, puis séchée sur du sulfate de magnésium, filtrée et évaporée sous pression réduite (2,7 kPa) pour donner 12,22 g d'ester méthylique de l'acide 2-(3-bromo-phényl)-3-diméthylamino-acrylique sous forme d'une huile jaune. Spectre IR (CCl₄) : 2947 ; 2813; 1691; 1603; 1432; 1285; 1221; 1098 et 694 cm⁻¹.

### Exemple 33

### Dichlorhydrate de 4-biphényl-3-yl-1-(2-diméthylamino-éthyl)-1H-pyrazol-3-ol

Une solution de 121 mg de {2-[3-benzyloxy-4-biphényl-3-yl-pyrazol-1-yl]-éthyl}-diméthyl-amine dans 20 cm³ d'éthanol est additionnée de 1 cm³ d'éther diéthylique chlorhydrique 3 N. Après 30 min d'agitation à une température voisine de 20°C, la solution est évaporée à sec sous pression réduite (2,7 kPa). Le résidu est repris par 20 cm³ d'éthanol. La solution obtenue est introduite dans un autoclave, et elle est additionnée de 11 mg de palladium sur charbon à 10 %, puis elle est placée sous hydrogène (7 bars). Après 5 h d'agitation à 30°C, le milieu réactionnel est filtré sur supercel, et le filtrat est évaporé. Le résidu est additionné d'éther diisopropylique conduisant à une suspension qui est chauffée au reflux du solvant et filtrée à chaud - Le solide résultant est séché sous vide (2,7 kPa) pour donner 69 mg de dichlorhydrate de 4-biphényl-3-yl-1-(2-diméthylamino-éthyl)-1H-pyrazol-3-ol sous forme d'une poudre blanche. Spectre IR (KBr) : 3054; 2959; 2685; 2299; 1606 ; 1522; 1457; 1298; 1182; 760; 698 et 671 cm⁻¹. Spectre de R.M.N. ¹H (300 MHz, (CD₃)₂SO d6, δ en ppm) : 2,79 (mf : 6H) ; 3,43 (mf : 2H) ; 4,31 (mf : 2H) ; de 7,25 à 7,55 (mt : 5H) ; 7,68 (mt : 3H) ; 7,94 (s large : 1H) ; 8,15 (s : 1H) ; de 9,45 à 9,65 (mf étalé : 1H) ; 10,49 (s large : 1H).

La {2-[3-benzyloxy-4-biphényl-3-yl-pyrazol-1-yl]-éthyl}-diméthyl-amine peut être préparée de la manière suivante :

A une solution agitée de 550 mg de {2-[3-benzyloxy-4-(3-bromo-phényl)-pyrazol-1-yl]-éthyl}-diméthyl-amine dans 25 cm³ de toluène sous atmosphère d'argon, sont additionnés 503 mg d'acide phényl boronique, 891 mg de phosphate de potassium, et 217 mg de chlorure de bis(triphénylphosphine)palladium. Après 15 h de chauffage au reflux du solvant, on ajoute 168 mg d'acide phényl boronique, 297 mg de phosphate de potassium, et 148 mg de chlorure de bis(triphénylphosphine)palladium au milieu réactionnel, et la réaction est poursuivie à la même température pendant 15 h. Le mélange est ensuite refroidi à une température voisine de 20°C, il est additionné d'acétate d'éthyle et d'eau, et il est filtré sur supercel. Le filtrat est décanté, puis la phase organique est lavée successivement par de l'hydroxyde de sodium 0,5 N, de l'eau et une solution aqueuse saturée de chlorure de sodium ; elle est séchée sur sulfate de magnésium, filtrée et évaporée sous pression réduite (2,7 kPa). L'huile brune obtenue (1,2 g) est purifiée par chromatographie-flash sur alumine CTB1 sous pression d'argon (50 kPa) [éluant : cyclohexane / acétate d'éthyle (80 / 20 en volumes)]. Après concentration des fractions sous pression réduite, on obtient 300 mg d'une huile orangée qui est remise en réaction pendant 15 h avec 25 cm³ de toluène, 503 mg d'acide phényl boronique, 891 mg de phosphate de potassium, et 217 mg de chlorure de bis(triphénylphosphine)palladium selon le protocole décrit ci-dessus. On obtient une huile brune (800 mg) qui est purifiée par chromatographie-flash sur alumine CTB1 sous pression d'argon (50 kPa) [éluant : cyclohexane / acétate d'éthyle (80 / 20 en volumes)]. Après concentration des fractions sous pression réduite (2,7 kPa), on obtient 120 mg de {2-[3-benzyloxy-4-biphényl-3-yl-pyrazol-1-yl]-éthyl}-diméthyl-amine sous forme d'une huile jaune. Spectre IR (CCl₄) : 3065; 3033; 2823; 2774; 1609; 1579; 1505; 1450; 1240 et 699 cm⁻¹.

### Exemple 34

### Dichlorhydrate de 1-(2-diméthylamino-éthyl)-4-(4'-fluoro-biphényl-4-yl)-1H-pyrazol-3-ol

Une solution de 200 mg de {2-[3-benzyloxy-4-(4'-fluoro-biphényl-4-yl)-pyrazol-1-yl]-éthyl}-diméthyl-amine dans 20 cm³ d'éthanol est additionnée de 0,06 cm³ d'acide chlorhydrique 12 N. Le mélange est introduit dans un autoclave, il est additionnée de 28 mg de palladium sur charbon à 10 %, puis il est placé sous hydrogène (5 bars). Après 4 h d'agitation à 40°C, le milieu réactionnel est filtré sur supercel, et le filtrat est évaporé. Le résidu est trituré dans de l'éther diisopropylique. Le solide obtenu, dissous dans 20 cm³ d'éthanol, est remis en réaction dans un autoclave avec 10 mg de palladium sur charbon à 10 % et sous hydrogène (7 bars). Après 5 h d'agitation à 35 °C, le mélange réactionnel est filtré sur supercel, et le filtrat est évaporé. Le résidu est trituré dans de l'éther diisopropylique, filtré et séché sous vide (2,7 kPa) pour donner 77 mg de dichlorhydrate de 1-(2-diméthylamino-éthyl)-4-(4'-fluoro-biphényl-4-yl)-1H-pyrazol-3-ol sous forme d'un solide beige. Spectre IR (KBr) : 2964; 2676; 2468; 1611; 1585; 1528; 1514; 1493; 1460; 1234; 1161; 826; 810 et 511 cm⁻¹. Spectre de R.M.N. ¹H (300 MHz, (CD₃)₂SO d6, δ en ppm) : 2,79 (s large : 6H) ; 3,48 (mf : 2H) ; 4,36 (t large, J = 6,5 Hz : 2H) ; 7,30 (t large, J = 9 Hz : 2H) ; 7,65 (d large, J = 8 Hz : 2H) ; de 7,70 à 7,80 (mt : 4H) ; 8,09 (s : 1H) ; 9,92 (mf : 1H) ; 10,52 (s large : 1H).

La {2-[3-benzyloxy-4-(4'-fluoro-biphényl-4-yl)-pyrazol-1-yl]-éthyl}-diméthyl-amine peut être préparée de la manière suivante

A une solution agitée de 500 mg de {2-[3-benzyloxy-4-(4-bromo-phényl)-pyrazol-1-yl]-éthyl}-diméthyl-amine dans 25 cm³ de toluène sous atmosphère d'argon, sont additionnés 770 mg d'acide 4-fluoro-phényl boronique, 1,19 g de phosphate de potassium, et 290 mg de chlorure de bis(triphénylphosphine)palladium. Après 15 h de chauffage au reflux du solvant, le mélange réactionnel est refroidi à une température voisine de 20°C, il est additionné d'acétate d'éthyle et d'eau, et il est filtré sur supercel. Le filtrat est décanté, puis la phase organique est lavée successivement par de l'hydroxyde de sodium 0,5 N, de l'eau et une solution aqueuse saturée de chlorure de sodium ; elle est séchée sur sulfate de magnésium, filtrée et évaporée sous pression réduite (2,7 kPa). L'huile brune obtenue (800 mg) est purifiée par chromatographie-flash sur alumine CTB1 sous pression d'argon (50 kPa) [éluant : cyclohexane / acétate d'éthyle (80 / 20 en volumes)]. Après concentration des fractions sous pression réduite (2,7 kPa), on obtient 220 mg de {2-[3-benzyloxy-4-(4'-fluoro-biphényl-4-yl)-pyrazol-1-yl]-éthyl}diméthyl-amine sous forme d'une huile jaune. Spectre de masse (IE) : m/z 415 (M^{+.}), m/z 344 [(M - C₄H₉N)⁺], m/z 91 (C₇H₇⁺), m/z 58 (C₃H₈N⁺).

La {2-[3-benzyloxy-4-(4-bromo-phényl)-pyrazol-1-yl]-éthyl}-diméthyl-amine peut être préparée de la manière suivante :

A une suspension de 1,36 g d'hydrure de sodium (à 75% dans l'huile de vaseline) dans 50 cm³ de diméthylformamide sous atmosphère d'argon et sous agitation, est ajoutée une solution de 5,2 g de 3-benzyloxy-4-(4-bromo-phényl)-1H-pyrazole dans 50 cm³ de diméthylformamide. Après 30 min de chauffage à 50°C, le mélange est agité 1 h à une température voisine de 20°C, puis il est refroidi dans un bain de glace et additionné d'une solution de 5,5 g de bromhydrate de (2-bromo-éthyl)-diméthyl-amine dans 50 cm³ de diméthylformamide. Le milieu réactionnel est agité 15 h à une température voisine de 20°C, puis il est refroidi à une température voisine de 20°C et jeté dans 400 cm³ d'eau. La phase aqueuse est extraite deux fois par de l'acétate d'éthyle. Les phases organiques sont réunies, lavées successivement par deux fois de l'eau et une solution aqueuse saturée de chlorure de sodium, séchées sur du sulfate de magnésium, filtrées et évaporées sous pression réduite (2,7 kPa) pour donner une huile brune qui est purifiée par chromatographie-flash sur silice sous pression d'argon (50 kPa) [éluant : acétate d'éthyle, acétate d'éthyle / méthanol (90 / 10 en volumes)]. Après concentration des fractions sous pression réduite, on obtient 2,3 g de {2-[3-benzyloxy-4-(4-bromo-phényl)-pyrazol-1-yl]-éthyl}-diméthyl-amine sous forme d'une huile jaune. Spectre de masse (IE) : m/z 399 (M^{+.}), m/z 328 [(M - C₄H₉N)⁺], m/z 91 (C₇H₇^{+.}), m/z 58 (C₃H₈N⁺).

Le 3-benzyloxy-4-(4-bromo-phényl)-1H-pyrazole peut être préparé de la manière suivante :

A une suspension de 6,58 g de 1-[3-benzyloxy-4-(4-bromo-phényl)-pyrazol-1-yl]-éthanone dans 70 cm³ de méthyléthylcétone sous agitation, sont ajoutés 3,88 g de carbonate de potassium et 3,1 cm³ de bromure de benzyle. Le mélange est chauffé au reflux du solvant pendant 2,5 h, refroidi à une température voisine de 20°C, et il est filtré. Le filtrat est évaporé sous pression réduite (2,7 kPa), et le résidu est repris par 50 cm³ tétrahydrofurane et 50 cm³ de méthanol, puis il est additionné de 1,5 cm³ d'hydroxyde de sodium 10 N. Après 30 min d'agitation à une température voisine de 20°C, le milieu réactionnel est évaporé sous pression réduite (2,7 kPa). Le brut de réaction est repris dans du dichlorométhane. La phase organique est lavée successivement par deux fois de l'eau et une solution aqueuse saturée de chlorure de sodium, séchée sur sulfate de magnésium, filtrée et évaporée sous pression réduite (2,7 kPa). Le solide obtenu est trituré dans de l'oxyde de diisopropyle., filtré et séché sous vide (2,7 kPa) pour donner 2,9 g de 3-benzyloxy-4-(4-bromo-phényl)-1H-pyrazole sous forme d'un solide beige. Le filtrat est évaporé sous pression réduite (2,7 kPa) et le résidu est repris par du dichlorométhane. La solution organique est lavée par de l'eau et une solution aqueuse saturée de chlorure de sodium, séchée sur sulfate de magnésium, filtrée et évaporée sous pression réduite (2,7 kPa). Le solide résultant est trituré dans de l'oxyde de diisopropyle, filtré et séché sous vide (2,7 kPa) pour donner 2,3 g supplémentaires de 3-benzyloxy-4-(4-bromo-phényl)-1H-pyrazole sous forme d'un solide beige. Spectre de R.M.N. ¹H (400 MHz, (CD₃)₂SO d6, δ en ppm) : 5,35 (s : 2H) ; 7,35 (t large, J = 7,5 Hz : 1H) ; 7,42 (t large, J = 7,5 Hz : 2H) ; 7,50 (d large, J = 7,5 Hz : 2H) ; 7,51 (d large, J = 8,5 Hz : 2H) ; 7,65 (d large, J = 8,5 Hz : 2H) ; 8,09 (s : 1H).

La 1-[4-(4-bromo-phényl)-3-hydroxy-pyrazol-1-yl]-éthanone peut être préparée de la manière suivante :

Une solution de 6 g de 4-(4-bromo-phényl)-1H-pyrazol-3-ol dans 50 cm³ de pyridine sous atmosphère d'argon et sous agitation est chauffée à 90°C, puis elle est additionnée goutte à goutte de 2,25 cm³ d'anhydride acétique. Après 1 h de chauffage à 90°C, le milieu réactionnel est refroidi à une température voisine de 20°C et il est jeté dans 150 cm³ d'eau glacée. Le précipité formé est filtré, lavé trois fois avec de l'eau et il est séché sous vide (2,7 kPa) pour donner 6,6 g de 1-[4-(4-bromo-phényl)-3-hydroxy-pyrazol-1-yl]-éthanone sous forme d'un solide blanc. Spectre IR (KBr) : 3132; 2968; 2696; 2653; 1714; 1621; 1533; 1417; 1392; 1328; 1279; 1231; 1008; 949; 822; 645 et 508 cm⁻¹.

Le 4-(4-bromo-phényl)-1H-pyrazol-3-ol peut être préparé de la manière suivante :

Une solution sous agitation de 11,5 g d'ester éthylique de l'acide 2-(4-bromo-phényl)-3-diméthylamino-acrylique et de 1,9 cm³ de monohydrate d'hydrazine dans 100 cm³ d'éthanol est chauffée 3 h au reflux du solvant. Le mélange réactionnel est évaporé à sec sous pression réduite (2,7 kPa) et le résidu est trituré dans de l'éther diisopropylique. Le solide formé est filtré et il est séché sous vide (2,7 kPa) pour donner 6 g de 4-(4-bromo-phényl)-1H-pyrazol-3-ol sous forme d'un solide blanc. Spectre IR (KBr) : 3299; 3123; 2958; 2674; 1606; 1579; 1517; 1488; 1399; 1163; 1080; 1008; 824 et 509 cm⁻¹.

L'ester éthylique de l'acide 2-(4-bromo-phényl)-3-diméthylamino-acrylique peut être préparé de la manière suivante :

Une solution de 10 g d'ester éthylique de l'acide 2-(4-bromo-phényl)-acrylique dans 100 cm⁻³ de tétrahydrofurane est additionnée de 13,5 cm³ de C-tert-butoxy-N,N,N',N'-tétraméthyl-méthanediamine et chauffée 3 h au reflux du solvant. Après 15 h d'agitation à une température voisine de 20°C, le mélange réactionnel est évaporé à sec sous pression réduite (2,7 kPa). Le résidu est repris par de l'acétate d'éthyle, et la solution organique est lavée par 3 fois de l'eau, puis séchée sur du sulfate de magnésium, filtrée et évaporée sous pression réduite (2,7 kPa) pour donner 11,3 g d'ester éthylique de l'acide 2-(4-bromo-phényl)-3-diméthylamino-acrylique sous forme d'une huile jaune. Spectre de masse (IC) : m/z 298 (MH⁺).

### Exemple 35

### Dichlorhydrate de 1-(2-pipéridin-1-yl-éthyl)-4-pyridin-2-yl-1H-pyrazol-3-ol

Une solution de 1,6 g de 2-[3-(cyclohex-2-ènyloxy)-1-(2-pipéridin-1-yl-éthyl)-1H-pyrazol-4-yl]-pyridine dans 20 cm³ de dioxane est additionnée de 10 cm³ de dioxane chlorhydrique 4 N. Après 15 h d'agitation à une température voisine de 20°C, la suspension est filtrée, et le solide est rinçé une fois avec du dioxane, puis trois fois avec de l'oxyde de diisopropyle, et il est séché sous vide (2,7 kPa) pour donner 55 mg de dichlorhydrate de 1-(2-pipéridin-1-yl-éthyl)-4-pyridin-2-yl-1H-pyrazol-3-ol sous forme d'une poudre blanche. Spectre IR (KBr) : 3037; 2943; 2644; 2541; 1633; 1606; 1577; 1454; 1179; 782 et 685 cm⁻¹. Spectre de R.M.N. ¹H (300 MHz, (CD₃)₂SO d6, δ en ppm) : 1,40 (mt : 1H) ; de 1,60 à 1,90 (mt : 5H) ; 2,95 (mt : 2H) ; de 3,35 à 3,55 (mt : 4H) ; 4,57 (t, J = 6,5 Hz : 2H) ; 7,58 (mt : 1H) ; 8,15 (d large, J = 7Hz : 1H) ; 8,31 (t large, J = 7 Hz : 1H) ; 8,61 (d large, J = 5 Hz : 1H) ; 8,69 (s large : 1H) ; 10,64 (mf : 1H).

La 2-[3-(cyclohex-2-ènyloxy)-1-(2 pipéridin-1-yl-éthyl)-1H-pyrazol-4-yl]-pyridine peut être préparée de la manière suivante :

A une suspension de 500 mg d'hydrure de sodium (à 75% dans l'huile de vaseline) dans 15 cm³ de diméthylformamide sous atmosphère d'argon et sous agitation, est ajoutée une suspension de 1,5 g de 2-[3-(cyclohex-2-ènyloxy)-1H-pyrazol-4-yl]-pyridine dans 20 cm³ de diméthylformamid.e. Après 30 min de chauffage à 50°C, le mélange est agité 30 min à une température voisine de 20°C, puis il est additionné d'une solution de 1,6 g de chlorhydrate de 1-(2-chloro-éthyl)-pipéridine. Le milieu réactionnel est agité 15 h à une température voisine de 20°C, puis il est jeté dans de l'eau. La phase aqueuse est extraite deux fois par de l'acétate d'éthyle. Les phases organiques sont réunies, lavées successivement par de l'eau et une solution aqueuse saturée de chlorure de sodium, séchées sur du sulfate de magnésium, filtrées et évaporées sous pression réduite (2,7 kPa) pour donner une huile jaune qui est purifiée par chromatographie-flash sur alumine CTB1 sous pression d'argon (50 kPa) [éluant : cyclohexane / acétate d'éthyle (90 / 10 en volumes)]. Après concentration des fractions sous pression réduite, on obtient 1,6 g de 2-[3-(cyclohex-2-ènyloxy)-1-(2-pipéridin-1-yl-éthyl)-1H-pyrazol-4-yl]-pyridine sous forme d'une huile jaune pâle. Spectre de masse (IE) : m/z 352 (M^{+.}), m/z 271 [(M - C₆)H₉)⁺], m/z 111 (C₇H₁₃N^{+.}), m/z 98 (C₆H₁₂N⁺).

La 2-[3-(cyclohex-2-ènyloxy)-1H-pyrazol-4-yl]-pyridine peut être préparée de la manière suivante :

A une suspension de 7,1 g de 1-(3-hydroxy-4-pyridin-2-yl-pyrazol-1-yl)-éthanone dans 70 cm³ de méthyléthylcétone sous agitation, sont ajoutés 5,4 g de carbonate de potassium, et 4,6 cm³ de 3-bromo-cyclohexène. Le mélange est chauffé au reflux du solvant pendant 4 h, refroidi à une température voisine de 20°C, et il est évaporé sous pression réduite (2,7 kPa). Le résidu est repris par 50 cm³ de tétrahydrofurane et 50 cm³ de méthanol, puis il est additionné de 7 cm³ d'hydroxyde de sodium 5 N et d'eau jusqu'à solubilisation totale du milieu. Après 15 h d'agitation à une température voisine de 20°C, le milieu réactionnel est évaporé sous pression réduite (2,7 kPa). Le brut de réaction est repris par de l'acétate d'éthyle et de l'eau. Le solide insoluble est filtré et séché sous pression réduite (2,7 kPa) pour donner 5,3 g de 2-[3-(cyclohex-2-ènyloxy)-1H-pyrazol-4-yl]-pyridine sous forme d'une poudre blanche. Spectre IR (KBr) : 3180; 2928; 2723; 1602; 1533; 1497; 1463; 1288; 1065; 786 et 700 cm⁻¹.

La 1-(3-hydroxy-4-pyridin-2-yl-pyrazol-1-yl)-éthanone peut être préparée de la manière suivante : :

Une solution agitée et sous atmosphère d'argon de 7,3 g de chlorhydrate de 4-pyridin-2-yl-1H-pyrazol-3-ol dans 70 cm³ de pyridine est chauffée à 100°C, puis elle est additionnée goutte à goutte de 3,75 cm³ d'anhydride acétique. Après 1,5 h de chauffage à 100°C, le milieu réactionnel est refroidi à une température voisine de 20°C et il est jeté dans 150 cm³ d'eau glacée. Le précipité formé est filtré, lavé trois fois avec de l'eau et il est séché sous vide (2,7 kPa) pour donner 7,1 g de 1-(3-hydroxy-4-pyridin-2-yl-pyrazol-1-yl)-éthanone sous forme d'un solide jaune pâle. Spectre IR (KBr) : 3157; 2396; 1719; 1608; 1391; 1274; 1223; 1000; 929; 790 et 618 cm⁻¹.

Le chlorhydrate de 4-pyridin-2-yl-1H-pyrazol-3-ol peut être préparé de la manière suivante :

Une solution sous agitation de 18 g d'ester éthylique de l'acide 3-diméthylamino-2-pyridin-2-yl-acrylique et de 3,95 cm³ de monohydrate d'hydrazine dans 120 cm³ d'éthanol est chauffée 3 h au reflux du solvant. Le mélange réactionnel est concentré sous pression réduite (2,7 kPa), additionné d'éthanol chlorhydrique 3 N, puis il est refroidi dans un bain de glace. Le solide formé est filtré, et il est séché sous vide (2,7 kPa) pour donner 11,3 g de chlorhydrate de 4-pyridin-2-yl-1H-pyrazol-3-ol sous forme d'un solide jaune. Spectre IR (KBr) : 3166; 1644; 1620; 1587; 1551; 1430; 1209; 1159; 907; 774 et 518 cm⁻¹.

L'ester éthylique de l'acide 3-diméthylamino-2 pyridin-2-yl-acrylique peut être préparé de la manière suivante :

Une solution de 13 g d'ester éthylique de l'acide 2-pyridin-2-yl-acrylique dans 100 cm³ de tétrahydrofurane est additionnée de 20 cm³ de C-tert-butoxy-N,N,N',N'-tétraméthyl-méthanediamine, et chauffée 15 h au reflux du solvant, puis le mélange réactionnel est refroidi à une température voisine de 20°C et évaporé à sec sous pression réduite (2,7 kPa). L'huile brune résultante est purifiée par chromatographie-flash sur silice sous pression d'argon (50 kPa) [éluant : cyclohexane / acétate d'éthyle (90 / 10 en volumes)]. Après concentration des fractions sous pression réduite, on obtient 18 g d'ester éthylique de l'acide 3-diméthylamino-2-pyridin 2-yl-acrylique sous forme d'une huile orangée. Spectre IR (CCl₄) : 2980; 2929; 1686; 1619; 1602; 1297; 1271; 1219; 1096 et 1085 cm⁻¹.

### Exemple 36

### Dichlorhydrate monohydrate de 1-(2-pipéridin-1-yl-éthyl)-4-pyridin-4-yl-1H-pyrazol-3-ol

Une solution agitée de 720 mg de 4-[3-benzyloxy-1-(2-pipéridin-1-yl-émyl)-1H-pyrazol-4-yl]-pyridine dans 7 cm³ d'éthanol est additionnée de 7 cm³ d'acide chlorhydrique 12 N. Après 7 h au reflux du solvant, puis 15 h à une température voisine de 20°C, le milieu réactionnel est évaporé sous pression réduite (2,7 kPa). Le résidu est repris par de l'éthanol, puis le mélange est évaporé à sec sous vide (2,7 kPa). L'opération est répétée deux fois, puis le solide est additionné d'éthanol et le mélange est chauffé au reflux du solvant. Après refroidissement de la solution dans un bain de glace, les cristaux formés sont filtrés et séchés sous vide (2,7 kPa) pour donner 300 mg de dichlorhydrate monohydrate de 1-(2-pipéridin-1-yl-éthyl)-4-pyridin-4-yl-1H-pyrazol-3-ol sous forme d'un solide blanc. Spectre IR (KBr) : 3495; 3414; 3197; 2934; 2652; 2545; 1637; 1599; 1540; 1513; 1206; 813 et 523 cm⁻¹. Spectre de R.M.N. ¹H (300 MHz, (CD₃)₂SO d6, δ en ppm) : de 1,30 à 1,60 (mf très étalé : 1H) ; de 1,60 à 1,90 (mt : 5H) ; de 2,85 à 3,05 (mf : 2H) ; de 3,30 à 3,45 (mt : 2H) ; 3,49 (t, J = 6,5 Hz : 2H) ; 4,54 (t, J = 6,5 Hz : 2H) ; 8,12 (d large, J = 7 Hz : 2H) ; 8,67 (s : 1H) ; 8,69 (d large, J = 7 Hz : 2H) ; 11,75 (mf : 1H).

La 4-[3-benzyloxy-1-(2-pipéridin-1-yl-éthyl)-1H-pyrazol-4-yl]-pyridine peut être préparée de la manière suivante :

A une suspension de 230 mg d'hydrure de sodium (à 75 % dans l'huile de vaseline) dans 10 cm³ de diméthylformamide sous atmosphère d'argon et sous agitation, est ajoutée une solution de 720 mg de 4-(3-benzyloxy-1H-pyrazol-4-yl)-pyridine dans 20 cm³ de diméthylformamide. Après 30 min de chauffage à 50°C, le mélange est agité 30 min à une température voisine de 20°C, puis il est additionné d'une solution de 740 mg de chlorhydrate de 1-(2-chloro-éthyl)-pipéridine. Le milieu réactionnel est agité 15 h à une température voisine de 20°C, puis il est jeté dans de l'eau. La phase aqueuse est extraite deux fois par de l'acétate d'éthyle. Les phases organiques sont réunies, lavées successivement par de l'eau et une solution aqueuse saturée de chlorure de sodium, séchées sur du sulfate de magnésium, filtrées et évaporées sous pression réduite (2,7 kPa) pour donner une huile orangée qui est purifiée par chromatographie-flash sur alumine CTB1 sous pression d'argon (50 kPa) [éluant : cyclohexane / acétate d'éthyle (90 / 10, puis 80 / 20 en volumes)]. Après concentration des fractions sous pression réduite, on obtient 720 mg de 4-[3-benzyloxy-1-(2-pipéridin-1-yl-éthyl)-1H-pyrazol-4-yl]-pyridine sous forme d'une huile jaune pâle. Spectre IR (CH₂Cl₂) : 2940; 1604; 1573; 1513; 1453; 1363; 1172; 992; 815; 676 et 534 cm⁻¹.

La 4-(3-benzyloxy-1H-pyrazol-4-yl)-pyridine peut être préparée de la manière suivante :

A une suspension de 2,4 g de 1-(3-hydroxy-4-pyridin-4-yl-pyrazol-1-yl)-éthanone dans 25 cm³ de méthyléthylcétone sous agitation, sont ajoutés 1,8 g de carbonate de potassium, et 1,55 cm³ de bromure de benzyle. Le mélange est chauffé au reflux du solvant pendant 3 h, refroidi à une température voisine de 20°C, et filtré. Le filtrat est évaporé sous pression réduite (2,7 kPa). L'huile brune résultante est purifiée par chromatographie-flash sur silice sous pression d'argon (50 kPa) [éluant : acétate d'éthyle / cyclohexane (80 / 20 en volumes)]. Après concentration des fractions sous pression réduite, on obtient 720 mg de 4-(3-benzyloxy-1H-pyrazol-4-yl)-pyridine sous forme d'un solide blanc. Spectre de R.M.N. ¹H (300 MHz, (CD₃)₂SO d6, δ en ppm) : 5,27 (s : 2H) ; 7,36 (tt, J = 7,5 et 1,5 Hz : 1H) ; 7,44 (tt, J = 7,5 et 1,5 Hz : 2H) ; 7,52 (d large, J = 7,5 Hz : 2H) ; 7,66 (dd, J = 5 et 2 Hz : 2H) ; 8,33 (s : 1H) ; 8,47 (dd, J = 5 et 2 Hz : 2H) ; de 12,25 à 12,50 (mf : 1H).

La 1-(3-hydroxy-4-pyridin-4-yl-pyrazol-1-yl)-éthanone peut être préparée de la manière suivante :

Une suspension de 2,5 g de chlorhydrate de 4-pyridin-4-yl-1H-pyrazol-3-ol dans 25 cm³ de pyridine sous agitation et sous atmosphère d'argon est chauffée à 100°C, puis elle est additionnée goutte à goutte de 1,25 cm³ d'anhydride acétique. Après 2 h de chauffage à 100°C, le milieu réactionnel est refroidi dans un bain de glace. Le solide formé est filtré, lavé avec de l'eau, puis avec de l'heptane, et il est séché sous vide (2,7 kPa) pour donner la 1-(3-hydroxy-4-pyridin-4-yl-pyrazol-1-yl)-éthanone qui est directement engagée dans l'étape suivante.

Le chlorhydrate de 4-pyridin-4-yl-1H-pyrazol-3-ol peut être préparé de la manière suivante :

Une solution sous agitation de 12,46 g d'ester éthylique de l'acide 3-diméthylamino-2-pyridin-4-yl-acrylique et de 2,75 cm³ de monohydrate d'hydrazine dans 80 cm³ d'éthanol est chauffée 3 h au reflux du solvant. Le mélange réactionnel est refroidi dans un bain de glace, le solide formé est filtré et repris par de l'eau. La suspension est ajustée à pH 6 par de l'acide chlorhydrique 1 N, puis elle est filtrée. Le solide obtenu est lavé par de l'eau et il est séché sous vide (2,7 kPa) pour donner 5,1 g de chlorhydrate de 4-pyridin-4-yl-1H-pyrazol-3-ol sous forme d'un solide jaune. Spectre IR (KBr) : 3355; 2464; 2059; 1965; 1637; 1575; 1527; 1207; 1193; 1075; 1022; 914; 838 et 519 cm⁻¹.

L'ester éthylique de l'acide 3-diméthylamino-2-pyridin-4-yl-acrylique peut être préparé de la manière suivante :

Une solution de 15 cm³ d'ester éthylique de l'acide 2-pyridin-4-yl-acrylique dans 100 cm³ de tétrahydrofurane est additionnée de 24 cm³ de C-tert-butoxy-N,N,N',N'-tétraméthyl-méthanediamine et chauffée 15 h au reflux du solvant, puis le mélange réactionnel est évaporé à sec sous pression réduite (2,7 kPa). Le résidu est repris par de l'acétate d'éthyle; et la solution organique est lavée par 2 fois avec de l'eau, et une solution aqueuse saturée de chlorure de sodium, elle est séchée sur du sulfate de magnésium, filtrée et évaporée sous pression réduite (2,7 kPa). L'huile brune obtenue est purifiée par chromatographie-flash sur silice sous pression d'argon (50 kPa) [éluant : cyclohexane / acétate d'éthyle (90 / 10 en volumes)]. Après concentration des fractions sous pression réduite, on obtient 12,46 g d'ester éthylique de l'acide 2-pyridin-4-yl-acrylique sous forme d'une huile orangée. Spectre IR (CCl₄) : 2981; 1690; 1596; 1280; 1218; 1095 et 1051 cm⁻¹.

### Exemple 37

### 4-(4-Fluoro-phényl)-1-(2-pipéridin-1-yl-éthyl)-1H-pyrazol-3-ol

0,5 g de chlorhydrate de 3-benzyloxy-4-(4-fluoro-phényl)-1-(2-pipéndin-1-yl-éthyl)-1H-pyrazole sont introduits dans un autoclave avec 12,8 mg de palladium sur charbon (à 10%) et 25 cm³ d'éthanol. L'appareil est placé sous une pression d'hydrogène de 500 kPa à une température de 25°C pendant 5 heures. Après refroidissement à une température proche de 20°C, le milieu réactionnel est filtré sur supercel ; le filtrat est lavé par 3 fois 100 cm³ d'éthanol, concentré à sec sous pression réduite (2 kPa). Le résidu obtenu est purifié par chromatographie, sous une pression d'azote de 50 kPa, sur une colonne de gel de silice (granulométrie 20-45µ ; diamètre 1 cm; hauteur 25 cm), en éluant par un mélange de dichlorométhane et d'une solution 2 N de méthanol ammoniacale (93 / 7 en volumes). On concentre sous pression réduite (2 kPa) ; on obtient 160 mg de 4-(4-fluoro-phényl)-1-(2-pipéridin-1-yl-éthyl)-1H-pyrazol-3-ol sous forme d'un solide blanc. Spectre de R.M.N. ¹H (300 MHz, (CD₃)₂SO d6, δ en ppm) : de 1,30 à 1,55 (mt : 6H) ; 2,40 (t large, J = 5,5 Hz : 4H) ; 2,65 (t, J = 7 Hz : 2H) ; 4,00 (t, J = 7Hz : 2H) ; 7,15 (t, J = 9 Hz : 2H) ; 7,65 (dd, J = 9 et 5,5 Hz : 2H) ; 7,91 (s : 1H) ; 10,32 (s large : 1H). Spectre de masse (IE) : m/z 289 (M^{+.}), m/z 98 (pic de base).

Le 3-benzyloxy-4-(4-fluoro-phényl)-1-(2-pipéridin-1-yl-éthyl)-1H-pyrazole peut être préparé de la manière suivante :

Une suspension de 0,80 g de 3-benzyloxy-4-bromo-1-(2-pipéridin-1-yl-éthyl)-1H-pyrazole, 1,12 g d'acide 4-fluoro-phényl boronique, 0,20 g de dichlorobis(triphénylphosphine)palladium et 1,88 g de phosphate de potassium dans 30 cm³ de 1,2-diméthoxyéthane est agitée, sous atmosphère d'argon, à la température d'ébullition du milieu réactionnel pendant 14 heures. Après refroidissement le mélange est additionné de 30 cm³ d'une solution saturée d'hydrogénocarbonate de sodium et extrait avec 30 cm³ d'acétate d'éthyle. La phase organique est séchée sur sulfate de magnésium, filtrée et concentrée à sec sous pression réduite (3 kPa). Le résidu est purifié par chromatographie sur gel de silice en éluant avec un mélange d'acétate d'éthyle et de méthanol (30 / 1 en volumes). Après concentration des fractions sous pression réduite on obtient 0,57 g d'une huile jaunâtre, qui est utilisée telle quelle dans l'étape suivante. 0,57 g de 3-benzyloxy-4-(4-fluoro-phényl)-1-(2-pipéridin-1-yl-éthyl)-1H-pyrazole sont repris par 5 cm³ d'oxyde de diéthyle et 0,5 cm³ d'une solution 4 N d'acide chlorhydrique dans l'oxyde de diéthyle. Le précipité formé est filtré sur séché. On obtient 0,5 g de chlorhydrate de 3-benzyloxy-4-(4-fluoro-phényl)-1-(2-pipéridin-1-yl-éthyl)-1H-pyrazole. Spectre de R.M.N. ¹H (300 MHz, (CD₃)₂SO d6, δ en ppm) : de 1,25 à 1,50 (mt : 1H) ; de 1,60 à 1,85 (mt : 5H) ; 2,91 (mt : 2H) ; 3,44 (d très large, J = 12,5 Hz : 2H) ; 3,52 (mt : 2H) ; 4,47 (t large, J = 6,5 Hz : 2H) ; 5,34 (s : 2H) ; 7,72 (t, J = 9 Hz : 2H) ; de 7,30 à 7,45 (mt : 3H) ; 7,51 (d large, J = 7,5 Hz : 2H) ; 7,68 (dd, J = 9 et 6 Hz : 2H) ; 8,16 (s : 1H) ; de 9,70 à 10,00 (mf : 1H).

Le 3-benzyloxy-4-bromo-1-(2-pipéridin-1-yl-éthyl)-1H-pyrazole peut être préparé de la manière suivante :

A une solution de 8 g de chlorhydrate de 3-benzyloxy-4-bromo-1H-pyrazole dans 100 cm³ de diméthylformamide anhydre on ajoute, progressivement et à une température voisine de 5°C, 5,6 g d'hydrure de sodium (à 75% en masse dans l'huile de vaseline) et 25 cm³ de diméthylformamide anhydre. Après 1 heure d'agitation à température ambiante on ajoute, par petites portions, 6,93 g de chlorhydrate de 1-(2-chloro-éthyl)-pipéridine et 30 cm³ de diméthylformamide anhydre. Après 21 heures d'agitation à température ambiante l'excès d'hydrure de sodium est détruit par addition lente d'eau, puis le milieu réactionnel est versé dans 1 dm³ d'eau et extrait avec 2 fois 200 cm³ d'acétate d'éthyle. Les phases organiques réunies sont séchées sur sulfate de magnésium, filtrées et concentrées à sec sous pression réduite (3 kPa). Le résidu huileux obtenu est repris par 20 cm³ d'acétone et versé sur une solution de 3,6 g d'acide oxalique dans 30 cm³ d'acétone. Le solide formé est trituré, puis isolé par filtration et séché à température ambiante, pour donner 11,3 g d'oxalate de 3-benzyloxy-4-bromo-1-(2-pipéridin-1-yl-éthyl)-1H-pyrazole. A 5 g de cet oxalate sont ajoutés 50 cm³ d'une solution saturée d'hydrogénocarbonate de sodium et le mélange est extrait avec 2x100 cm³ d'acétate d'éthyle. Les phases organiques réunies sont séchées sur sulfate de magnésium, filtrées et concentrées à sec sous pression réduite (3 kPa). On obtient ainsi 3,84 g de 3-benzyloxy-4-bromo-1-(2-pipéridin-1-yl-éthyl)-1H-pyrazole sous forme d'une huile claire. Spectre de R.M.N. ¹H (300 MHz, (CD₃)₂SO d6, δ en ppm) : de 1,25 à 1,55 (mt : 6H) ; 2,35 (t large, J = 5 Hz : 4H) ; 2,61 (t, J = 6,5 Hz : 2H) ; 4,04 (t, J = 6,5 Hz : 2H) ; 5,20 (s : 2H) ; de 7,30 à 7,50 (mt : 5H) ; 7,81 (s : 1H).

Le chlorhydrate de 3-benzyloxy-4-bromo-1H-pyrazole peut être préparé de la manière suivante :

A une suspension de 8,76 g de 3-benzyloxy-1H-pyrazole et de 11 g de carbonate de sodium dans 100 cm³ de dichlorométhane, refroidie et maintenue aux environs de 5°C sous agitation, on ajoute, goutte à goutte en 0,5 heure, une solution de 2,6 cm³ de brome dans 50 cm³ de dichlorométhane. Après 0,5 heure d'agitation à cette température, le mélange est additionné de 20 cm³ d'une solution 0,1 N de thiosulfate de sodium et agité encore 1 heure vers 5°C, puis est additionné de 100 cm³ de dichlorométhane et décanté. La phase organique est extraite à nouveau par 50 cm³ d'eau et les phases organiques sont réunies et séchées sur sulfate de magnésium, filtrées et concentrées à sec sous pression réduite (3 kPa). Le résidu huileux obtenu est repris par 10 cm³ de dioxane chlorhydrique 6 N et le solide formé est trituré dans l'éther éthylique et isolé par filtration. On obtient ainsi 12,5 g de chlorhydrate de 3-benzyloxy-4-bromo-1H-pyrazole sous forme d'un solide blanc fondant vers 80°C (avec décomposition). Spectre de masse (IE) : m/z 252 (M^{+.}), m/z 91 (pic de base). Spectre de R.M.N. ¹H (300 MHz, (CD₃)₂SO d6, δ en ppm) : 5,22 (s : 2H) ; de 7,30 à 7,50 (mt : 5H) ; 7,81 (s : 1H) ; de 11,80 à 12,70 (mf très étalé : 1H).

Le 3-benzyloxy-1H-pyrazole peut être préparé de la manière suivante :

A 10,6 g de chlorhydrate de 3-benzyloxy-1H-pyrazole sont ajoutés 50 cm³ d'une solution saturée d'hydrogénocarbonate de sodium et le mélange est extrait avec 2x150 cm³ de dichlorométhane. Les phases organiques réunies sont séchées sur sulfate de magnésium, filtrées et concentrées à sec sous pression réduite (3 kPa) pour donner 8,76 g de 3-benzyloxy-1H-pyrazole sous forme d'une huile qui est utilisée telle quelle dans l'étape suivante.

Le chlorhydrate de 3-benzyloxy-1H-pyrazole peut être préparé de la manière suivante :

Une suspension de 11 g de 1-(3-hydroxy-pyrazol-1-yl)-éthanone, 12,5 g de carbonate de potassium et 11,3 cm³ (16,25 g) de bromure de benzyle dans 250 cm³ de 2-butanone est agitée à la température d'ébullition du mélange pendant 1,25 heure. L'insoluble minéral est ensuite éliminé par filtration et le filtrat est concentré à sec sous pression réduite (3 kPa). Le résidu huileux obtenu est dissous dans un mélange de 150 cm³ de tétrahydrofurane et 100 cm³ de méthanol, puis est additionné de 4 cm³ d'une solution 10 M d'hydroxyde de sodium. Après 0,65 heure d'agitation à température ambiante le mélange est concentré à sec sous pression réduite (3 kPa). Le résidu pâteux obtenu est repris par 250 cm³ d'acétate d'éthyle et lavé avec 2 fois 10 cm³ de saumure. La phase organique est séchée sur sulfate de magnésium, filtrée et concentrée sous pression réduite (3 kPa). Le résidu est additionné de 100 cm³ d'éther chlorhydrique 1N et le solide formé est trituré, puis isolé par filtration. Le solide est solubilisé dans 250 cm³ d'isopropanol vers 60°C, puis le mélange est concentré partiellement jusqu'à l'apparition des premiers cristaux, additionné de 5 cm³ d'acétate d'isopropyle et refroidi vers 0°C. Après filtration et séchage on obtient 10,6 g de chlorhydrate de 3-benzyloxy-1H-pyrazole sous forme de cristaux rose saumon fondant à 100°C. Spectre de R.M.N. ¹H (300 MHz, (CD₃)₂SO d6, δ en ppm) : 5,16 (s : 2H) ; 5,75 (d, J = 3 Hz : 1H) ; de 7,30 à 7,50 (mt : 5H) ; 7,57 (d, J = 3 Hz : 1H).

La 1-(3-hydroxy pyrazol-1-yl)-éthanone peut être préparée de la manière suivante :

A la solution de 8,4 g de 1H-pyrazol-3-ol (N° CAS 60456-92-0) dans 38 cm³ de pyridine préalablement chauffée à 95°C on ajoute lentement, en 0,33 heure, une solution de 9,5 cm³ d'anhydride acétique dans 18 cm³ de pyridine, puis maintient cette température pendant 1 heure. Le mélange est ensuite concentré sous pression réduite (3 kPa). La suspension résiduelle est additionnée de 100 cm³ d'éther éthylique et triturée pour achever la cristallisation. Après filtration et séchage on obtient 11 g de 1-(3-hydroxy-pyrazol-1-yl)-éthanone sous forme de cristaux blanchâtres se sublimant vers 215°C. Spectre de R.M.N. ¹H (300 MHz, (CD₃)₂SO d6, δ en ppm : 2,50 (s : 3H) ; 6,02 (d, J = 3 Hz : 1H) ; 8,15 (d, J = 3 Hz : 1H) ; de 10,80 à 11,20 (mf : 1H).

### Exemple 38

### Dichlorhydrate de 4-(4-trifluorométhoxy-phényl)-1-(2-pipéridin-1-yl-éthyl)-1H-pyrazol-3-ol

600 mg de 3-benzyloxy-4-(4-trifluorométhoxy-phényl)-1-(2-pipéridin-1-yl-éthyl)-1H-pyrazole dans 4,5 cm³ d'éthanol et 4,5 cm³ d'acide chlorhydrique 12 N sont portés au reflux à une température proche de 100°C pendant 7 heures. Après refroidissement à une température proche de 20°C, le milieu réactionnel est concentré à sec sous pression réduite (2 kPa) ; le résidu est précipité dans un mélange d'oxyde de diisopropyle et d'acétone. On obtient 464 mg de Dichlorhydrate de 4-(4-trifluorométhoxy-phényl)-1-(2-pipéridin-1-yl-éthyl)-1H-pyrazol-3-ol sous forme d'une poudre blanche. Spectre IR (KBr) : 3428; 2951; 2642; 2538; 1615; 1591; 1533; 1456; 1275; 1219; 1159; 1012; 856 et 806 cm⁻¹. Spectre de R.M.N. ¹H (300 MHz, (CD3)2SO d6, δ en ppm) : 1,41 (mt : 1H) ; de 1,60 à 1,85 (mt : 5H) ; 2,94 (mf : 2H) ; 3,46 (mt : 4H) ; 4,41 (mt : 2H) ; 7,34 (d large, J = 8 Hz : 2H) ; 7,76 (d, J = 8 Hz : 2H) ; 8,09 (s : 1H) ; 10,04 (mf étalé : 1H) ; 10,62 (s large : 1H).

Le 3-benzyloxy-4-(4-tifluorométhoxy-phényl)-1-(2-pipéridin-1-yl-éthyl)-1H-pyrazole peut être préparé de la manière suivante :

On refroidit, sous agitation, à une température proche de -5°C et sous atmosphère inerte, 560 mg de 3-benzyloxy-4-(4-trifluorométhoxy phényl)-1H-pyrazole dans 15 cm³ de diméthylformamide anhydre; 140 mg d'hydrure de sodium à 75% dans l'huile de vaseline sont ajoutés par portion au milieu réactionnel et on laisse revenir la température à environ 20°C. On ajoute ensuite 431 mg de chlorhydrate de 1-(2-chloroéthyl)-pipéridine et maintient l'agitation à cette température pendant 15 heures. Le milieu réactionnel est repris par 300 cm³ d'eau glacée et 300 cm³ d'acétate d'éthyle. La phase organique est décantée, lavée par 200 cm³ d'eau et concentrée à sec sous pression réduite (2 kPa). Le résidu est purifié sur cartouche de silice FC50SI-HP en éluant avec un mélange de dichlorométhane et de méthanol (95 / 5 en volumes). Le produit obtenu est à nouveau purifié sur cartouche de silice FC50SI-HP en éluant avec un mélange de dichlorométhane et de méthanol (98 / 2 en volumes). On obtient 600 mg de 3-benzyloxy-4-(4-trifluorométhoxy-phényl)-1-(2-pipéridin-1-yl-éthyl)-1H-pyrazole sous forme d'une huile incolore. Spectre de masse (IC) : m/z 446 ([M+H]⁺) (pic de base).

Le 3-benzyloxy-4-(4-trifluorométhoxy-phényl)-1H-pyrazole peut être préparé de la manière suivante :

1,07 g de 1-(toluène-4-sulfonyl)-3-benzyloxy-4-(4-trifluorométhoxy-phényl)-1H-pyrazole, 5 cm³ d'une solution 1 N de fluorure de n-tétrabutylammonium dans le tétrahydrofurane et 50 cm³ de tétrahydrofurane sont chauffés, sous agitation, au reflux du solvant pendant 15 heures. Le milieu réactionnel est refroidi à une température proche de 20°C, repris par 300 cm³ d'acétate d'éthyle et 100 cm³ d'eau. La phase organique est décantée, séchée sur du sulfate de magnésium anhydre, filtrée et concentrée à sec sous pression réduite (2 kPa). Le résidu obtenu est purifié sur cartouche de silice FC50SI-HP en éluant avec du dichlorométhane. On obtient 560 mg de 3-benzyloxy-4-(4-trifluorométhoxy-phényl)-1H-pyrazole sous forme d'une poudre. Spectre de masse (IE) : m/z 334 (M^{+.}), m/z 91 (pic de base).

Le 3-benzyloxy-1-(toluène-4-sulfonyl)-4(4-trifluorométhoxy-phényl)-1H-pyrazole peut être préparé de la manière suivante :

A une solution de 1,5 g de 3-benzyloxy-4-iodo 1-(toluène-4-sulfonyl)-1H-pyrazole dans un mélange de 40 cm³ de toluène et d'éthanol (4 / 1 en volumes) dans un tricol, on ajoute 2,04 g d'acide 4-trifluorométhoxy-phényl boronique, 4,96 cm³ d'une solution aqueuse 2 N de carbonate de potassium et 496 mg de tétrakis(triphénylphosphine)palladium. Le tricol contenant le milieu réactionnel est placé dans un bain préchauffé à une température proche de 120°C ; on continue l'agitation pendant 90 minutes à cette température. Le mélange est ensuite refroidi à une température proche de 20°C, filtré sur supercel. Le filtrat est repris par 300 cm³ d'acétate d'éthyle et 100 cm³ d'eau. La phase organique est décantée et concentrée à sec sous pression réduite (2 kPa). Le résidu obtenu est purifié sur cartouche de silice FCS50SI-HP en éluant avec un mélange de cyclohexane et d'acétate d'éthyle (90 / 10 en volumes). On obtient 1,07 g de 3-benzyloxy-l-(toluène-4-sulfonyl)-4-(4-trifluorométhoxy-phényl)-1H-pyrazole sous forme d'une poudre jaune. Spectre de masse (IC) : m/z 489 ([M+H]⁺), m/z 335 (pic de base).

Le 3-benzyloxy-4-iodo-1-(toluène-4-sulfonyl)-1H-pyrazole peut être préparé de la manière suivante :

On refroidit en agitant, sous atmosphère inerte, à une température proche de -5°C, 5 g de 3-benzyloxy-4-(4-iodo-phényl)-1H-pyrazole dans 110 cm³ de diméthylformamide. On ajoute par portions 587 mg d'hydrure de sodium à 75% dans l'huile de vaseline et laisse revenir à une température proche de 20°C. On ajoute ensuite 4,4 g de chlorure de para-toluène sulfonyle et maintient l'agitation à cette température pendant 15 heures. Le milieu réactionnel est repris par 300 cm³ d'eau glacée et 500 cm³ d'acétate d'éthyle. La phase organique est décantée, lavée par 300 cm³ d'eau et 300 cm³ d'une solution aqueuse saturée de chlorure de sodium, séchée sur du sulfate de magnésium anhydre, filtrée et concentrée à sec sous pression réduite (2 kPa). Le résidu est cristallisé dans de l'oxyde de diisopropyle. On obtient 7,24 g de 3-benzyloxy-4-iodo-1-(toluène-4-sulfonyl)-1H-pyrazole sous forme d'une poudre. Spectre de masse (IE): m/z 454 (M^{+.}), m/z 299, m/z 91 (pic de base).

Le 3-benzyloxy-4-iodo-1H-pyrazole peut être préparé de la manière suivante:

Une suspension de 0,32 g de 3-benzyloxy-1H-pyrazole, 0,3 g d'acétate de sodium et 0,65 g d'iode dans 50 cm³ de chloroforme est agitée à température ambiante pendant 26 heures. Le mélange est ensuite additionné de 50 cm³ d'une solution 0,5 N de thiosulfate de sodium, agité jusqu'à décoloration et décanté. La phase aqueuse est extraite à nouveau avec 25 cm³ de chloroforme. Les phases organiques réunies sont séchées sur sulfate de magnésium, filtrées et concentrées sous pression réduite (3 kPa). Le résidu obtenu est purifié par chromatographie sur gel de silice en éluant avec un mélange de cyclohexane et d'acétate d'éthyle (80 / 20 en volumes). Après concentration des fractions sous pression réduite on obtient une huile incolore qui cristallise rapidement et fournit 0,4 g de 3-benzyloxy-4-iodo-1H-pyrazole sous forme d'un solide blanc, ayant un Rf de 0,6 [mélange de cyclohexane et d'acétate d'éthyle (50 / 50 en volumes), plaque de gel de silice 60 F254 référence 105719, Merck]. Spectre de masse (IE) : m/z 300 (M+.) (pic de base). Spectre de RM.N. ¹H (300 MHz, (CD3)2SO d6, δ en ppm) : 5,22 (s : 2H) ; de 7,30 à 7,50 (mt : 5H) ; 7,74 (s : 1H) ; de 12,20 à 12,60 (mf étalé : 1H).

### Exemple 39

### Dichlorhydrate de 4-phényl-1-(2-pipéridin-1-yl-propyl)-1H-pyrazol-3-ol

A un mélange de 150 mg de 1-{2-[3-(cyclohex-2-ènyloxy)-4-phényl-pyrazol-1-yl]-1-méthyl-éthyl}-pipéridine dans 5 cm³ de méthanol anhydre, on ajoute, à une température proche de 20°C, 2 cm³ d'une solution 4 N d'acide chlorhydrique dans le dioxane. Le milieu réactionnel est agité à cette température pendant 20 heures, concentré à sec sous pression réduite (2 kPa), repris par 20 cm³ de dichlorométhane. La solution est concentrée à sec sous pression réduite (2 kPa). Le résidu est précipité par 20 cm³ d'oxyde de diisopropyle. On obtient 110 mg de dichlorydrate de 4-phényl-1-(2-pipéridin-1-yl-propyl)-1H-pyrazol-3-ol , sous forme d'un solide jaune. Spectre IR (KBr) : 3431; 2949; 2651; 2521; 1606; 1580; 1527; 1451; 1175; 1121; 1012; 765; 698 et 672 cm⁻¹. Spectre de R.M.N. ¹H (300 MHz, (CD3)2SO d6, δ en ppm) : 1,24 (d, J = 6,5 Hz : 3H); 1,44 (mt : 1H) ; de 1,65 à 1,95 (mt : 5H); 2,95 (mt : 1H) ; 3,10 (mt : 1H) ; de 3,35 à 3,55 (mt : 2H) ; 3,71 (mt : 1H) ; 4,21 (dd, J = 14,5 et 7,5 Hz : 1H) ; 4,43 (dd, J =14,5 et 5,5 Hz : 1H) ; 7,15 (t large, J = 7,5 Hz : 1H); 7,34 (t large, J = 7,5 Hz : 2H) ; 7,66 (d large, J = 7,5 Hz : 2H) ; 8,01 (s : 1H); 9,51 (mf : 1H) ; 10,51 (s : 1H).

La 1-{2-[3-(Cyclohex-2-ènyloxy)-4-phényl-pyrazol-1-yl]-1-méthyl-éthyl}-pipéridine peut être préparée de la manière suivante :

Un mélange de 0,5 g d'ester du 2-[3-(cyclohex-2-ènyloxy)-4-phényl-pyrazol-1-yl]-1-méthyl-éthyl de l'acide méthane sulfonique, 0,4 cm³ de pipéridine, 1,0 g de carbonate de potassium dans 20 cm³ de diméthylformamide, est chauffé en agitant à une température proche de 80°C pendant 6 heures puis 15 heures à une température proche de 20°C. Le milieu réactionnel est repris par 100 cm³ d'eau et 100 cm³ d'acétate d'éthyle. La phase organique est décantée, lavée par 2 fois 100 cm³ d'eau et 100 cm³ d'une solution aqueuse saturée de chlorure de sodium, séchée sur du sulfate de magnésium anhydre, filtrée et concentrée à sec sous pression réduite (2 kPa). Le résidu est purifié par chromatographie, sous une pression d'azote de 50 kPa, sur une colonne de gel de silice (granulométrie 20-45µ; diamètre 2 cm; hauteur 40 cm), en éluant par un mélange de cyclohexane et d'acétate d'éthyle (80/20 en volumes). Après concentration des fractions sous pression réduite (2 kPa), on obtient 150 mg de 1-{2-[3-(cyclohex-2-ènyloxy)-4-phényl-pyrazol-1-yl]-1-méthyl-éthyl}-pipéridine sous forme d'une huile jaune. Spectre de masse (IE) : m/z 365 (M^{+.}), m/z 112 (pic de base).

L'ester du 2-[3-(cyclohex-2-ènyloxy)-4-phényl-pyrazol-1-yl]-1-méthyl-éthyl de l'acide méthane sulfonique peut être préparé de la manière suivante :

A une solution agitée de 550 mg de 1-[3-(cyclohex-2-ènyloxy)-4-phényl-pyrazol-1-yl]-propan-2-ol dans 30 cm³ de dichlorométhane, à une température proche de 20°C, on ajoute 1 cm³ de chlorure de méthane sulfonyle et 2,59 cm³ de triéthylamine. Le milieu réactionnel est agité 7 heures à une température proche de 20°C, repris par 50 cm³ d'eau distillée et 50 cm³ d'acétate d'éthyle. La phase organique est décantée, séchée sur du sulfate de magnésium anhydre, filtrée et concentrée à sec sous pression réduite (2 KPa). Le résidu est purifié par chromatographie, sous une pression d'azote de 50 kPa, sur une colonne de gel de silice (granulométrie 20-45µ ; diamètre 4 cm; hauteur 60 cm), en éluant par un mélange de cyclohexane et d'acétate d'éthyle (80 / 20 en volumes). Après concentration des fractions sous pression réduite (2 kPa), on obtient 300 mg d'ester du 2-[3-(cyclohex-2-ènyloxy)-4-phényl-pyrazol-1-yl]-1-méthyl-éthyl de l'acide méthane sulfonique sous forme d'une huile incolore. Spectre de masse (IE): m/z 376 (M^{+.}), m/z 296 (pic de base).

Le 1-[3-(Cyclohex-2-ènyloxy)4-phényl-pyrazol-1-yl]-propan-2-ol peut être préparé de la manière suivante :

On dissout, sous atmosphère inerte, et en agitant, 2,4 g de 3-(cyclohex-2-ènyloxy)-4-phényl-1H-pyrazole dans 25 cm³ de diméthylformamide anhydre. On ajoute, à une température proche de 20°C, 2,24 g de tertiobutylate de potassium puis 0,7 cm³ de méthyl oxirane. Le milieu réactionnel est chauffé à une température proche de 60°C pendant 1 heure. Puis on ajoute encore 0,7 cm³ de méthyl oxirane et chauffe 1 heure à une température proche de 60°C. Le mélange est refroidi à une température proche de 20°C, repris par 200 cm³ d'eau et 200 cm³ d'acétate d'éthyle. La phase organique est décantée, lavée par 3 fois 200 cm³ d'eau et 200 cm³ d'une solution aqueuse saturée de chlorure de sodium, séchée sur du sulfate de magnésium anhydre, filtrée et concentrée à sec sous pression réduite (2 kPa). Le résidu est purifié par chromatographie, sous une pression d'azote de 50 kPa, sur une colonne de gel de silice (granulométrie 20-45µ ; diamètre 4 cm; hauteur 50 cm), en éluant par un mélange de cyclohexane et d'acétate d'éthyle (80 / 20 en volumes). Après concentration des fractions sous pression réduite (2 kPa), on obtient 450 mg de 1-[3-(cyclohex-2-ènyloxy)-4-phényl-pyrazol-1-yl]-propan-2-ol sous forme d'une huile incolore. Spectre de masse (IE): m/z 298 (M^{+.}), m/z 218, m/z 173 (pic de base)

### Exemple 40

### Dichlorhydtate de 3-(4-phényl-pyrazol-1-yl-méthyl)-1-aza-bicyclo[2.2.2]octane

On ajoute une solution de 0,4 g de 3-(pyrazol-1-yl-méthyl)-1-aza-bicyclo[2.2.2]octane dans 5 cm³ de diméthylformamide) à un mélange de 0,4 g d'hydrure de sodium à 75% dans l'huile de vaseline et de 10 cm³ de diméthylformamide. Le milieu réactionnel est chauffé à une température proche de 50°C pendant environ 1 heure puis on refroidit la solution à une température proche de 20°C. On ajoute lentement 1,75 g de 3-chloro-méthylquinuclidine, chauffe le milieu réactionnel à une température proche de 50°C pendant 16 heures puis refroidit à une température proche de 20°C. Le mélange est repris par 100 cm³ d'eau et 100 cm³ d'acétate d'éthyle. La phase organique est décantée, lavée par 2 fois 100 cm³ d'eau et 100 cm³ d'une solution saturée aqueuse de chlorure de sodium puis concentrée à sec sous pression réduite (2 kPa). Le résidu est purifié par chromatographie, sous une pression d'azote de 50 kPa, sur une colonne d'alumine CTB1 (diamètre 3 cm; hauteur 40 cm), en éluant par de l'acétate d'éthyle puis par un mélange d'acétate d'éthyle et de méthanol (98/2, 95/5 puis 90 / 10 en volumes) et en recueillant des fractions de 60 cm³. On concentre sous pression réduite les fractions 14 à 20. Le résidu obtenu est purifié une seconde fois par chromatographie, sous pression d'azote (50 kPa), sur une colonne d'alumine CTB1 (diamètre 3 cm; hauteur 40 cm), en éluant par de l'acétate d'éthyle puis par un mélange d'acétate d'éthyle et de méthanol (98 / 2, 95/5 puis 90/10 en volumes) et en recueillant des fractions de 60 cm³. On concentre sous pression réduite les fractions 14 à 20. On obtient 150 mg de 3-(4-phényl-pyrazol-1-yl-méthyl)-1-aza-bicyclo[2.2.2]octane. Le dichlorhydrate est préparé avec 1,2 cm³ d'une solution 4,7 N d'acide chlorhydrique dans de l'oxyde d'isopropyle et de 5 cm³ d'éthanol. On obtient 230 mg de dichlorhydrate de 3-(4-phényl-pyrazol-1-yl-méthyl)-1-aza-bicyclo[2.2.2]octane.

Spectre de R.M.N. ¹H (300 MHz, (CD3)2SO d6, δ en ppm) : de 1,65 à 1,95 (mt : 4H); de 2,00 à 2,15 (mt : 1H) ; 2,62 (mt : 1H) ; 2,97 (dd large, J =13 et 7 Hz : 1H); de 3,10 à 3,40 (mt : 5H) ; 4,25 (dd, J = 13 et 8 Hz : 1H) ; 4,32 (dd, J = 13 et 8 Hz : 1H) ; 7,21 (t large, J = 7,5 Hz : 1H) ; 7,38 (t, J = 7,5 Hz : 2H) ; 7,59 (d large, J = 7,5 Hz : 2H) ; 7,94 (s : 1H) ; 8,25 (s : 1H) ; 10,44 (mf : 1H). Spectre de masse (IE): m/z 267 (M^{+·}) (pic de base), m/z 183, m/z 123.

Le 4-phényl-1H-pyrazole peut être préparé de la manière suivante :

1,044 g de 4-phényl-1-(toluène-4-sulfonyl)-1H-pyrazole, 7 cm³ d'une solution 1 N de fluorure de n-tétrabutylammonium dans le tétrahydrofurane et 35 cm³ de tétrahydrofurane sont chauffés à une température proche de 70°C pendant 6 heures. On ajoute encore 3,5 cm³ d'une solution 1 N de fluorure de n-tétrabutylammonium dans le tétrahydrofurane et continue de chauffer pendant 15 heures à cette température. Le milieu réactionnel est refroidi à une température proche de 20°C, concentré à sec sous pression réduite (2 kPa) puis repris par 100 cm³ d'acétate d'éthyle et 100 cm³ d'eau. La phase organique est décantée, lavée par 100 cm³ d'eau et 100 cm³ d'une solution saturée aqueuse de chlorure de sodium, séchée sur du sulfate de magnésium anhydre, filtrée et concentrée à sec sous pression réduite (2 kPa). Le résidu obtenu est repris par 20 cm³ de dichlorométhane. Le précipité est filtré et séché. On obtient 0,4 g de 4-phényl-1H-pyrazole sous forme d'une poudre blanche. Spectre de masse (IE): m/z 144 (M^{+.}) (pic de base).

Le 4-phényl-1-(toluène-4-sulfonyl)-1H-pyrazole peut être préparé de la manière suivante :

A une solution agitée de 8,7 g de 4-iodo-1-(toluène-4-sulfonyl)-1H-pyrazole dans 200 cm³ de 1,2-diméthoxyéthane sous atmosphère inerte, on ajoute 11,72 g d'acide phényl boronique. Le milieu réactionnel est chauffé à 110°C puis on ajoute 20,63 g de phosphate de potassium tribasique finement broyé et 2,18 g de chlorure de bis(triphénylphosphine)palladium; le mélange est chauffé au reflux du solvant pendant 3 heures puis refroidi à une température proche de 20°C et ensuite filtré sur supercel. Le filtrat est repris par 250 cm³ d'acétate d'éthyle et lavé par 8 fois 100 cm³ d'eau et 100 cm³ d'une solution aqueuse saturée de chlorure de sodium. La phase organique est décantée, séchée sur du sulfate de magnésium anhydre, filtrée et concentrée à sec sous pression réduite (2 kPa). Le résidu est purifié par chromatographie, sous une pression d'azote de 50 kPa, sur une colonne de gel de silice (granulométrie 20-45µ; diamètre 6 cm; hauteur 45 cm), en éluant par un mélange de cyclohexane et d'acétate d'éthyle (70 / 30 en volumes) et en recueillant des fractions de 20 cm³. On concentre sous pression réduite les fractions 6 à 12. On obtient 4,04 g de 4-phényl-1-(toluène-4-sulfonyl)-1H-pyrazole sous la forme de cristaux blancs. Spectre de masse (IE): m/z 298 (M^{+.}) (pic de base), m/z 234, m/z 91.

Le 4-iodo-1-(toluène-4-sulfonyl)-1H-pyrazole peut être préparé de la manière suivante :

Une solution agitée de 10 g de 4-iodo-1H-pyrazole dans 300 cm³ de diméthylformamide, sous atmosphère inerte, est refroidie à une température proche de -3°C. On ajoute en 5 minutes 1,8 g d'hydrure de sodium à 75% dans l'huile de vaseline et laisse revenir la température à environ 20°C. On ajoute ensuite 13,9 g de chlorure de paratoluène sulfonyle et maintient l'agitation pendant 3 heures à cette température. Le milieu réactionnel est repris par 100 g de glace puis 700 cm³ d'eau et 700 cm³ d'acétate d'éthyle. La phase organique est décantée, lavée par 9 fois 300 cm³ d'eau et 2 fois 100 cm³ d'une solution aqueuse de chlorure de sodium, séchée sur du sulfate de magnésium anhydre, filtrée puis concentrée sous pression réduite (2 kPa). Le résidu est recristallisé dans 1000 cm³ d'oxyde de diisopropyle. On obtient 10,9 g de 4-iodo-1-(toluène-4-sulfonyl)-1H-pyrazole sous forme de cristaux blancs. Spectre de masse (IE) : m/z 348 (M^{+.}), m/z 284, m/z 91 (pic de base).

### Exemple 41

### Chlorhydrate de 4-(5-chloro-thiophèn-2-yl)-1-(2-pipéridin-1-yl-éthyl)-1H-pyrazol-3-ol

Le produit est préparé en suivant le mode opératoire décrit pour la préparation du 4-(4-trifluorométhoxy-phényl)-1-(2-pipéridin-1-yl-éthyl)-1H-pymzol-3-ol, à partir de 400 mg de 3-benzyloxy-4-(5-chloro-thiophèn-2-yl)-1-(2-pipéridin-1-yl-éthyl)-1H-pyrazole, 3,3 cm³ d'acide chlorhydrique 12 N et 3,3 cm³ d'éthanol. Le milieu est repris par de l'oxyde de diisopropyle et filtré sur verre fritté. Le filtrat est précipité dans de l'éthanol. On obtient 160 mg du produit attendu sous forme d'une poudre. Spectre de R.M.N. ¹H (300 MHz, (CD3)2SO d6, δ en ppm) : de 1,25 à 1,50 (mt : 1H) ; de 1,60 à 1,90 (mt : 5H) ; de 2,80 à 3,00 (mf : 2H) ; de 3,35 à 3,50 (mt : 4H) ; 4,40 (t large, J = 6,5 Hz : 2H) ; 7,00 (d, J = 5,5 Hz : 1H) ; 7,03 (d, J = 5,5 Hz : 1H) ; 7,97 (s : 1H) ; de 10,00 à 10,30 (mf : 1 H) ; 10,78 (s large : 1H). Spectre de masse (IC) : m/z 312 ([M+H]⁺) (pic de base).

Le 3-benzyloxy-4-(5-chloro-thiophèn-2-yl)-1-(2-pipéridin-1-yl-éthyl)-1H-pyrazole peut être préparé de la manière suivante :

Le produit est préparé en suivant le mode opératoire décrit pour la préparation du 3-benzyloxy-4-(4-trifluorométhoxy-phényl)-1-(2-pipéridin-1-yl-éthyl)-1H-pyrazole, à partir de 345 mg de 3-benzyloxy-4-(5-chloro-thiophèn-2-yl)-1H-pyrazole, 100 mg d'hydrure de sodium à 75 % dans l'huile de vaseline, 305 mg de 1-(2-chloroéthyl)-pipéridine et 10 cm³ de diméthylformamide anhydre. Après purification sur cartouche de silice FC50SI-HP en éluant avec un mélange de dichlorométhane et de méthanol (95 / 5 en volumes), on obtient 400 mg du produit attendu sous forme d'une huile orangée. Spectre de masse (IC) : m/z 402 ([M+H]⁺) (pic de base).

Le 3-benzyloxy-4-(5-chloro-thiophèn-2-yl)-1H-pyrazole peut être préparé de la manière suivante :

Le produit est préparé en suivant le mode opératoire décrit pour la préparation du 3-benzyloxy-4-(4-trifluorométhoxy-phényl)-1H-pyrazole, à partir de 740 mg de 3-benzyloxy-(5-chloro-thiophèn-2-yl)-1-(toluène-4-sulfonyl)-1-pyrazole, 3,8 cm³ d'une solution 1 N de fluorure de n-tétrabutylammonium dans le tétrahydrofurane et 38 cm³ de tétrahydrofurane. Après purification sur cartouche de silice FC50SI-HP en éluant avec un mélange de dichlorométhane et de méthanol (95 / 5 en volumes), on obtient 345 mg du produit attendu sous forme d'une poudre écrue. Spectre de masse (IE) : m/z 290 (M^{+.}), m/z 91 (pic de base)

Le 3-benzyloxy 4-(5-chloro-thiophèn-2-yl)-1-(toluène-4-sulfonyl)-1H-pyrazole peut être préparé de la manière suivante :

A une solution de 1,8 g de 3-benzyloxy-l-(toluène-4-sulfonyl)-4-tributylstannanyl-1H-pyrazole dans 20 cm³ de dioxane, on ajoute, en agitant et sous atmosphère inerte, 589 mg de 2-bromo-5-chlom-thiophène, 32,5 mg de tris(trifuryl)phosphine et 34,7 mg de tris(dibenzylidèneacétone)dipalladium. Le milieu réactionnel est chauffé à une température proche de 100°C pendant 15 heures. Le mélange est ensuite refroidi à une température proche de 20°C, filtré sur supercel. Le filtrat est concentré à sec sous pression réduite (2 kPa), repris par du cyclohexane ; l'insoluble est filtré sur verre fritté, le filtrat est concentré à sec sous pression réduite (2 kPa) ; le résidu obtenu est purifié sur cartouche de silice FC50SI-HP en éluant avec un mélange de cyclohexane et d'acétate d'éthyle (90 / 10 en volumes). On obtient 200 mg de 3-benzyloxy-4-(5-chloro-thiophèn-2-yl)-1-(toluène-4-sulfonyl)-1H-pyrazole sous forme d'une poudre jaune. Spectre de masse (IE) : m/z 444 (M^{+.})m/z 289 et m/z 91 (pic de base).

Le 3-benzyloxy-1-(toluène-4-sulfonyl)-4-tributylstannanyl-1H-pyrazole peut être préparé de la manière suivante :

A une solution de 4,4 g de 3-benzyloxy-4-iodo 1-(toluène-4-sulfonyl)-1H-pyrazole dans 45 cm³ de diméthylformamide, on ajoute, en agitant et sous atmosphère inerte, 325 mg de triphénylphosphine, 5,9 cm³ de 1,1,1,2,2,2-hexabutyl-distannane et 141,3 mg de diacétate de palladium. Le milieu réactionnel est chauffé à une température proche de 80°C pendant 1 heure. Le mélange est ensuite refroidi à une température proche de 20°C, filtré sur supercel. Le filtrat est repris par 200 cm³ d'eau et 100 cm³ d'acétate d'éthyle. La phase organique est décantée, lavée par 3 fois 100 cm³ d'eau, séchée sur du sulfate de magnésium anhydre, filtrée et concentrée à sec sous pression réduite (2 kPa). Le résidu est purifié par chromatographie, sous une pression d'azote de 50 kPa, sur une colonne de gel de silice (granulométrie 20-45µ ; diamètre 2 cm; hauteur 40 cm), en éluant par un mélange de cyclohexane et d'acétate d'éthyle (95 / 5 en volumes). On obtient 4,5 g de 3-benzyloxy-1-(toluène-4-sulfonyl)-4-tributylstannanyl-1H-pyrazole sous forme d'une huile jaune. Spectre de masse (IE) : m/z 618 (M^{+.}), m/z 561 (pic de base).

### Exemple 42

### Chlorhydrate de 4-(3-méthoxy-phényl)-1-(2-pipéridin-1-yl-éthyl)-1H-pyrazol-3-ol

Le produit est préparé en suivant le mode opératoire décrit pour la préparation du 4-(4-trifluorométhoxy-phényl)-1-(2-pipéridin-1-yl-éthyl)-1H-pyrazol-3-ol, à partir de 760 mg de 3-benzyloxy-4-(3-mémoxy-phényl)-1-(2-pipéridin-1-yl-éthyl)-1H-pyrazole, 6,5 cm³ d'acide chlorhydrique 12 N et 6,5 cm³ d'éthanol. Le milieu est concentré à sec sous pression réduite (2 kPa) ; le résidu est précipité dans de l'éthanol. On obtient 232 mg du produit attendu sous forme d'une poudre. Spectre de R.M.N. ¹H (300 MHz, (CD₃)₂SO d6, δ en ppm) : de 1,25 à 1,50 (mf : 1H) ; de 1,60 à 1,90 (mt : 5H) ; de 2,80 à 3,05 (mf : 2H) ; de 3,35 à 3,55 (mf : 4H) ; 3,78 (s : 3H) ; 4,38 (mt : 2H) ; 6,72 (ddd, J = 7 - 6 et 3 Hz : 1H) ; de 7,15 à 7,30 (mt : 3H) ; 8,05 (s : 1H) ; de 9,80 à 10,10 (mf étalé : 1H) ; 10,45 (mf : 1H). Spectre de masse (IE) : m/z 301 (M^{+.}), m/z 98 (pic de base).

Le 3-benzyloxy-4-(3-méthoxy-phényl)-1-(2-pipéridin-1-yl-éthyl)-1H-pyrazole peut être préparé de la manière suivante :

Le produit est préparé en suivant le mode opératoire décrit pour la préparation du 3-benzyloxy-4-(4-trifluorométhoxy-phényl)-1-(2-pipéridin-1-yl-éthyl)-1H-pyrazole, à partir de 590 mg de 3-benzyloxy-4-(3-méthoxy-phényl)-1H-pyrazole, 176 mg d'hydrure de sodium à 75% dans l'huile de vaseline, 542 mg de 1-(2-chloroéthyl)-pipéridine et 10 cm³ de diméthylformamide anhydre. Après purification sur cartouche de silice FCSOSI-HP en éluant avec un mélange de dichlorométhane et de méthanol (98 / 2 en volumes), on obtient 760 mg du produit attendu sous forme d'une huile incolore. Spectre de masse (IE) : ml 391 (M^{+.}), m/z 98 (pic de base).

Le 3-benzyloxy-4-(3-méthoxy-phényl)-1H-pyrazole peut être préparé de la manière suivante :

Le produit est préparé en suivant le mode opératoire décrit pour la préparation du 3-benzyloxy-4-(4-trifluorométhoxy-phényl)-1-pyrazole, à partir de 1,32 g de 3-benzyloxy-4-(3-méthoxy-phényl)-1-(toluène-4-sulfonyl)-1H-pyrazole, 6,94 cm³ d'une solution 1 N de fluorure de n-tétrabutylammonium dans le tétrahydrofurane et 80 cm³ de tétrahydrofurane. Après purification par précipitation dans de l'oxyde de diéthyle et purification sur cartouche de silice FC50SI-HP en éluant avec un mélange de dichlorométhane et de méthanol (95 / 5 en volumes), on obtient 590 mg du produit attendu. Spectre de masse (IE) : m/z 280 (M^{+.}), m/z 91 (pic de base).

Le 3-benzyloxy-4-(3-méthoxy-phényl)-1-(toluène-4-sulfonyl)-1H-pyrazole peut être préparé de la manière suivante :

Le produit est préparé en suivant le mode opératoire décrit pour la préparation du 3-benzyloxy-1-(toluène-4-sulfonyl)-4-(4-trifluorométhoxy-phényl)-1H-pyrazole, à partir de 1,5 g de 3-benzyloxy-4-iodo-1-(toluène-4-sulfonyl)-1H-pyrazole, 1,5 g d'acide 3-méthoxy-phényl boronique, 496 mg de tétrakis(triphénylphosphine)palladium, 4,96 cm³ d'une solution aqueuse 2 N de carbonate de potassium dans un mélange de 30 cm³ de toluène et d'éthanol (4 / 1 en volumes). Après purification sur cartouche de silice FC50SI-HP en éluant avec un mélange de cyclohexane et d'acétate d'éthyle (90/10 en volumes), on obtient 1,13 g du produit attendu sous forme de cristaux jaunes pâles. Spectre de masse (IE) : m/z 434 (M^{+.}), m/z 279 et m/z 91 (pic de base).

### Exemple 43

### Chlorhydrate de 4-(2-méthoxy-phényl)-1-(2-pipéridin-1-yl-éthyl)-1H-pyrazol-3-ol

Le produit est préparé en suivant le mode opératoire décrit pour la préparation du 4-(4-tirifluorométhoxy-phényl)-1-(2-pipéridin-1-yl-éthyl)-1H-pyrazol-3-ol, à partir de 450 mg de 3-benzyloxy-4-(2-méthoxy-phényl)-1-(2-pipéridin-1-yl-éthyl)-1H-pyrazole, 3,8 cm³ d'acide chlorhydrique 12 N et 3,8 cm³ d'éthanol. Le milieu est concentré à sec sous pression réduite (2 kPa) ; le résidu est précipité dans de l'acétonitrile. On obtient 380 mg du produit attendu sous forme d'une poudre jaune. Spectre de R.M.N. ¹H (300 MHz, (CD₃)₂SO d6, δ en ppm) : de 1,25 à 1,50 (mt : 1H) ; de 1,60 à 1,90 (mt : 5H) ; 2,94 (mt : 2H) ; de 3,30 à 3,60 (mt : 4H) ; 3,86 (s : 3H) ; 4,44 (t, J = 6,5 Hz : 2H) ; 6,95 (t dédoublé, J = 7,5 et 1 Hz : 1H) ; 7,05 (d large, J = 7,5 Hz : 1H) ; 7,17 (t dédoublé, J = 7,5 et 1,5 Hz : 1H) ; 7,91 (dd, J = 7,5 et 1,5 Hz : 1H) ; 8,03 (s : 1H) ; de 10,10 à 10,30 (mf : 1H) ; de 10,20 à 10,45 (mf étalé : 1H). Spectre de masse (IC) : m/z 302 ([M+H]⁺) (pic de base).

Le 3-benzyloxy-4-(2-méthoxy phényl)-1-(2-pipéridin-1-yl-éthyl)-1H-pyrazole peut être préparé de la manière suivante :

Le produit est préparé en suivant le mode opératoire décrit pour la préparation du 3-benzyloxy-4-(4-trifluorométhoxy-phényl)-1-(2-pipéridin-1-yl-éthyl)-1H-pyrazole, à partir de 382 mg de 3-benzyloxy-4-(2-méthoxy-phényl)-1H-pyrazole, 114 mg d'hydrure de sodium à 75% dans l'huile de vaseline, 351 mg de 1-(2-chloroéthyl)-pipéridine et 11 cm³ de diméthylformamide anhydre. Après purification sur cartouche de silice FC50SI-HP en éluant avec un mélange de dichlorométhane et de méthanol (95 / 5 en volumes), on obtient 450 mg du produit attendu sous forme d'une huile jaune pâle. Spectre de masse (IE) : m/z 391 (M^{+.}), m/z 98 (pic de base).

Le 3-benzyloxy-4-(2-méthoxy-phényl)-1H-pyrazole peut être préparé de la manière suivante :

Le produit est préparé en suivant le mode opératoire décrit pour la préparation du 3-benzyloxy-4-(4-trifluorométhoxy-phényl)-1H-pyrazole, à partir de 720 mg de 3-benzyloxy-4-(2-méthoxy-phényl)-1-(toluène-4-sulfonyl)-1H-pyrazole, 3,5 cm³ d'une solution N de fluorure de n-tétrabutylammonium dans le tétrahydrofurane et 50 cm³ de tétrahydrofurane. Après purification sur cartouche de silice FC50SI-HP. en éluant avec un mélange de dichlorométhane et de méthanol (95 / 5 en volumes), on obtient 382 mg du produit attendu sous forme d'un solide beige rosé. Spectre de masse (IE) : m/z 280 (M+.), m/z 91 (pic de base).

Le 3-benzyloxy-4-(2-méthoxy-phényl)-1-(toluène-4-sulfonyl)-1H-pyrazole peut être préparé de la manière suivante :

Le produit est préparé en suivant le mode opératoire décrit pour la préparation du 3-benzyloxy-1-(toluène-4-sulfonyl)-4-(4-trifluorométhoxy-phényl)-1-pyrazole, à partir de 1 g de 3-benzyloxy-4-iodo-1-(toluène-4-sulfonyl)-1H-pyrazole, 1 g d'acide 2-méthoxy-phényl boronique, 330 mg de tétrakis(triphénylphosphine)palladium, 3,3 cm³ d'une solution aqueuse 2 N de carbonate de potassium dans un mélange de 15 cm³ de toluène et d'éthanol (4 / 1 en volumes). Après purification sur cartouche de silice FC50SI-HP en éluant avec un mélange de cyclohexane et d'acétate d'éthyle (80 / 20 en volumes), on obtient 720 mg du produit attendu sous forme d'une poudre beige. Spectre de masse (IC) : m/z 435 ([M+H]+) et m/z 281 (pic de base).

### Exemple 44

### Chlorhydrate de 4-(3-hydroxy-phényl)-1-(2-pipéridin-1-yl-éthyl)-1H-pymzol-3-ol

Une solution agitée de 516 mg de chlorhydrate de 4-(3-méthoxy-phényl)-1-(2-pipéridin-1-yl-éthyl)-1H-pyrazol-3-ol dans 13 cm³ de dichlorométhane, sous atmosphère inerte, est refroidie à une température proche de -78°C. On ajoute 4,38 cm³ de tribromure de bore et continue l'agitation 15 heures à une température proche de 20°C. La solution est reprise par du méthanol puis concentrée à sec sous pression réduite (2 kPa). Le résidu est repris par 20 cm³ d'eau et 20 cm³ de dichlorométhane. La phase organique est décantée ; la phase aqueuse est lavée par une solution aqueuse saturée de bicarbonate de sodium, jusqu'à pH 8-8,4 (pH mètre) puis reprise par du dichlorométhane. La phase organique est décantée et concentrée à sec sous pression réduite (2 kPa). La poudre blanche obtenue est reprise par 0,4 cm³ d'acide chlorhydrique 12 N et 5 cm³ de dioxane. Le mélange est agité 10 minutes puis concentré à sec sous pression réduite (2 kPa). On obtient 198 mg de chlorhydrate de 4-(3-hydroxy-phényl)-1-(2-pipéridin-1-yl-éthyl)-1H-pyrazol-3-ol sous forme d'une poudre écrue. Spectre de RM.N. ¹H (300 MHz, (CD3)2SO d6, δ en ppm) : de 1,25 à 1,50 (mt : 1H) ; de 1,60 à 1,90(mt : 5H) ; 2,94 (mt : 2H) ; de 3,30 à 3,55 (mt : 4H) ; 4,40 (t, J = 6,5 Hz : 2H) ; 6,55 (ddd, J = 8 - 3 et 1,5 Hz : 1H) ; de 7,00 à 7,15 (mt : 3H) ; 8,06 (s : 1H); de 9,10 à 9,40 (mf étalé : 1H) ; de 9,90 à 10,10 (mf: 1H) ; de 10,30 à 10,45 (mf : 1H). Spectre de masse (IE) : m/z 287 (M+.), m/z 98 (pic de base).

### Exemple 45

### Chlorhydrate de 4-(4-hydroxy-phényl)-1-(2-pipézidin-1-yl-éthyl)-1H-pyrazol-3-ol

On amène à pH 7 (papier Lyphan), avec une solution aqueuse de soude 1 N, 200 mg de chlorhydrate de 4-(4-méthoxy-phényl)-1-(2-pipéridin-1-yl-éthyl)-1H-pyrazol-3-ol. La phase organique est extraite par du dichlorométhane et concentrée à sec sous pression réduite (2kPa). Le résidu est repris par 5 cm³ de dichlorométhane. La solution agitée, est refroidie à une température proche de -78°C. On ajoute 1,7 cm³ de tribromure de bore et continue l'agitation 15 heures à une température proche de 20°C. La solution est reprise par 20 cm³ d'eau glacée et 10 cm³ de dichlorométhane. La phase organique est décantée ; la phase aqueuse est lavée par du dichlorométhane puis par une solution aqueuse saturée de bicarbonate de sodium jusqu'à pH 8-8,4 (pH mètre), puis reprise par du dichlorométhane. La phase organique est décantée et concentrée à sec sous pression réduite (2 kPa). La poudre blanche obtenue est reprise par 300 µl d'acide chlorhydrique 12 N et 5 cm³ de dioxane. Le mélange est agité 10 minutes puis concentré à sec sous pression réduite (2 kPa). On obtient 101 mg de chlorhydrate de 4-(4-hydroxy-phényl)-1-(2-pipéridin-1-yl-éthyl)-1H-pyrazol-3-ol sous forme d'une poudre écrue. Spectre de R.M.N. ¹H (300 MHz, (CD3)2SO d6, δ en ppm) : de 1,25 à 1,50 (mt : 1H) ; de 1,60 à 1,90 (mt : 5H) ; 2,92 (mt: 2H) ; de 3,30 à 3,50 (mt : 4H) ; 4,38 (t, J = 6,5 Hz : 2H) ; 6,75 (d large, J = 8,5 Hz : 2H) ; 7,45 (d large, J = 8,5 Hz : 2H) ; 7,85 (s : 1H) ; de 9,10 à 9,35 (mf étalé : 1H) ; de 10,00 à 10,20 (mf: 1H) ; de 10,15 à 10,30 (mf: 1H). Spectre de masse (IE) : m/z 287 (M+.), m/z 98 (pic de base).

### Exemple 46

### Dichlorhydrate de 4-(4-méthoxy-phényl)-1-(2-pipéridin-1-yl-éthyl)-1H-pyrazol-3-ol

Une solution de 640 mg de 1-{2-[3-benzyloxy-4-(4-méthoxy phényl)-pyrazol-1-yl]-éthyl}-pipéridine dans 20 cm³ d'éthanol est additionnée de 7 cm³ d'éther diéthylique chlorhydrique 1 N. Après 30 min d'agitation à une température voisine de 20°C, la solution est évaporée à sec sous pression réduite (2,7 kPa). Le résidu est repris par 20 cm³ d'éthanol . La solution obtenue est introduite dans un autoclave, et elle est additionnée de 87 mg de palladium sur charbon à 10 %, puis elle est placée sous hydrogène (8 bars). Après 8 h d'agitation à une température voisine de 30°C, le milieu réactionnel est filtré sur supercel et le filtrat est évaporé. Le résidu est additionné d'éther diisopropylique conduisant à une suspension qui est chauffée au reflux du solvant et filtrée à chaud. Le solide résultant est séché sous vide (2,7 kPa) pour donner 400 mg de dichlorhydrate de 4-(4-méthoxy-phényl)-1-(2-pipéridin-1-yl-éthyl)-1H-pyrazol-3-ol sous forme d'une poudre blanche. Spectre IR (KBr) : 3052; 2933; 2655; 2559; 1578; 1569; 1518; 1501; 1453; 1248; 1170; 1020; 837; 810; 652 et 528 cm⁻¹. Spectre de R.M.N. ¹H (300 MHz, (CD3)2SO d6, δ en ppm) : de 1,30 à 1,50 (mt : 1H) ; de 1,60 à 1,90 (mt : 5H) ; de 2,80 à 3,05 (mt : 2H) ; 3,46 (mt : 4H) ; 3,76 (s : 3H) ; 4,37 (t large, J = 6 Hz : 2H) ; 6,93 (d, J = 8,5 Hz : 2H) ; 7,57 (d, J = 8,5 Hz : 2H) ; 7,92 (s large : 1H) ; de 9,75 à 9,95 (mf étalé: 1H) ; 10,32 (s large : 1H)..

La 1-{2-[3-benzyloxy-4-(4-méthoxy-phényl)-pyrazol-1-yl]-éthyl}-pipéridine peut être préparée de la manière suivante :

A une solution agitée de 1,2 g de 1-[2-(3-benzyloxy-4-bromo-pyrazol-1-yl)-éthyl]-pipéridine dans 60 cm³ de 1,2-diméthoxy-éthane sous atmosphère d'argon, on ajoute 2 g d'acide 4-méthoxy-phényl boronique, 2,85 g de phosphate de potassium et 750 mg de chlorure de bis(triphénylphosphine)palladium. Après 15 h de chauffage au reflux du solvant, le milieu réactionnel est évaporé sous pression réduite (2,7 kPa). Le résidu est additionné d'acétate d'éthyle et d'eau, et il est filtré sur supercel. Le filtrat est décanté, puis la phase organique est lavée successivement par de l'eau, une solution aqueuse saturée d'hydrogénocarbonate, et une solution aqueuse saturée de chlorure de sodium ; elle est séchée sur sulfate de magnésium, filtrée et évaporée sous pression réduite (2,7 kPa). L'huile brune obtenue (3,6 g) est purifiée par chromatographie-flash sur alumine CTB1 sous pression d'argon (50 kPa) [éluant : cyclohexane / acétate d'éthyle (90 / 10 en volumes)]. Après concentration des fractions sous pression réduite (2,7 kPa), on obtient 700 mg de -{2-[3-benzyloxy-4-(4-méthoxy-phényl)-pyrazol-1-yl]-éthyl}-pipéridine sous forme d'une huile jaune. Spectre de masse (IE) : m/z 391 (M^{+.}), m/z 280 [(M - C₇H₁₃N)^{+.}], m/z 111 C₇H₁₃N)^{+.}], m/z 98 (C₆H₁₂N⁺), m/z 91(C₇H₇⁺).

### Exemple 47

### Dichlorhydrate de 4-(3-fluoro-phényl)-1-(2-pipéridin-1-yl-éthyl)-1H-pyrazol-3-ol

Une solution agitée de 400 mg de 1-{2-[3-benzyloxy-4-(3-fluoro-phényl)-pyrazol-1-yl]-éthyl}-pipéridine dans 3,5 cm³ d'éthanol est additionnée de 3,5 cm³ d'acide chlorhydrique 12 N. Après 7 h au reflux du solvant, puis 15 h à une température voisine de 20°C, le milieu réactionnel est évaporé à sec sous pression réduite (2,7 kPa). Le résidu est séché sous vide (2,7 kPa) à 45 °C pendant 1 h, et il est trituré dans de l'oxyde de diisopropyle. Le précipité formé est filtré et séché sous vide (2,7 kPa) pour donner 350 mg de dichlorhydrate de 4-(3-fluoro-phényl)-1-(2-pipéridin-1-yl-éthyl)-1H-pyrazol-3-ol sous forme d'un solide blanc. Spectre IR (KBr) : 2951; 2647; 2540; 1619; 1586; 1530; 1456; 1267; 1178; 882; 785; 687 et 523 cm⁻¹. Spectre de R.M.N. ¹H (300 MHz, (CD₃)₂SO d6 avec ajout de quelques gouttes de CD₃COOD d4, δ en ppm) : 1,42 (mt : 1H) ; de 1,60 à 1,90 (mt : six ; 2,95 (mt : 2H) ; de 3,40 à 3,55 (mt : 2H) ; 3,50 (t, J = 6,5 Hz : 2H) ; 4,39 (t, J = 6,5 Hz : 2H) ; 6,95 (tdd, J = 7,5 - 3 et 1 Hz : 1H) ; de 7,30 à 7,50 (mt : 3H) ; 8,09 (s : 1H).

La 1-{2-[3-benzyloxy-4-(3-fluoro-phényl)-pyrazol-1-yl]-éthyl}-pipéridine peut être préparée de la manière suivante :

A une solution agitée sous atmosphère d'argon de 1 g de 1-[2-(3-benzyloxy-4-bromo-pyrazol-1-yl)-éthyl]-pipéridine dans un mélange de 20 cm³ de toluène et de 5 cm³ d'éthanol, on ajoute 1,15 g d'acide 3-fluoro-phényl boronique, 4,1 cm³ d'une solution aqueuse de carbonate de potassium 2 N, et 475 mg de tétrakis(triphénylphosphine)palladium. Après 3 h de chauffage au reflux du solvant et 15 h à une température voisine de 20°C, le milieu réactionnel est additionné d'acétate d'éthyle et d'eau, et il est filtré sur supercel. Le filtrat est décanté, puis la phase organique est lavée successivement par deux fois de l'eau et une solution aqueuse saturée de chlorure de sodium ; elle est séchée sur sulfate de magnésium, filtrée et évaporée sous pression réduite (2,7 kPa). L'huile brune obtenue (2,1 g) est purifiée par chromatographie-flash sur silice sous pression d'argon (50 kPa) [éluant : acétate d'éthyle]. Après concentration des fractions sous pression réduite, on obtient 400 mg de 1-{2-[3-benzyloxy-4-(3-fluoro-phényl)-pyrazol-1-yl]-éthyl}-pipéridine sous forme d'une huile jaune. Spectre IR (CCl₄) : 2939; 2854; 2802; 1617; 1586; 1509; 1463; 1432; 1359; 1272; 1187; 1169;1160; 1043; 883; 695 et 687 cm⁻¹.

### Exemple 48

### Dichlorhydrate de 1-(2-pipéridin-1-yl-éthyl)-4-(3-trifluorométhyl-phényl)-1H-pyrazol-3-ol

Une solution agitée de 470 mg de 1-{2-[3-benzyloxy-4-(3-trifluorométhyl-phényl)-pyrazol-1-yl]-éthyl}-pipéridine dans 5 cm³ d'éthanol est additionnée de 5 cm³ d'acide chlorhydrique 12 N. Après 7 h au reflux du solvant, puis 15 h à une température voisine de 20°C, le milieu réactionnel est évaporé à sec sous pression réduite (2,7 kPa). Le résidu est séché sous vide (2,7 kPa) à 45 °C pendant 1 h, et il est trituré dans de l'oxyde de diisopropyle. Le précipité formé est filtré et séché sous vide (2,7 kPa) pour donner 350 mg de dichlorhydrate de 1-(2-pipéridin-1-yl-éthyl)-4-(3-trifluorométhyl-phényl)-1H-pyrazol-3-ol sous forme d'un solide jaune pâle. Spectre IR (KBr) : 2955; 2629; 2533; 1619; 1533; 1325; 1188; 1170; 1117; 1076; 800; 696 et 688 cm⁻¹. Spectre de R.M.N. ¹H (300 MHz, (CD₃)₂SO d6, δ en ppm) : 1,40 (mt : 1H) ; de 1,60 à 1,90 (mt : 5H) ; 2,94 (mt : 2H) ; de 3,30 à 3,55 (mt : 4H) ; 4,43 (t, J = 6,5 Hz : 2H) ; 7,50 (d large, J = 7,5 Hz : 1H) ; 7,59 (t, J = 7,5 Hz : 1H) ; 7,95 (d large, J = 7,5 Hz : 1H) ; 8,01 (s large : 1H) ; 8,22 (s : 1H) ; 10,24 (mf : 1H) ; de 10,60 à 10,90 (mf étalé : 1H).

La 1-{2-[3-benzyloxy-4-(3-trifluorométhyl-phényl)-pyrazol-1-yl]-éthyl}-pipéridine peut être préparée de la manière suivante :

A une solution agitée sous atmosphère d'argon de 1 g de 1-[2-(3-benzyloxy-4-bromo-pyrazol-1-yl)-éthyl]-pipéridine dans un mélange de 20 cm³ de toluène et de 5 cm³ d'éthanol, on ajoute 1,58 g d'acide 3-trifluorométhyl-phényl boronique, 4,1 cm³ d'une solution aqueuse de carbonate de potassium 2 N, et 475 mg de tétrakis(triphénylphosphine)palladium. Après 3 h de chauffage au reflux du solvant, le milieu réactionnel est évaporé sous pression réduite (2,7 kPa). Le résidu est additionné d'acétate d'éthyle et d'eau, et il est filtré sur supercel. Le filtrat est décanté, puis la phase organique est lavée successivement par deux fois de l'eau et une solution aqueuse saturée de chlorure de sodium ; elle est séchée sur sulfate de magnésium, filtrée et évaporée sous pression réduite (2,7 kPa). L'huile brune obtenue (3 g) est purifiée par chromatographie-flash sur silice sous pression d'argon (50 kPa) [éluant : acétate d'éthyle / méthanol (95 / 5 en volumes)]. Après concentration des fractions sous pression réduite (2,7 kPa), on obtient un résidu qui est repris par de l'acétate d'éthyle. La solution est traitée par du noir de charbon, filtrée et évaporée sous pression réduite (2,7 kPa) pour donner 470 mg de 1-{2-[3-benzyloxy-4-(3-trifluorométhyl-phényl)-pyrazol-1-yl]-éthyl}-pipéridine sous forme d'une huile orangée. Spectre de R.M.N. ¹H (300 MHz, (CD₃)₂SO d6, δ en ppm) : de 1,30 à 1,55 (mt : 6H) ; 2,40 (mf : 4H) ; 2,70 (mf : 2H) ; 4,10 (mf : 2H) ; 5,34 (s : 2H) ; de 7,30 à 7,55 (mt : 6H) ; 7,58 (t, J = 7,5 Hz : 1H) ; 7,92 (d large, J = 7,5 Hz : 1H) ; 8,03 (s large : 1H) ; 8,25 (s : 1H).

### Exemple 49

### Dichlorhydrate de 1-(2-pipéridin-1-yl-éthyl)-4-pyridin-3-yl-1H-pyrazol-3-oL

Une solution agitée de 720 mg de 3-[3-benzyloxy-1-(2-pipéridin-1-yl-éthyl)-1H-pyrazol-4-yl]-pyridine dans 7 cm³ d'éthanol est additionnée de 7 cm³ d'acide chlorhydrique 12 N. Après 7 h au reflux du solvant, puis 15 h à une température voisine de 20°C, le milieu réactionnel est évaporé à sec sous pression réduite (2,7 kPa). Le résidu est repris par de l'éthanol, puis le mélange est évaporé à sec sous vide (2,7 kPa). L'opération est répétée deux fois, puis le solide est séché sous vide (2,7 kPa) à 45 °C pendant 1 h, et il est trituré dans l'acétone. Le précipité formé est filtré et séché sous vide (2,7 kPa) pour donner 190 mg de dichlorhydrate de 1-(2-pipéridin-1-yl-éthyl)-4-pyridin-3-yl-1H-pyrazol-3-ol sous forme d'un solide blanc. Spectre IR (KBr) : 2970; 2434; 2931; 1601; 1551; 1460; 1307; 1178; 825; 691 et 624 cm⁻¹. Spectre de R.M.N. ¹H (300 MHz, (CD3)2SO d6, δ en ppm) : 1,40 (mt : 1H) ; de 1,60 à 1,90 (mt : 5H) ; 2,94 (mt : 3H) ; de 3,25 à 3,65 (mt : 3H) ; 4,50 (t, J = 6,5 Hz : 2H) ; 7,88 (dd large, J = 8 et 5 Hz : 1H) ; 8,39 (s : 1H) ; 8,55 (d large, J = 8 Hz : 1H) ; 8,60 (d large, J = 5 Hz : 1H) ; 9,02 (d large, J = 1,5 Hz : 1H) ; 10,60 (mf : 1H) ; 11,20 (mf : 1H).

La 3-[3-benzyloxy-1-(2-pipéridin-1-yl-éthyl)-1H-pyrazol-4-yl]-pyridine peut être préparée de la manière suivante :

A une solution agitée de 950 mg de 1-[2-(3-benzyloxy-4-bromo-pyrazol-1-yl)-éthyl]-pipéridine dans 100 cm³ de dioxane et sous atmosphère d'argon, on ajoute 580 mg de 3-diéthylboranyl-pyridine, 690 mg de carbonate de sodium dissous dans 20 cm³ d'eau, et 390 mg de tétrakis(triphénylphosphine)palladium. Après 3 h de chauffage au reflux du solvant, le milieu réactionnel est refroidi à une température voisine de 20°C, il est additionné d'acétate d'éthyle et d'eau, et il est filtré sur supercel. Le filtrat est décanté, puis la phase organique est lavée par de l'eau et une solution aqueuse saturée de chlorure de sodium ; elle est séchée sur sulfate de magnésium, filtrée et évaporée sous pression réduite (2,7 kPa). L'huile brune obtenue (2 g) est purifiée par chromatographie-flash sur alumine CTB1 sous pression d'argon (50 kPa) [éluant : cyclohexane / acétate d'éthyle (97 / 3 en volumes), puis acétate d'éthyle]. Après concentration des fractions sous pression réduite (2,7 kPa), on obtient un résidu qui est purifié par chromatographie-flash sur silice sous pression d'argon (50 kPa) [éluant : acétate d'éthyle / méthanol (90 / 10 en volumes)]. Après concentration des fractions sous pression réduite (2,7 kPa), on obtient 220 mg de 3-[3-benzyloxy-1-(2-pipéridin-1-yl-éthyl)-1H-pyrazol-4-yl]-pyridine sous forme d'une huile jaune. Spectre IR (CCl₄) 2940; 2854; 2801; 1599; 1575; 1505; 1453; 1362; 1167; 1020; 708 et 702 cm⁻¹.

### Exemple 50

### Dichlorhydrate de 4-(4-chloro-phényl)-1-(2-pipéridin-1-yl-éthyl)-1H-pyrazol-3-ol

Une solution agitée de 470 mg de 1-{2-[3-benzyloxy-4-(4-chloro-phényl)-pyrazol-1-yl]-éthyl}-pipéridine dans 3,5 cm³ d'éthanol est additionnée de 3,5 cm³ d'acide chlorhydrique 12 N. Après 7 h au reflux du solvant, puis 15 h à une température voisine de 20°C, le milieu réactionnel est évaporé à sec sous pression réduite (2,7 kPa). Le résidu est séché sous vide (2,7 kPa) à 45 °C pendant 2 h, puis il est trituré dans de l'éther diisopropylique. Le précipité formé est filtré et séché sous vide (2,7 kPa) pour donner 430 mg de dichlorhydrate 4-(4-chloro-phényl)-1-(2-pipéridin-1-yl-éthyl)-1H-pyrazol-3-ol sous forme d'un solide blanc. Spectre IR (KBr) : 2952; 2640; 2534; 1607; 1578; 1552; 1521; 1455; 1191; 1093; 1011; 830; 818 et 516 cm⁻¹. Spectre de R.M.N. ¹H (300 MHz, (CD₃)₂SO d6, δ en ppm) : 1,39 (mt : 1H) ; de 1,60 à 1,85 (mt : 5H) ; 2,92 (mt : 2H) ; de 3,35 à 3,45 (mt : 2H) ; 3,46 (t large, J = 6,5 Hz : 2H) ; 4,42 (t, J = 6,5 Hz : 2H) ; 7,39 (dmt, J = 8,5 Hz : 2H) ; 7,67 (dmt, J = 8,5 Hz : 2H) ; 8,08 (s : 1H) ; 10,39 (mf : 1H).

La 1-{2-[3-benzyloxy-4-(4-chloro-phény)-pyraol-1-yl]-éthyl}-pipéridine peut être préparée de la manière suivante :

A une suspension de 132 mg d'hydrure de sodium (à 75% dans l'huile de vaseline) dans 15 cm³ de diméthylformamide sous atmosphère d'argon et sous agitation, est ajoutée une solution de 440 mg de 3-benzyloxy-4-(4-chloro-phényl)-1H pyrazole dans 20 cm³ de diméthylformamide. Après 30 min de chauffage à 50°C, le mélange est agité 30 min à une température voisine de 20°C, puis il est additionné de 400 mg de chlorhydrate de 1-(2-chloro-éthyl)-pipéridine. Le milieu réactionnel est agité 15 h à une température voisine de 20°C, puis il est jeté dans de l'eau. La phase aqueuse est extraite deux fois par de l'acétate d'éthyle. Les phases organiques sont réunies, lavées successivement par de l'eau et une solution aqueuse saturée de chlorure de sodium, séchées sur du sulfate de magnésium, filtrées et évaporées sous pression réduite (2,7 kPa) pour donner une huile jaune pâle qui est purifiée par chromatographie-flash sur alumine CTB1 sous pression d'argon (50 kPa) [éluant : cyclohexane / acétate d'éthyle (95 / 5. puis 90 / 10 en volumes)]. Après concentration des fractions sous pression réduite, on obtient 470 mg de 1-{2-[3-benzyloxy-4-(4-chloro-phényl)-pyrazol-l-yl]-éthyl}-pipéridine sous forme d'une huile jaune pâle. Spectre IR (CCl₄) : 2938; 1574 ; 1509 ; 1482 ; 1452 ; 1358 ; 1171 ; 1094 ; 1037 ; 1014 ; 834 ; 695 et S 11 cm⁻¹.

Le 3-benzyloxy-4-(4-chloro-phényl)-1H-pyrazole peut être préparé de la manière suivante :

A une solution agitée sous atmosphère d'argon de 800 mg de 3-benzyloxy-4-(4-chloro-phényl)-1-(toluène-4-sulfbnyl)-1H-pyrazole dans 20 cm³ de tétrahydrofurane, on ajoute 2 cm³ d'une solution 1 N de fluorure de tétrabutyl-ammonium dans le tétrahydrofurane. Après 2 h de chauffage au reflux du solvant, 0,5 cm³ de solution 1 N de fluorure de tétrabutyl-ammonium dans le tétrahydrofurane est additionné au milieu réactionnel qui est maintenu 15 h à 60°C. On ajoute encore 0,5 cm³ de solution 1 N de fluorure de tétrabutyl-ammonium dans le tétrahydrofurane à la solution qui est chauffée au reflux du solvant 2h supplémentaires. Le milieu réactionnel est alors refroidi à une température voisine de 20°C et évaporé sous pression réduite (2,7 kPa). Le résidu est additionné d'acétate d'éthyle, et la phase organique est lavée successivement par deux fois de l'eau et une solution aqueuse saturée de chlorure de sodium ; elle est séchée sur sulfate de magnésium, filtrée et évaporée sous pression réduite (2,7 kPa). Le solide jaune résultant est purifié par chromatographie-flash sur silice sous pression d'argon (50 kPa) [éluant : cyclohexane / acétate d'éthyle (80 / 20 en volumes), puis acétate d'éthyle]. Après concentration des fractions sous pression réduite (2,7 kPa), on obtient 440 mg de 3-benzyloxy-4-(4-chloro-phényl)-1H-pyrazole sous la forme d'un solide blanc. Spectre de masse (IE) : m/z 284 (M^{+.}), m/z 206 [(M - C₆H₆)^{+.}], m/z 91 (C₇H₇⁺).

Le 3-benzyloxy-4-(4-chloro-phényl)-1-(toluène-4-sulfonyl)-1H-pyrazole peut être préparé de la manière suivante :

A une solution agitée sous atmosphère d'argon de 1,1 g de 3-benzyloxy-4-iodo-1-(toluène-4-sulfonyl)-1H-pyrazole dans 20 cm³ de toluène additionnés de 5 cm³ d'éthanol, on ajoute 1,15 g d'acide 4-chloro-phényl boronique, 3,6 cm³ d'une solution aqueuse de carbonate de potassium 2 N, et 360 mg de tétrakis(triphénylphosphine)palladium. Après 15 h de chauffage au reflux du solvant, le milieu réactionnel est évaporé sous pression réduite (2,7 kPa). Le résidu est additionné d'acétate d'éthyle, d'eau et de noir de charbon, et il est filtré sur supercel. Le filtrat est décanté, puis la phase organique est lavée successivement par deux fois de l'eau et une solution aqueuse saturée de chlorure de sodium ; elle est séchée sur sulfate de magnésium, filtrée et évaporée sous pression réduite (2,7 kPa). L'huile brune obtenue (2,6 g) est purifiée par chromatographie-flash sur silice sous pression d'argon (50 kPa) [éluant : cyclohexane / acétate d'éthyle (90 / 10 en volumes)]. Après concentration des fractions sous pression réduite (2,7 kPa), on obtient 800 mg de 3-benzyloxy-4-(4-chloro-phényl)-1-(toluène-4-sulfonyl)-1H-pyrazole sous forme d'un solide orangé. Spectre de masse (IC) : m/z 456 (MNH4⁺), m/z 439 (MH⁺).

### Exemple 51

### Dichlorhydrate de 4-(3-chloro-phényl)-1-(2-pipéridin-1-yl-éthyl)-1H-pyrazol-3-ol

Une solution agitée de 550 mg de 1-{2-[3-benzyloxy-4-(3-chloro-phényl)-pyrazol-1-yl]-éthyl}-pipéridine dans 5 cm³ d'éthanol est additionnée de 5 cm³ d'acide chlorhydrique 12 N. Après 7 h au reflux du solvant, puis 15 h à une température voisine de 20°C, le milieu réactionnel est évaporé à sec sous pression réduite (2,7 kPa). Le résidu est séché sous vide (2,7 kPa) à 45 °C pendant 2 h, puis il est trituré dans de l'éther diisopropylique. Le précipité formé est filtré et séché sous vide (2,7 kPa) pour donner 460 mg de dichlorhydrate 4-(3-chloro-phényl)-1-(2-pipéridin-1-yl-éthyl)-1H-pyrazol-3-ol sous forme d'un solide blanc. Spectre IR (KBr) : 2951 ; 2637 ; 2436 ; 1394 ; 1603 ; 1565 ;1521 ;1454 ; 1180 ;778 et 689 cm⁻¹. Spectre de R.M.N. ¹H (300 MHz, (CD3)2SO d6, δ en ppm) : 1,40 (mt : 1H) ; de 1,60 à 1,90 (mt : 5H) ; 2,95 (mt : 2H) ; de 3,35 à 3,55 (mt : 4H) ; 4,40 (t, J = 6,5 Hz : 2H) ; 7,20 (dmt, J = 8 Hz : 1H) ; 7,37 (t, J = 8 Hz : 1H) ; 7,62 (d large, J = 8 Hz : 1H) ; 7,73 (t, J = 2 Hz : 1H) ; 8,14 (s : 1H) ; 9,90 (mf : 1H) ; 10,69 (mf : 1H).

La 1-{2-[3-benzyloxy-4-(3-chloro-phényl)-pyrazol-1-yl]-éthyl}-pipéridine peut être préparée de la manière suivante :

A une suspension de 142 mg d'hydrure de sodium (à 75% dans l'huile de vaseline) dans 15 cm³ de diméthylformamide sous atmosphère d'argon et sous agitation, est ajoutée une solution de 500 mg de 3-benzyloxy-4-(3-chloro-phényl)-1H-pyrazole dans 20 cm³ de diméthylformamide. Après 30 min de chauffage à 50°C, le mélange est agité 30 min à une température voisine de 20°C, puis il est additionné d'une solution de 500 mg de chlorhydrate de 1-(2-chloro-éthyl)-pipéridine. Le milieu réactionnel est agité 15 h à une température voisine de 20°C, puis il est jeté dans de l'eau. La phase aqueuse est extraite deux fois par de l'acétate d'éthyle. Les phases organiques sont réunies, lavées successivement par de l'eau et une solution aqueuse saturée de chlorure de sodium, séchées sur du sulfate de magnésium, filtrées et évaporées sous pression réduite (2,7 kPa) pour donner une huile jaune qui est purifiée par chromatographie-flash sur alumine CTB1 sous pression d'argon (50 kPa) [éluant : cyclohexane / acétate d'éthyle (95 / 5 en volumes)]. Après concentration des fractions sous pression réduite, on obtient 550 mg de 1-{2-[3-benzyloxy-4-(3-chloro-phényl)-pyrazol-1-yl]-éthyl}-pipéridine sous forme d'une huile jaune. Spectre IR (CCl₄) :2938; 2853; 1603; 1574; 1507; 1452; 1357; 1260; 1174; 1046; 997; 695 et 687 cm⁻¹.

Le 3-benzyloxy-4-(3-chloro-phényl)-1H-pyrazole peut être préparé de la manière suivante :

A une solution agitée sous atmosphère d'argon de 810 mg de 3-benzyloxy-4-(3-chloro-phényl)-1-(toluène-4-sulfonyl)-1H-pyrazole dans 20 cm³ de tétrahydrofurane, on ajoute 4,6 cm³ d'une solution 1 N de fluorure de tétrabutyl-ammonium dans le tétrahydrofurane. Après 15 h de chauffage au reflux du solvant, le milieu réactionnel est évaporé sous pression réduite (2,7 kPa) et le résidu est additionné d'acétate d'éthyle. La phase organique est lavée successivement par deux fois de l'eau et une solution aqueuse saturée de chlorure de sodium ; elle est séchée sur sulfate de magnésium, filtrée et évaporée sous pression réduite (2,7 kPa). L'huile résultante (0,7 g) est purifiée par chromatographie-flash sur silice sous pression d'argon (50 kPa) [éluant : cyclohexane / acétate d'éthyle (70 / 30 en volumes)] . Après concentration des fractions sous pression réduite (2,7 kPa), on obtient 500 mg de 3-benzyloxy-4-(3-chloro-phényl)-1H-pyrazole sous la forme d'un solide blanc. Spectre IR (KBr) : 3148; 2957; 1601; 1505; 1446; 1422; 1355; 1237; 1229; 1046; 785; 729; 682 et 597 cm⁻¹.

Le 3-benzyloxy-4-(3-chloro-phényl)-1-(toluène-4-sulfonyl-1H-pyrazole peut être préparé de la manière suivante :

A une solution agitée sous atmosphère d'argon de 1 g de 3-benzyloxy-4-iodo-1-(toluène-4-sulfonyl)-1H-pyrazole dans 20 cm³ de toluène additionnés de 5 cm³ d'éthanol, on ajoute 1,03 g d'acide 3-chloro-phényl boronique, 3,3 cm³ d'une solution aqueuse de carbonate de potassium 2 N, et 380 mg de tétrakis(triphénylphosphine)palladium. Après 2,5 h de chauffage au reflux du solvant, le milieu réactionnel est évaporé sous pression réduite (2,7 kPa). Le résidu est additionné d'acétate d'éthyle, d'eau et de noir de charbon, et il est filtré sur supercel. Le filtrat est décanté, puis la phase organique est lavée successivement par deux fois de l'eau et une solution aqueuse saturée de chlorure de sodium ; elle est séchée sur sulfate de magnésium, filtrée et évaporée sous pression réduite (2,7 kPa). L'huile orangée obtenue (2 g) est purifiée par chromatographie-flash sur silice sous pression d'argon (50 kPa) [éluant : cyclohexane / acétate d'éthyle (97 / 3 en volumes)]. Après concentration des fractions sous pression réduite (2,7 kPa), on obtient 810 mg de 3-benzyloxy-4-(3-chloro-phényl)-1-(toluène-4-sulfonyl)-1H-pyrazole sous forme d'un solide jaune. Spectre IR (KBr) : 3098; 1604; 1508; 1372; 1357; 1189; 1180; 1094; 991; 790; 672; 586; 554 et 536 cm⁻¹.

### Exemple 52

### Dichlorhydrate de 4-(2-fluoro-phényl)-1-(2-pipéridin-1-yl-éthyl)-1H-pyrazol-3-ol:

Une solution agitée de 800 mg de 1-{2-[3-benzyloxy-4-(2-fluoro-phényl)-pyrazol-1-yl]-éthyl}-pipéridine dans 10 cm³ d'éthanol est additionnée de 7 cm³ d'acide chlorhydrique 12 N. Après 7 h au reflux du solvant, puis 15 h à une température voisine de 20°C, le milieu réactionnel est évaporé à sec sous pression réduite (2,7 kPa). Le brut est repris par de l'éthanol, puis le mélange est évaporé à sec sous pression réduite (2,7 kPa); l'opération est répétée deux fois. Le résidu est trituré dans de l'éther diisopropylique, et le précipité formé est filtré, puis il est dissous à chaud dans de l'éthanol. Les cristaux apparus après refroidissement de la solution dans un bain de glace, sont filtrés et séchés sous vide (2,7 kPa) pour donner 470 mg de dichlorhydrate 4-(2-fluoro-phényl)-1-(2-pipéridin-1-yl-éthyl)-1H-pyrazol-3-ol sous forme d'un solide blanc. Spectre IR (KBr) : 2947; 2621; 2540; 1620; 1538; 1463; 1231; 1186; 1093; 970; 761 et 656 cm⁻¹. Spectre de R.M.N. ¹H (300 MHz, (CD3)2SO d6, δ en ppm) : 1,41 (mt : 1H) ; de 1,60 à 1,90 (mt : 5H) ; 2,94 (mt : 2H) ; de 3,35 à 3,55 (mt : 4H) ; 4,46 (t large, J = 6,5 Hz : 2H) ; de 7,15 à 7,30 (mt : 3H) ; de 7,90 à 8,05 (mt : 2H) ; 10,15 (mf : 1H) ; 10,65 (s large : 1H).

La 1-{2-[3-benzyloxy-4-(2-fluoro-phényl)-pyrazol-1-yl]-éthyl}-pipéridine peut être préparée de la manière suivante :

A une suspension de 200 mg d'hydrure de sodium (à 75 % dans l'huile de vaseline) dans 10 cm³ de diméthylformamide sous atmosphère d'argon et sous agitation, est ajoutée une solution de 650 mg de 3-benzyloxy-4-(2-fluoro-phényl)-1H-pyrazole dans 20 cm³ de diméthylformamide. Après 30 min de chauffage à 50°C, le mélange est agité 30 min à une température voisine de 20°C, puis il est additionné de 625 mg de chlorhydrate de 1-(2-chloro-éthyl)-pipéridine. Le milieu réactionnel est agité 15 h à une température voisine de 20°C, puis il est jeté dans 100 cm³ d'eau. La phase aqueuse est extraite deux fois par de l'acétate d'éthyle. Les phases organiques sont réunies, lavées successivement par deux fois de l'eau et une solution aqueuse saturée de chlorure de sodium, séchées sur du sulfate de magnésium filtrées et évaporées sous pression réduite (2,7 kPa) pour donner une huile jaune (1,1 g) qui est purifiée par chromatographie-flash sur alumine CTB1 sous pression d'argon (50 kPa) [éluant : cyclohexane / acétate d'éthyle (95 / 5 en volumes)]. Après concentration des fractions sous pression réduite, on obtient 800 mg de 1-{2-[3-benzyloxy-4-(2-fluoro-phényl)-pyrazol-1-yl]-éthyl}-pipéridine sous forme d'une huile jaune. Spectre IR (CCl₄) : 2938; 2855; 2801; 1572; 1512; 1486; 1358; 1175; 1044; 1027 et 696 cm⁻¹.

Le 3-benzyloxy-4-(2-fluoro-phényl)-1H-pyrazole peut être préparé de la manière suivante :

A une solution agitée sous atmosphère d'argon de 1,25 g de 3-benzyloxy-4-(2-fluoro-phényl)-1-(toluène-4-sulfonyl)-1H-pyrazole dans 30 cm³ de tétrahydrofurane, on ajoute 7,4 cm³ d'une solution 1 N de fluorure de tétrabutyl-ammonium dans le tétrahydrofurane. Après 15 h de chauffage au reflux du solvant, le milieu réactionnel est évaporé sous pression réduite (2,7 kPa) et le résidu est additionné d'acétate d'éthyle. La phase organique est lavée successivement par deux fois de l'eau et une solution aqueuse saturée de chlorure de sodium ; elle est séchée sur sulfate de magnésium, filtrée et évaporée sous pression réduite (2,7 kPa). L'huile résultante (0,92 g) est purifiée par chromatographie-flash sur silice sous pression d'argon (50 kPa) [éluant : cyclohexane / acétate d'éthyle (70 / 30 en volumes)]. Après concentration des fractions sous pression réduite (2,7 kPa), on obtient 650 mg de 3-benzyloxy-4-(2-fluoro-phényl)-1H-pyrazole sous la forme d'un solide blanc. Spectre IR (KBr) : 3161; 2954; 2691; 1572; 1474; 1440; 1353; 1264; 1045; 1036; 1027; 759; 729 et 654 cm⁻¹.

Le 3-benzyloxy-4-(2-fluoro-phényl)-1-(toluène-4-sulfonyl)-1H-pyrazole peut être préparé de la manière suivante :

A une solution agitée sous atmosphère d'argon de 1,5 g de 3-benzyloxy-4-iodo-1-(toluène-4-sulfonyl)-1H-pyrazole dans un mélange de 20 cm³ de toluène et de 5 cm³ d'éthanol, on ajoute 1,4 g d'acide 2-fluoro-phényl boronique, 5 cm³ d'une solution aqueuse de carbonate de potassium 2 N, et 500 mg de tétrakis(triphénylphosphine)palladium. Après 3 h de chauffage au reflux du solvant, le milieu réactionnel est refroidi à une température voisine de 20°C et évaporé sous pression réduite (2,7 kPa). Le résidu est additionné d'acétate d'éthyle, d'eau et de noir de charbon, et il est filtré sur supercel. Le filtrat est décanté, puis la phase organique est lavée successivement par deux fois de l'eau et une solution aqueuse saturée de chlorure de sodium ; elle est séchée sur sulfate de magnésium, filtrée et évaporée sous pression réduite (2,7 kPa). L'huile orangée obtenue (2 g) est purifiée par chromatographie-flash sur silice sous pression d'argon (50 kPa) [éluant : cyclohexane / acétate d'éthyle (95 / 5 en volumes)]. Après concentration des fractions sous pression réduite (2,7 kPa), on obtient 1,25 g de 3-benzyloxy-4-(2-fluoro-phényl)-1-(toluène-4-sulfonyl)-1H-pyrazole sous forme d'une huile orangée. Spectre de masse (IE) : m/z 422 (M⁺), m/z 267 [(M - C7H7O2S)⁺], m/z 91 (C7H7⁺).

### Exemple 53

### Chlorhydrate de 4-(2-chloro-phényl)-1-(2-pipéridin-1-yl-éthyl)-1H-pyrazol-3-ol

Une solution agitée de 570 mg de 1-{2-[3-benzyloxy-4-(2-chloro-phényl)-pyrazol-1-yl]-éthyl}-pipéridine dans 7 cm³ d'éthanol est additionnée de 5 cm³ d'acide chlorhydrique 12 N. Après 7 h au reflux du solvant, puis 15 h à une température voisine de 20°C, le milieu réactionnel est évaporé à sec sous pression réduite (2,7 kPa). Le résidu est repris par de l'éthanol, puis le mélange est évaporé à sec sous vide (2,7 kPa). L'opération est répétée deux fois, puis la laque obtenue est séchée sous vide (2,7 kPa) à 45°C pendant 30 min, puis elle est dissoute à chaud dans de l'éthanol. Les cristaux formés après refroidissement de la solution dans un bain de glace, sont filtrés et séchés sous vide (2,7 kPa) pour donner 380 mg de chlorhydrate de 4-(2-chloro-phényl)-1-(2-pipéridin-1-yl-éthyl)-1H-pyrazol-3-ol sous forme d'un solide blanc. Spectre IR (KBr) : 3097; 2939; 2674; 2545; 1579; 1517; 1439; 1224; 1171; 935; 758 et 653 cm⁻¹. Spectre de R.M.N. ¹H (200 MHz, (CD₃)₂SO d6 avec ajout de quelques gouttes de CD₃COOD d4, à une température de 363 K, δ en ppm) : 1,60 (mt : 2H) ; 1,81 (mt : 4H) ; 3,20 (mf : 4H) ; 3,49 (t large, J = 6,5 Hz : 2H) ; 4,42 (t large, J = 6,5 Hz : 2H) ; 7,26 (t large, J = 7,5 Hz : 1H) ; 7,35 (t large, J = 7,5 Hz : 1H) ; 7,48 (d large, J = 7,5 Hz : 1H) ; 7,66 (d large, J = 7,5 Hz : 1H) ; 7,93 (s large : 1H).

La 1-{2-[3-benzyloxy-4-(2-chloro-phényl)-pyrazol-1-yl]-éthyl}-pipéridine peut être préparée de la manière suivante :

A une suspension de 140 mg d'hydrure de sodium (à 75 % dans l'huile de vaseline) dans 10 cm³ de diméthylformamide sous atmosphère d'argon et sous agitation, est ajoutée une solution de 500 mg de 3-benzyloxy-4-(2-chloro-phényl)-1H-pyrazole dans 20 cm³ de diméthylformamide. Après 30 min de chauffage à 50°C, le mélange est agité 30 min à une température voisine de 20°C, puis il est additionné de 453 mg de chlorhydrate de 1-(2-chloro-éthyl)-pipéridine. Le milieu réactionnel est agité 15 h à une température voisine de 20°C, puis il est jeté dans 100 cm³ d'eau. La phase aqueuse est extraite deux fois par de l'acétate d'éthyle. Les phases organiques sont réunies, lavées successivement par deux fois de l'eau et une solution aqueuse saturée de chlorure de sodium, séchées sur du sulfate de magnésium, filtrées et évaporées sous pression réduite (2,7 kPa) pour donner une huile jaune (0,8 g) qui est purifiée par chromatographie-flash sur alumine CTB1 sous pression d'argon (50 kPa) [éluant : cyclohexane / acétate d'éthyle (95 / 5 en volumes)]. Après concentration des fractions sous pression réduite, on obtient 570 mg de 1-{2-[3-benzyloxy-4-(2-chloro-phényl)-pyrazol-1-yl]-éthyl}-pipéridine sous forme d'une huile incolore. Spectre IR (CCl₄) : 2938; 2853; 2801; 1573; 1506; 1456; 1450; 1357; 1174; 1125; 1036; 1025; 716 et 695 cm⁻¹.

Le 3-benzyloxy-4-(2-chloro-phényl)-1H-pyrazole peut être préparé de la manière suivante :

A une solution agitée sous atmosphère d'argon de 1,2 g de 3-benzyloxy-4-(2-chloro-phényl)-1-(toluène-4-sulfonyl)-1H-pyrazole dans 30 cm³ de tétrahydrofurane, on ajoute 6,9 cm³ d'une solution 1 N de fluorure de tétrabutyl-ammonium dans le tétrahydrofurane. Après 15 h de chauffage au reflux du solvant, le milieu réactionnel est évaporé sous pression réduite (2,7 kPa) et le résidu est additionné d'acétate d'éthyle. La phase organique est lavée successivement par deux fois de l'eau et une solution aqueuse saturée de chlorure de sodium ; elle est séchée sur sulfate de magnésium, filtrée et évaporée sous pression réduite (2,7 kPa). L'huile résultante est triturée dans du pentane. Le précipité formé est filtré et séché sous pression réduite pour donner 500 mg de 3-benzyloxy-4-(2-chloro-phényl)-1H-pyrazole sous la forme d'un solide jaune. Spectre de masse (IE) : m/z 284 (M^{+.}), m/z 249 [(M - Cl)⁺], m/z 91 (C7H7⁺).

Le 3-benzyloxy-4-(2-chloro-phényl)-1-(toluène-4-sulfonyl)-1H-pyrazole peut être préparé de la manière suivante :

A une solution agitée sous atmosphère d'argon de 1,5 g de 3-benzyloxy-4-iodo-1-(toluène-4-sulfonyl)-1H-pyrazole dans 20 cm³ de toluène additionnés de 5 cm³ d'éthanol, on ajoute 1,55 g d'acide 2-chloro-phényl boronique, 5 cm³ d'une solution aqueuse de carbonate de potassium 2 N, et 500 mg de tétrakis(triphénylphosphine)palladium. Après 5 h de chauffage au reflux du solvant, le milieu réactionnel est évaporé sous pression réduite (2,7 kPa). Le résidu est additionné d'acétate d'éthyle, d'eau et de noir de charbon, et il est filtré sur supercel. Le filtrat est décanté, puis la phase organique est lavée successivement par deux fois de l'eau et une solution aqueuse saturée de chlorure de sodium ; elle est séchée sur sulfate de magnésium, filtrée et évaporée sous pression réduite (2,7 kPa). L'huile orangée obtenue est purifiée par chromatographie-flash sur silice sous pression d'argon (50 kPa) [éluant : cyclohexane / acétate d'éthyle (95 / 5 en volumes)]. Après concentration des fractions sous pression réduite (2,7 kPa), on obtient 1,2 g de 3-benzyloxy-4-(2-chloro-phényl)-1-(toluène-4-sulfonyl)-1H-pyrazole sous forme d'une huile orangée. Spectre de masse (IE): m/z 438 (M^{+.}), m/z 283 [(M - C7H7SO2)⁺], m/z 91 (C7H7⁺).

### Exemple 54

### Dichlorhydrate de 1-(1-aza-bicyclo2.2.2]oct-3-yl)-4(4-chloro-phényl)-1H-pyrazol-3-ol

Une solution agitée de 220 mg de 3-[3-benzyloxy-4-(4-chloro-phényl)-pyrazol-1-yl]-1-aza-bicyclo[2.2.2]octane dans 5 cm³ d'éthanol est additionnée de 2,5 cm³ d'acide chlorhydrique 12 N. Après 7 h au reflux du solvant, puis 15 h à une température voisine de 20°C, le milieu réactionnel est évaporé à sec sous pression réduite (2,7 kPa). Le résidu est repris par de l'éthanol, puis le mélange est évaporé à sec sous vide (2,7 kPa). L'opération est répétée deux fois, puis la meringue obtenue est triturée dans de l'oxyde de diisopropyle. Le précipité formé est filtré et séché sous vide (2,7 kPa) pour donner 170 mg de dichlorhydrate de 1-(1-aza-bicyclo[2.2.2]oct-3-yl)-4-(4-chloro-phényl)-1H-pyrazol-3-ol sous forme d'un solide blanc. Spectre IR (KBr): 3052; 2926; 2793; 2559; 1606; 1576; 1520; 1486; 1454; 1195; 1167; 1090; 1010; 827; 626 et 515 cm⁻¹. Spectre de R.M.N. ¹H (300 MHz, (CD₃)₂SO d6, δ en ppm) : de 1,65 à 2,05 (mt : 4H) ; 2,42 (mt : 1H); de 3,15 à 3,50 (mt : 4H) ; de 3,70 à 3,85 (mt : 2H) ; 4,67 (mt : 1H) ; 7,40 (d large, J = 8 Hz : 2H) ; 7,71 (d large, J = 8 Hz : 2H) ; 8,35 (s : 1H) ; de 10,50 à 10,70 (mf étalé : 1H) ; 10,73 (mf : 1H).

Le 3-[3-benzyloxy-4-(4-chloro-phényl)-pyrazol-1-yl]-1-aza-bicyclo[2.2.2]octane peut être préparé de la manière suivante :

A une solution agitée sous atmosphère d'argon de 500 mg de 3-benzyloxy-4-(4-chloro-phényl)-1H-pyrazole dans 20 cm³ de diméthylformamide, sont additionnés 500 mg de tert-butylate de potassium, puis une solution de 660 mg d'ester 1-aza-bicyclo[2.2.2]oct-3-yl d'acide toluène-4-sulfonique dans 20 cm³ de diméthylformamide. Après 15 h de chauffage à 110°C, le milieu réactionnel est jeté dans 100 cm³ d'eau, et le mélange est extrait par deux fois de l'acétate d'éthyle. Les phases organiques sont lavées successivement par deux fois de l'eau et une solution aqueuse saturée de chlorure de sodium, séchées sur sulfate de magnésium, filtrées et évaporées sous pression réduite (2,7 kPa). L'huile orangée (650 mg) obtenue est purifiée par chromatographie-flash sur silice sous pression d'argon (50 kPa) [éluant : acétate d'éthyle, puis dichlorométhane / méthanol (80 / 20 en volumes)]. Après concentration des fractions sous pression réduite (2,7 kPa), on obtient 220 mg de 3-[3-benzyloxy-4-(4-chloro-phényl)-pyrazol-1-yl]-1-aza-bicyclo[2.2.2]octane sous forme d'une huile jaune pâle. Spectre IR (CC₄) : 3035; 2941; 2873; 1605; 1574; 1508; 1481; 1454; 1165; 1095; 1014; 834; 695 et 513 cm⁻¹.

### Exemple 55

### Chlorhydrate de 1-(1-aza-bicyclo[2.2.2]oct-3-yl)-4-(3-chloro-phényl)-1H-pyrazol-3-ol

Une solution agitée de 270 mg de 3-[3-benzyloxy-4-(3-chloro-phényl)-pyrazol-1-yl]-1-aza-bicyclo[2.2.2]octane dans 6 cm³ d'éthanol est additionnée de 3 cm³ d'acide chlorhydrique 12 N. Après 7 h au reflux du solvant, le milieu réactionnel est évaporé à sec sous pression réduite (2,7 kPa). Le résidu est repris par de l'éthanol, puis le mélange est évaporé à sec sous vide (2,7 kPa). L'opération est répétée deux fois, puis la meringue obtenue est triturée dans de l'oxyde de diisopropyle. Le précipité formé est filtré et séché sous vide (2,7 kPa) pour donner 180 mg de chlorhydrate de 1-(1-aza-bicyclo[2.2.2]oct-3-yl)-4-(3-chloro-phényl)-1H-pyrazol-3-ol sous forme d'un solide blanc. Spectre IR (KBr) : 2931; 2801; 2660; 2557; 1599; 1563; 1517; 1459; 1425; 1165; 1095; 950; 891; 840; 788; 685; 627 et 440 cm⁻¹. Spectre de R.M.N. ¹H (300 MHz, (CD₃)₂SO d6, δ en ppm) : de 1,65 à 2,05 (mt : 4H) ; 2,43 (mt : 1H) ; de 3,15 à 3,45 (mt : 4H) ; de 3,70 à 3,85 (mt : 2H) ; 4,67 (mt : 1H) ; 7,20 (ddd, J = 8 - 2 et 1 Hz : 1H) ; 7,38 (t, J = 8 Hz : 1H) ; 7,67 (d large, J = 8 Hz : 1H) ; 7,75 (t, J = 2 Hz : 1H) ; 8,31 (s : 1H) ; de 10,30 à 10,60 (mf étalé : 1H) ; 10,68 (s large : 1H).

Le 3-[3-benzyloxy-4-(3-chloro-phényl)-pyrazol-l-yl]-1-aza-bicyclo[2.2.2]octane peut être préparé de la manière suivante :

A une solution agitée sous atmosphère d'argon de 570 mg de 3-benzyloxy-4-(3-chloro-phényl)-1H-pyrazole dans 20 cm³ de diméthylformamide, sont additionnés 560 mg de tert-butylate de potassium, puis une solution de 740 mg d'ester 1-aza-bicyclo[2.2.2]oct-3-yl d'acide toluène-4-sulfonique dans 20 cm³ de diméthylformamide. Après 15 h de chauffage à 110°C, le milieu réactionnel est jeté dans 100 cm³ d'eau, et le mélange est extrait par deux fois de l'acétate d'éthyle. Les phases organiques sont lavées successivement par deux fois de l'eau et une solution aqueuse saturée de chlorure de sodium, séchées sur sulfate de magnésium, filtrées et évaporées sous pression réduite (2,7 kPa). L'huile orangée (800 mg) obtenue est purifiée par chromatographie-flash sur silice sous pression d'argon (50 kPa) [éluant : acétate d'éthyle / méthanol (90 / 10 en volumes), puis dichlorométhane / méthanol (80 / 20 en volumes)]. Après concentration des fractions sous pression réduite (2,7 kPa), on obtient 270 mg de 3-[3-benzyloxy-4-(3-chloro-phényl)-pyrazol-1-yl]-1-aza-bicyclo[2.2.2]octane sous forme d'une huile jaune pâle. Spectre IR (CCl₄) : 3034; 1602; 1574; 1507; 1454; 1356; 1176; 1097; 1048; 695 et 687 cm⁻¹.

### Exemple 56

### Chlorhydrate de 1-(1-aza-bicylo[2.2.2]oct-3-yl)-4-3-fluoro-phényl)-1H-pyrazol-3-ol

Une solution agitée de 85 mg de 3-[3-benzyloxy-4-(3-fluoro-phényl)-pyrazol-1-yl]-1-aza-bicyclo[2.2.2]octane dans 4 cm³ d'éthanol est additionnée de 2 cm³ d'acide chlorhydrique 12 N. Après 7 h au reflux du solvant, puis 15 h à une température voisine de 20°C, le milieu réactionnel est évaporé à sec sous pression réduite (2,7 kPa). Le résidu est repris par de l'éthanol, puis le mélange est évaporé à sec sous vide (2,7 kPa). L'opération est répétée deux fois, puis la meringue obtenue est triturée dans de l'oxyde de diisopropyle. Le précipité formé est filtré et séché sous vide (2,7 kPa) pour donner 63 mg de chlorhydrate de 1-(1-aza-bicyclo[2.2.2]oct-3-yl)-4-(3-fluoro-phényl)-1H pyrazol-3-ol sous forme d'un solide beige. Spectre IR (KBr) : 2932; 2765; 2663; 2577; 1617; 1596; 1521; 1457; 1436; 1265; 1180; 1165; 876; 783; 666; 625 et 521 cm⁻¹. Spectre de R.M.N. ¹H (300 MHz, (CD₃)₂SO d6, δ en ppm) : de 1,65 à 2,05 (mt : 4H) ; 2, 43 (mt : 1H) ; de 3,10 à 3,50 (mt : 4H) ; 3,78 (d large, J = 7 Hz : 2H) ; 4,67 (mt : 1H) ; 6,96 (t dédoublé large, J = 8 et 2,5 Hz : 1H) ; de 7,25 à 7,55 (mt : 3H) ; 8,28 (s : 1H) ; 10,12 (mf : 1H) ; 10,65 (s : 1H).

Le 3-[3-benzyloxy-4-(3-fluoro-phényl)-pyrazol-1-yl]-1-aza-bicyclo[2.2.2]octane peut être préparé de la manière suivante :

A une solution agitée sous atmosphère d'argon de 250 mg de 3-benzyloxy-4-(3-fluoro-phényl)-1H-pyrazole dans 20 cm³ de diméthylformamide, sont additionnés 260 mg de tert-butylate de potassium, puis une solution de 400 mg d'ester 1-aza-bicyclo[2.2.2]oct-3-yl d'acide toluène-4-sulfonique dans 20 cm³ de diméthylformamide. Après 15 h de chauffage à 110°C, le milieu réactionnel est jeté dans 100 cm³ d'eau, et le mélange est extrait par deux fois de l'acétate d'éthyle. Les phases organiques sont lavées successivement par deux fois de l'eau et une solution aqueuse saturée de chlorure de sodium, séchées sur sulfate de magnésium, filtrées et évaporées sous pression réduite (2,7 kPa). L'huile orangée (345 mg) obtenue est purifiée par chromatographie-flash sur silice sous pression d'argon (50 kPa) [éluant : cyclohexane / acétate d'éthyle (90 /10 en volumes), puis dichlorométhane / méthanol (80 / 20 en volumes)]. Après concentration des fractions sous pression réduite (2,7 kPa), on obtient 270 mg de 3-[3-benzyloxy-4-(3-fluoro-phényl)-pyrazol-1-yl]-1-aza-bicyclo[2.2.2]octane sous forme d'une huile jaune pâle. Spectre de R.M.N. ¹H (300 MHz, (CD₃)₂SO d6, δ en ppm) : 0,80 (mt : 1H) ; 1,53 (mt : 1H) ; 1,67 (mt : 2H) ; 2,09 (mt : 1H) ; de 2,60 à 2,80 (mt : 3H) ; 2,98 (mt : 1H) ; 3,21 (ddd, J = 14 - 10 et 1,5 Hz : 1H) ; 3,37 (dd large, J = 14 et 5,5 Hz : 1H) ; 4,25 (mt : 1H) ; 5,35 (s : 2H) ; 6,95 (t dédoublé large, J = 8 et 2,5 Hz : 1H) ; de 7,25 à 7,55 (mt : 8H) ; 8,31 (s : 1H).

### Exemple 57

### Chlorhydrate de 1-(1-méthyl-pyrrolidin-3-yl)-4-phényl-1H-pyrazol-3-ol

A un mélange de 165 mg de 1-(1-méthyl-pyrrolidin-3-yl)-4-phényl-1H-pyrazol-3-ol dans 2 cm³ d'acétate d'éthyle sont ajoutées quelques gouttes de méthanol pour solubiliser le milieu qui est refroidi à 0°C avant l'addition de 5 cm³ d'une solution d'acide chlorhydrique 3 M dans l'acétate d'éthyle. Le milieu réactionnel est agité 5 mn à 0°C, laissé remonter à une température voisine de 20°C puis agité de nouveau à cette température pendant 20 mn avant d'être concentré sous pression réduite (2,7 kPa). Le produit brut est séché sur une pompe à palettes (10⁻³ kPa) pour donner 160 mg de chlorhydrate de 1-(1-méthyl-pyrrolidin-3-yl)-4-phényl-1H-pyrazol-3-ol sous forme d'un solide très hygroscopique. LCMS (electrospray) : m/z 244 (MH⁺). Spectre de RMN ¹H (300MHz, (CD₃)₂SO d6 à 80°C, δ en ppm) : de 2,30 à 2,65 (m : 2H) ; 2,91 (s : 3H) ; de 3,10 à 4,00 (m : 4H) ; 5,03 (m : 1H) ; 7,14 (t, J = 7,5 Hz : 1H) ; 7,32 (t, J = 7,5 Hz : 2H) ; 7,65 (t, J = 7,5 Hz : 2H) ; 8,00 (s : 1H).

Le 1-(1-méthyl-pyrrolidin-3-yl)-4-phényl-1H-pyrazol-3-ol peut être préparé de la manière suivante :

A une solution de 505 mg de 3-benzyloxy-1-(1-méthyl-pyrrolidin-3-yl)-4-phényl-1H-pyrazole dans 3,53 cm³ d'éthanol sont additionnés 7,55 cm³ d'une solution aqueuse d'acide chlorhydrique 4 M. Le milieu réactionnel est agité au reflux pendant 8 h puis concentré sous pression réduite (2,7 kPa). L'huile violette obtenue est reprise par trois fois avec de l'éther diéthylique, évaporée à sec sous pression réduite (2,7 kPa), reprise par trois fois avec de l'isopropanol et concentrée sous pression réduite, et enfin reprise par trois fois avec du dichlorométhane pour donner une huile figée, qui, après séchage sur une pompe à palettes (10⁻³ kPa), donne 524 mg d'un solide. Le résidu est est purifié par chromatographie sur colonne de 30 g de silice (irrégulière 15-40 µm Merck) [éluant : dichlorométhane / méthanol / hydroxyde d'ammonium 39% (95 / 5 / 0,4 en volumes) ; débit : 8 cm³ / min ; détection : 250 nm]. Après concentration des fractions sous pression réduite, on obtient 279 mg de 1-(1-méthyl-pyrrolidin-3-yl)-4-phényl-1H-pyrazol-3-ol sous forme d'un solide amporphe incolore. LCMS (electrospray) : m/z 334 (MH⁺).

Le 3-benzyloxy-1-(1-méthyl-pyrrolidin-3-yl)-4-phényl 1H-pyrazole peut être obtenu de la manière suivante :

A une solution de 428 mg de 3-benzyloxy-4-phényl-1H-pyrazole dans 8,5 cm³ de diméthylformamide agitée sous atmosphère d'azote et à 0°C, sont ajoutés 123 mg d'hydrure de sodium (à 50 % dans l'huile). Après 30 min d'agitation à une température voisine de 20°C est additionnée une solution de 398 mg d'ester (1-méthyl-pyrrolidin-3-yl) de l'acide méthane sulfonique dans 5,6 cm³ de diméthylformamide. Le milieu réactionnel est agité 1 h à 80°C puis jeté dans un mélange eau / acétate d'éthyle. Après 5 min d'agitation, le milieu est décanté et la phase aqueuse est extraite trois fois par de l'acétate d'éthyle. Les phases organiques sont réunies, lavées par une solution aqueuse saturée de chlorure de sodium, séchées sur du sulfate de magnésium, filtrées sur iéna et évaporées sous pression réduite (2,7 kPa) pour donner 657 mg d'une huile qui est purifiée par chromatographie sur colonne de 30 g de silice (irrégulière 15-40 µm Merck) [éluant : dichlorométhane / méthanol (97 / 3 en volumes) ; débit : 8 cm³ / min ; détection : 250 nm]. Après concentration des fractions sous pression réduite , on obtient 511 mg de 3-benzyloxy-1-(1-méthyl-pyrrolidin-3-yl)-4-phényl 1H-pyrazole sous forme d'un solide amorphe incolore. LCMS (electrospray) : 334 (MH⁺).

L'ester (1-méthyl-pyrrolidin-3-yl) de l'acide méthane sulfonique peut être préparé de la manière suivante :

A une solution agitée de 0,39 cm³ de 1-méthyl-3-hydroxypyrrolidine et 0,62 cm³ de triéthylamine dans 7,7 cm³ de dichlorométhane sous atmosphère d'azote, à -10 °C, est additionnée goutte à goutte une solution de 0,33 cm³ de chlorure de méthane sulfonyle dans 7,07 cm³ de dichlorométhane. Le milieu réactionnel est agité 5 min à - 10 °C puis 2 h à une température voisine de 20°C avant d'être concentré à sec sous pression réduite (2,7 kPa). Le résidu obtenu est repris par de l'eau et de l'acétate d'éthyle. La solution est agitée 5 min puis décantée. La phase aqueuse est extraite trois fois par de l'acétate d'éthyle. Les phases organiques sont réunies, lavées successivement par une solution aqueuse de bicarbonate de sodium à 5 % et une solution aqueuse saturée de chlorure de sodium, séchées sur du sulfate de magnésium, filtrées et évaporées sous pression réduite (2,7 kPa) pour donner 399 mg d'ester (1-méthyl-pyrrolidin-3-yl) de l'acide méthane sulfonique sous forme d'une huile incolore. LCMS (electrospray) : m/z 180 (MH⁺), m/z 84 [MH⁺- (SO₂CH₃)].

### Exemple 58

### Dichlorhydrate de 1-[2-(1-méthyl-pyrrolidin-2-yl)-éthyl]-4-phényl-1H-pyrazol-3-ol

Une suspension de 200 mg de 3-benzyloxy-1-[2-(1-méthyl-pyrrolidin-2-yl)-éthyl]-4-phényl-1H-pyrazole et d'une pointe de spatule de palladium sur charbon à 10% dans 6 cm³ d'éthanol est hydrogénée à une température voisine de 20°C sous une atmosphère de 1600 mbar pendant 3 h 30. Le milieu réactionnel est repris par un mélange dichlorométhane / méthanol, filtré sur clarcel. Le filtrat est concentré à sec sous pression réduite (2,7 kPa) pour donner 120 mg de 1-[2-(1-méthyl-pyrrolidin-2-yl)-éthyl]-4-phényl 1H-pyrazol-3-ol sous forme d'un produit cristallisé. Un second lot de 100 mg de 1-[2-(1-méthyl-pyrrolidin-2-yl)-éthyl]-4-phényl] 1H-pyrazol-3-ol est préparé selon le même procédé mais à partir de 140 mg de 3-benzyloxy-1-[2-(1-méthyl-pyrrolidin-2-yl)-éthyl]-4-phényl-1H-pyrazole.

Une solution de 160 mg de 1-[2-(1-méthyl-pyrrolidin-2-yl)-éthyl]-4-phényl-1H-pyrazol-3-ol dans 5 cm³ de méthanol est acidifiée (pH 1) par une solution d'acide chlorhydrique dans le méthanol. Le milieu réactionnel est agité 10 mn à une température voisine de 20°C puis concentré sous pression réduite (2,7 kPa) et placé une nuit au congélateur. Le résidu est repris par de l'acétonitrile, essoré puis lavé par de l'acétonitrile avant d'être séché sous vide pour donner 160 mg de dichlorhydrate de 1-[2-(1-méthyl-pyrrolidin-2-yl)-éthyl]-4-phényl-1H-pyrazol-3-ol sous forme d'une poudre blanche amorphe hygroscopique. LCMS (electrospray) : m/z 272 (MH⁺). Spectre de RMN ¹H (400 MHz, (CD₃)₂SO d6, δ en ppm) : 1,65 (m : 1H); 1,92 (m : 2H); 2,09 (m : 1H); 2,21 (m : 1H); 2,41 (m : 1H); 2,77 (d, J = 5,0 Hz : 3H); 3,01 (m : 1H); 3,15 (m : 1H); 3,52 (m : 1H); 4,04 (dt, J = 14,0 et 6,5 Hz : 1H); 4,07 (dt, J = 14,0 et 6,5 Hz : 1H); 7,11 (t, J = 7,5 Hz : 1H); 7,31 (t, J = 7,5 Hz : 2H); 7,63 (t, J = 7,5 Hz : 2H); 7,98 (s : 1H); 10,70 (s : 1H).

Le 3-benzyloxy-1-[2-(1-méthyl-pyrrolidin-2-yl)-éthyl]-4-phényl-1H-pyrazole peut être préparé de la manière suivante :

A une solution de 425 mg de 3-benzyloxy-4-phényl-1H-pyrazole dans 2,5 cm³ de diméthylformamide sont additionnés en trois fois 82 mg d'hydrure de sodium (à 50% dans l'huile). A la fin du dégazement gazeux, le milieu réactionnel est agité à une température voisine de 20 °C pendant 15 min supplémentaires avant l'ajout d'une solution de 250 mg de 1-méthyl-2-(2-chloro-éthyl)-pyrrolidine dans 0,5 cm³ de diméthylformamide. Le milieu réactionnel est agité à une température voisine de 20°C pendant 1 h puis à 50°C pendant 3 h avant d'être jeté sur de l'eau. La solution est extraite par de l'acétate d'éthyle. La phase organique est lavée par une solution aqueuse saturée de chlorure de sodium, séchée sur du sulfate de magnésium, filtrée et évaporée sous pression réduite (2,7 kPa) pour donner 580 mg d'un produit brut qui est purifié par chromatographie sur 25 g de gel de silice [éluant : dichlorométhane puis dichlorométhane / méthanol (90 / 10 en volumes)]. Après concentration des fractions sous pression réduite, on obtient 200 mg de 3-benzyloxy-1-[2-(1-méthyl-pyrrolidin-2-yl)-éthyl]-4-phényl-1H-pyrazole sous forme d'une poudre blanche amorphe ainsi que 240 mg d'un mélange constitué de 3-benzyloxy-1-[2-(1-méthyl-pyrrolidin 2-yl)-éthyl]-4-phényl-1H-pyrazole et de 3-benzyloxy-4-phényl-1H-pyrazole. Le mélange est de nouveau purifié par chromatographie sur 10 g de gel de silice [éluant : dichlorométhane / méthanol (50 / 50 puis 90 / 10 en volumes)] pour donner 90 mg de 3-benzyloxy-4-phényl-1H-pyrazole et 140 mg de 3-benzyloxy-1-[2-(1-méthyl-pyrrolidin-2-yl)-éthyl]-4-phényl-1H-pyrazole de même aspect que le lot précédent. LCMS (electrospray) : m/z 362 (MH⁺).

Le 1-méthyl-2-(2-chloro-éthyl)-pyrrolidine peut être préparé de la manière suivante :

Une solution de 330 mg de chlorhydrate de 1-méthyl-2-(2-chloro-éthyl)-pyrrolidine et de 5 cm³ d'hydroxyde de sodium 1 N dans 20 cm³ de dichlorométhane est agitée 1 h à une température voisine de 20°C. Le milieu réactionnel est extrait par du dichlorométhane. La phase organique est lavée par une solution aqueuse saturée de chlorure de sodium, séchée sur du sulfate de magnésium, filtrée et concentrée sous pression réduite (2,7 kPa) pour donner 255 mg de 1-méthyl-2-(2-chloro-éthyl)-pyrrolidine qui sont immédiatement mis en réaction.

### Exemple 59

### Dichlorhydrate de 1-(pyrrolidin-3-yl)-4-phényl-1H-pyrazol-3-ol

Une suspension de 608 mg de dichlorhydrate de 3-benzyloxy-4-phényl-1-(pyrrolidin-3-yl)-1H-pyrazole et 60 mg de palladium sur charbon à 10 % dans 18 cm³ d'éthanol est hydrogénée à une température voisine de 20°C sous une atmosphère de 1300 mbar pendant 3 h. Le milieu réactionnel est dilué par du méthanol, filtré sur hyflosupercel et rincé par du méthanol. Le filtrat est évaporé sous pression réduite (2,7 kPa) pour donner 365 mg d'une poudre blanche. Ce brut réactionnel est recristallisé dans 20 cm³ d'éthanol au reflux. La solution obtenue est laissée revenir à une température voisine de 20°C puis plongée dans un bain de glace. Les cristaux obtenus sont filtrés à froid sur iéna, rincés successivement avec de l'éthanol puis de l'éther éthylique et séchés sous vide (13 kPa) pour donner 185 mg de dichlorhydrate de 1-(pyrrolidin-3-yl)-4-phényl-1H-pyrazol-3-ol sous forme d'une poudre blanche. LCMS (electrospray) : m/z 230 (MH⁺). Spectre de RMN ¹H (400 MHz, (CD₃)₂SO d6, δ en ppm) : 2,32 (m : 2H); 3,37 (m, sous l'eau : 2H); 3,45 (dd, J = 12,5 et 5,0 Hz : 1H); 3,60 (dd, J = 12,5 et 7,0 Hz : 1H); 4,91 (m : 1H); 7,13 (t, J = 7,5 Hz : 1H); 7,32 (t, J = 7,5 Hz : 2H), 7,66 (t, J = 7,5 Hz : 2H); 8,11 (s : 1H); 9,44 (s : 2H); 10,40 (s : 1H).

Le dichlorhydrate de 3-benzyloxy-4-phényl-1-(pyrrolidin-3-yl)-1H-pyrazole peut être obtenu de la manière suivante :

A une solution de 569 mg de 3-benzyloxy-1-(1-tert-butoxycarbonyl-pyrrolidin-3-yl)-4-phényl-1H-pyrazole dans 6 cm³ d'acétate d'éthyle agitée à 0°C est additionnée goutte à goutte 6 cm³ d'une solution d'acide chlorhydrique 3 M dans l'acétate d'éthyle. Le milieu réactionnel est agité 2 h à une température voisine de 20°C avant d'être concentré sous pression réduite pour donner 608 mg de dichlorhydrate de 3-benzyloxy-4-phényl-1-(pyrrolidin-3-yl)-1H-pyrazole, sous forme d'une poudre blanche, qui sont immédiatement utilisés.

Le 3-benzyloxy-1-(1-tert-butoxycarbonyl-pyrrolidin-3-yl)-4-phényl-1H-pyrazole peut être préparé de la manière suivante :

A une solution de 450 mg de 3-benzyloxy-4-phényl-1H-pyrazole dans 9 cm³ de diméthylformamide agitée sous atmosphère d'azote à 0°C sont additionnés 129 mg d'hydrure de sodium (à 50% dans l'huile). Après 30 min d'agitation à une température voisine de 20°C sont additionnés 621 mg d'ester (1-tert-butoxycarbonyl-pyrrolidin-3-yl) de l'acide méthane sulfonique. Le milieu réactionnel est agité 1 h à 80°C puis jeté dans un mélange eau / acétate d'éthyle. Après 5 min d'agitation, le milieu est décanté et la phase aqueuse est extraite trois fois par de l'acétate d'éthyle. Les phases organiques sont réunies, lavées par une solution aqueuse saturée de chlorure de sodium, séchées sur du sulfate de magnésium, filtrées sur iéna, rincées avec de l'acétate d'éthyle et évaporées sous pression réduite (2,7 kPa) pour donner 998 mg d'une huile qui est purifiée par chromatographie sur colonne de 70 g de silice (irrégulière 15-40 µm Merck) [éluant : dichlorométhane / méthanol (98 / 2 en volumes) ; débit : 15 cm³ / min ; détection : 250 nm]. Après concentration des fractions sous pression réduite, on obtient 956 mg d'un produit qui est de nouveau purifié par chromatographie sur colonne de 90 g de silice (irrégulière 15-40 µm Merck) [éluant : dichlorométhane / acétate d'éthyle (98 / 2 en volumes) ; débit : 15 cm³ / min ; détection : 250 nm]. Après concentration des fractions sous pression réduite, on récupère 575 mg de 3-benzyloxy-1-(1-tert-butoxycarbonyl -pyrrolidin-3-yl)-4-phényl-1H-pyrazole sous forme d'une mousse incolore. LCMS (electrospray) : m/z 420 (MH⁺), m/z 364 [MH⁺ - tBu], m/z 320 [MH⁺ - Boc].

L'ester (1-tert-butoxycarbonyl-pyrrolidin-3-yl) de l'acide méthane sulfonique peut être préparé de la manière suivante :

A une solution de 710 mg de 1-tert-butoxycarbonyl-3-hydroxypyrrolidine et 0,62 cm³ de triéthylamine dans 14,2 cm³ de dichlorométhane agitée sous atmosphère d'azote à -10 °C est additionnée goutte à goutte une solution de 0,33 cm³ de chlorure de méthane sulfonyle dans 3,2 cm³ de dichlorométhane. Le milieu réactionnel est agité 5 min à -10°C puis 2 h à une température voisine de 20°C avant d'être concentré à sec sous pression réduite (2,7 kPa). Le résidu obtenu est repris par de l'eau et de l'acétate d'éthyle. La solution est agitée 5 min puis décantée. La phase aqueuse est extraite trois fois par de l'acétate d'éthyle. Les phases organiques sont réunies, lavées successivement par une solution aqueuse de bicarbonate de sodium à 5% et de chlorure de sodium, séchées sur du sulfate de magnésium, filtrées et évaporées sous pression réduite (2,7 kPa) pour donner 938 mg d'ester (1-tert-butoxycarbonyl-pyrrolidin-3-yl) de l'acide méthane sulfonique sous forme d'une huile jaune pâle. LCMS (electrospray) : m/z 266 (MH⁺), m/z 210 [MH⁺ - tBu].

Le 1-tert-butoxycarbonyl-3-hydroxypyrrolidine peut être obtenu de la manière suivante :

A 0,848 cm³ de 3-hydroxypyrrolidine dans un mélange de 30 cm³ de tétrahydrofurane et 9,6 cm³ d'eau est additionnée une solution de 2,78 cm³ de triéthylamine et 3,27 g de di-tert-butyldicarbonate. Le milieu réactionnel est agité à une température voisine de 20°C pendant 3 h puis concentré à sec sous pression réduite (2,7 kPa). Le résidu obtenu est repris avec de l'eau et de l'acétate d'éthyle. La solution est agitée pendant 5 min puis décantée. La phase aqueuse est extraite trois fois par de l'acétate d'éthyle. Les phases organiques sont réunies, séchées sur du sulfate de magnésium, filtrées sur iéna puis concentrées à sec sous pression réduite (2,7 kPa) pour donner 1,823 g de 1-tert-butoxycarbonyl-3-hydroxypyrrolidine sous forme de cristaux incolores. m/z 188 (MH⁺), m/z 132 [MH⁺ - tBu].

### Exemple 60

### 1-[(1-méthyl-pyrrolidin-2-(S)-yl)-méthyl]-4-phényl-1H-pyrazol-3-ol

A une solution de 263 mg de 3-benzyloxy-1-[(1-méthyl-pyrrolidin-2-(S)-yl)-méthyl]-4-phényl-1H-pyrazole dans 2 cm³ d'éthanol sont additionnés 3,75 cm³ d'une solution d'acide chlorhydrique 4 M. Le milieu réactionnel est agité au reflux pendant 7 h puis concentré sous pression réduite. L'huile violette obtenue est reprise par trois fois avec 10 cm³ d'isopropanol puis évaporée à sec sous pression réduite, pour donner 259 mg de résine violette. Cette résine est dissoute dans un mélange de 0,6 cm³ d'éthanol et 3 cm³ de 1,4-dioxane. Après ajout de 0,665 cm³ d'une solution de chlorure d'hydrogène 4 M dans le 1,4-dioxane et agitation à température ambiante le milieu est concentré sous pression réduite à 40°C. Le résidu est dissous dans 10 cm³ d'eau et la solution obtenue est lavée au dichlorométhane (3x1 cm³) puis amenée à pH 9-10 par ajout de carbonate de sodium. Après extraction au dichlorométhane, les phases organiques sont réunies puis séchées sur sulfate de magnésium, filtrées et concentrées sous pression réduite à 35 °C. Le solide rose pâle obtenu (124 mg) est recristallisé à chaud dans l'éthanol pour conduire à 73 mg de 1-[(1-méthyl-pyrrolidin-2(S)-yl)-méthyl]-4-phényl-1H-pyrazol-3-ol sous forme de solide blanc. LCMS (electrospray) : m/z 258 (MH⁺). Spectre de RMN ¹H (400MHz, DMSO d6, δ en ppm) : 1,63 (m : 1H) ; 1,76 (m : 2H) ; 1,95 (m : 1H) ; 2,30 (m partiellement masqué : 1H) ; 2,34 (s : 3H) ; 2,82 (m : 1H) ; 3,14 (m : 1H) ; 3,90 (dd, J= 7-14 Hz : 1H) ; 4,11 (dd, J=6-14 Hz : 1H) ; 7,18 (tl, J=8 Hz : 1H) ; 7,35 (tl, J= 8 Hz : 2H) ; 7,57 (s : 1H) ; 7,70 (dl, J= 8Hz : 2H).

Le 3-benzyloxy-1-[(1-méthyl-pyrrolidin-2-(S)-yl)-méthyl]-4-phényl-1H-pyrazole peut être obtenu de la manière suivante :

A une solution de 283 mg de 3-benzyloxy-4-phényl-1H-pyrazole dans 6 cm³ de diméthylformamide agitée sous atmosphère d'azote à température ambiante, sont ajoutés 136 mg d'hydrure de sodium (à 50% dans l'huile). Après 30 min d'agitation à température ambiante est additionnée une solution de 250 mg de chlorhydrate de 1-méthyl-2-(S)-chlorométhyl pyrrolidine dans 6 cm³ de diméthylformamide. Le milieu réactionnel est agité 1 h à 80°C puis refroidi à température ambiante et hydrolysé. Après extraction à l'acétate d'éthyle les phases organiques sont réunies, lavées avec une solution aqueuse saturée de bicarbonate de sodium, séchées sur sulfate de magnésium, filtrées et concentrées sous pression réduite à 35 °C pour donner 554 mg d'une huile jaune. Après purification par chromatographie sur colonne de 30 g de silice (irrégulière 15-40 µm Merck) [éluant : dichlorométhane / méthanol (98 / 2) ; débit : 15 cm³ / min ] et concentration des fractions sous pression réduite, on obtient 263 mg de 3-benzyloxy-1-[(1-méthyl-pyrrolidin-2-(S)-yl)-méthyl]-4-phényl-1H-pyrazole. LCMS (electrospray) : m/z 348 (MH⁺).

Le chlorhydrate de 1-méthyl-2-(S)-chlorométhyl-pyrrolidine peut être préparé de la manière suivante :

A une solution de 250 mg de (S)-(-)-1-méthyl-2-pyrrolidine méthanol dans 2 cm³ de dichlorométhane refroidie dans un bain d'eau glacée sont ajoutés lentement 388 µl de chlorure de thionyle. La solution obtenue est chauffée au reflux pendant trois heures puis agitée à température ambiante pendant 18 heures. Après évaporation sous pression réduite à 35°C le résidu brun obtenu est dissous dans l'éthanol puis concentré à sec sous pression réduite. L'extrait sec obtenu est dissous dans 1 cm³ d'éthanol puis précipité par ajout progressif de 6 cm³ d'éther éthylique. La suspension obtenue est refroidie par un bain d'eau glacée et le solide filtré puis lavé à l'éther éthylique. Après séchage sous vide, 258 mg de chlorhydrate de 1-méthyl-2-(S)-chlorométhyl pyrrolidine sont obtenus sous forme d'un solide ocre très hygroscopique. Spectre de masse (EI) : m/z 133 (M^{+.}).

### Exemple 61

### Chlorhydrate de 4-phényl-1-pyrrolidin-3-yl-méthyl-1H-pyrazol-3-ol

Dans un réacteur pour four à micro-ondes muni d'une agitation magnétique sont additionnés 163,5 mg de chlorhydrate de 1-(1-benzyl-pyrrolidin-3-ylméthyl)-4-phényl-1H-pyrazol-3-ol dans 5 cm³ de méthanol, 139,4 mg de formiate d'ammonium et 10 mg de palladium sur charbon à 10%. Le tube est scellé et placé dans un appareil à micro-ondes pour 60 secondes à une température de 100°C sous une pression de 10,5 bar. Le milieu réactionnel est filtré sur Acodisc GHP Polypro (PALL) puis rincé avec du méthanol. Le filtrat est concentré à sec sous pression réduite pour donner une gomme qui est concrétisée à l'éthanol. On obtient ainsi 40 mg d'une poudre blanche. L'opération est répétée au filtrat éthanolique précédent pour donner, après réunification des deux lots, 52 mg d'une poudre blanche. Le dernier filtrat éthanolique est de nouveau concentré à sec, le résidu obtenu est repris par 10 cm³ d'eau. La solution est glacée puis lyophilisée toute la nuit. Les différents lots sont réunis pour donner 80 mg de chlorhydrate de 4-phényl-1-pyrrolidin-3-yl-méthyl-1H-pyrazol-3-ol sous forme d'une poudre blanche. Spectre de R.M.N. ¹H (300MHz, (CD₃)₂SO d6, δ en ppm) : 1,66 (m, 1H) ; 1,99 (m, 1H) ; 2,73 (m, 1H) ; 2,92 (m, 1H) ; de 3,02 à 3,25 (m, 2H) ; 4,01 (d, J = 7,0 Hz, 2H) ; 7,12 (t large, J = 7,5 Hz, 1H) ; 7,32 (t large, J = 7,5 Hz, 2H) ; 7,65 (d large, J = 7,5 Hz, 2H) ; 7,99 (s, 1H) ; 8,36 (s large, 1H) ; de 6,70 à 8,70 (m très très étalé, 1H). Spectre de masse (IE) : m/z 244⁺ (M+H)⁺.

Le chlorhydrate de 1-(1-benzyl-pyrrolidin-3-ylméthyl)-4-phényl-1H-pyrazol-3-ol peut être préparé de la manière suivante :

Une suspension de 937 mg de chlorhydrate de 3-benzyloxy-1-(1-benzyl-pyrrolidin-3-ylméthyl)-4-phényl-1H-pyrazole et 93 mg de palladium sur charbon à 10% dans 9,4 cm³ d'éthanol est hydrogénée à une température voisine de 20°C sous une atmosphère de 1500 mbar pendant 16 h. Le milieu réactionnel est filtré sur hyflosupercel et rincé avec de l'éthanol. Après concentration à sec du filtrat, on obtient 350 mg d'une gomme beige qui est recristallisée dans l'éthanol. Après filtration sur iéna, rinçage à l'éther éthylique puis séchage en étuve sous vide industriel, on obtient 178 mg de chlorhydrate de 1-(1-benzyl-pyrrolidin-3-ylméthyl)-4-phényl-1H-pyrazol-3-ol. Spectre de masse (IE) : m/z 334⁺ [(M+H)⁺-HCl].

Le chlorhydrate de 3-benzyloxy-1-(1-benzyl-pyrrolidin-3-ylméthyl)-4-phényl-1H-pyrazole peut être préparé de la manière suivante :

A une solution de 450 mg de 3-benzyloxy-4-phényl-1H-pyrazole dans 9 cm³ de diméthylformamide anhydre agitée sous atmosphère d'argon à 0°C sont addditionnés en une fois 129 mg d'hydrure de sodium (à 50% dans l'huile). Après 30 mn d'agitation à une température voisine de 20°C, une solution de 630 mg d'ester (1-benzyl-pyrrolidin-3-ylméthyl) de l'acide méthane sulfonique dans 9 cm³ de diméthylformamide anhydre est additionnée. Le milieu réactionnel est agité 4 h à 80°C puis jeté dans un mélange eau / acétate d'éthyle. Après 5 min d'agitation, le milieu est décanté et la phase aqueuse est extraite trois fois par de l'acétate d'éthyle. Les phases organiques sont réunies, lavées deux fois par de l'eau puis une fois par une solution aqueuse saturée de chlorure de sodium, séchées sur du sulfate de magnésium, filtrées sur iéna. Le sulfate de magnésium est rincé par de l'acétate d'éthyle. Les phases organiques réunies sont évaporées sous pression réduite et le résidu obtenu est séché une nuit sur une pompe à palette pour donner 939 mg d'une huile jaune pâle qui est purifiée par chromatographie sur colonne de 90 g de silice (irrégulière 15-40 µm Merck) [éluant : dichlorométhane / méthanol (98 / 2 en volumes) ; débit : 10 cm³ / min ; détection : 250 nm]. Après concentration des fractions sous pression réduite, on obtient 586 mg de 3-benzyloxy-1-(1-benzyl-pyrrolidin-3-ylméthyl)-4-phényl-1H-pyrazole pur à 83% sous forme d'une mousse. Celle-ci est reprise par de l'eau (pH=1) puis additionnée d'acétate d'éthyle. Après 5 min d'agitation, le milieu est décanté et la phase aqueuse est extraite par de l'acétate d'éthyle. Les phases organiques sont amenées à pH=9 avec une solution d'hydroxyde d'ammonium, extraites trois fois par de l'acétate d'éthyle, réunies, séchées sur du sulfate de magnésium, filtrées, rincées puis concentrées à sec sous pression réduite pour fournir 497 mg de 3-benzyloxy-1-(1-benzyl-pyrrolidin 3ylméthyl)-4-phényl-1H-pyrazole sous forme de base libre. Le produit est repris par 5 cm³ d'acétate d'éthyle. Le milieu est refroidi à 0°C avant addition de 5 cm³ d'une solution d'acide chlorhydrique 3M dans l'acétate d'éthyle. La solution est concentrée à sec sous pression réduite pour donner une huile brune. Des essais de recristallisation du résidu brut obtenu ont été tentés en vain (acétate d'éthyle, éthanol, méthanol, éther diéthylique ou hexane). On récupère ainsi 940 mg de chlorhydrate de 3-benzyloxy-1-(1-benzyl-pyrrolidin-3-ylméthyl]-4-phényl-1H-pyrazole. Spectre de masse (IE) : m/z 424⁺ (M+H)⁺.

L'ester (1-benzyl-pyrrolidin-3-ylméthyl) de l'acide méthane sulfonique peut être préparé de la manière suivante :

A une solution de 1 g de (1-benzyl-pyrrolidin-3-yl) méthanol et 0,844 cm³ de triéthylamine dans 20 cm³ de dichlorométhane agitée sous atmosphère d'argon à 0°C est additionnée goutte à goutte une solution de 0,455 cm³ de chlorure de méthane sulfonique dans 9,7 cm³ de dichlorométhane anhydre. Le milieu réactionnel est agité 5 min à 0°C puis 2 h à une température proche de 20°C avant d'être concentré à sec sous pression réduite. Le résidu obtenu est repris par de l'eau et de l'acétate d'éthyle. La solution est agitée 5 min puis décantée. La phase aqueuse est extraite trois fois par de l'acétate d'éthyle. Les phases organiques sont réunies, lavées successivement par une solution aqueuse de bicarbonate de sodium à 5% et par une solution aqueuse saturée de chlorure de sodium, séchées sur du sulfate de magnésium, filtrées et évaporées sous pression réduite pour donner 1,2 g d'ester (1-benzyl-pyrrolidin-3-ylméthyl) de l'acide méthane sulfonique. Spectre de masse (IE) : m/z 270⁺ (M+H)⁺.

Le (1-benzyl-pyrrolidin-3-yl) méthanol peut être préparé de la manière suivante :

A une solution de 2 g d'ester méthylique de l'acide 1-benzyl-5-oxo-pyrrolidine-3 carboxylique dans 40 cm³ de tétrahydrofuranne agitée à 0°C sous atmosphère d'azote sont additionnés 17,1 cm³ d'une solution d'hydrure de lithium et aluminium 1M dans le tétrahydrofuranne. Après 15 min d'agitation à 0°C, le milieu réactionnel est laissé revenir à une température voisine de 20°C et agité pendant 4 h. Un mélange constitué de 0,65 cm³ d'eau et de 6,5 cm³ de tétrahydrofuranne est additionné goutte à goutte au milieu réactionnel. Puis sont successivement ajoutés 0,65 cm³ d'une solution aqueuse d'hydroxyde de sodium à 15 % et 1,95 cm³ d'eau. Le milieu est agité à une température voisine de 20°C jusqu'à formation d'un solide filtrable, auquel on additionne deux spatules de sulfate de magnésium. Après filtration sur iéna, rinçage et concentration à sec sous pression réduite, on obtient 1,72 g de (1-benzyl-pyrrolidin-3-yl) méthanol sous forme d'une huile incolore. Spectre de masse (IE) : m/z 192⁺ (M+H)⁺.

### Exemple 62

### 1-((2R)-1-méthyl-pyrrolidin-2-ylméthyl)-4-phényl-1H-pyrazol-3-ol

A une solution de 211 mg de 3-benzyloxy-1-((2R)-1-méthyl-pyrrolidin-2-ylméthyl)-4-phényl-1H-pyrazole dans 1,5 cm³ d'éthanol sont ajoutés 2,89 cm³ d'acide chlorhydrique 6 N. Le milieu réactionnel est agité pendant 5h 30 à 110°C avant d'être concentré à sec sous pression réduite. Le résidu est repris par de l'isopropanol et concentré à sec pour donner 136 mg d'une mousse qui est recristallisée dans un minimum d'éthanol à chaud. Après glaçage pendant une nuit, aucune cristallisation n'est apparue. Le résidu est repris par 5 cm³ d'eau et extrait trois fois par 1 cm³ de dichlorométhane, amené à pH 9-10 avec du carbonate de sodium solide. La phase aqueuse est de nouveau extraite trois fois par du dichlorométhane. Les phases organiques sont réunies, séchées sur du sulfate de magnésium, filtrées et concentrées à sec sous pression réduite pour donner 100 mg d'un produit qui est recristallisé dans un minimum d'éthanol. Après une nuit au réfrigérateur, filtration et séchage, on obtient 58 mg de 1-((2R)-1-méthyl-pyrrolidin-2-ylméthyl)-4-phényl-1H-pyrazol-3-ol sous forme d'un solide blanc. Spectre de R.M.N. ¹H (400MHz, (CD₃)₂SO d6 avec une goutte AcOD d4, δ en ppm) : de 1,62 à 2,00 (m, 4H) ; 2,42 (s, 3H) ; 3,02 (m, 1H) ; 3,21 (m, 2H) ; 3,96 (dd, J = 7,0 et 14,0 Hz, 1H) ; 4,13 (dd, J = 5,5 et 14,0 Hz, 1H) ; 7,13 (t large, J = 7,5 Hz, 1H) ; 7,33 (t large, J = 7,5 Hz, 2H) ; 7,65 (d large, J = 7,5 Hz, 2H) ; 7,97 (s, 1H). Spectre de masse (IE) : 258(+)=(M+H)(+). Le 3-benzyloxy-1-((2R)-1-méthyl-pyrrolidin-2-ylméthyl)-4-phényl-1H-pyrazole peut être préparé de la manière suivante :

Une solution de 400 mg de chlorhydrate de 3-benzyloxy-4-phényl-((2R)-pyrrolidin-2-ylméthyl)-1H-pyrazole, 297 mg de carbonate de potassium, 0,101 cm³ d'iodure de méthyle dans 4 cm³ de diméthylformamide est agitée une nuit à une température voisine de 20 °C. Le milieu est dilué avec de l'eau puis extrait par de l'acétate d'éthyle. La phase organique est séchée sur sulfate de magnésium, filtrée et concentrée à sec sous pression réduite pour donner un brut réactionnel qui est purifié par chromatographie sur gel de silice (éluant : dichlorométhane à 3% de méthanol). Après concentration à sec des fractions, on obtient 215 mg de 3-benzyloxy-1-((2R)-1-méthyl-pyrrolidin-2-ylméthyl]-4-phényl-1H-pyrazole.

Le chlorhydrate de 3-benzyloxy-4-phényl-1-((2R)-pyrrolidin-2-ylméthyl)-1H-pyrazole peut être préparé de la manière suivante :

A une solution de 500 mg de 3-benzyloxy-1-((2R)-1-tert-butoxycarbonyl-pyrrolidin-2-ylméthyl)-4-phényl-1H-pyrazole dans 5 cm³ de dioxane sont additionnés 3,17 cm³ d'une solution d'acide chlorhydrique 4 N dans le dioxane. Le milieu réactionnel est agité une nuit à une température voisine de 20°C puis concentré à sec sous pression réduite pour donner 400 mg de chlorhydrate de 3-benzyloxy-4-phényl-1-((2R)-pyrrolidin-2-ylméthyl)-1H-pyrazole sous forme d'un solide blanc. Spectre de masse (IE) : m/z 334⁺ (M+H)⁺, m/z 667⁺ (2M+H)⁺.

Le 3-benzyloxy-4-phényl-1-((2R)-1-tert-butoxycarbonyl-pyrrolidin-2-ylméthyl)-1H-pyrazole peut être préparé de la manière suivante :

Une solution de 251 mg de 3-benzyloxy-4-phényl 1H-pyrazole et 72 mg d'hydrure de sodium (à 50 % dans l'huile) dans 5 cm³ de diméthylformamide est agitée pendant 1 h avant d'additionner une solution de 364 mg de l'ester ((2R)-1-tert-butoxycarbonyl-pyrrolidin-2-ylméthyl) de l'acide méthane sulfonique dans 5 cm³ de diméthylformamide. Le milieu réactionnel est agité à 80°C pendant 3 h puis versé dans de l'eau. Après extraction par de l'acétate d'éthyle, la phase organique est lavée trois fois par une solution aqueuse saturée de chlorure de sodium, séchée sur du sulfate de magnésium, filtrée puis concentrée à sec pour fournir 440 mg d'un produit brut qui est purifié par chromatographie sur gel de silice (éluant : heptane / acétate d'éthyle 80 / 20). Après concentration à sec des fractions, on obtient 202 mg de 3-benzyloxy-4-phényl-1-((2R)-1-tert-butoxycarbonyl-pyrrolidin-2-ylméthyl)-1H-pyrazole. Spectre de masse (IE) : m/z 456⁺ (M+Na)⁺, m/z 434⁺ (M+H)⁺, m/z 334⁺ [(M+H)⁺- CO₂tBu + H].

L'ester ((2R)-1-tert-butoxycarbonyl-pyrrolidin-2-ylméthyl) de l'acide méthane sulfonique peut être préparé de la manière suivante :

A une solution de 3 g de (2R)-1-tert-butoxycarbonyl-2-hydroxyméthylpyrrolidine et 2,27 cm³ de triéthylamine dans 65 cm³ de dichlorométhane agitée sous atmosphère d'azote à -10°C est additionnée goutte à goutte une solution de 1,2 cm³ de chlorure de méthane sulfonyle dans 20 cm³ de dichlorométhane. Le milieu réactionnel est laissé remonter à 20°C avant concentration à sec sous pression réduite. Le résidu obtenu est repris par de l'eau, extrait deux fois par 20 cm³ d'acétate d'éthyle. Les phases organiques réunies sont lavées trois fois par 20 cm³ d'une solution aqueuse de bicarbonate de sodium à 5%, séchées sur du sulfate de magnésium, filtrées et concentrées à sec sous pression réduite pour donner 3,75 g d'un mélange d'ester ((2R)-1-tert-butoxycarbonyl-pyrrolidin-2-ylméthyl) de l'acide méthane sulfonique et de (2R)-1-tert-butoxycarbonyl-2-hydroxyméthylpyrrolidine. Le mélange est remis en réaction dans les mêmes conditions que précédemment mais avec 0,3 eq de triéthylamine et 0,3 eq de chlorure de méthane sulfonyle. Après un traitement similaire, on obtient 3,63 g d'ester ((2R)-1-tert-butoxycarbonyl-pyrrolidin-2-ylméthyl) de l'acide méthane sulfonique sous forme d'un liquide incolore. Spectre de masse (IE) : m/z 280⁺ (M+H)⁺.

### Exemple 63

### Chlorhydrate de 4-phényl-1-(piperidin-3-yl)-1H-pyrazol-3-ol

Une suspension de 130 mg de chlorhydrate de 3-benzyloxy-4-phényl-1-(piperidin-3-yl)-1H-pyrazole et 13 mg de palladium sur charbon à 10% dans 4 cm³ d'éthanol est hydrogénée à une température voisine de 20°C sous une atmosphère de 1500 mbar pendant 3 h. Le milieu réactionnel est repris par un mélange de 15 cm³ de dichlorométhane / méthanol 80 / 20 en volumes, essoré sur Clarcel puis lavé deux fois par 10 cm³ d'un mélange dichlorométhane / méthanol 80 / 20 en volumes. Après concentration à sec, le produit cristallisé est repris par 5 cm³ d'acétate d'éthyle, essoré et repris deux fois par 0,5 cm³ d'acétate d'éthyle pour fournir 70 mg de chlorhydrate de 4-phényl-1-(piperidin-3-yl)-1H-pyrazol-3-ol sous forme d'un produit cristallisé. Spectre de R.M.N. ¹H (300MHz, (CD₃)₂SO d6, δ en ppm) : de 1,72 à 2,22 (m, 4H) ; 2,86 (m, 1H) ; de 3,10 à 3,57 (m, partiellement masqué, 3H) ; 4,36 (m, 1H) ; 7,14 (t large, J = 7,5 Hz, 1H) ; 7,33 (t large, J = 7,5 Hz, 2H) ; 7,68 (d large, J = 7,5 H, 2H) ; 8,07 (s, 1H) ; 9,18 (m étalé, 2H) ; 10,4 (s large, 1H). Spectre de masse (IE) : m/z 244⁺ (M+H)⁺.

Le chlorhydrate de 3-benzyloxy-4-phényl-1-(piperidin-3-yl)-1H-pyrazole peut être préparé de la manière suivante :

A une solution de 230 mg de 3-benzyloxy-1-(1-tert-butoxycarbonyl-piperidin-3-yl)-4-phényl 1H-pyrazole dans 2 cm³ d'acétate d'éthyle refroidie à l'aide d'un bain de glace sont additionnés 2 cm³ d'une solution d'acide chlorhydrique 4N dans l'acétate d'éthyle. Le milieu réactionnel est laissé revenir à une température voisine de 20°C puis agité pendant 2 h 30 avant concentration de l'acétate d'éthyle. Le résidu est repris trois fois par 2 cm³ d'éther diéthylique et l'insoluble est filtré pour donner 150 mg de chlorhydrate de 3 benzyloxy-4-phényl-1-(piperidin-3-yl)-1H-pyrazole. Spectre de masse (IE) : m/z 334⁺ (M+H)⁺, m/z 36⁺/38⁺ HCl⁺.

Le 3-benzyloxy-1-(1-tert-butoxycarbonyl-piperidin-3-yl)-4-phényl-1H-pyrazole peut être préparé de la manière suivante :

A une solution de 725 mg de 3-benzyloxy-4-phényl-1H-pyrazole dans 7 cm³ de diméthylformamide anhydre agitée sous atmosphère d'azote sont addditionnés en trois fois 153 mg d'hydrure de sodium (à 50% dans l'huile). Après 45 mn d'agitation à une température voisine de 20°C, une solution de 890 mg d'ester (1-tert-butoxycarbonyl-piperidin-3-yl) de l'acide méthane sulfonique dans 4,5 cm³ de diméthylformamide anhydre est additionnée. Le milieu réactionnel est agité 3 h à 80°C puis, après refroidissement, jeté dans de l'eau. La phase aqueuse est extraite quatre fois par 50 cm³ d'acétate d'éthyle. Les phases organiques réunies sont lavées par une solution aqueuse saturée de chlorure de sodium, séchées sur du sulfate de magnésium, filtrées puis concentrées à sec sous pression réduite. Le produit obtenu est repris par du dichlorométhane, qui, après essorage, fournit 380 mg de 3-benzyloxy-4-phényl-1H-pyrazole sous forme d'un solide beige. Le filtrat, après concentration à sec, est purifié par chromatographie sur colonne de 70 g de silice (Merck, éluant: dichlorométhane / acétate d'éthyle 95 / 5 en volumes). Après concentration des fractions sous pression réduite, on obtient 280 mg de 3-benzyloxy-1-(1-tert-butoxycarbonyl-piperidin-3-yl)-4-phényl-1H-pyrazole avec une pureté de 70%. Ces 280 mg sont de nouveau purifiés par chromatographie sur colonne de 30 g de silice (Merck, éluant : dichlorométhane / heptane 70 / 30 en volumes). Après concentration des fractions sous pression réduite, on obtient 230 mg de 3-benzyloxy-1-(1-tert-butoxycarbonyl-piperidin-3-yl)-4-phényl-1H-pyrazole. Spectre de R.M.N. ¹H (300MHz, (CD₃)₂SO d6, δ en ppm) : 1,20 (m, 1H) ; 1,41 (s, 9H) ; de 1,45 à 2,16 (m, 3H) ; 2,93 (m, 1H) ; de 3,00 à 3,40 (m étalé, 1H) ; 3,79 (m, 1H) ; de 3,98 à 4,16 (m, 2H) ; 5,31 (s large, 2H) ; 7,15 (t large, J = 7,5 Hz, 1H) ; de 7,30 à 7,45 (m, 5H) ; 7,51 (d large, J = 7,5 Hz, 2H) ; 7,65 (d large, J = 7,5 Hz, 2H) ; 8,14 (s, 1H).

L'ester (1-tert-butoxycarbonyl-piperidin-3-yl) de l'acide méthane sulfonique peut être préparé de la manière suivante :

A une solution de 750 mg de 1-tert-butoxycarbonyl-3-hydroxypipéridine et 0,570 cm³ de triéthylamine dans 7 cm³ de dichlorométhane agitée sous atmosphère d'azote à -10°C est additionnée goutte à goutte une solution de 0,305 cm³ de chlorure de méthane sulfonyle. Le milieu réactionnel est laissé remonter à une température voisine de 20°C et agiter pendant 3 h avant concentration à sec sous pression réduite. Le résidu obtenu est repris par de l'acétate d'éthyle. La phase organique est successivement lavée par une solution aqueuse de bicarbonate de sodium à 5 % puis une solution aqueuse saturée de chlorure de sodium, séchée sur du sulfate de magnésium, filtrée et concentrée à sec sous pression réduite pour donner 0,9 g d'ester (1-tert-butoxycarbonyl-piperidin-3-yl) de l'acide méthane sulfonique sous forme d'une huile incolore. Spectre de masse (IE) : m/z 280⁺ (M+H)⁺, m/z 224⁺ [(M+H)⁺ - tBu + H].

### Exemples 64

### Chlorhydrate de 1-(1-méthyl-pipéridin-2-ylméthyl)-4-phényl-1H-pyrazol-3-ol

Une solution de 610 mg de 3-benzyloxy-1-(1-méthyl-pipéridin-2-ylméthyl)-4-phényl-1H-pyrazole dans 12 cm³ d'acétate d'éthyle et 6 cm³ d'une solution chlorhydrique 4M dans l'acétate d'éthyle est agitée 15 min à une température voisine de 20°C. Après concentration à sec sous pression réduite, on obtient 669 mg du chlorhydrate de 3-benzyloxy-1-(1-méthyl-pipéridin-2-ylméthyl)-4-phényl-1H-pyrazole qui est immédiatement remis en réaction. Une suspension de 669 mg du chlorhydrate précédent et 66 mg de palladium sur charbon à 10% dans 15 cm³ d'éthanol est hydrogénée à une température voisine de 20°C sous une atmosphère de 1500 mbar pendant 3 h. Le milieu réactionnel est repris par un mélange de 25 cm³ de dichlorométhane / méthanol 80 / 20 puis essoré sur Clarcel. Après concentration à sec, le produit obtenu est dissous dans 20 cm³ d'eau puis lyophilisé pour donner 500 mg de chlorhydrate de 1-(1-méthyl-pipéridin-2-ylméthyl)-4-phényl-1H-pyrazol-3-ol. Spectre de R.M.N. ¹H (300MHz, (CD₃)₂SO d6 à 353K, δ en ppm) : de 1,37 à 1,87 (m, 6H) ; 2,86 (m large, 4H) ; de 3,32 à 3,60 (m très étalé, 2H) ; 4,24 (m large, 1H) ; 4,50 (m large, 1H) ; 7,16 (t large, J = 7,5 Hz, 1H) ; 7,34 (t large, J = 7,5 Hz, 2H) ; 7,67 (d large, J = 7,5 Hz, 2H) ; 7,97 (s, 1H) ; de 10,0 à 10,6 (m très étalé, 2H). Spectre de masse (IE) : m/z 272⁺ (M+H)⁺, m/z 36⁺/38⁺ HCl⁺.

### Exemple 65

### Chlorhydrate de 1-(1-méthyl-azepan-3-yl)-4-phényl-1H-pyazol-3-ol

Le chlorhydrate de 1-(1-méthyl-azepan-3-yl)-4-phényl-1H-pyrazol-3-ol peut être préparé selon la même méthode utilisée pour la préparation du chlorhydrate de 1-(1-méthyl-pipéridin-2-ylméthyl)-4-phényl-1H-pyrazol-3-ol, mais à partir de 470 mg de 3-benzyloxy-1-(1-méthyl-azépan-3-yl)-4-phényl-1H-pyrazole, 10 cm³ d'acétate d'éthyle, 5 cm³ d'une solution d'acide chlorhydrique 4M dans l'acétate d'éthyle, puis de 51 mg de palladium sur charbon à 10% et 15 cm³ d'éthanol. Selon les mêmes conditions de traitement, on obtient ainsi 400 mg de chlorhydrate de 1-(1-méthyl-azépan-3-yl)-4-phényl-1H-pyrazol-3-ol. Spectre de RM.N. ¹H (400MHz, (CD₃)₂SO d6 à 343K, δ en ppm) : de 1,62 à 2,32 (m, 6H) ; 2,86 (s large, 3H) ; de 2,92 à 3,76 (m partiellement masqué, 4H) ; de 4,62 à 4,92 (m très étalé, 1H) ; 7,15 (t large, J = 7,5 Hz, 1H) ; 7,33 (t large, J = 7,5 Hz, 2H) ; 7,65 (d large, J = 7,5 H, 2H) ; 7,93 (s large, 1H) ; de 9,85 à 10,3 (m étalé, 1H) ; de 10,7 à 11,4 (m très étalé, 1H). Spectre de masse (IE) : m/z 272⁺ (M+H)⁺.

Les 3-benzyloxy-1-(1-méthyl-pipéridin-2-ylméthyl]-4-phényl-1H-pyrazole et 3-benzyloxy-1-(1-méthyl-azépan-3-yl)-4-phényl-1H-pyrazole peuvent être préparés de la manière suivante :

A une solution de 2 g de 3-benzyloxy-4-phényl-1H-pyrazole dans 20 cm³ de diméthylformamide anhydre agitée sous atmosphère d'azote sont addditionnés en trois fois 426 mg d'hydrure de sodium (à 50% dans l'huile). Après 30 min d'agitation à une température voisine de 20°C, est additionné goutte à goutte 1,3 g d'un mélange 75 / 25 de 1-méthyl-2-chlorométhylpipéridine et d'ester (1-méthyl-pipéridin-2-ylméthyl) de l'acide méthane sulfonique. Le milieu réactionnel est agité 3 h à 80°C puis, après refroidissement, jeté dans un mélange eau / glace. La phase aqueuse est extraite par de l'acétate d'éthyle. La phase organique est lavée par une solution aqueuse saturée de chlorure de sodium, séchée sur du sulfate de magnésium, filtrée puis concentrée à sec sous pression réduite. Le produit est purifié par deux chromatographies successives sur colonne de 119 g de silice (Merck, éluant : dichlorométhane / méthanol 97 / 3 en volumes). Après concentration des fractions sous pression réduite, on obtient 460 mg de 3-benzyloxy-1-(1-méthyl-azépan-3-yl)-4-phényl-1H-pyrazole et 650 mg de 3-benzyloxy-1-(1-méthyl-pipéridin 2-ylméthyl)-4-phényl-1H-pyrazole.

3-Benzyloxy-1-(1-méthyl-pipéridin-2-ylméthyl]-4-phényl-1H-pyrazole. Spectre de R.M.N. ¹H (400MHz, (CD₃)₂SO d6, δ en ppm) : de 1,12 à 1,65 (m, 6H) ; 2,06 (m, 1H) ; 2,29 (s, 3H) ; 2,34 (m, 1H) ; 2,77 (m, 1H) ; 3,86 (dd, J = 7,5 et 14,0 Hz, 1H) ; 4,25 (dd, J = 5,5 et 14,0 Hz, 1H) ; 5,30 (s, 2H) ; 7,14 (t large, J = 7,5 Hz, 1H) ; de 7,30 à 7,44 (m, 5H) ; 7,50 (d large, J = 7,5 Hz, 2H) ; 7.64 (d large, J = 7,5 Hz, 2H) ; 8,06 (s, 1H).

3-Benzyloxy-1-(1-méthyl-azépan-3-yl)-4-phényl-1H-pyrazole. Spectre de R.M.N. ¹H (400MHz, (CD₃)₂SO d6, δ en ppm) : de 1,56 à 1,82 (m, 4H) ; de 1,96 à 2,08 (m, 2H) ; 2,33 (s, 3H) ; de 2,50 à 2,93 (m partiellement masqué, 4H) ; 4,26 (m, 1H) ; 5,30 (s, 2H) ; 7,13 (t large, J = 7,5 Hz, 1H) ; de 7,28 à 7,45 (m, 5H) ; 7,51 (d large, J = 7,5 Hz, 2H) ; 7,65 (d large, J = 7,5 H, 2H) ; 8,11 (s, 1H).

Le mélange de 2-chlorométhyl-1-méthyl-pipéridine et d'ester (1-méthyl-pipéridin-2-ylméthyl) de l'acide méthane sulfonique peut être préparé de la manière suivante :

A une solution de 1,31 cm³ de (1-méthyl-pipéridin-2-yl)-méthanol et 1,53 cm³ de triéthylamine dans 26 cm³ de dichlorométhane agitée sous atmosphère d'azote à - 10°C est additionnée goutte à goutte une solution de 0,815 cm³ de chlorure de méthane sulfonyle. Le milieu réactionnel est laissé remonter à une température voisine de 20°C et agiter pendant 3 h avant concentration à sec sous pression réduite. Le résidu obtenu est repris par de l'acétate d'éthyle. La phase organique est successivement lavée par une solution aqueuse de bicarbonate de sodium à 5% puis une solution aqueuse saturée de chlorure de sodium, séchée sur du sulfate de magnésium, filtrée et concentrée à sec sous pression réduite pour donner 1,3 g d'un mélange 75 / 25 de 2-chlorométhyl-1-méthyl-pipéridine et d'ester (1-méthyl-pipéridin-2-ylméthyl) de l'acide méthane sulfonique. Spectre de masse (IE) : m/z 148⁺ (M+H)⁺, m/z 208⁺ (M+H)⁺.

### Exemple 66

### Chlorydrate de 4-phényl-1-(2-pipéridin-1-yl-éthyl)-1H-pyrazol-3-ol

On opère comme à l'exemple 38 mais avec 0,75 g d' oxalate de 3-benzyloxy-4-phényl-1-(2-pipéridin-1-yl-éthyl)-1H-pyrazole, 5,9 cm³ d'acide chlorhydrique 12 N et 5,9 cm³ d'éthanol. Le mélange est chauffé pendant 4 heures à une température proche de 100°C. Après refroidissement à une température proche de 20°C, le milieu réactionnel est repris par de l'éthanol, concentré à sec sous pression réduite (2 kPa) ; le résidu est précipité dans un mélange d'oxyde de diisopropyle et d'éthanol. On obtient 0,357 g de chlorhydrate de 4-phényl-1-(2-pipéridin-1-yl-éthyl)-1H-pyrazol-3-ol sous forme d'un solide blanc. Spectre RMN 1H (300MHz ) - δ en ppm - dans le DMSO-d6 : 1,40 (m, 1H) ; de 1,63 à 1,87 (m, 5H) ; 2,92 (m, 2H) ; de 3,36 à 3,52 (m, 4H) ; 4,43 (t, J = 6,5 Hz, 2H) ; 7,14 (tt, J = 1,5 et 7,5 Hz, 1H) ; 7,33 (t large, J = 7,5 Hz, 2H) ; 7,64 (d large, J = 7,5 Hz, 2H) ; 8,05 (s, 1H) ; de 10,35 à 10,72 (m étalé, 2H). Spectre IR (KBr) : 2939; 1606; 1581; 1520; 1454; 1444; 1170; 771; 700; 673 et 427 cm⁻¹.

L' oxalate de 3-benzyloxy-4-phényl-1-(2-pipéridin-1-yl-éthyl)-1H-pyrazole peut être obtenu de la manière suivante :

On opère comme à l'exemple 15 mais avec 0,166 g d'hydrure de sodium (à 75% en masse dans l'huile de vaseline), 0,515 g de chlorhydrate de 1-(2-chloroéthyl)-pipéridine et 0,5 g de 3-benzyloxy-4-phényl-pyrazole. On obtient ainsi 0,754 g d' oxalate de 3-benzyloxy-4-phényl-1-(2-pipéridin-1-yl-éthyl)-1H-pyrazole sous forme d'une poudre blanche. Spectre IR (KBr) : 2930; 2638; 2542; 1606; 1511; 1454; 1357; 1280; 1181; 763; 721; 697 et 501 cm⁻¹. Spectre masse (IC) : m/z=362 (MH⁺) pic de base.

### Oxalate de de 4-phényl-1-(2-pipéridin-1-yl-éthyl)-1H-pyrazole

On opère comme à l'exemple 15 mais avec 0,231 g d'hydrure de sodium (à 75% en masse dans l'huile de vaseline), 0,715 g de chlorhydrate de 1-(2-chloroéthyl)-pipéridine et 0,4 g de 4-phényl-pyrazole. On obtient ainsi 0,832 g d' oxalate de 4-phényl-1-(2-pipéridin-1-yl-éthyl)-1H-pyrazole sous forme de cristaux blancs.

Spectre RMN 1H (300MHz ) - δ en ppm - dans le DMSO-d6 : 1,60 (m, 2H) ; 1,79 (m, 4H) ; 3,06 (m, 4H) ; 3,43 (t, J = 6,5 Hz, 2H) ; 4,58 (t, J = 6,5 Hz, 2H) ; 7,32 (t large, J = 7,5 Hz, 1H) ; 7,48 (t large, J = 7,5 Hz, 2H) ; 7,69 (d large, J = 7,5 Hz, 2H) ; 8,06 (s, 1H) ; 8,33 (s, 1H) . Spectre IR (KBr) : 2949; 1679; 1713; 1606; 1460; 1187; 955; 763; 703 et 476 cm⁻¹.

### Exemple 67

### Chlorhydrate de 4-(thiophèn-2-yl)-1-(2-pipéridin-1-yl-éthyl)-1H-pyrazol-3-ol

Une suspension de 0,15 g de chlorhydrate de 4-(5-chloro-thiophèn-2-yl)-1-(2-pipéridin-1-yl-éthyl)-1H-pyrazol-3-ol et 5 mg de palladium sur charbon (à 10%) dans 15 cm³ de méthanol est agitée en autoclave sous une pression d'hydrogène de 3000 kPa, à une température de 60°C pendant 20 heures. Le milieu réactionnel est ensuite filtré sur Célite®, rincé avec du méthanol et concentré à sec sous pression réduite (3 kPa). Le résidu est trituré par de l'oxyde de d'isopropyle ; après filtration du solide apparu et séchage sous vide (70 Pa) à une température de 60°C, on obtient 0,1 g de chlorhydrate de 4-(thiophèn-2-yl)-1-(2-pipéridin-1-yl-éthyl)-1H-pyrazol-3-ol sous forme d'une poudre grise fondant vers 180°C (avec décomposition). Spectre

RMN 1H (300MHz ) - δ en ppm - dans le DMSO-d6 : 1,40 (m, 1H) ; de 1,62 à 1,85 (m, 5H) ; de 2,82 à 3,02 (m large, 2H) ; de 3,30 à 3,52 (m partiellement masqué, 4H) ; 4,40 (t large, J = 6,5 Hz, 2H) ; 7,02 (m, 1H) ; 7,19 (m, 1H) ; 7,32 (m, 1H) ; 7,93 (s large, 1H) ; de 10,1 à 10,65 (m étalés, 2H) . Spectre IR, KBr : 2952; 2539; 1605; 1545; 1455; 1404; 1175; 969 et 697 cm⁻¹.

### Exemple 68

### Chlorhydrate de 4-(3,4-dichloro-phényl)-1-(2-pipéridin-1-yl-éthyl)-1H-pyrazol-3-ol

On opère comme à l'exemple 38 mais avec 0,47 g de 3-benzyloxy-4-(3,4-dichloro-phényl-1-(2-pipéridin-1-yl-éthyl)-1H-pyrazole, 3 cm³ d'acide chlorhydrique 12 N et 10 cm³ d'éthanol. Le mélange est chauffé pendant 24 heures à une température proche de 100°C. Après refroidissement à une température proche de 20°C, le milieu réactionnel est repris par 3 fois 30 cm³ de toluène puis 3 fois 30 cm³ d'acétone l'éthanol, concentré à sec sous pression réduite (2 kPa) ; le résidu est précipité dans 30 cm³ d'acétone. On obtient 0,26 g de chlorhydrate de 4-(3,4-dichloro-phényl)-1-(2-pipéridin-1-yl-éthyl)-1H-pyrazol-3-ol sous forme d'une poudre blanchâtre. Spectre RMN 1H (300MHz ) - δ en ppm - dans le DMSO-d6 : de 1,32 à 1,92 (m, 6H) ; de 2,85 à 3,60 (m, 6H) ; 4,36 (m large, 2H) ; de 7,55 à 7,68 (m, 2H) ; 7,90 (d, J = 2,0 Hz, 1H) ; 8,15 (s large, 1H) ; de 9,35 à 9,48 (m étalé, 1H) ; 10,8 (s large, 1H). Spectre IR, KBr: 2945; 2533; 1604; 1525; 1448; 1180; 1028 et 806 cm⁻¹.

### 3-Benzyloxy-4-(3,4-dichloro-phényl)-1-(2-pipéridin-1-yl-éthyl]-1H-pyrazole

On opère comme à l'exemple 15 mais avec 0,135 g d'hydrure de sodium (à 75% en masse dans l'huile de vaseline), 0,519 g de chlorhydrate de 1-(2-chloroéthyl)-pipéridine et 0,45 g de 3-benzyloxy-4-(3,4-dichloro-phényl)-pyrazole. Après chauffage du milieu réactionnel pendant 1 heure à 50°C puis à 20°C pendant 16 heures , le milieu est repris par 150 cm³ d'acétate d'éthyle et 150 cm³ d'eau ; la phase organique est décantée, lavée par 2 fois 100 cm³ d'eau distillée et 100 cm³ d'une solution aqueuse saturée de chlorure de sodium, séchée sur du sulfate de sodium, filtrée et concentrée à sec sous pression réduite (3 kPa). On obtient ainsi 0,6 g de 3-benzyloxy-4-(3,4-dichloro-phényl)-1-(2-pipéridin-1-yl-éthyl]-1H-pyrazole sous forme d'une huile jaune orangée. Spectre RMN 1H (400MHz) - δ en ppm - dans le DMSO-d6 : 1,38 (m, 2H) ; 1,48 (m, 4H) ; 2,38 (m, 4H) ; 2,68 (t, J = 6,5 Hz, 2H) ; 4,07 (t, J = 6,5 Hz, 2H) ; 5,33 (s, 2H) ; 7,36 (tt, J = 1,5 et 7,5 Hz, 1H) ; 7,41 (t large, J = 7,5 Hz, 2H) ; 7,51 (d, J = 7,5 Hz, 2H) ; de 7,57 à 7,65 (m, 2H) ; 7,88 (d, J = 2,5 Hz, 1H) ; 8,21 (m, 1H). Spectre masse (IC) : m/z=430 (MH⁺) pic de base.

### 3-Benzyloxy-4-(3,4-dichloro-phényl)-1H-pyrazole

On opère comme à l'exemple 38 mais avec 0,3 g de 1-(toluène-4-sulfonyl)-3-benzyloxy-4-(3,4-dichloro-phényl)-1H-pyrazole et 1,6 cm³ d'une solution 1 N de fluorure de tétrabutylammonium dans le tétrahydrofurane et 15 cm³ de tétrahydrofurane. On obtient ainsi 0,2 g de 3-benzyloxy-4-(3,4-dichloro-phényl)-1H-pyrazole sous forme d'une huile qui cristallise. Spectre RMN 1H (300MHz ) - δ en ppm - dans le DMSO-d6 : 5,35 (s, 2H) ; de 7,32 à 7,46 (m, 3H) ; 7,50 (d large, J = 7,5 Hz, 2H) ; 7,59 (d, J = 9,0 Hz, 1H) ; 7,70 (dd, J = 2,5 et 9,0 Hz, 1H) ; 7,95 (d, J = 2,5 Hz, 1H) ; 8,23 (s, 1H) ; 12,3 (m étalé, 1H) . Spectre masse (IE) m/z=318 (M^{+.} ), m/z=91(C₇H₇⁺) pic de base.

### 3-Benzyloxy-1-(toluéne-4-sulfonyl)-4-(3,4-dichloro-phényl)-1H-pyrazole

On opère comme à l'exemple 38 mais avec 0,3 g de 1-(toluène-4-sulfonyl)-3-benzyloxy-4-iodo-1H-pyrazole, 2,29 g d'acide 3,4-dichloro-phényl boronique, 2,547 g de phosphate de tripotassium, 0,421 g de dichloro-bis(triphénylphoshine)palladium dans 40 cm³ de diméthoxyéthane. Après purification par chromatographie, sous une pression d'azote de 50 kPa, sur une colonne de gel de silice (granulométrie 20-45 µ; diamètre 4 cm; hauteur 60 cm), en éluant avec un mélange d'acétate d'éthyle et de cyclohexane (5/95 puis 10/90 en volumes), les fractions 9 à 12 sont réunies, concentrées à sec sous pression réduite (3 kPa).On obtient ainsi 0,3 g de 3-benzyloxy-1-(toluène-4-sulfonyl)-4-(3,4-dichloro-phényl)-1H-pyrazole sous forme d'une poudre blanche. Spectre masse (IE) : m/z=472 (M^{+.}), m/z=317 [(M - C₇H₇SO₂)⁺], m/z=91 (C₇H₇⁺) pic de base.

### Exemple 69

### 4-(4-Bromo-phényl)-1-(2-pipéridin-1-yl-éthyl)-1H-pyrazol-3-ol

On opère comme à l'exemple 38 mais avec 0,32 g de 3-benzyloxy-4-(4-bromo-phényl)-1-(2-pipéridin-1-yl-éthyl]-1H-pyrazole, 3 cm³ d'acide chlorhydrique 12 N et 10 cm³ d'éthanol. Le mélange est chauffé pendant 20 heures à une température proche de 100°C. Après refroidissement à une température proche de 20°C, le milieu réactionnel est repris par 5 fois 30 cm3 d'acétone. Après concentration à sec sous pression réduite (3 kPa), le résidu est trituré par 30 cm³ d'oxyde de diisopropyle puis purifié par chromatographie, sous une pression d'azote de 50 kPa, sur une colonne de gel de silice (granulométrie 20-45 µ ; diamètre 2 cm; hauteur 20 cm), en éluant avec de l'acétate d'éthyle puis un mélange d'acétate d'éthyle et de méthanol (95/5 puis 90/10 puis 80/20 en volumes). Les fractions 10 à 28 sont réunies, concentrées à sec sous pression réduite (3 kPa). On obtient ainsi 0,11 mg de 4-(4-bromo-phényl)-1-(2-pipéridin-1-yl-éthyl)-1H-pyrazol-3-ol sous forme d'une poudre blanche. Spectre RMN 1H (300MHz) - δ en ppm - dans le DMSO-d6 : de 1,32 à 1,55 (m, 6H) ; 2,39 (m, 4H) ; 2,65 (t, J = 6,5 Hz, 2H) ; 4,00 (t, J = 6,5 Hz, 2H) ; 7,50 (d large, J = 8,5 Hz, 2H) ; 7,62 (d large, J = 8,5 Hz, 2H) ; 7,96 (s, 1H) ; de 10,3 à 10,50 (m étalé, 1H). Spectre IR, KBr : 2941; 1631; 1601; 1529; 1173; 1007; 824 et 510 cm⁻¹.

### 3-Benzyloxy-4-(4-bromo-phényl)-1-(2-pipéridin-1-yl-éthyl]-1H-pyrazole

On opère comme à l'exemple 15 mais avec 2,77 g d'hydrure de sodium (à 75 % en masse dans l'huile de vaseline), 1,063 g de chlorhydrate de 1-(2-chloroéthyl)-pipéridine et 0,95 g de 3-benzyloxy-4-(4-bromo-phényl)-pyrazole. Après chauffage du milieu réactionnel pendant 1 heure à 50°C, le milieu est refroidi à une température proche de 20°C, repris par 300 cm³ d'acétate d'éthyle et 300 cm³ d'eau ; la phase organique est décantée, séchée sur du sulfate de magnésium, filtrée et concentrée à sec sous pression réduite (3 kPa). Le résidu est purifié par chromatographie, sous une pression d'azote de 50 kPa, sur une colonne de gel de silice (granulométrie 20-45 µ ; diamètre 3 cm; hauteur 40 cm), en éluant avec un mélange d'acétate d'éthyle et de cyclohexane (5/95 puis 10/95 en volumes). Les fractions 19 à 35 sont réunies, concentrées à sec sous pression réduite (3 kPa). On obtient ainsi 0,32 g de 3-benzyloxy-4-(4-bromo-phényl)-1-(2-pipéridin-1-yl-éthyl]-1H-pyrazole sous forme d'une huile incolore. Spectre RMN 1H (300MHz) - δ en ppm - dans le DMSO-d6 :

De 1,34 à 1,53 (m, 6H) ; 2,38 (m, 4H) ; 2,68 (t, J = 6,5 Hz, 2H) ; 4,07 (t, J = 6,5 Hz, 2H) ; 5,32 (s, 2H) ; de 7,32 à 7,55 (m, 7H) ; 7,60 (d large, J = 8,5 Hz, 2H) ; 8,10 (s, 1H). Spectre masse (ES) : m/z=440 (MH⁺) pic de base.

### 3-Benzyloxy-4-(4-bromo-phényl)-1H-pyrazole

On opère comme à l'exemple 38 mais avec 1,5 g de 1-(toluène-4-sulfonyl)-3-benzyloxy-4-(4-bromo-phényl)-1H-pyrazole et 7,1 cm³ d'une solution 1 N de fluorure de tétrabutylammonium dans le tétrahydrofurane et 50 cm³ de tétrahydrofurane. On obtient ainsi 0,93g de 3-benzyloxy-4-(4-bromo-phényl)-1H-pyrazole sous forme d'une poudre blanchâtre. Spectre RMN 1H (400MHz) - δ en ppm - dans le DMSO-d6 : 5,34 (s, 2H) ; 7,35 (tt, J = 1,5 et 7,5 Hz, 1H) ; 7,41 (t large, J = 7,5 Hz, 2H) ; de 7,47 à 7,54 (m, 4H) ; 7,66 (m, 2H) ; 8,13 (s, 1H) ; 12,2 (m large, 1H). Spectre masse (IE) : m/z=328 (M^{+.}), m/z=91 (C₇H₄⁺) pic de base.

### 3-Benzyloxy-1-(toluène-4-sulfonyl)-4-(4-bromo-phényl)-1H-pyrazole

On opère comme à l'exemple 38 mais avec 1,817 g de 1-(toluène-4-sulfonyl)-3-benzyloxy-4-iodo-1H-pyrazole, 2,41 g d'acide 4-bromo-phényl boronique, 2,54 g de phosphate de tripotassium, 0,421 g de dichloro-bis(triphénylphoshine)palladium dans 40 cm³ de diméthoxyéthane. Après purification par chromatographie, sous une pression d'azote de 50 kPa, sur une colonne de gel de silice (granulométrie 20-45 µ ; diamètre 4 cm; hauteur 60 cm), en éluant avec un mélange d'acétate d'éthyle et de cyclohexane (5/95 en volumes), les fractions 15 à 30 sont réunies, concentrées à sec sous pression réduite (3 kPa). On obtient ainsi 1,5 g de 3-benzyloxy-1-(toluène-4-sulfonyl)-4-(4-bromo-phényl)-1H-pyrazole sous forme d'une huile jaune qui cristallise. Spectre RMN 1H (300MHz ) - δ en ppm - dans le DMSO-d6 pour 77 % du mélange : 2,42 (s, 3H) ; 5,33 (s, 2H) ; de 7,33 à 7,50 (m, 7H) ; 7,59 (d large, J = 8,5 Hz, 2H) ; 7,72 (d large, J = 8,5 Hz, 2H) ; 7,84 (m, 2H) ; 8,86 (s, 1H) . Spectre masse (IE) : m/z=48 (M^{+.}), m/z=327 [(M - C₇H₇SO₂)⁺], m/z=91 (C₇H₇⁺), pic de base.

### Exemple 70

### 4-(1H-Indol-5-yl)-1-(2 pipéridin-1-yl-éthyl)-1H-pyrazol-3-ol

On agite pendant 30 minutes, sous atmosphère inerte, à une temperature proche de 70°C, un mélange de 0,5 g de 3-benzyloxy-4-(1H-indol-5-yl)-1-(2-pipéridin-1-yl-éthyl]-1H-pyrazole, 0,5 g de formiate d'ammonium et 0,5 g de palladium sur charbon (à 10%) dans 50 cm³ d'éthanol. Le milieu réactionnel est ensuite refroidi à une température proche de 20°C, filtré sur Célite®, rincé avec de l'éthanol et concentré à sec sous pression réduite (3 kPa). Le résidu est purifié sur cartouche de silice (granulométrie 20-40 µm) en éluant avec un mélange de dichlorométhane et d'une solution 2 N de méthanol ammoniacale (90/ 10 en volumes). On obtient 0,258 g de 4-(1H-Indol-5-yl)-1-(2-pipéridin-1-yl-éthyl)-1H-pyrazol-3-ol sous forme de solide floconneux blanc. Spectre RMN 1H (300MHz ) - 8 en ppm - dans le DMSO-d6 : de 1,33 à 1,55 (m, 6H) ; 2,40 (m, 4H) ; 2,66 (t, J = 6,5 Hz, 2H) ; 4,00 (t, J = 6,5 Hz, 2H) ; 6,39 (t large, J = 2,5 Hz, 1H) ; de 7,27 à 7,40 (m, 3H) ; 7,81 (m, 2H) ; 10,05 (s large, 1H) ; 10,95 (m large, 1H). Spectre IR, KBr : 3265; 2944; 1593; 1524; 1242; 1184; 1044; 891; 803; 762; 725 et 437 cm⁻¹. Spectre masse (IE) : m/z=310 (M^{+.}), m/z=98 (C₆H₁₂N⁺) pic de base.

### 3-Benzyloxy-4-(1H-indol-5-yl)-1-(2-pipéridin-1-yl-éthyl]-1H-pyrazole

On opère comme à l'exemple 37 mais avec 3,39 g de 3-benzyloxy-4-bromo-1-(2-pipéridin-1-yl-éthyl)-1H-pyrazole, 3,14 g d'acide 5-indolyl boronique, 3,87 g de carbonate de potassium, 1,2 g de tétrakis(triphényl)phosphine palladium dans 70 cm³ de toluène et 20 cm3 d'éthanol. Après purification 2 fois sur cartouche de silice (granulométrie 20-40 µm) en éluant avec un mélange de dichlorométhane et de méthanol (95/ 5 en volumes) , on obtient ainsi 2,17 g de 3-benzyloxy-4-(1H-indol-5-yl)-1-(2-pipéridin-1-yl-éthyl]-1H-pyrazole sous forme d'une huile beige qui cristallise. Spectre RMN 1H (300MHz ) - δ en ppm - dans le DMSO-d6 : de 1,33 à 1,55 (m, 6H) ; de 2,32 à 2,50 (m étalé, 4H) ; 2,72 (m large, 2H) ; 4,10 (t, J = 6,5 Hz, 2H) ; 5,32 (s, 2H) ; 6,39 (t large, J = 2,5 Hz, 1H) ; de 7,29 à 7,46 (m, 6H) ; 7,52 (d large, J = 8,5 Hz, 2H) ; 7,81 (s large, 1H) ; 7,93 (s, 1H) ; 11,0 (m large, 1H) .

### Exemple 71

### 4-(5-Bromo-thiophèn-2-yl)-1-(2-pipéridin-1-yl-éthyl)-1H-pyrazol-3-ol

On opère comme à l'exemple 38 mais avec 0,592 g d' oxalate de 3-benzyloxy-4-(5-bromo-thiophèn-2-yl)-1-(2-pipéridin-1-yl-éthyl)-1H-pyrazole, 3,65 cm³ d'acide chlorhydrique 12 N et 4 cm³ d'éthanol. Le mélange est chauffé pendant 2 heures à une température proche de 100°C. Après refroidissement à une température proche de 20°C, le milieu réactionnel est repris par de l'éthanol, concentré à sec sous pression réduite (2 kPa) ; le résidu est précipité dans de l'oxyde de diisopropyle puis purifié sur cartouche de silice (granulométrie 20-40 µm) en éluant avec un mélange de dichlorométhane et d'une soluiton 2 N de méthanol ammoniacale (90/10 en volumes). On obtient 0,068 g de 4-(5-bromo-thiophèn-2-yl)-1-(2-pipéridin-1-yl-éthyl)-1H-pyrazol-3-ol sous forme d'une poudre jaune. Spectre RMN 1H (300MHz ) - δ en ppm - dans le DMSO-d6 : de 1,33 à 1,53 (m, 6H) ; 2,37 (m, 4H) ; 2,62 (t, J = 6,5 Hz, 2H) ; 3,99 (t, J = 6,5 Hz, 2H) ; 6,93 (d, J = 3,5 Hz, 1H) ; 7,10 (d, J = 3,5 Hz, 1H) ; 7,85 (s, 1H) ; de 10,45 à 10,75 (m très étalé, 1H) . Spectre IR, KBr : 2938; 1593; 1536; 1471; 1173; 981; 798; 758 et 496 cm⁻¹. Spectre masse (IE) : m/z=355 (M^{+.}), m/z=98 (C₆H₁₂N⁺) pic de base.

### Oxalate de 3-benzyloxy-4-(5-bromo-thiophèn-2-yl)-1-(2-pipéridin-1-yl-éthyl)-1H-pyrazole

On opère comme à l'exemple 15 mais avec 0,154 g d'hydrure de sodium (à 75 % en masse dans l'huile de vaseline), 0,477 g de chlorhydrate de 1-(2-chloroéthyl)-pipéridine et 0,62 g de 3-benzyloxy-4-(5-bromo-thiophèn-2-yl)-pyrazole dans 13 cm³ de diméthylformamide. Après agitation pendant 1 heure à une température proche de 20°C, le milieu est repris par 50 cm³ d'acétate d'éthyle et 50 cm³ d'eau ; la phase organique est décantée, lavée par 3 fois 50 cm³ d'une solution aqueuse saturée de chlorure de sodium, séchée sur du sulfate de magnésium, filtrée et concentrée à sec sous pression réduite (3 kPa). Le résidu est repris par 10 cm³ d'acétone et 170 mg d'acide oxalique en solution dans 2 cm³ d'acétone. Le précipité est filtré sur verre fritté, lavé par de l'acétone, séché puis purifié sur cartouche de silice (granulométrie 20-40 µm) en éluant avec un mélange de dichlorométhane et de méthanol (95/5 puis 90/ 10 en volumes). On obtient ainsi 0,716 g d'oxalate de 3-benzyloxy-4-(5-bromo-thiophèn-2-yl)-1-(2-pipéridin-1-yl-éthyl]-1H-pyrazole sous forme d'une poudre jaune. Spectre RMN 1H (300MHz ) - δ en ppm - dans le

DMSO-d6 : 1,48 (m, 2H) ; 1,65 (m, 4H) ; de 2,86 à 3,00 (m étalé, 4H) ; 3,25 (m large, partiellement masqué, 2H) ; 4,30 (t large, J = 6,5 Hz, 2H) ; 5,32 (s, 2H) ; 7,01 (d, J = 3,5 Hz, 1H) ; 7,15 (d, J = 3,5 Hz, 1H) ; de 7,32 à 7,54 (m, 5H) ; 8,09 (s, 1H). Spectre IR, KBr: 2948; 2536; 1724; 1641; 1595; 1532; 1498; 1451; 1363; 1173; 1008; 795 et 702 cm⁻¹. Spectre masse (IE) : m/z=445 (M^{+.}), m/z=98 (C₆H₁₂N⁺) pic de base.

### 3-Benzyloxy-4-(5-bromo-thiophèn-2-yl)-pyrazole

On opère comme à l'exemple 38 mais avec 1,1 g de 1-(toluène-4-sulfonyl)-3-benzyloxy-4-(5-bromo-thiophèn-2-yl)-1H-pyrazole et 5 cm³ d'une solution 1 N de fluorure de tétrabutylammonium dans le tétrahydrofurane et 40 cm³ de tétrahydrofurane. Après purification sur cartouche de silice (granulométrie 20-40 µm) en éluant avec un mélange de dichlorométhane et d'acétone (95/ 5 en volumes), on obtient ainsi 0,624 g de 3-benzyloxy-4-(5-bromo-thiophèn-2-yl)-1H-pyrazole sous forme d'un solide jaune. Spectre IR, KBr : 3193; 1599; 1503; 1438; 1362; 1238; 1023; 795; 731; 694 et 496 cm⁻¹. Spectre masse (IE) : m/z=334 (M^{+.} ), m/z=91 (C₇H₇⁺) pic de base.

### 1-(Toluène-4-sulfonyl)-3-benzyloxy-4-(5-bromo-thiophèn-2-yl)-1H-pyrazole

On opère comme à l'exemple 38 mais avec 1 g de 1-(toluène-4-sulfonyl)-3-benzyloxy-4-iodo-1H-pyrazole, 1,32 g d'acide 5-bromo-thiophèn-2-yl boronique, 1,22 g de carbonate de potassium et 309 mg de dichloro-bis(triphénylphoshine)palladium dans 20 cm³ de toluène et de 5 cm³ d'éthanol. Après purification par sur cartouche de silice (granulométrie 20-40 µm) en éluant avec un mélange d'acétate d'éthyle et de cyclohexane (10/90 en volumes), on obtient ainsi 0,85 g de 1-(toluène-4-sulfonyl)-3-benzyloxy-4-(5-bromo-thiophèn-2-yl)-1H-pyrazole sous forme d'une gomme orange. Spectre IR, CCl₄ : 1597; 1527; 1494; 1391; 1190; 1179; 1096; 1081; 695; 671; 595 et 540 cm⁻¹. Spectre masse (IC) : m/z=489 (MH⁺), m/z=263 (HPPh₃⁺) pic de base.

### Exemple 72

### 2-[1-(2-Pipéridin-1-yl-éthyl)-1H-pyrazol-4-yl] benzamide

0,373 g d'oxalate de 4-(4-cyano-phényl)-1-(2 pipéridin-1-yl-éthyl)-1H-pyrazole et 11 cm³ de soude 0,1 N dans 20 cm³ de dichlorométhane sont agités à une température proche de 20°C pendant 15 minutes. La phase organique est décantée, séchée sur du sulfate de magnésium anhydre, filtrée et concentrée à sec sous pression réduite (3 kPa). Le résidu est repris par 11 cm³ de toluène. On ajoute 0,287 g de triméthylsilanolate de potassium et chauffe le milieu réactionnel au reflux du solvant pendant 6h30. Le mélange est refroidi à une température proche de 20°C, repris par 40 cm³ d'acétate d'éthyle et 40 cm³ d'eau. La phase organique est décantée, lavée par de l'eau, séchée sur du sulfate de magnésium anhydre, filtrée et concentrée à sec sous pression réduite (3 kPa). Le résidu est purifié sur cartouche de silice (granulométrie 20-40 µm) en éluant avec un mélange de dichlorométhane et une solution 2 N de méthanol ammoniacale (95/ 5 en volumes). On obtient 0,081 g de 2-[1-(2-pipéridin-1-yl-éthyl)-1H-pyrazol-4-yl] benzamide sous forme d'un solide blanc fondant à 168°C.

Spectre RMN 1H (300MHz ) - δ en ppm - dans le DMSO-d6 : de 1,33 à 1,55 (m, 6H) ; 2,40 (m, 4H) ; 2,68 (t, J = 6,5 Hz, 2H) ; 4,22 (t, J = 6,5 Hz, 2H) ; de 7,23 à 7,45 (m, 4H) ; 7,50 (d large, J = 8,5 Hz, 1H) ; 7,70 (d, J = 1,0 Hz, 1H) ; 7,75 (m large, 1H) ; 7,98 (d, J = 1,0 Hz, 1H). Spectre IR, KBr: 3380; 3162; 2921; 1646; 1402; 954; 858; 754 et 633 cm⁻¹. Spectre masse (IC) : m/z=299 (MH⁺) pic de base.

### Oxalate de 4-(4-cyano-phényl)-1-(2-pipéridin-1-yl-éthyl)-1H-pyrazole

On opère comme à l'exemple 15 mais avec 0,098 g d'hydrure de sodium (à 75 % en masse dans l'huile de vaseline), 0,305 g de chlorhydrate de 1-(2-chloroéthyl)-pipéridine et 0,2 g de 4-(4-cyano-phényl)-1H-pyrazole. On obtient ainsi 0,373 g d' oxalate de 4-(4-cyano-phényl)-1-(2-pipéridin-1-yl-éthyl)-1H-pyrazole sous forme d'une poudre blanche. Spectre RMN 1H (300MHz ) - δ en ppm - dans le DMSO-d6 :

1,50 (m, 2H) ; 1,68 (m, 4H) ; 2,96 (m, 4H) ; 3,33 (t large, J = 6,5 Hz, 2H) ; 4,55 (t, J = 6,5 Hz, 2H) ; 7,45 (m, 1H) ; 7,73 (m, 2H) ; 7,89 (m, 1H) ; 8,06 (d, J = 1,0 Hz, 1H) ; 8,42 (d, J = 1,0 Hz, 1H). Spectre IR, KBr: 2949; 2223; 1747; 1641; 1600; 1225; 1207; 990; 952; 764; 705 et 504 cm⁻¹Spectre masse (IC) : m/z=281 (MH⁺) pic de base.

### 4-(4-Cyano-phényl)-1H-pyrazole

On opère comme à l'exemple 38 mais avec 0,613 g de 1-(toluène-4-sulfonyl)-4-(4-cyano-phényl)-1H-pyrazole et 3,8 cm³ d'une solution 1 N de fluorure de tétrabutylammonium dans le tétrahydrofurane et 30 cm³ de tétrahydrofurane. Après purification sur cartouche de silice (granulométrie 20-40 µm) en éluant avec un mélange de dichlorométhane et d'acétone (90/ 10 en volumes), on obtient ainsi 0,202 g de 4-(4-cyano-phényl)-1H-pyrazole sous forme d'un solide blanc. Spectre RMN 1H (300MHz ) - δ en ppm - dans le DMSO-d6 : 7,43 (m, 1H) ; de 7,69 à 7,80 (m, 2H) ; 7,88 (m, 1H) ; de 8,00 à 8,30 (m large, 2H) ; 13,3 (m large, 1H). Spectre IR, KBr : 3153; 2966; 2218; 1601; 1516; 1347; 1044; 949; 763; 656 et 501 cm⁻¹. Spectre masse (IE) : m/z=169 (M^{+.}) pic de base, m/z=142 [(M - CHN)⁺], m/z=115 [(m/z=142 - CHN)⁺].

### 1-(Toluène-4-sulfonyl)-4-(4-cyano-phényl)-1H-pyrazole

On opère comme à l'exemple 41 pour la préparation du 3-benzyloxy-4-(5-chloro-thiophèn-2-yl)-1-(toluène-4-sulfonyl)-1H-pyrazole mais à partir de 0,943 g de 1-(toluène-4-sulfonyl)-4-tributylstannanyl-1H-pyrazole, 0,43 g de 2-cyano-1-iodo benzène, 84 mg de tris(dibenzylidèneacétone)palladium et 77 mg de tris(trifuryl)phosphine dans 11 cm³ de dioxane. Après purification sur cartouche de silice (granulométrie 20-40 µm) en éluant avec un mélange de cyclohexane et d'acétate d'éthyle (90/10 puis 80/20 puis 50/50 en volumes), on obtient ainsi 0,613 g del-(toluène-4-sulfonyl)-4-(4-cyano-phényl)-1H-pyrazole sous forme d'un solide pâteux jaune orangé. pectre RMN 1H (400MHz) - 5 en ppm - dans le DMSO-d6 : 2,42 (s, 3H) ; de 7,50 à 7,58 (m, 3H) ; 7,78 (dt, J = 1, 5 et 8,0 Hz, 1H) ; 7,84 (d large, J = 8,0 Hz, 1H) ; de 7,93 à 7,98 (m, 3H) ; 8,38 (d, J = 1,0 Hz, 1H) ; 8,98 (d, J = 1,0 Hz, 1H). Sectre IR, KBr : 2225; 1382; 1192; 1176; 1091; 1051; 812; 761; 702; 679; 664; 593 et 541 cm⁻¹. Spectre masse (IE) : m/z=323 (M^{+.}), m/z=259 [(M - SO₂⁺)], m/z=91 (C₇H₇⁺) pic de base.

### 1-(Toluène-4-sulfonyl)-4-tributylstannanyl-1H-pyrazole

On opère comme à l'exemple 41 pour la préparation du 3-benzyloxy-1-(toluène-4-sulfonyl)-4-tributylstannanyl-1H-pyrazole mais à partir de 1,5 g de 1-(toluène-4-sulfonyl)-4-iodo-1H-pyrazole, 2,65 cm³ de 1,1,1,2,2,2-hexabutyl-distannane, 58 mg de diacétate de palladium et 136 mg de triphénylphosphine dans 20 cm³ de DMF. Après 2 purifications sur cartouche de silice (granulométrie 20-40 µm) en éluant avec du cyclohexane puis un mélange de cyclohexane et d'acétate d'éthyle (95/5 en volumes), on obtient ainsi 0,743 g del-(toluène-4-sulfonyl)-4-tributylstannanyl-1H-pyrazole sous forme d'une huile incolore. Spectre IR, CH₂Cl₂ : 2959; 2925; 2873; 2854; 1378; 1175; 1064; 957; 673; 594 et 543 cm⁻¹. Spectre masse (IE) : m/z=511 (M^{+.}), m/z=455 [(M - C₄H₈)⁺] pic de base, m/z=399 [(m/z=455 - C₄H₈)⁺], m/z=343 [(m/z=399 - C₄H₈)^{+.}], m/z=91 (C₇H₇⁺).

### Exemple 73

### Chlorhydrate de 4-(2-hydroxy-phényl)-1-(2-pipéridin-1-yl-éthyl)-1H-pyrazole

Une solution agitée de 0,582 g d'oxalate de 4-(2-méthoxy-phényl)-1-(2-pipéridin-1-yl-éthyl)-1H-pyrazole dans 12 cm³ de dichlorométhane, sous atmosphère inerte, est refroidie à une température proche de -78°C. On ajoute 4,3 cm³ de tribromure de bore et continue l'agitation 4 heures à une température proche de -70°C puis 15 heures à une température proche de 20°C. Le milieu réactionnel est repris par 10 cm³ d'eau. La phase organique est décantée puis lavée par une solution 1 N de soude jusqu'à pH 8-8,4 (papier Lyphan) et reprise par 20 cm³ d'eau. La phase organique est décantée, séchée sur du sulfate de magnésium anhydre, filtrée et concentrée à sec sous pression réduite (3 kPa). Le résidu est précipité dans de l'oxyde de diisopropyle ; le précipité est purifié sur cartouche de silice (granulométrie 20-40 µm) en éluant avec un mélange de dichlorométhane et de méthanol (90/10 en volumes) ; la gomme obtenue est reprise par une solution 1 N d'oxyde de diéthyle chlorhydrique. On obtient 0,172 g de chlorhydrate de 4-(2-hydroxy-phényl)-1-(2-pipéridin-1-yl-éthyl)-1H-pyrazole sous forme d'une poudre rose. Spectre RMN 1H (300MHz ) - δ en ppm - dans le DMSO-d6 : de 1,30 à 1,82 (m étalé, 6H) ; de 2,38 à 3,62 (m très étalé, 6H) ; de 4,22 à 4,60 (m étalé, 2H) ; 6,82 (dt, J = 1, 5 et 8,0 Hz, 1H) ; 6,92 (d large, J = 8,0 Hz, 1H) ; 7,03 (dt, J = 1,5 et 8,0 Hz, 1H) ; 7,54 (dd, J = 1,5 et 8,0 Hz, 1H) ; 7,98 (s large, 1H) ; 8,23 (s large, 1H) ; de 9,05 à 9,45 (m très étalé, 1H) ; 9,76 (s large, 1H). spectre IR, KBr: 3144; 2938; 2539; 1560; 1461; 1351; 1282; 1238; 1111; 954; 856; 747 et 478 cm⁻¹. Spectre masse (IE) : m/z=271 (M^{+.}), m/z=98 [C₆H₁₂N⁺] pic de base.

### Oxalate de 4-(2-méthoxy-phényl)-1-(2-pipéridin-1-yl-éthyl)-1H-pyrazole

On opère comme à l'exemple 15 mais avec 0,22 mg d'hydrure de sodium (à 75% en masse dans l'huile de vaseline), 0,681 g de chlorhydrate de 1-(2-chloroéthyl)-pipéridine et 0,46 mg de 4-(2-méthoxy-phényl)-1H-pyrazole. On obtient ainsi 0,582 g d' oxalate de 4-(2-méthoxy-phényl)-1-(2-pipéridin-1-yl-éthyl)-1H-pyrazole sous forme d'un solide blanc. Spectre RMN 1H (300MHz ) - δ en ppm - dans le DMSO-d6 pour 50 % du mélange : 1,50 (m, 2H) ; de 1,62 à 1,75 (m étalé, 4H) ; de 2,88 à 3,09 (m, 4H) ; 3,15 (t, J = 6,5 Hz, 2H) ; 3,89 (s, 3H) ; 4,52 (t, J = 6,5 Hz, 2H) ; 6,99 (t large, J = 8,0 Hz, 1H) ; 7,10 (d large, J = 8,0 Hz, 1H) ; 7,23 (dt, J = 1,5 et 8,0 Hz, 1H) ; 7,63 (dd, J = 1,5 et 8,0 Hz, 1H) ; 8,01 (s large, 1H) ; 8,23 (s large, 1H) . Spectre IR, KBr : 2948; 2537; 1719; 1635; 1493; 1246; 1184; 1028; 952; 756; 721; 704 et 497 cm⁻¹. Spectre masse (IC) : m/z=281 (MH⁺) pic de base, m/z= 148 (M'H⁺).

### 4-(2-Méthoxy-phényl)-1H-pyrazole

On opère comme à l'exemple 38 mais avec 1,08 g de 1-(toluène-4-sulfonyl)-4-(2-méthoxy-phényl)-1H-pyrazole et 7,3 cm³ d'une solution 1 N de fluorure de tétrabutylammonium dans le tétrahydrofurane et 58 cm³ de tétrahydrofurane. Après purification sur cartouche de silice (granulométrie 20-40 µm) en éluant avec un mélange de dichlorométhane et du méthanol (90/ 10 en volumes), on obtient ainsi 0,463 g de 4-(2-méthoxy-phényl)-1H-pyrazole sous forme d'un solide blanc cassé. Spectre RMN 1H (300MHz ) - δ en ppm) - dans le DMSO-d6 : 3,89 (s, 3H) ; 6,97 (dt, J = 1,5 et 8,5 Hz, 1H) ; 7,06 (d large, J = 8,5 Hz, 1H) ; 7,21 (m, 1H) ; 7,63 (dd, J = 1,5 et 8,5 Hz, 1H) ; de 7,85 à 8,20 (m très étalé, 2H) ; 12,9 (m large, 1H). Spectre IR, KBr: 3156; 2936; 2832; 1569; 1488; 1466; 1263; 1247; 1148; 1027; 950; 753; 661 et 628 cm⁻¹. Spectre masse (IE) : m/z=174 (M^{+.}) pic de base, m/z=159 [(M - CH₃)⁺], m/z=131 [(m/z=159 - CO)⁺].

### 1-(Toluène-4-sulfonyl)-4-(2-méthoxy-phényl)-1H-pyrazole

On opère comme à l'exemple 38 mais avec 1,5 g de 1-(toluène-4-sulfonyl)-4-iodo-1H-pyrazole, 1,31 g d'acide 2-méthoxy-phényl boronique, 1,74 g de carbonate de potassium et 0,605 g de dichloro-bis(triphénylphoshine)palladium dans 30 cm³ de toluène et de 7,5 cm³ d'éthanol. Après purification sur cartouche de silice (granulométrie 20-40 µm) en éluant avec un mélange d'acétate d'éthyle et de cyclohexane (20/80 en volumes), on obtient ainsi 1,081 g de 1-(toluène-4-sulfonyl)-4-(2-méthoxy-phényl)-1H-pyrazole sous forme d'une gomme orange. Spectre RMN 1H (300MHz ) - δ en ppm - dans le DMSO-d6 : 2,41 (s, 3H) ; 3,92 (s, 3H) ; 7,01 (dt, J = 1,5 et 8,5 Hz, 1H) ; 7,13 (d large, J = 8,5 Hz, 1H) ; 7,33 (m, 1H) ; 7,50 (d large, J = 8,5 Hz, 2H) ; 7,73 (dd, J = 1,5 et 8,5 Hz, 1H) ; 7,93 (d large, J = 8,5 Hz, 2H) ; 8,41 (s large, 1H) ; 8,72 (s large, 1H) . Spectre IR, KBr : 2835; 1497; 1372; 1177; 1097; 1039; 1023; 950; 753; 681; 598 et 550 cm⁻¹. Spectre masse (IE) : m/z=328 (M^{+.}) pic de base, m/z=264 [(M - SO₂⁺], m/z=173 [(M - C₇H₇SO₂)⁺], m/z=91 (C₇H₇⁺).

### Exemple 74

### 4-(1H-Indol-5-yl)-1-(2 pipéridin-1-yl-éthyl)-1H-pyrazole

On opère comme à l'exemple 38 mais avec 1,22 g de 4-iodo-1-(2-pipéridin-1-yl-éthyl)-1H-pyrazole, 1,93 g d'acide 1H-indol-5-yl boronique, 2,547 g de phosphate de tripotassium, 0,421 g de dichloro-bis(triphénylphoshine)palladium dans 50 cm³ de diméthoxyéthane. Après purification par chromatographie, sous une pression d'azote de 50 kPa, sur une colonne de gel de silice (granulométrie 20-45 µ ; diamètre 3 cm; hauteur 60 cm), en éluant avec un mélange d'acétate d'éthyle et de méthanol (95/5 puis 90/10 en volumes), les fractions 18 à 30 sont réunies, concentrées à sec sous pression réduite (3 kPa). On obtient ainsi 0,23 g de 4-(1H-indol-5-yl)-1-(2-pipéridin-1-yl-éthyl)-1H-pyrazole sous forme d'une poudre blanchâtre. Spectre RMN 1H (300MHz ) - δ en ppm - dans le DMSO-d6 : de 1,34 à 1,56 (m, 6H) ; 2,41 (m, 4H) ; 2,72 (t, J = 6,5 Hz, 2H) ; 4,22 (t, J = 6,5 Hz, 2H) ; 6,41 (m, 1H) ; de 7,27 à 7,40 (m, 3H) ; 7,71 (m,1H) ; 7,80 (d, J =1,0 Hz, 1H) ; 8,06 (s large, 1H) ; 11,0 (m large, 1H) .

spectre IR, KBr : 2937; 1436; 1363; 1167; 1119; 994; 887; 792; 763; 614 et 430 cm⁻¹

spectre masse (IE) : m/z=294 (M^{+.}), m/z=98 (C₆H₁₂N⁺) pic de base.

### 4-Iodo-1-(2-pipéridin-1-yl-éthyl)-1H-pyrazole

On opère comme à l'exemple 15 mais avec 4,94 g d'hydrure de sodium (à 75% en masse dans l'huile de vaseline), 19 g de chlorhydrate de 1-(2-chloroéthyl)-pipéridine et 10 g de 4-iodo-pyrazole. Après agitation 16 heures à une température proche de 20°C, le milieu réactionnel est repris par 1000 cm³ d'acétate d'éthyle et 1000 cm³ d'eau ; la phase organique est décantée, lavée par 3 fois 1000 cm³ d'eau et 500 cm³ d'une solution aqueuse saturée de chlorure de sodium, séchée sur du sulfate de magnésium, filtrée et concentrée à sec sous pression réduite (3 kPa). Le résidu est purifié par chromatographie, sous une pression d'azote de 50 kPa, sur une colonne de gel de silice (granulométrie 20-45 µ ; diamètre 6 cm; hauteur 60 cm), en éluant avec un mélange d'acétate d'éthyle et de cyclohexane (30/70 en volumes) puis de l'acétate d'éthyle. Les fractions 16 à 20 sont réunies, concentrées à sec sous pression réduite (3 kPa). On obtient ainsi 8,2 g de 4-iodo-1-(2-pipéridin-1-yl-éthyl)-1H-pyrazole sous forme d'une huile jaune claire. Spectre RMN 1H (300MHz ) - δ en ppm - dans le DMSO-d6 : de 1,34 à 1,52 (m, 6H) ; 2,36 (m, 4H) ; 2,64 (t, J = 6,5 Hz, 2H) ; 4,22 (t, J = 6,5 Hz, 2H) ; 7,51 (s large, 1H) ; 7,92 (s large, 1H). Spectre masse (IC) : m/z=306 (MH⁺) pic de base.

### Exemple 75

### Chlorydrate de 4-(4-méthyl-phényl)-1-(2-pipéridin-1-yl-éthyl)-1H-pyrazol-3-ol

On opère comme à l'exemple 38 mais avec 0,63 g d' oxalate de 3-benzyloxy-4-(4-méthyl-phényl)-1-(2-pipéridin-1-yl-éthyl]-1H-pyrazole, 4,8 cm³ d'acide chlorhydrique 12 N et 4,8 cm³ d'éthanol. Le mélange est chauffé pendant 4 heures à une température proche de 100°C. Après refroidissement à une température proche de 20°C, le milieu réactionnel est repris par de l'éthanol, concentré à sec sous pression réduite (2 kPa) ; le résidu est précipité dans de l'oxyde de diisopropyle. On obtient 0,385 g de chlorydrate de 4-(4-méthyl-phényl)-1-(2-pipéridin-1-yl-éthyl)-1H-pyrazol-3-ol sous forme d'un solide blanc. Spectre RMN 1H (300MHz) - δ en ppm - dans le DMSO-d6 : 1,40 (m, 1H) ; de 1,63 à 1,87 (m, 5H) ; 2,30 (s, 3H) ; de 2,82 à 3,02 (m étalé, 2H) ; de 3,27 à 3,53 (m, 4H) ; 4,38 (t large, J = 6,5 Hz, 2H) ; 7,15 (d large, J = 8,5 Hz, 2H) ; 7,54 (d large, J = 8,5 Hz, 2H) ; 7,98 (s, 1H) ; 10,05 (m très étalé, 1H) ; 10,4 (m étalé, 1H). Spectre IR, KBr: 2941; 2646; 1597; 1534; 1447; 1179; 1010; 818; 627 et 515 cm⁻¹. Spectre masse (IE) : m/z=285 (M^{+.}), m/z=98 (C₆H₁₂N⁺) pic de base.

### Oxalate de 3-benzyloxy-4-(4-méthyl-phényl)-1-(2-pipéridin-1-yl-éthyl]-1H-pyrazole

On opère comme à l'exemple 15 mais avec 0,123 g d'hydrure de sodium (à 75% en masse dans l'huile de vaseline), 0,38 g de chlorhydrate de 1-(2-chloroéthyl)-pipéridine et 0,39 g de 3-benzyloxy-4-(4-méthyl-phényl)-pyrazole. On obtient ainsi 0,632 g d' oxalate de 3-benzyloxy-4-(4-méthyl-phényl)-1-(2-pipéridin-1-yl-éthyl]-1H-pyrazole sous forme d'une poudre blanche. Spectre IR, KBr : 2931; 2639; 2543; 1719; 1618; 1580; 1519; 1452; 1279; 1180; 818; 721 et 500 cm⁻¹. Spectre masse (IC) : m/z=376 (MH⁺) pic de base.

### 3-Benzyloxy-4-(4-méthyl-phényl)-1H-pyrazole

On opère comme à l'exemple 38 mais avec 0,8 g de 1-(toluène-4-sulfonyl)-3-benzyloxy-4-(4-méthyl-phényl)-1H-pyrazole et 4 cm³ d'une solution 1 N de fluorure de tétrabutylammonium dans le tétrahydrofurane et 40 cm³ de tétrahydrofurane. On obtient ainsi 0,397 g de 3-benzyloxy-4-(4-méthyl-phényl)-1H-pyrazole sous forme d'une poudre blanche. Spectre IR, KBr : 3187; 2980; 1586; 1498; 1450; 1380; 1233; 1043; 814; 737; 695 et 514 cm⁻¹. Spectre masse (IE) : m/z=264 (M^{+.}), m/z=186 [(M - C₆H₆)⁺], m/z=91 (C₇H₇⁺) pic de base.

### 3-Benzyloxy-1-(toluène-4-sulfonyl)-4-(4-méthyl-phényl)-1H-pyrazole

On opère comme à l'exemple 38 mais avec 1 g de 1-(toluène-4-sulfonyl)-3-benzyloxy-4-iodo-1H-pyrazole, 0,898 g d'acide 4-méthyl-phényl boronique, 0,913 g de carbonate de potassium et 0,331 g de tétrakis(triphénylphosphine)palladium dans 13 cm³ de toluène, 3 cm³ d'éthanol et 3,3 cm³ d'eau. On obtient ainsi 0,817 g de 3-benzyloxy-1-(toluène-4-sulfonyl)-4-(4-méthyl-phényl)-1H-pyrazole sous forme d'un solide cotonneux beige rosé. Spectre IR, KBr. 1589; 1485; 1377; 1191; 1179; 1098; 813; 702; 672; 580 et 538 cm⁻¹. Spectre masse (IE) : m/z=418 (M^{+.}), m/z=263 [(M - C₇H₇SO₂)⁺], m/z=91 (C₇H₇⁺) pic de base.

### Exemple 76

### 1-(1-Aza-bicyclo[2.2.2]oct-3-yl)-4-(1H-indol-5-yl)-1H-pyrazole

On opère comme à l'exemple 38 mais avec 1 g de 1-(1-aza-bicyclo[2.2.2]oct-3-yl)-4-iodo-1H-pyrazole, 0,797 g d'acide 1H-indol-5-yl boronique, 1,026 g de carbonate de potassium, 0,463 g de dichloro-bis(triphénylphoshine)palladium dans 30 cm³ de toluène, 6 cm³ d'éthanol et 3 cm³ d'eau. Après purification par chromatographie, sous une pression d'azote de 50 kPa, sur une colonne d'alumine CBT1 en éluant avec de l'acétate d'éthyle puis un mélange d'acétate d'éthyle et de méthanol (95/5 puis 90/10 puis 80/20 en volumes), les fractions 97 à 110 sont réunies, concentrées à sec sous pression réduite (3 kPa). Le résidu est précipité dans un mélange de 5 cm³ d'acétate d'éthyle et 25 cm³ d'oxyde de diisopropyle. On obtient ainsi 0,18 g de 1-(1-aza-bicyclo[2.2.2]oct-3-yl)-4-(1H-indol-5-yl)-1H-pyrazole sous forme d'une poudre jaune. Spectre RMN 1H (300MHz) - δ en ppm - dans le DMSO-d6 1,34 (m, 1H) ; de 1,53 à 1,75 (m, 3H) ; 2,13 (m, 1H) ; de 2,67 à 2,81 (m, 3H) ; 3,02 (m, 1H) ;3,26 (m partiellement masqué, 1H) ; 3,49 (m, 1H) ; 4,44 (m, 1H) ; 6,40 (m, 1H) ; de 7,29 à 7,41 (m, 3H) ; 7,75 (s large, 1H) ; 7,87 (s large, 1H) ; 8,22 (s large, 1H) ; 11,0 (m large, 1H) . Spectre IR, KBr : 3113; 2939; 1587; 1454; 1362; 1165; 1058; 976; 881; 792; 729; 619 et 435 cm⁻¹. Spectre masse (ES) : m/z=293 (MH⁺) pic de base.

Les énantiomères sont séparés par HPLC sur chiralpak AD 20 µm avec comme éluant un mélange d'heptane, d'éthanol et de butylamine (40 / 60 / 0,2 en volumes). On obtient 2 énantiomères A et B qui sont purifiés selon le protocole suivant : l'énantiomère A est purifié par extraction avec de l'acétate d'éthyle puis solubilisé dans 100 ml d'eau. Le pH de la solution est ajusté à 10 avec de la soude 0,1 N. La phase organique est extraite avec 100 ml d'acétate d'éthyle. La phase aqueuse est décantée avec 2 x 50ml de dichlorométhane. La phase organique est séchée avec du sulfate de sodium anhydre, filtrée et concentrée à sec sous pression réduite. La phase organique est contrôlée par HPLC en polarité de phase inversée sur colonne Thermo hypersil Hypurity C18 250*4.6*5µm ; éluant : gradient 95/5 : tampon acétate / acétonitrile pendant 50 minutes. On obtient 42,6 mg de (-)-1-(1-aza-bicyclo[2.2.2]oct-3-yl)-4-(1H-indol-5-yl)-1H pyrazole, énantiomère A ([α]²⁰_{D} = -37,3° (solvant : diméthylsulfoxyde, concentration : 0,3)).

L'énantiomère B est purifié par extraction avec de l'acétate d'éthyle puis solubilisé dans 100 ml d'eau. Le pH de la solution est ajusté à 10 avec de la soude 0,1 N. La phase organique est extraite avec 100 ml d'acétate d'éthyle. La phase aqueuse est décantée avec 2 x 50ml de dichlorométhane. La phase organique est séchée avec du sulfate de sodium anhydre, filtrée et concentrée à sec sous pression réduite. La phase organique est contrôlée par HPLC en polarité de phase inversée sur colonne Thermo hypersil Hypurity C18 250*4.6*5µm ; éluant: gradient 95 / 5 : tampon acétate / acétonitrile pendant 50 minutes. On obtient 56,3 mg de (+)-1-(1-aza-bicyclo[2.2.2]oct-3-yl)-4-(1H-indol-5-yl)-1H-pyrazole, énantiomère B ( [α]²⁰_{D} = +36,2° (solvant : diméthylsulfoxyde, concentration : 0,42)).

### 1-(1-aza-bicyclo[2.2.2]oct-3-yl)-4-iodo-1H-pyrazole

On opère comme à l'exemple 5 mais avec 0,48 g d'hydrure de sodium (à 75 % en masse dans l'huile de vaseline), 3,079 g d'ester 1-aza-bicyclo[2.2.2]oct-3-yl d'acide toluène-4-sulfonique et 1,94 g de 4-iodo-pyrazole dans 30 cm³ de diméthylformamide. Le résidu est purifié par chromatographie, sous une pression d'azote de 50 kPa, sur une colonne d'alumine CBT1, en éluant avec de l'acétate d'éthyle puis un mélange d'acétate d'éthyle et de méthanol (95/5 puis 90/10 en volumes). Les fractions 29 à 39 sont réunies, concentrées à sec sous pression réduite (3 kPa). L'huile obtenue est à nouveau purifiée sur une colonne d'alumine CBT1, en éluant avec de l'acétate d'éthyle. Les fractions 11 à 15 sont réunies, concentrées à sec sous pression réduite (3 kPa). On obtient ainsi 0,33 g de 1-(1-aza-bicyclo[2.2.2]oct-3-yl)-4-iodo-1H-pyrazole sous forme d'une huile qui cristallise. Spectre RMN 1H (400MHz) - δ en ppm - dans le DMSO-d6 : 1,30 (m, 1H) ; 1,46 (m, 1H) ; 1,66 (m, 2H) ; 2,05 (m, 1H) ; de 2,65 à 2,78 (m, 4H) ; 2,91 (m, 1H) ; 3,21 (m partiellement masqué, 1H) ; 4,44 (m, 1H) ; 7,58 (s large, 1H) ; 8,06 (s large, 1H) . Spectre masse (IE) : m/z=303 (M^{+.}), m/z=220 [(M - C₅H₉N)⁺], m/z=109 (C₇H₁₁N⁺), m/z=97 (C₆H₁₁N⁺) pic de base.

### Exemple 77

### Chlorhydrate de 1-(1-aza-bicyclo[2.2.2]oct-3-yl)-4-(5-chloro-thiophèn 2-yl)-1H-pyrazol-3-ol, isomère A

On opère comme à l'exemple 38 mais avec 0,13 g de 1-(1-aza-bicyclo[2.2.2]oct-3-yl)-3-benzyloxy-4-(5-chloro-thiophèn-2-yl)-1H-pyrazole, isomère A, 10 cm³ d'acide chlorhydrique 12 N et 15 cm³ d'éthanol. Le mélange est chauffé pendant 22 heures à une température proche de 100°C puis refroidi à une température proche de 20°C ; le milieu réactionnel est concentrée à sec sous pression réduite (3 kPa) puis repris par 2 fois 20 cm³ d'éthanol et concentré à sec sous pression réduite (2 kPa) ; le résidu est précipité dans 20 cm³ d'oxyde de diisopropyle. On obtient 80 mg de chlorhydrate de 1-(1-aza-bicyclo[2.2.2]oct-3-yl)-4-(5-chloro-thiophèn-2-yl)-1H-pyrazol-3-ol, isomère A sous forme d'une poudre grisâtre. Spectre RMN 1H (300MHz ) - δ en ppm - dans le DMSO-d6 : de 1,62 à 1,98 (m, 4H) ; 2,36 (m, 1H) ; de 3,07 à 3,55 (m partiellement masqué, 4H) ; 3,71 (m, 2H) ; 4,62 (m, 1H) ; 7,03 (m, 2H) ; 8,10 (s large, 1H) ; 10,75 (m large, 1H) . Spectre IR, KBr: 1602; 1536; 1459; 1164; 1005; 795 et 502 cm⁻¹

Spectre de masse (ES) : m/z=310 (MH⁺) pic de base. [α]_{D} = -14° (solvant : MeOH, concentration : 0,1266).

### Exemple 78

### Chlorhydrate de 1-(1-aza-bicyclo[2.2.2]oct-3-yl)-4-(5-chloro-thiophèn-2-yl)-1H-pyrazol-3-ol, isomère B

On opère comme à l'exemple 38 mais avec 0,13 g de 1-(1-aza-bicyclo[2.2.2]oct-3-yl)-3-benzyloxy-4-(5-chloro-thiophèn-2-yl)-1H-pyrazole, isomère B, 5 cm³ d'acide chlorhydrique 12 N et 10 cm³ d'éthanol. Le mélange est chauffé pendant 16 heures à une température proche de 100°C puis refroidi à une température proche de 20°C ; le milieu réactionnel est concentré à sec sous pression réduite (3 kPa) puis repris par 2 fois 20 cm³ de toluène et concentré à sec sous pression réduite (2 kPa) ; le résidu est repris par 3 fois 20 cm³ d'éthanol et concentré à sec sous pression réduite (2 kPa). Le résidu ainsi obtenu est précipité dans 20 cm³ d'oxyde de diisopropyle. On obtient 100 mg de chlorhydrate de 1-(1-aza-bicyclo[2.2.2]oct-3-yl)-4-(5-chloro-thiophèn-2-yl)-1H-pyrazol-3-ol, isomère B sous forme d'une poudre grisâtre. Spectre RMN 1H (300MHz ) - δ en ppm - dans le DMSO-d6 : de 1,62 à 2,00 (m, 4H) ; 2,38 (m, 1H) ; de 3,05 à 3,53 (m, 4H) ; 3,73 (m, 2H) ; 4,63 (m, 1H) ; 7,03 (m, 2H) ; 8,11 (s large, 1H) ; 10,75 (m large, 1H). Spectre IR, KBr: 1601; 1536; 1457; 1163; 1004; 794 et 502 cm⁻¹. Spectre masse (IC) : m/z=310 (MH⁺) pic de base. [α]_{D} = -18° (solvant : MeOH, concentration : 0,2168).

### 1-(1-Aza-bicyclo[2.2.2]oct-3-yl)-3-benzyloxy-4-(5-chloro-thiophèn-2-yl)-1H-pyrazole, isomères A et B

On opère comme à l'exemple 5 mais avec 0,495 g d'hydrure de sodium (à 75 % en masse dans l'huile de vaseline), 3,178 g d'ester 1-aza-bicyclo[2.2.2]oct-3-yl d'acide toluène-4-sulfonique et 1,94 g de 3-benzyloxy-4-(5-chloro-thiophèn-2-yl)-pyrazole dans 70 cm³ de diméthylformamide. Le résidu est purifié par chromatographie, sous une pression d'azote de 50 kPa, sur une colonne de gel de silice (granulométrie 20-45 µ ; diamètre 3 cm; hauteur 50 cm), en éluant avec du dichlorométhane puis un mélange de dichlorométhane et de méthanol (95/5 puis 90/10 puis 80/20 en volumes). Les fractions 52 à 74 sont réunies, concentrées à sec sous pression réduite (3 kPa). L'huile obtenue est à nouveau purifiée sur une colonne d'alumine CBT1, en éluant avec de l'acétate d'éthyle puis un mélange d'acétate d'éthyle et de méthanol (90/10 en volumes). Les fractions 22, 32 et 40 à 50 sont réunies, concentrées à sec sous pression réduite (3 kPa). On obtient ainsi 0,5 g de 1-(1-aza-bicyclo[2.2.2]oct-3-yl)-3-benzyloxy-4-(5-chloro-thiophén-2-yl)-1H-pyrazole sous forme d'une huile verte qui cristallise pour laquelle les 2 énantiomères sont séparés par HPLC. Spectre masse (IE) : m/z=399 (M^{+.}), m/z=308 [(M - C₇H₇)⁺], m/z=110 (C₇H₁₂N⁺), m/z=91 (C₇H₇⁺) pic de base.

A partir de 0,45 g de 1-(1-aza-bicyclo[2.2.2]oct-3-yl)-3-benzyloxy-4-(5-chloro-thiophèn-2-yl)-1H-pyrazole, les énantiomères sont séparés par HPLC sur chiralpak AD 20 µm en éluant avec un mélange de 70 % heptane / 15% éthanol / 15% méthanol / 0,1% triéthylamine. On obtient 138 mg de 1-(1-aza-bicyclo[2.2.2]oct-3-yl)-3-benzyloxy-4-(5-chloro-thiophèn-2-yl)-1H-pyrazole, isomère A ([α]_{D} = +28,2° (solvant : MeOH, concentration: 0,5)) et 129 mg de 1-(1-aza-bicyclo[2.2.2]oct-3-yl)-3-benzyloxy-4-(5-chloro-thiophèn-2-yl)-1H-pyrazole, isomère B ([α]_{D} = -24,6° (solvant : MeOH, concentration : 0,5)). Spectre masse (IE) : m/z=399 (M⁺), m/z=308 [(M - C₇H₇)⁺], m/z=110 (C₇H₁₂N⁺), m/z=91 (C₇H₇⁺) pic de base.

### Exemple 79

### Chlorhydrate de 1-(1-aza-bicyclo[2.2.2]oct-2-ylméthyl)-4-phényl-1H-pyrazol-3-ol

Une solution de 0,8 g de 2-(3-benzyloxy-4-phényl-pyrazol-1-ylméthyl)-1-aza-bicyclo[2.2.2]octane dans 20 cm³ d'éthanol est additionnée de 1,8 cm³ d'acide chlorhydrique 6 N, agitée 30 minutes à température ambiante puis concentrée à sec sous pression réduite (3 kPa). La suspension du résidu obtenu et de 114 mg de palladium sur charbon à 10% dans 20 cm³ d'éthanol est agitée en autoclave sous une pression d'hydrogène de 100 kPa, à une température de 20°C pendant 8 heures. Le milieu réactionnel est ensuite filtré sur Célite^{®} et concentré à sec sous pression réduite (3 kPa), pour donner un résidu pâteux, qui est recouvert par 40 cm³ d'acétone et trituré jusqu'à cristallisation complète. Après filtration du solide apparu et séchage sous vide (70 Pa) à une température de 60°C, on obtient 0,6 g de chlorhydrate de 1-(1-aza-bicydo[2.2.2]oct-2-ylméthyl)-4-phényl-1H-pyrazol-3-ol, sous forme de cristaux beiges fondant à une température supérieure. à 260°C. Spectre RMN 1H (300MHz) - δ en ppm - dans le DMSO-d6 (référencé à 2,50 ppm) :1,55 (m, 1H) ; de 1,71 à 1,95 (m, 5H) ; 2,08 (m, 1H) ; de 3,12 à 3,35 (m partiellement masqué, 3H) ; 3,50 (m, 1H) ; 3,88 (m, 1H) ; 4,27 (dd, J = 7,5 et 14,0 Hz, 1H) ; 4,42 (dd, J = 7,5 et 14,0 Hz, 1H) ; 7,12 (t large, J = 7,5 Hz, 1H) ; 7,32 (t large, J = 7,5 Hz, 2H) ; 7,63 (d large, J = 7,5 Hz, 2H) ; 8,01 (s, 1H) ; 9,80 (m étalé, 1H) ; 10,45 (m large, 1H). Spectre de masse (IE) : m/z=283 (M^{+.}), m/z=201 [(M- C₅H₈N)^{+.}], m/z=173 [(M - C₇H₁₂N)^{+.}], m/z=124 (C₈H₁₄N⁺) pic de base, m/z=82 (C₅H₈N⁺), m/z=36 (HCl^{+.}).

Le 2-(3-benzyloxy-4-phényl-pyrazol-1-ylméthyl)-1-aza-bicylo[2.2.2]octane peut être obtenu de la manière suivante :

A une solution de 0,95 g de 3-benzyloxy-4-phényl-pyrazole dans 20 cm³ de diméthylformamide anhydre on ajoute progressivement, sous atmosphère d'argon et à température ambiante, 182 mg d'hydrure de sodium (à 75 % en masse dans l'huile de vaseline). Après 0,75 heure d'agitation à une température voisine de 50°C, on ajoute progressivement la solution de 1,25 g de méthanesulfonate de 1-aza-bicyclo[2.2.2]oct-2-ylméthyle dans 20 cm³ de diméthylformamide anhydre, puis chauffe pendant 24 heures à une température voisine de 110°C. Le mélange est refroidi à température ambiante, puis est additionné lentement de 10 cm³ d'eau et enfin concentré au rotavapor. Le résidu obtenu est additionné de 25 cm³ d'eau et extrait par 250 cm³ d'acétate d'éthyle. La phase organique est lavée par 3 fois 25 cm³ d'eau, puis filtrée sur filtre séparateur de phase (Whatman^{®}, référence : 2200 185) et concentrée à sec sous pression réduite (3kPa). Le résidu huileux obtenu est purifié par chromatographie sur alumine, en éluant par du dichlorométhane. Après concentration des fractions sous pression réduite, on obtient 0,8 g de 2-(3-benzyloxy-4-phényl-pyrazol-1-ylméthyl)-1-aza-bicydo[2.2.2]octane sous forme d'une huile qui concrétise lentement en solide amorphe. Spectre de masse (IE) : m/z=373 (M^{+.}), m/z=282 [(M - C₇H₇)⁺], m/z=124 (C₈H₁₄N⁺), m/z=91 (C₇H₇⁺) pic de base, m/z=82 (C₅H₈N⁺).

Le méthanesulfonate de 1-aza-bicyclo[2.2.2]oct-2-ylméthyle peut être obtenu de la manière suivante :

A la solution de 1 g de (1-aza-bicyclo[2.2.2]oct-2-yl)-méthanol dans 40 cm³ de dichlorométhane on ajoute, à une température voisine de 0°C et sous atmosphère d'argon, 0,69 cm³ de pyridine puis goutte à goutte 0,66 cm³ de chlorure de méthanesulfonyle. La suspension est agitée 20 minutes vers 0°C puis 18 heures à température ambiante. Le mélange est ensuite additionné de 15 cm³ d'une solution saturée de carbonate de potassium et extrait par 3 fois 50 cm³ d'acétate d'éthyle. Les phases organiques réunies sont séchées sur sulfate de magnésium anhydre, filtrées et concentrées à sec sous pression réduite (3kPa). Le résidu obtenu est purifié par chromatographie sur alumine, en éluant par de l'acétate d'éthyle. Après concentration des fractions sous pression réduite, on obtient 1,1 g de méthanesulfonate de 1-aza-bicyclo[2.2.2]oct-2-ylméthyle sous forme d'une huile incolore. Spectre de masse (IE) : m/z=219 (M^{+.}), m/z=140 [(M - SO₂CH₃)⁺], m/z=124 (C₈H₁₄N⁺) pic de base.

Le (1-aza-bicyclo[2.2.2]oct-2-yl)-méthanol peut être obtenu selon la méthode décrite dans le brevet DE 1938546.

### Exemple 80

### Chlorhydrate de 3-[4-(3,5-difluoro-phényl)-pyrazol-1-yl]-1-aza-bicyclo[2.2.2]octane :

A une solution de 0,65 g de 4-(3,5-difluoro-phényl)-1H-pyrazole dans 30 cm³ de diméthylformamide anhydre on ajoute progressivement, sous atmosphère d'argon et à température ambiante, 0,173 g d'hydrure de sodium (à 75 % en masse dans l'huile de vaseline). Après 0,75 heure d'agitation à une température voisine de 50°C, on ajoute goutte à goutte la solution de 1,17 g de 3-[(méthanesulfonyl)oxy]-1-aza-bicyclo[2.2.2]octane dans 10 cm³ de diméthylformamide anhydre, puis chauffe pendant 20 heures à une température voisine de 110°C. Le mélange est refroidi à température ambiante, puis est additionné lentement de 5 cm³ d'eau et concentré sous pression réduite (3 kPa). Le résidu est repris par 20 cm³ d'eau et extrait avec 250 cm³ d'acétate d'éthyle. La phase organique est lavée avec 4 fois 20 cm³ d'eau, puis séchée, filtrée et concentrée à sec sous pression réduite (3 kPa). Le résidu obtenu est purifié par chromatographie sur alumine en éluant avec un mélange de dichlorométhane et d'acétate d'éthyle (70 / 30 en volumes). Après concentration des fractions sous pression réduite on obtient une huile, qui est dissoute dans 35 cm³ d'acétone et additionnée de 20 cm³ d'éther chlorhydrique 1 M. Après 1 heure d'agitation à température ambiante le solide apparu est isolé par filtration et séché sous vide (70kPa) à une température de 40°C. On obtient ainsi 0,44 g de chlorhydrate de 3-[4-(3,5-difluoro-phényl)-pyrazol-1-yl]-1-aza-bicyclo[2.2.2]octane sous forme de cristaux blancs hygroscopiques. Spectre RMN 1H (300MHz) - δ en ppm - dans le DMSO-d6 (référencé à 2,50 ppm) : 1,71 (m, 2H) ; 2,00 (m, 2H) ; 2,42 (m, 1H) ; de 3,20 à 3,48 (m, 4H) ; de 3,75 à 3,95 (m, 2H) ; 4,90 (m, 1H) ; 7,05 (tt, J = 2,5 et 9,5 Hz, 1H) ; 7,40 (m, 2H) ; 8,17 (s large, 1H) ; 8,60 (s large, 1H) ; 10,6 (m étalé, 1H). Spectre de masse (IE) : m/z=289 (M^{+.}) pic de base, m/z =206 [(M - C₅H₉N)^{+.}], m/z= 109 (C₇H₁₁N^{+.}), m/z=36 (HCl^{+.}).

Le 4-(3,5-difluoro-phényl)-1H-pyrazole peut être obtenu de la manière suivante :

A une solution de 2,1 g de 4-(3,5-difluoro-phényl)-1-(toluène-4-sulfonyl)-1H-pyrazole dans 70 cm³ de tétrahydrofurane on ajoute, à température ambiante, 15,7 cm³ d'une solution de fluorure de tétrabutylammonium dans le tétrahydrofurane 1 M. Le mélange est chauffé au reflux pendant 4,5 heures, puis refroidi à température ambiante et concentré à sec sous pression réduite (3kPa). Le résidu est additionné de 50 cm³ d'eau et extrait par 200 cm³ d'acétate d'éthyle. La phase organique est lavée par 50 cm³ d'eau puis par 25 cm³ de saumure et enfin séchée sur sulfate de magnésium anhydre, filtrée et concentrée à sec sous pression réduite (3 kPa). Le résidu est trituré dans 40 cm³ de dichlorométhane, puis isolé par filtration sous vide. On obtient ainsi 0,65 g de 4-(3,5-difluoro-phényl)-1H-pyrazole sous forme de cristaux blancs fondant vers 185°C. Spectre de masse (IE) : m/z=180 (M^{+.}) pic de base, m/z= 153 [(M - HCN)⁺], m/z= 126 [(m/z= 153 - HCN)⁺,].

Le 4-(3,5-difluoro-phényl)-1-(toluène-4-sulfonyl)-1H-pyrazole peut être préparé de la manière suivante :

A la solution de 3,5 g de 4-iodo-1-(toluène-4-sulfonyl)-1H-pyrazole dans 100 cm³ de 1,2-diméthoxyéthane on ajoute, sous atmosphère d'argon et à température ambiante, 6,31 g d'acide 3,5-difluoro-phényl-boronique. Le milieu réactionnel est chauffé à 110°C et on ajoute alors 8,5 g de phosphate de potassium tribasique finement broyé et 0,91 g de chlorure de bis(triphénylphosphine)palladium, puis maintient le chauffage au reflux pendant 3,5 heures. Le mélange est refroidi à température ambiante, filtré sur Celite^{®}, que l'on lave ensuite avec 500 cm³ d'acétate d'éthyle. La phase organique est lavée par 5 fois 100 cm³ d'eau puis par 2 fois 100 cm³ de saumure, séchée sur sulfate de magnésium anhydre, filtrée et concentrée à sec sous pression réduite (3 kPa). Le résidu est purifié par chromatographie sur silice en éluant avec du cyclohexane puis du dichlorométhane. Après concentration des fractions sous pression réduite on obtient 2,2 g de 4-(3,5-ditluoro-phényl)-1-(toluène-4-sulfonyl)-1H-pyrazole sous forme d'une poudre blanche. Spectre de masse (IE) : m/z=334 (M^{+.}), m/z=270 [(M - SO₂)^{+.}], m/z=155 (C₇H₇SO₂⁺), m/z=91 (C₇H₇⁺) pic de base.

### Exemple 81

### Chlorhydrate de 4-benzo[b]thiophèn-2-yl-1-(2-pipéridin-1-yl-éthyl)-1H-pyrazol-3-ol

Une solution sous agitation de 930 mg de 1-[2-(4-benzo[b]thiophèn-2-yl-3-benzyloxy-pyrazol-1-yl)-éthyl]-pipéridine dans 15 cm³ d'éthanol est additionnée de 8 cm³ d'acide chlorhydrique 12 N. Après 7 h au reflux du solvant, puis 15 h à une température voisine de 20°C, le milieu réactionnel est évaporé à sec sous pression réduite (2,7 kPa). Le résidu est, à trois reprises, successivement dissous dans de l'éthanol et évaporé à sec sous pression réduite (2,7 kPa), puis il est trituré dans 20 cm³ d'éther diisopropylique. Le précipité formé est filtré et séché sous vide (2,7 kPa) pour donner 790 mg de chlorhydrate de 4 benzo[b]thiophèn-2-yl-1-(2-pipéridin-1-yl-éthyl)-1H-pyrazol-3-ol sous forme d'un solide jaune pâle. Spectre de masse (CI): 328(+)=(M+H)(+); présence 36(+)/38(+)=HCl(+). Spectre RMN 1H (300MHz) - δ en ppm - dans le DMSO-d6 : 1,42 (m, 1H) ; de 1,65 à 1,89 (m, 511) ; 2,95 (m, 2H) ; de 3,38 à 3,54 (m, 4H) ; 4,44 (t, J = 6,5 Hz, 2H) ; de 7,23 à 7,38 (m, 2H) ; 7,50 (s, 1H) ; 7,78 (d large, J = 7,5 Hz, 1H) ; 7,90 (d large, J = 7,5 Hz, 1H) ; 8,07 (s, 1H) ; de 10,05 à 10,2 (m très étalé, 1H) ; 10,9 (m large, 1H).

La 1-[2-(4-benzo[b]thiophèn-2-yl-3-benzyloxy-pyrazol-1-yl)-éthyl]-pipéridine peut être préparée de la manière suivante :

A une suspension de 225 mg d'hydrure de sodium (à 75 % dans l'huile de vaseline) dans 30 cm³ de diméthylformamide sous atmosphère d'argon et sous agitation, sont ajoutés par portion 860 mg de 4-benzo[b]thiophèn-2-yl-3-benzyloxy-1H-pyrazole. Après 30 min de chauffage à 50°C, le mélange est agité 1 h à une température voisine de 20°C, puis il est additionné par portion de 725 mg de chlorhydrate de 1-(2-chloro-éthyl)-pipéridine. Le milieu réactionnel est agité 15 h à une température voisine de 20°C, puis il est jeté dans 100 cm³ d'eau. La phase aqueuse est extraite deux fois par de l'acétate d'éthyle. Les phases organiques sont réunies, lavées successivement par deux fois de l'eau et une solution aqueuse saturée de chlorure de sodium, séchées sur du sulfate de magnésium, filtrées et évaporées sous pression réduite (2,7 kPa) pour donner une huile jaune qui est purifiée par chromatographie-flash sur alumine CTB1 sous pression d'argon (50 kPa) [éluant : cyclohexane / acétate d'éthyle (95 / 5 en volumes)]. Après concentration des fractions sous pression réduite, on obtient 930 mg de l-[2-(4-benzo[b]thiophèn-2-yl-3-benzyloxy-pyrazol-1-yl)-éthyl]-pipéridine sous forme d'une huile jaune pâle. Spectre RMN 1H (300MHz) - δ en ppm - dans le DMSO-d6 : de 1,34 à 1,54 (m, 6H) ; 2,40 (m, 4H) ; 2,69 (t, J = 6,5 Hz, 2H) ; 4,11 (t, J = 6,5 Hz, 2H) ; 5,37 (s, 2H) ; de 7,23 à 7,48 (m, 6H) ; 7,56 (d large, J = 8,5 Hz, 2H) ; 7,73 (d large, J = 7,5 Hz, 1H) ; 7,90 (d large, J = 7,5 Hz, 1H) ; 8,10 (s, 1H) .

Le 4-benzo[b]thiophèn-2-yl-3-benzyloxy-1H-pyrazole peut être préparé de la manière suivante :

A une solution sous atmosphère d'argon et sous agitation de 1,4 g de 4-benzo[b]thiophèn-2-yl-3-benzyloxy-1-(toluène-4-sulfonyl)-1H-pyrazole dans 30 cm³ de tétrahydrofurane, on ajoute 7,6 cm³ d'une solution 1 N de fluorure de tétrabutyl-ammonium dans le tétrahydrofurane. Après 15 h de chauffage au reflux du solvant, le milieu réactionnel est évaporé sous pression réduite (2,7 kPa) et le résidu est additionné d'acétate d'éthyle. La phase organique est lavée successivement par deux fois de l'eau et une solution aqueuse saturée de chlorure de sodium ; elle est séchée sur sulfate de magnésium et évaporée sous pression réduite (2,7 kPa). L'huile jaune pâle obtenue (0,98 g) est purifiée par chromatographie-flash sur silice sous pression d'argon (50 kPa) [éluant : cyclohexane / acétate d'éthyle (70 / 30 en volumes)]. Après concentration des fractions sous pression réduite (2,7 kPa), on obtient 860 mg de 4-benzo[b]thiophèn-2-yl-3-benzyloxy-1H-pyrazole sous la forme d'un solide blanc. Spectre IR (KBr) : 3173; 2950; 1586; 1530; 1501; 1445; 1363; 1304; 1215; 1166; 1022; 818; 745; 736; 727; 693 et 564 cm⁻¹.

Le 4-benzo[b]thiophèn-2-yl-3-benzyloxy-1-(toluène-4-sulfonyl)-1H-pyrazole peut être préparé de la manière suivante :

A une solution sous atmosphère d'argon et sous agitation de 2 g de 3-benzyloxy-4-iodo-1-(toluène-4-sulfonyl)-1H-pyrazole dans 40 cm³ de toluène additionnés de 10 cm³ d'éthanol, on ajoute 2,4 g d'acide 2-benzothiènyl boronique, 6,6 cm³ d'une solution aqueuse de carbonate de potassium 2 N et 660 mg de tétrakis(triphénylphosphine)palladium. Après 5 h de chauffage au reflux du solvant et 16 h à une température voisine de 20°C, le milieu réactionnel est évaporé sous pression réduite (2,7 kPa). Le résidu est additionné d'acétate d'éthyle, d'eau et de noir de charbon, et il est filtré sur supercel. Le filtrat est décanté, puis la phase organique est lavée successivement par deux fois de l'eau et une solution aqueuse saturée de chlorure de sodium ; elle est séchée sur sulfate de magnésium et évaporée sous pression réduite (2,7 kPa). L'huile orangée obtenue (3,8 g) est purifiée par chromatographie-flash sur silice sous pression d'argon (50 kPa) [éluant : cyclohexane / acétate d'éthyle (90 / 10 en volumes)]. Après concentration des fractions sous pression réduite (2,7 kPa), le solide crème résultant est trituré dans de l'éther diisopropylique. On obtient, après filtration, 1,4 g de 4-benzo[b]thiophèn-2-yl-3-benzyloxy-1-(toluène-4-sulfonyl)-1H-pyrazole sous forme d'un solide blanc. Spectre de masse (EI) : 460(+)=M(+) ; 305(+)=M(+)-Ts.

### Exemple 82

### Chlorhydrate de 1-(2-pipéridin-1-yl-éthyl)-4-thiophèn-3-yl-1H-pyrazol-3-ol

Une solution sous agitation de 640 mg de 1-[2-(3 benzyloxy-4-thiophèn 3-yl-pyrazol-1-yl)-éthyl]-pipéridine dans 10 cm³ d'éthanol est additionnée de 6 cm³ d'acide chlorhydrique 12 N. Après 7 h au reflux du solvant, puis 15 h à une température voisine de 20°C, le milieu réactionnel est évaporé à sec sous pression réduite (2,7 kPa). Le résidu est, à trois reprises, successivement dissous dans de l'éthanol et évaporé à sec sous pression réduite (2,7 kPa), puis il est trituré dans de l'éther diisopropylique. Le précipité formé est filtré et séché sous vide (2,7 kPa) pour donner 470 mg de chlorhydrate de 1-(2-pipéridin-1-yl-éthyl)-4-thiophén-3-yl-1H-pyrawl-3-ol sous forme d'un solide blanc. Spectre de masse (CI) : 278(+)=(M+ED(+) ; présence 36(+)/38(+)=HCl(+). Spectre RMN 1H (300MHz) - δ en ppm - dans le DMSO-d6 : 1,40 (m, 1H) ; de 1,64 à 1,88 (m, 5H) ; 2,93 (m, 2H) ; de 3,35 à 3,52 (m, 4H) ; 4,39 (t, J = 6,5 Hz, 2H) ; 7,35 (dd, J = 1,0 et 5,0 Hz, 1H) ; 7,49 (dd, J = 1,0 et 3,0 Hz, 1H) ; 7,55 (dd, J = 3,0 et 5,0 Hz, 1H) ; 7,94 (s, 1H) ; de 9,85 à 10,05 (m très étalé, 1H) ; 10,4 (m large, 1H).

La 1-[2-(3-benzyloxy-4-thiophèn-3-yl-pyrazol-1-yl)-éthyl]-pipéridine peut être préparée de la manière suivante :

A une suspension de 200 mg d'hydrure de sodium (à 75 % dans l'huile de vaseline) dans 30 cm³ de diméthylformamide sous atmosphère d'argon et sous agitation, sont ajoutés par portion 650 mg de 3-benzyloxy-4-thiophèn-3-yl-1H-pyrazole. Après 30 min de chauffage à 50°C, le mélange est agité 30 min à une température voisine de 20°C, puis il est additionné par portion de 654 mg de chlorhydrate de 1-(2-chloro-éthyl)-pipéridine. Le milieu réactionnel est agité 15 h à une température voisine de 20°C, puis il est jeté dans 100 cm³ d'eau. La phase aqueuse est extraite deux fois par de l'acétate d'éthyle. Les phases organiques sont réunies, lavées successivement par deux fois de l'eau et une solution aqueuse saturée de chlorure de sodium, séchées sur du sulfate de magnésium, filtrées et évaporées sous pression réduite (2,7 kPa) pour donner une huile orangée qui est purifiée par chromatographie-flash sur alumine CTB1 sous pression d'argon (50 kPa) [éluant : cyclohexane / acétate d'éthyle (95 / 5 en volumes)]. Après concentration des fractions sous pression réduite, on obtient 640 mg de 1-[2-(3-benzyloxy-4-thiophèn-3-yl-pyrazol-1-yl)-éthyl]-pipéridine sous forme d'une huile jaune. Spectre RMN 1H (300MHz) - δ en ppm - dans le DMSO-d6 : de 1,32 à 1,54 (m, 6H) ; 2,38 (m, 4H) ; 2,67 (t, J = 6,5 Hz, 2H) ; 4,06 (t, J = 6,5 Hz, 2H) ; 5,30 (s, 2H) ; de 7,30 à 7,55 (m, 8H) ; 7,98 (s, 1H) .

Le 3-benzyloxy-4-thiophèn-3-yl-1H-pyrazole peut être préparé de la manière suivante :

A une solution sous atmosphère d'argon et sous agitation de 1,35 g de 3-benzyloxy-4-thiophèn 3-yl-1-(toluène-4-sulfonyl)-1H-pyrazole dans 30 cm³ de tétrahydrofurane, on ajoute 8,5 cm³ d'une solution 1 N de fluorure de tétrabutyl-ammonium dans le tétrahydrofurane. Après 15 h de chauffage au reflux du solvant, le milieu réactionnel est évaporé sous pression réduite (2,7 kPa) et le résidu est additionné d'acétate d'éthyle. La phase organique est lavée successivement par trois fois de l'eau et une solution aqueuse saturée de chlorure de sodium ; elle est séchée sur sulfate de magnésium et évaporée sous pression réduite (2,7 kPa). Le résidu obtenu (0,96 g) est purifié par chromatographie-flash sur silice sous pression d'argon (50 kPa) [éluant : cyclohexane / acétate d'éthyle (70 / 30 en volumes)]. Après concentration des fractions sous pression réduite (2,7 kPa), on obtient 650 mg de 3-benzyloxy-4-thiophèn 3-yl-1H-pyrazole sous la forme d'un solide crème. Spectre de masse (EI) : 256(+)=M(+) ; 91(+)=C7H7(+).

Le 3-benzyloxy-4-thiophèn-3-yl-1-(toluène-4-sulfonyl)-1H-pyrazole peut être préparé de la manière suivante :

A une solution sous atmosphère d'argon et sous agitation de 2 g de 3-benzyloxy-4-iodo-1-(toluène-4-sulfonyl)-1H-pyrazole dans 40 cm³ de toluène additionnés de 10 cm³ d'éthanol, on ajoute 1,7 g d'acide 3-thiènyl boronique, 6,6 cm³ d'une solution aqueuse de carbonate de potassium 2 N et 660 mg de tétrakis(triphénylphosphine)palladium. Après 5 h de chauffage au reflux du solvant et 16 h à une température voisine de 20°C, le milieu réactionnel est évaporé sous pression réduite (2,7 kPa). Le résidu est additionné d'acétate d'éthyle, d'eau et de noir de charbon, et il est filtré sur supercel. Le filtrat est décanté, puis la phase organique est lavée successivement par deux fois de l'eau et une solution aqueuse saturée de chlorure de sodium ; elle est séchée sur sulfate de magnésium et évaporée sous pression réduite (2,7 kPa). Le solide résultant est trituré dans de l'éther diisopropylique, filtré et cristallisé dans 10 cm³ d'éthanol. On obtient ainsi 1,35 g de 3-benzyloxy-4-thiophèn-3-yl-1-(toluène-4-sulfonyl)-1H-pyrazole sous forme d'un solide beige. Spectre de masse (EI) : 410(+)=M(+) ; 255(+)=M(+)-Ts.

### Exemple 83

### Chlorhydrate de 4-[3-hydroxy-1-(2-pipéridin-1-yl-éthyl)-1H-pyrazol-4-yl]-benzamide

Une solution de 442 mg de 4-[3-benzyloxy-1-(2-pipéridin-1-yl-éthyl)-1H-pyrazol-4-yl]-benzamide dans 20 cm³ d'éthanol est additionnée de 4 cm³ d'éther diéthylique chlorhydrique 3 N. Après 1 h d'agitation à une température voisine de 20°C, la solution est évaporée à sec sous pression réduite (2,7 kPa). Le résidu est repris par 25 cm³ d'éthanol. La solution obtenue est introduite dans un autoclave, et elle est additionnée de 52 mg de palladium sur charbon à 10 %, puis elle est placée sous hydrogène (10 bars). Après 8 h d'agitation à 22°C, le milieu réactionnel est filtré sur supercel, le filtrat est évaporé et le résidu est trituré dans de l'éther diisopropylique. Le solide résultant est filtré, séché sous vide (2,7 kPa) pour donner 118 mg de chlorhydrate de 4-[3-hydroxy-1-(2-pipéridin-1-yl-éthyl)-1H-pyrazol-4-yl]-benzamide sous la forme d'un solide crème. Spectre de masse (CI) : 315(+)=(M+H)(+). Spectre RMN 1H (300MHz) - δ en ppm - dans le DMSO-d6 : 1,41 (m, 1H) ; de 1,64 à 1,89 (m, 5H) ; 2,94 (m, 2H) ; de 3,25 à 3,53 (m, 4H) ; 4,41 (t, J = 6,5 Hz, 2H) ; 7,23 (m étalé, 1H) ; 7,73 (d large, J = 8,5 Hz, 2H) ; de 7,83 à 7,91 (m, 3H) ; 8,14 (s, 1H) ; de 9,80 à 9,95 (m très étalé, 1H) ; 10,65 (s large, 1H).

Le 4-[3-benzyloxy-1-(2-pipéridin-1-yl-éthyl)-1H-pyrazol-4-yl]-benzamide peut être préparé de la manière suivante :

A une suspension de 144 mg d'hydrure de sodium (à 75% dans l'huile de vaseline) dans 30 cm³ de diméthylformamide sous atmosphère d'argon et sous agitation, sont ajoutés par portion 527 mg de 4-(3-benzyloxy-1H-pyrazol-4-yl)-benzamide. Après 30 min de chauffage à 50°C, le mélange est agité 30 min à une température voisine de 20°C, puis il est additionné par portion de 465 mg de chlorhydrate de 1-(2-chloro-éthyl)-pipéridine. Le milieu réactionnel est agité 15 h à une température voisine de 20°C, puis il est jeté dans 100 cm³ d'eau. La phase aqueuse est extraite deux fois par de l'acétate d'éthyle. Les phases organiques sont réunies, lavées successivement par deux fois de l'eau et une solution aqueuse saturée de chlorure de sodium, séchées sur du sulfate de magnésium, filtrées et évaporées sous pression réduite (2,7 kPa). Le solide résultant est trituré dans de l'éther diisopropylique, filtré et cristallisé dans de l'acétone. On obtient ainsi 445 mg de 4-[3-benzyloxy-1-(2-pipéridin-1-yl-éthyl)-1H-pyrazol-4-yl]-benzamide sous forme d'un solide blanc. Spectre RMN 1H (300MHz) - δ en ppm - dans le DMSO-d6 : de 1,32 à 1,54 (m, 6H) ; 2,39 (m, 4H) ; 2,69 (t, J = 6,5 Hz, 2H) ; 4,09 (t, J = 6,5 Hz, 2H) ; 5,33 (s, 2H) ; 7,24 (m étalé, 1H) ; de 7,32 à 7,46 (m, 3H) ; 7,52 (d large, J = 8,5 Hz, 2H) ; 7,70 (d large, J = 7,5 Hz, 2H) ; de 7,81 à 7,88 (m, 3H) ; 8,17 (s, 1H) .

Le 4-(3-benzyloxy-1H-pyrazol-4-yl)-benzamide peut être préparé de la manière suivante :

A une solution sous atmosphère d'argon et sous agitation de 1,1 g de 4-[3-benzyloxy-1-(toluène-4-sulfonyl)-1H-pyrazol-4-yl]-benzamide dans 30 cm³ de tétrahydrofurane, on ajoute 6,5 cm³ d'une solution 1 N de fluorure de tétrabutyl-ammonium dans le tétrahydrofurane. Après 15 h de chauffage au reflux du solvant, le milieu réactionnel est refroidi dans un bain de glace. Après filtration puis séchage des cristaux résultants sous vide (2,7 kPa), on obtient 527 mg de 4-(3-benzyloxy-1H-pyrazol-4-yl)-benzamide sous la forme d'un solide blanc. Spectre IR (KBr) : 3403; 3176; 1667; 1611; 1576; 1553; 1517; 1485; 1393; 1379; 1226; 1040; 1027 et 739 cm⁻¹.

Le 4-[3-benzyloxy-1-(toluène-4-sulfonyl)-1H-pyrazol-4-yl]-benzamide peut être préparé de la manière suivante :

A une solution sous atmosphère d'argon et sous agitation de 2,5 g de 3-benzyloxy-4-iodo-1-(toluène-4-sulfonyl)-1H-pyrazole dans 40 cm³ de toluène additionnés de 10 cm³ d'éthanol, on ajoute 2,72 g d'acide 4-aminocarbonylphényl boronique, 8,3 cm³ d'une solution aqueuse de carbonate de potassium 2 N et 830 mg de tétrakis(triphénylphosphine)palladium. Après 15 h de chauffage au reflux du solvant, le milieu réactionnel est refroidi à une température voisine de 20°C et évaporé sous pression réduite (2,7 kPa). Le résidu est additionné d'acétate d'éthyle, d'eau et de noir de charbon, et il est filtré sur supercel. Le filtrat est décanté, puis la phase organique est lavée successivement par deux fois de l'eau et une solution aqueuse saturée de chlorure de sodium ; elle est séchée sur sulfate de magnésium et évaporée sous pression réduite (2,7 kPa). L'huile jaune obtenue (3,7 g) est purifiée par chromatographie-flash sur silice sous pression d'argon (50 kPa) [éluant : cyclohexane / acétate d'éthyle (50 / 50 puis 20 / 80 en volumes)]. Après concentration des fractions sous pression réduite (2,7 kPa), on obtient 1,1 g de 4-[3-benzyloxy-1-(toluéne-4-sulfonyl)-1H-pyrazol-4-yl]-benzamide sous forme d'un solide blanc. Spectre de masse (EI) : 447(+)=M(+) ; 91(+)=C7H7(+).

### Exemple 84

### Chlorhydrate de 3-[3-hydroxy-1-(2-pipéridin-1-yl-éthyl)-1H-pyrazol-4-yl]-benzamide

Une solution de 690 mg de 3-[3-benzyloxy-1-(2-pipéridin-1-yl-éthyl)-1H-pyrazol-4-yl]-benzamide dans 20 cm³ d'éthanol est additionnée de 4 cm³ d'éther diéthylique chlorhydrique 3 N. Après 45 min d'agitation à une température voisine de 20°C, la solution est évaporée à sec sous pression réduite (2,7 kPa). Le résidu est repris par 25 cm³ d'éthanol. La solution obtenue est introduite dans un autoclave, et elle est additionnée de 80 mg de palladium sur charbon à 10 %, puis elle est placée sous hydrogène (10 bars). Après 8 h d'agitation à 22°C, le milieu réactionnel est filtré sur supercel, le filtrat est évaporé et le résidu est trituré dans de l'éther diisopropylique. Le solide résultant est filtré, séché sous vide (2,7 kPa) pour donner 528 mg de chlorhydrate de 3-[3-hydroxy-1-(2-pipéridin-1-yl-éthyl)-1H-pyrazol-4-yl]-benzamide sous la forme d'un solide jaune pâle- Spectre de masse (CI) : 315(+)=(M+H)(+). Spectre RMN 1H (300MHz ) - δ en ppm - dans le DMSO-d6 : 1,40 (m, 1H) ; de 1,65 à 1,88 (m, 5H) ; 2,94 (m, 2H) ; de 3,41 à 3,54 (m, 4H) ; 4,42 (t, J = 6,5 Hz, 2H) ; 7,33 (m étalé, 1H) ; 7,42 (t, J = 8,0 Hz, 1H) ; 7,64 (d large, J = 8,0 Hz, 1H) ; 7,82 (d large, J = 8, 0 Hz, 1H) ; 7,94 (m étalé, 1H) ; 8,10 (s, 1H) ; 8,14 (s large, 1H) ; de 9,90 à 10,05 (m très étalé, 1H) ; 10,55 (s large, 1H).

Le 3-[3-benzyloxy-1-(2-pipéridin-1-yl-éthyl)-1H-pyrazol-4-yl]-benzamide peut être préparé de la manière suivante :

A une suspension de 315 mg d'hydrure de sodium (à 75 % dans l'huile de vaseline) dans 30 cm³ de diméthylformamide sous atmosphère d'argon et sous agitation, sont ajoutés par portion 1,16 g de 3-(3-benzyloxy-1H-pyrazol-4-yl)-benzamide. Après 30 min de chauffage à 50°C, le mélange est agité 30 min à une température voisine de 20°C, puis il est additionné par portion de 1 g de chlorhydrate de 1-(2-chloro-éthyl)-pipéridine. Le milieu réactionnel est agité 15 h à une température voisine de 20°C, puis il est jeté dans 100 cm³ d'eau. La phase aqueuse est extraite deux fois par de l'acétate d'éthyle. Les phases organiques sont réunies, lavées successivement par deux fois de l'eau et une solution aqueuse saturée de chlorure de sodium, séchées sur du sulfate de magnésium, filtrées et évaporées sous pression réduite (2,7 kPa) pour donner une huile jaune (1,6 g) qui est purifiée par chromatographie-flash sur silice sous pression d'argon (50 kPa) [éluant : dichlorométhane puis dichlorométhane / méthanol (98 / 2, 95 / 5 puis 90 / 10 en volumes)]. Après concentration des fractions sous pression réduite, on obtient 700 mg de 3-[3-benzyloxy-1-(2-pipéridin-1-yl-éthyl)-1H-pyrazol-4-yl]-benzamide sous forme d'une huile jaune pâle. Spectre RMN 1H (300MHz) - δ en ppm - dans le DMSO-d6 : de 1,33 à 1,54 (m, 6H) ; 2,40 (m, 4H) ; 2,70 (t, J = 6,5 Hz, 2H) ; 4,10 (t, J = 6,5 Hz, 2H) ; 5,33 (s, 2H) ; de 7,30 à 7,44 (m, 5H) ; 7,53 (d large, J = 8,5 Hz, 2H) ; 7,63 (d large, J = 8,0 Hz, 1H) ; 7,80 (d large, J = 8,0 Hz, 1H) ; 7,92 (m étalé, 1H) ; 8,11 (s, 1H) ; 8,15 (s large, 1H).

Le 3-(3-benzyloxy-1H-pyrazol-4-yl)-benzamide peut être préparé de la manière suivante :

A une solution sous atmosphère d'argon et sous agitation de 2,1 g de 3-[3-benzyloxy-1-(toluène-4-sulfonyl)-1H-pyrazol-4-yl]-benzamide dans 40 cm³ de tétrahydrofurane, on ajoute 11,8 cm³ d'une solution 1 N de fluorure de tétrabutyl-ammonium dans le tétrahydrofurane. Après 15 h de chauffage au reflux du solvant, le milieu réactionnel est évaporé sous pression réduite (2,7 kPa) et le résidu est additionné d'acétate d'éthyle. La phase organique est lavée successivement par deux fois de l'eau et une solution aqueuse saturée de chlorure de sodium ; elle est séchée sur sulfate de magnésium et évaporée sous pression réduite (2,7 kPa). Le résidu est trituré dans de l'éther diisopropylique, filtré et séché sous vide (2,7 kPa) pour donner 1,16 g de 3-(3-benzyloxy-1H-pyrazol-4-yl)-benzamide sous forme d'un solide crème. Spectre IR (KBr) : 3332; 3196; 2975; 1682; 1606; 1581; 1572; 1505; 1447; 1389; 1280; 1232; 1049; 732; 695; 672 et 637 cm⁻¹.

Le 3-[3-benzyloxy-1-(toluène-4-sulfonyl)-1H-pyrazol-4-yl]-benzamide peut être préparé de la manière suivante :

A une solution sous atmosphère d'argon et sous agitation de 2,5 g de 3-benzyloxy-4-iodo-1-(toluène-4-sulfonyl)-1H-pyrazole dans 40 cm³ de toluène additionnés de 10 cm³ d'éthanol, on ajoute 2,72 g d'acide 3-aminocarbonylphényl boronique, 9,3 cm³ d'une solution aqueuse de carbonate de potassium 2 N et 830 mg de tétrakis(triphénylphosphine)palladium. Après 15 h de chauffage au reflux du solvant, le milieu réactionnel est refroidi dans un bain de glace. Après filtration puis séchage des cristaux résultants sous vide (2,7 kPa), on obtient 2,1 g de 3-[3-benzyloxy-1-(toluène-4-sulfonyl)-1H-pyrazol-4-yl]-benzamide sous la forme d'un solide blanc. Spectre de masse (EI) : 447(+)=M(+) ; 91(+)=C7H7(+).

### Exemple 85

### Chlorhydrate de (-)-1-(1-aza-bicyclo[2.2.2]oct-3-yl)-4-phényl-1H-pyrazol-3-ol

Une solution sous agitation de 1,44 g de (+)-3-(3-benzyloxy-4-phényl-pyrazol-1-yl)-1-aza-bicyclo[2.2.2]octane dans 20 cm³ d'éthanol est additionnée de 15 cm³ d'acide chlorhydrique 12 N. Après 7 h au reflux du solvant, puis 15 h à une température voisine de 20°C, le milieu réactionnel est évaporé à sec sous pression réduite (2,7 kPa). Le résidu est, à trois reprises, successivement dissous dans de l'éthanol et évaporé à sec sous pression réduite (2,7 kPa), puis il est trituré dans 20 cm³ d'éther diisopropylique. Le précipité formé est filtré et séché sous vide (2,7 kPa) pour donner 1,2 g de chlorhydrate de (-)-1-(1-aza-bicyclo[2.2.2]oct-3-yl)-4-phényl-1H-pyrazol-3-ol sous forme d'un solide blanc. Spectre de masse (EI) 269(+)=M(+); présence 36(+)/38(+)=HCl(+). Spectre RMN 1H (300MHz) - δ en ppm - dans le DMSO-d6 : de 1,69 à 2,04 (m, 4H) ; 2,43 (m, 1H) ; de 3,22 à 3,47 (m, 4H) ; de 3,72 à 3,85 (m, 2H) ; 4,67 (m, 1H) ; 7,15 (tt, J = 1,5 et 7,5 Hz, 1H) ; 7,34 (t large, J = 7,5 Hz, 2H) ; 7,67 (d large, J = 7,5 Hz, 2H) ; 8,18 (s, 1H) ; 10,2 (m étalé, 1H) ; 10,4 (m étalé, 1H). [α]²⁰_{D} = -17.4 +/- 0.6° (c 0.5, MeOH).

Le (+)-3-(3-benzyloxy-4-phényl-pyrazol-1-yl)-l-aza-bicyclo[2.2.2]octane et le (-)-3-(3-benzyloxy-4-phényl-pyrazol-1-yl)-1-aza-bicyclo[2.2.2]octane peuvent être préparés de la manière suivante : Une solution éthanolique contenant 1,5 g de 3-(3-benzyloxy-4-phényl-pyrazol-1-yl)-1-aza-bicyclo[2.2.2]octane est introduite sur une colonne de 6 cm de diamètre contenant 800 g de phase stationnaire chirale Chiralpak AD™. L'élution est réalisée à l'aide d'un mélange de 10% éthanol, 10% de méthanol, 0.1% de triéthylamine et de 80% d'heptane. Le débit de la phase mobile étant de 90 ml/min. L'énantiomère dextrogyre est élué en première position, la solution est évaporée à sec sous pression réduite (2,7 kPa) pour donner 0,66 g d'huile.

Spectre RMN 1H (300MHz) - δ en ppm - dans le DMSO-d6 :1,30 (m, 1H) ; de 1,50 à 1,71 (m, 3H) ; 2,08 (m, 1H) ; de 2,65 à 2,79 (m, 3H) ; 2,99 (m, 1H) ; de 3,15 à 3,42 (m, 2H) ; 4,27 (m, 1H) ; 5,33 (s, 2H) ; 7,15 (tt, J = 1,5 et 7,5 Hz, 1H) ; de 7,30 à 7,44 (m, 5H) ; 7,51 (d large, J = 7,5 Hz, 2H) ; 7,69 (d large, J = 7,5 Hz, 2H) ; 8,20 (s, 1H). [α]²⁰_{D} = +27.0 +/- 0.8° (c 0.5, MeOH). La solution contenant l'énantiomère lévogyre élué en deuxième position est évaporée à sec sous pression réduite (2,7 kPa) pour donner 0,65 g d'huile.

Spectre RMN 1H (300MHz) - δ en ppm - dans le DMSO-d6 : 1,31 (m, 1H) ; de 1,50 à 1,72 (m, 3H) ; 2,09 (m, 1H) ; de 2,66 à 2,80 (m, 3H) ; 2,99 (m, 1H) ; de 3,16 à 3,43 (m, 2H) ; 4,29 (m, 1H) ; 5,34 (s, 2H) ; 7,14 (tt, J = 1,5 et 7,5 Hz, 1H) ; de 7,30 à 7,44 (m, 5H) ; 7,51 (d large, J = 7,5 Hz, 2H) ; 7,70 (d large, J = 7,5 Hz, 2H) ; 8,20 (s, 1H). [α]²⁰_{D} = -27.0 +/- 0.8° (c 0.5, MeOH).

### Exemple 86

### Chlorhydrate de (+)-1-(1-aza-bicyclo[2.2.2]oct-3-yl)-4-phényl-1H-pyrazol-3-ol

Une solution sous agitation de 1,44 g de (-)-3-(3-benzyloxy-4-phényl-pyrazol-1-yl)-1-aza-bicyclo[2.2.2]octane dans 20 cm³ d'éthanol est additionnée de 15 cm³ d'acide chlorhydrique 12 N. Après 7 h au reflux du solvant, puis 15 h à une température voisine de 20°C, le milieu réactionnel est évaporé à sec sous pression réduite (2,7 kPa). Le résidu est, à trois reprises, successivement dissous dans de l'éthanol et évaporé à sec sous pression réduite (2,7 kPa), puis il est trituré dans 20 cm³ d'éther diisopropylique. Le précipité formé est filtré et séché sous vide (2,7 kPa) pour donner 1,2 g de chlorhydrate de (+)-1-(1-aza-bicyclo[2.2.2]oct-3-yl)-4-phényl-1H-pyrazol-3-ol sous forme d'un solide beige. Spectre de masse (EI) : 269(+)=M(+) ;presence 36(+)/38(+)=HCl(+). Spectre RMN 1H (300MHz) - δ en ppm - dans le DMSO-d6 : de 1,70 à 2,03 (m, 4H) ; 2,43 (m, 1H) ; de 3,22 à 3,47 (m, 4H) ; de 3,72 à 3,86 (m, 2H) ; 4,68 (m, 1H) ; 7,15 (tt, J = 1,5 et 7,5 Hz, 1H) ; 7,34 (t large, J = 7,5 Hz, 2H) ; 7,67 (d large, J = 7,5 Hz, 2H) ; 8,19 (s, 1H) ; 10,2 (m étalé, 1H) ; de 10,3 à 10,5 (m très étalé, 1H). [α]²⁰_{D} = +13.7 +/- 0.6° (c 0.5, MeOH).

### Exemple 87

### Chlorhydrate de (-)-1-(1-aza-bicyclo[2.2.2]oct-3-ylméthyl)-4-phényl-1H-pyrazol-1-ol

Une solution sous agitation de 825 mg de (-)-3-(3-benzyloxy-4-phényl-pyrazol-1-ylméthyl)-1-aza-bicyclo[2.2.2]octane dans 10 cm³ d'éthanol est additionnée de 10 cm³ d'acide chlorhydrique 12 N. Après 7 h au reflux du solvant, puis 15 h à une température voisine de 20°C, le milieu réactionnel est évaporé à sec sous pression réduite (2,7 kPa). Le résidu est, à trois reprises, successivement dissous dans de l'éthanol et évaporé à sec sous pression réduite (2,7 kPa), puis il est trituré dans de l'éther diisopropylique. Le précipité formé est filtré et séché sous vide (2,7 kPa) pour donner 700 mg de chlorhydrate de (-)-1-(1-aza-bicyclo[2.2.2]oct-3-ylméthyl)-4-phényl-1H-pyrazol-1-ol sous forme d'un solide blanc. Spectre de masse (EI) : 283(+)=M(+). Spectre RMN 1H (300MHz) - δ en ppm - dans le DMSO-d6 : de 1,66 à 1,93 (m, 4H) ; 2,05 (m, 1H) ; 2,52 (m masqué, 1H) ; 2,94 (m, 1H) ; de 3,12 à 3,40 (m, 5H) ; de 3,98 à 4,12 (m, 2H) ; 7,13 (tt, J = 1,5 et 7,5 Hz, 1H) ; 7,33 (t large, J = 7,5 Hz, 2H) ; 7,64 (d large, J = 7,5 Hz, 2H) ; 7,99 (s' 1H) ; 10,1 (m étalé, 1H). [α]²⁰_{D} = -36.8 +/- 0.8° (c 0.5, MeOH).

Le (-)-3-(3-benzyloxy-4-phényl-pyrazol-1-ylméthyl)-1-aza-bicyclo[2.2.2]octane et le (+)-3-(3-benzyloxy-4-phényl-pyrazol-1-ylméthyl)-1-aza-bicyclo[2.2.2]octane peuvent être préparés de la manière suivante :

A une suspension de 1,9 g d'hydrure de sodium (à 75% en masse dans l'huile de vaseline) dans 50 cm³ de diméthylformamide on ajoute progressivement, sous atmosphère d'argon et à une température voisine de 20°C, une solution de 5 g de 3-benzyloxy-4-phényl-pyrazole dans 30 cm³ de diméthylformamide. Après 1 h d'agitation à une température voisine de 50°C on ajoute par petites portions 5,8 g de chlorhydrate de 3-chlorométhyl-1-aza-bicyclo[2.2.2]octane, puis chauffe pendant 15 heures à une température voisine de 90°C. Le mélange est refroidi à une température voisine de 20°C puis il est jeté dans 400 cm³ d'eau. La phase aqueuse est extraite avec 2 fois de l'acétate d'éthyle. Les phases organiques réunies sont lavées avec 2 fois de l'eau et de la saumure, puis séchées sur sulfate de magnésium, filtrées et concentrées à sec sous pression réduite (2,7 kPa). L'huile orangée obtenue est purifiée par chromatographie-flash sur alumine CTB1 sous pression d'argon (50 kPa) [éluant : cyclohexane / acétate d'éthyle (10 / 90 en volumes), acétate d'éthyle puis acétate d'éthyle / méthanol (95 / 5 en volumes)]. Après concentration des fractions sous pression réduite, on obtient 1,7 g d'une huile jaune pâle. Une solution éthanolique de cette huile est introduite sur une colonne de 8 cm de diamètre contenant 1180 g de phase stationnaire chirale Chiracel OD™. L'élution est réalisée à l'aide d'un mélange de 50% éthanol, 0.1 % de triéthylamine et de 50 % d'heptane. Le débit de la phase mobile étant de 120 ml/min. L'énantiomère lévogyre est élué en première position, la solution est évaporée à sec sous pression réduite (2,7 kPa) pour donner 0,82 g d'huile.

Spectre RMN 1H (300MHz) - δ en ppm - dans le DMSO-d6 : de 1,24 à 1,59 (m, 4H) ; 1,78 (m, 1H) ; 2,14 (m, 1H) ; 2,37 (m, 1H) ; de 2,59 à 2,90 (m, 5H) ; 3,89 (d, J = 8,0 Hz, 2H) ; 5,31 (s, 2H) ; 7,14 (tt, J = 1,5 et 7,5 Hz, 1H) ; de 7,30 à 7,44 (m, 5H) ; 7,50 (d large, J = 8,5 Hz, 2H) ; 7,64 (d large, J = 8,5 Hz, 2H) ; 8,09 (s, 1H). [α]²⁰_{D} = -37.8 +/- 0.8° (c 0.5, MeOH). La solution contenant l'énantiomère dextrogyre élué en deuxième position est évaporée à sec sous pression réduite (2,7 kPa) pour donner 0,74 g d'huile.

Spectre RMN 1H (300MHz) - δ en ppm - dans le DMSO-d6 : de 1,24 à 1,60 (m, 4H) ; 1,78 (m, 1H) ; 2,14 (m, 1H) ; 2,36 (m, 1H) ; de 2,60 à 2,90 (m, 5H) ; 4,00 (d, J = 8,0 Hz, 2H) ; 5,31 (s, 2H) ; 7,14 (tt, J = 1,5 et 7,5 Hz, 1H) ; de 7,30 à 7,44 (m, 5H) ; 7,50 (d large, J = 8,5 Hz, 2H) ; 7,64 (d large, J = 8,5 Hz, 2H) ; 8,09 (s, 1H). [α]²⁰_{D} = -39.1 +/- 0.9° (c 0.5, MeOH).

### Exemple 88 Chlorhydrate de (+)-1-(1-aza-bicyclo[2.2.2]oct-3-ylméthyl)-4-phényl-1H-pyrazol-1-ol

Une solution sous agitation de 740 mg de (+)-3-(3-benzyloxy-4-phényl-pyrazol-1-ylméthyl)-1-aza-bicyclo[2.2.2]octane dans 10 cm³ d'éthanol est additionnée de 10 cm³ d'acide chlorhydrique 12 N. Après 7 h au reflux du solvant, puis 15 h à une température voisine de 20°C, le milieu réactionnel est évaporé à sec sous pression réduite (2,7 kPa). Le résidu est, à trois reprises, successivement dissous dans de l'éthanol et évaporé à sec sous pression réduite (2,7 kPa), puis il est trituré dans de l'éther diisopropylique. Le précipité formé est filtré et séché sous vide (2,7 kPa) pour donner 600 mg de chlorhydrate de (+)-1-(1-aza-bicyclo[2.2.2]oct-3-ylméthyl)-4-phényl-1H-pyrazol-1-ol sous forme d'un solide blanc. Spectre de masse (EI) : 283(+)=M(+). Spectre RMN 1H (300MHz) - δ en ppm - dans le DMSO-d6 : de 1,67 à 1,94 (m, 4H) ; 2,06 (m, 1H) ; 2,52 (m masqué, 1H) ; 2,94 (m, 1H) ; de 3,12 à 3,40 (m, 5H) ; de 3,99 à 4,12 (m, 2H) ; 7,13 (tt, J = 1,5 et 7,5 Hz, 1H) ; 7,33 (t large, J = 7,5 Hz, 2H) ; 7,64 (d large, J = 7,5 Hz, 2H) ; 7,99 (s, 1H) ; 10,05 (m étalé, 1H) . [α]²⁰_{D} = +36.5 +/- 0.8° (c 0.5, MeOH).

### Exemple 89

### Chlorhydrate de 1-(1-aza-bicyclo[2.2.2]oct-3-ylméthyl)-4-(4-chloro-phényl)-1H-pyrazol-3-ol

Une solution sous agitation de 430 mg de 3-[3-benzyloxy-4-(4-chloro-phényl)-pyrazol-1-ylméthyl]-1-aza-bicyclo[2.2.2]octane-borane dans 10 cm³ d'éthanol est additionnée de 10 cm³ d'acide chlorhydrique 12 N. Après 15 h au reflux du solvant, le mileu réactionnel est refroidi à une température voisine de 20°C et évaporé à sec sous pression réduite (2,7 kPa). Le résidu est repris par de l'eau. La solution résultante est amenée à pH voisin de 8 avec de la soude 1 N et extraite avec de l'acétate d'éthyle. La phase organique est séchée sur sulfate de magnésium, filtrée et concentrée à sec sous pression réduite (2,7 kPa). Le solide résiduel est trituré dans de l'oxyde de diisopropyle, filtré et séché sous pression réduite (2,7 kPa) pour donner 137 mg de chlorhydrate de 1-(1-aza-bicyclo[2.2.2]oct-3-ylméthyl)-4-(4-chloro-phényl)-1H-pyrazol-3-ol sous forme d'un solide blanc. Spectre de masse (EI) : 317(+)/...=M(+)/...(1 CI). Spectre RMN 1H (400MHz) - δ en ppm - dans le DMSO-d6 : de 1,30 à 1,61 (m, 4H) ; 1,78 (m, 1H) ; 2,15 (m, 1H) ; 2,38 (m, 1H) ; de 2,66 à 2,81 (m, 4H) ; 2,88 (m, 1H) ; 3,91 (d, J = 8,0 Hz, 2H) ; 7,37 (d large, J = 8,5 Hz, 2H) ; 7,67 (d large, J = 8,5 Hz, 2H) ; 8,00 (s, 1H) ; de 10,35 à 10, 5 (m très étalé, 1H).

Le 3-[3-benzyloxy-4-(4-chloro-phényl)-pyrazol-1-ylméthyl]-1-aza-bicyclo[2.2.2]octane-borane peut être préparé de la manière suivante :

Une solution de 3 g de 3-benzyloxy-4-(4-chloro-phényl)-1H-pyrazole et de 3 g de tert-butylate de potassium dans 30 cm³ de diméthylformamide, sous atmosphère inerte et sous agitation, est chauffée 30 min à 50°C puis elle est additionnée de 3,1 g de chlorhydrate de 3-chlorométhyl-1-aza-bicyclo[2.2.2]octane. Après 15 h de chauffage au reflux, le milieu réactionnel est jeté dans de l'eau, et le mélange est extrait par deux fois de l'acétate d'éthyle. Les phases organiques sont lavées successivement par deux fois de l'eau et une solution aqueuse saturée de chlorure de sodium, séchées sur sulfate de magnésium et évaporées sous pression réduite (2,7 kPa). L'huile beige (5,3 g) obtenue est purifiée par chromatographie-flash sur alumine CTB1 sous pression d'argon (50 kPa) [éluant : cyclohexane / acétate d'éthyle (20 / 80 en volumes), acétate d'éthyle puis acétate d'éthyle / méthanol (90 / 10 en volumes)]. Après concentration des fractions sous pression réduite (2,7 kPa), on obtient un solide qui est dissous dans 10 cm³ de tétrahydrofurane sous atmosphère inerte. La solution, sous agitation et refroidie à -60°C, est additionnée de 3 cm³ d'une solution 1 N de borane de dans le tétrahydrofurane. Après 2,5 h de réaction à -60°C, on ajoute 15 cm³ d'eau au milieu réactionnel, laisse remonter la température du mélange à une température voisine de 20°C et extrait la solution avec de l'acétate d'éthyle. La phase organique est lavée par deux fois de l'eau et une solution aqueuse saturée de chlorure de sodium, séchée sur sulfate de magnésium et évaporée sous pression réduite (2,7 kPa) pour donner une huile (1 g) qui est purifiée par chromatographie-flash sur alumine CTB1 sous pression d'argon (50 kPa) [éluant : acétate d'éthyle, acétate d'éthyle / méthanol (90 / 10 en volumes) puis dichlorométhane / méthanol (80 / 20 en volumes)]. Après concentration des fractions sous pression réduite (2,7 kPa), on obtient 430 mg de 3-[3-benzyloxy-4-(4-chloro-phényl)-pyrazol-1-ylméthyl]-1-aza-bicyclo[2.2.2]octane-borane sous la forme d'une huile utilisée sans autre purification pour la suite de la synthèse.

### Exemple 90

### Chlorhydrate de (-)-1-(1-aza-bicyclo[2.2.2]oct-3-yl)-4-(4-chloro-phényl)-1H-pyrazol-3-ol

Une solution sous agitation de 690 mg de (+)-3-[3-benzyloxy-4-(4-chloro-phényl)-pyrazol-1-yl]-1-aza-bicyclo[2.2.2]octane dans 10 cm³ d'éthanol est additionnée de 7 cm³ d'acide chlorhydrique 12 N. Après 7 h au reflux du solvant, le mileu réactionnel est refroidi à une température voisine de 20°C et évaporé à sec sous pression réduite (2,7 kPa). Le résidu est trituré dans de l'oxyde de diisopropyle, filtré et séché sous pression réduite (2,7 kPa) pour donner 580 mg de chlorhydrate de (-)-1-(1-aza-bicyclo[2.2.2]oct-3-yl)-4-(4-chloro-phényl)-1H-pyrazol-3-ol sous forme d'un solide beige. Spectre de masse (EI) : 303(+)/...=M(+)/... (1 CI). Spectre RMN 1H (300MHz) - δ en ppm - dans le DMSO-d6 : de 1,68 à 2,03 (m, 4H) ; 2,42 (m, 1H) ; de 3,20 à 3,55 (m, 4H) ; 3,78 (m, 2H) ; 4,66 (m, 1H) ; 7,41 (d large, J = 8,5 Hz, 2H) ; 7,71 (d large, J = 8,5 Hz, 2H) ; 8,25 (s, 1H) ; 10,35 (m étalé, 1H) ; 10,65 (m étalé, 1H). [α]²⁰_{D} = -19.4 +/- 0.7° (c 0.5, MeOH).

Le (+)-3-[3-benzyloxy-4-(4-chloro-phényl)-pyrazol-1-yl]-1-aza-bicyclo[2.2.2]octane et le (-)-3-[3-benzyloxy-4-(4-chloro-phényl)-pyrazol-1-yl]-1-aza-bicyclo[2.2.2]octane peuvent être préparés de la manière suivante :

Une solution éthanolique contenant 0,29 g de (+/-)-3-[3-benzyloxy-4-(4-chloro-phényl)-pyrazol-1-yl]-1-aza-bicyclo[2.2.2]octane est introduite sur colonne de 8 cm de diamètre contenant 1180 g de phase stationnaire chirale Chiralpak AD™. L'élution est réalisée à l'aide d'un mélange d'heptane, d'éthanol, de méthanol et de triéthylamine (80 / 10 / 10 / 0,1) en volumes), le débit de la phase mobile étant de 120 ml / min. L'énantiomère dextrogyre est élué en première position, la solution est évaporée à sec sous pression réduite (2,7 kPa) pour obtenir 0,69 g de (+)-3-[3-benzyloxy-4-(4-chloro-phényl)-pyrazol-1-yl]-1-aza-bicyclo[2.2.2]octane sous forme d'huile.

Spectre de masse (EI) : 393(+)/...=M(+)/...(1Cl) ; 91(+)=C7H7(+). [α]²⁰_{D} = +25,4 +/- 0,8- (c 0.5, MeOH). La solution contenant l'énantiomère lévogyre élué en deuxième position est évaporée à sec sous pression réduite (2,7 kPa) pour obtenir 0,69 g de (-)-3-[3-benzyloxy-4-(4-chloro-phényl)-pyrazol-1-yl]-1-aza-bicyclo[2.2.2]octane sous forme d'huile.

Spectre de masse (EI) : 393(+)/...=M(+)/...(1 CI) ; 91(+)=C7H7(+). Pouvoir rotatoir : [α]²⁰ _{D} = -26,2 +/- 0,8° (c 0.5, MeOH).

### Exemple 91

### Chlorhydrate de (+)-1-(1-aza-bicyclo[2.2.2]oct-3-yl)-4-(4-chloro-phényl)-1H-pyrazol-3-ol

Une solution sous agitation de 690 mg de (-)-3-[3-benzyloxy-4-(4-chloro-phényl)-pyrazol-1-yl]-1-aza-bicyclo[2.2.2]octane dans 10 cm³ d'éthanol est additionnée de 7 cm³ d'acide chlorhydrique 12 N. Après 7 h au reflux du solvant, le mileu réactionnel est refroidi à une température voisine de 20°C et évaporé à sec sous pression réduite (2,7 kPa). Le résidu est trituré dans de l'oxyde de diisopropyle, filtré et séché sous pression réduite (2,7 kPa) pour donner 580 mg de chlorhydrate de (+)-1-(1-aza-bicyclo[2.2.2]oct-3-yl)-4-(4-chloro-phényl)-1H-pyrazol-3-ol sous forme d'un solide beige. Spectre de masse (EI) : 303(+)/...=M(+)/...(1 Cl). Spectre RMN 1H (300MHz) - δ en ppm - dans le DMSO-d6 : de 1,67 à 2,02 (m, 4H) ; 2,42 (m, 1H) ; de 3,22 à 3,50 (m, 4H) ; 3,79 (m, 2H) ; 4,66 (m, 1H) ; 7,41 (d large, J = 8,5 Hz, 2H) ; 7,71 (d large, J = 8,5 Hz, 2H) ; 8,25 (s, 1H) ; de 10,2 à 10,3 (m très étalé, 1H) ; 10,65 (m étalé, 1H). [α]²⁰_{D} = +17.7 +/- 0.6° (c 0.5, MeOH).

### Exemple 92

### Chlorhydrate de (-)-1-(1-aza-bicyclo[2.2.2]oct-3-yl)-4-(4-fluoro-phényl)-1H-pyrazol-3-ol

Une solution sous agitation de 135 mg de (+)-3-[3-benzyloxy-4-(4-fluoro-phényl)-pyrazol-1-yl]-1-aza-bicyclo[2.2.2]octane dans 5 cm³ d'éthanol est additionnée de 5 cm³ d'acide chlorhydrique 12 N. Après 7 h au reflux du solvant, le mileu réactionnel est refroidi à une température voisine de 20°C et évaporé à sec sous pression réduite (2,7 kPa). Le résidu est trituré dans de l'oxyde de diisopropyle, filtré et séché sous pression réduite (2,7 kPa) pour donner 91 mg de chlorhydrate de (-)-1-(1-aza-bicyclo[2.2.2]oct-3-yl)-4-(4-fluoro-phényl)-1H-pyrazol-3-ol sous forme d'un solide blanc. Spectre de masse (EI) 287(+)=M(+). Spectre RMN 1H (300MHz) - δ en ppm - dans le DMSO-d6 : de 1,66 à 2,03 (m, 4H) ; 2,42 (m, 1H) ; de 3,20 à 3,49 (m, 4H) ; 3,79 (m, 2H) ; 4,66 (m, 1H) ; 7,19 (t large, J = 9,0 Hz, 2H) ; 7,71 (dd large, J = 6,0 et 9,0 Hz, 2H) ; 8,17 (s, 1H) ; 10,15 (m étalé, 1H) ; 10,5 (s, 1H). [α]²⁰_{D} = -14.9 +/- 0.7° (c 0.5, MeOH).

Le (+)-3-[3-benzyloxy-4-(4-fluoro-phényl)-pyrazol-1-yl]-1-aza-bicyclo[2.2.2]octane et le (-)-3-[3-benzyloxy-4-(4-fluoro-phényl)-pyrazol-1-yl]-1-aza-bicyclo[2.2.2]octane peuvent être préparés de la manière suivante :

Une solution de 2 g de 3-benzyloxy-4-(4-fluoro-phényl)-1H-pyrazole et de 1 g de tert-butylate de potassium dans 20 cm³ de diméthylformamide, sous atmosphère inerte, est agitée 1,5 h à une température voisine de 20°C puis elle est additionnée d'une solution de 2,3 g d'ester 1-aza-bicyclo[2.2.2]oct-3-yl d'acide toluène-4-sulfonique dans 25 cm³ de diméthylformamide. Après 15 h de chauffage à 100°C, le milieu réactionnel est jeté dans de l'eau, et le mélange est extrait par deux fois de l'acétate d'éthyle. Les phases organiques réunies sont lavées successivement par deux fois de l'eau et une solution aqueuse saturée de chlorure de sodium, séchées sur sulfate de magnésium et évaporées sous pression réduite (2,7 kPa). L'huile orangée obtenue (2,3 g) est purifiée par chromatographie-flash sur alumine CTB1 sous pression d'argon (50 kPa) [éluant : acétate d'éthyle puis acétate d'éthyle / méthanol (98 / 2 en volumes)]. Après concentration des fractions sous pression réduite (2,7 kPa), on obtient une huile orangée (1,5 g) qui est purifiée par chromatographie-flash sur silice sous pression d'argon (50 kPa) [éluant : acétate d'éthyle / méthanol (95 / 5 en volumes) puis dichlorométhane / méthanol (98/2, 95/5 puis 90 / 10 en volumes)]. La concentration des fractions appropriées sous pression réduite (2,7 kPa) fournit une huile orange (500 mg) qui est à nouveau purifiée par chromatographie-flash sur alumine CTB1 sous pression d'argon (50 kPa) [éluant : cyclohexane / acétate d'éthyle (10 / 90 en volumes)]. Après concentration des fractions sous pression réduite (2,7 kPa), on obtient 290 mg de (R,S)-3-[3-benzyloxy-4-(4-fluoro-phényl)-pyrazol-1-yl]-1-aza-bicyclo[2.2.2]octane sous la forme d'une huile jaune pâle. Spectre RMN 1H (300MHz ) - δ en ppm - dans le DMSO-d6 : 1,28 (m, 1H) ; de 1,49 à 1,71 (m, 3H) ; 2,08 (m, 1H) ; de 2,65 à 2,79 (m, 3H) ; 2,98 (m, 1H) ; de 3,14 à 3,44 (m, 2H) ; 4,25 (m, 1H) ; 5,33 (m, 2H) ; 7,18 (t large, J = 9,0 Hz, 2H) ; de 7,30 à 7,44 (m, 3H) ; 7,50 (d large, J = 7,5 Hz, 2H) ; 7,71 (dd large, J = 6,0 et 9,0 Hz, 2H) ; 8,20 (s, 1H).

Une solution éthanolique contenant 0,29 g de (+/-)-3-[3-benzyloxy-4-(4-fluoro-phényl)-pyrazol-1-yl]-1-aza-bicyclo[2.2.2]octane est introduite sur colonne de 8 cm de diamètre contenant 1180 g de phase stationnaire chirale Chiralpak AD™. L'élution est réalisée à l'aide d'un mélange d'heptane, d'éthanol, de méthanol et de triéthylamine (80 /10 /10 / 0,1) en volumes), le débit de la phase mobile étant de 120 ml / min. L'énantiomère dextrogyre est élué en première position, la solution est évaporée à sec sous pression réduite (2,7 kPa) pour donner 0,135 g de (+)3-[3-benzyloxy-4-(4-fluoro-phényl)-pyrazol-1-yl]-1-aza-bicyclo[2.2.2]octane sous forme d'huile.

Spectre RMN 1H (300MHz ) - δ en ppm - dans le DMSO-d6 : 1,30 (m, 1H) ; de 1,50 à 1,71 (m, 3H) ; 2,08 (m, 1H) ; de 2,64 à 2,80 (m, 3H) ; 2,99 (m, 1H) ; de 3,16 à 3,43 (m, 2H) ; 4,26 (m, 1H) ; 5,33 (m, 2H) ; 7,17 (t large, J = 9,0 Hz, 2H) ; de 7,30 à 7,44 (m, 3H) ; 7,50 (d large, J = 7,5 Hz, 2H) ; 7,71 (dd large, J = 6,0 et 9,0 Hz, 2H) ; 8,20 (s, 1H). [α]²⁰_{D} = +21,5 +/- 0,5° (c 0.5, MeOH). La solution contenant l'énantiomère lévogyre élue en deuxième position est évaporée à sec sous pression réduite (2,7 kPa) pour obtenir 0,137 g de (-)-3-[3-benzyloxy-4-(4-fluoro-phényl)-pyrazol-1-yl]-1-aza-bicyclo[2.2.2]octane d'huile.

Spectre RMN 1H (300MHz ) - δ en ppm - dans le DMSO-d6 : 1,30 (m, 1H) ; de 1,49 à 1,71 (m, 3H) ; 2,08 (m, 1H) ; de 2,65 à 2,80 (m, 3H) ; 2,98 (m, 1H) ; de 3,15 à 3,42 (m, 2H) ; 4,25 (m, 1H) ; 5,33 (m, 2H) ; 7,18 (t large, J = 9,0 Hz, 2H) ; de 7,30 à 7,44 (m, 3H) ; 7,50 (d large, J = 7,5 Hz, 2H) ; 7,71 (dd large, J = 6,0 et 9,0 Hz, 2H) ; 8,20 (s, 1H). [α]²⁰_{D} = -20,2 +/- 0,6° (c 0.5, MeOH).

Le 3-benzyloxy-4-(4-fluoro-phényl)-1H-pyrazole peut être préparé de la manière suivante :

A une solution sous atmosphère d'argon et sous agitation de 3,5 g de 3-benzyloxy-4-(4-fluoro-phényl)-1-toluène-4-sulfonyl)-1H-pyrazole dans 50 cm³ de tétrahydrofurane, on ajoute 20 cm³ d'une solution 1 N de fluorure de tétrabutyl-ammonium dans le tétrahydrofurane. Après 15 h de chauffage au reflux du solvant, le milieu réactionnel est évaporé sous pression réduite (2,7 kPa) et le résidu est additionné d'acétate d'éthyle. La phase organique est lavée successivement par deux fois de l'eau et une solution aqueuse saturée de chlorure de sodium ; elle est séchée sur sulfate de magnésium et évaporée sous pression réduite (2,7 kPa). Le solide jaune obtenu (2,3 g) est purifié par chromatographie-flash sur silice sous pression d'argon (50 kPa) [éluant : cyclohexane / acétate d'éthyle (70 / 30 en volumes)]. Après concentration des fractions sous pression réduite (2,7 kPa), on obtient 2 g de 3-benzyloxy-4-(4-fluoro-phényl)-1H-pyrazole sous la forme d'un solide blanc. Spectre de masse (EI) : 268(+)=M(+).

Le 3-benzyloxy-4-(4-fluoro-phényl)-1-(toluène-4-sulfonyl)-1H-pyrazole peut être préparé de la manière suivante :

A une solution sous atmosphère d'argon et sous agitation de 4,8 g de 3-benzyloxy-4-iodo-l-(toluène-4-sulfonyl)-1H-pyrazole dans 40 cm³ de toluène additionnés de 10 cm³ d'éthanol, on ajoute 4,5 g d'acide 4-fluoro-phényl boronique, 15 cm³ d'une solution aqueuse de carbonate de potassium 2 N, et 1,6 g de tétrakis(triphénylphosphine)palladium. Après 5 h de chauffage à 100°C et 16 h à une température voisine de 20°C, le milieu réactionnel est évaporé sous pression réduite (2,7 kPa). Le résidu est additionné d'acétate d'éthyle, d'eau et de noir de charbon, et il est filtré sur supercel. Le filtrat est décanté, puis la phase organique est lavée successivement par trois fois de l'eau et une solution aqueuse saturée de chlorure de sodium ; elle est séchée sur sulfate de magnésium et évaporée sous pression réduite (2,7 kPa). Le solide jaune obtenu (9 g) est purifié par chromatographie-flash sur silice sous pression d'argon (50 kPa) [éluant : cyclohexane / acétate d'éthyle (95 / 5 en volumes)]. Après concentration des fractions sous pression réduite (2,7 kPa), on obtient 3,5 g de 3-benzyloxy-4-(4-fluoro-phényl)-1-(toluène-4-sulfonyl)-1H-pyrazole sous forme d'un solide jaune. Spectre de masse (EI): 422(+)=M(+) ; 267(+)=422(+)-Ts ; 91(+)=C7H7(+).

### Exemple 93

### Chlorhydrate de (+)-1-(1-aza-bicyclo[2.2.2]oct-3-yl)-4-(4-fluoro-phényl)-1H-pyrazol-3-ol

Une solution sous agitation de 137 mg de (-)-3-[3-benzyloxy-4-(4-fluoro-phényl)-pyrazol-1-yl]-1-aza-bicyclo[2.2.2]octane dans 5 cm³ d'éthanol est additionnée de 5 cm³ d'acide chlorhydrique 12 N. Après 7 h au reflux du solvant, le mileu réactionnel est refroidi à une température voisine de 20°C et évaporé à sec sous pression réduite (2,7 kPa). Le résidu est trituré dans de l'oxyde de diisopropyle, filtré et séché sous pression réduite (2,7 kPa) pour donner 99 mg de chlorhydrate de (+)-1-(1-aza-bicyclo[2.2.2]oct-3-yl)-4-(4-fluoro-phényl)-1H-pyrazol-3-ol sous forme d'un solide blanc. Spectre de masse (EI): 287(+)=M(+). Spectre RMN 1H (300MHz) - δ en ppm - dans le DMSO-d6 : de 1,69 à 2,03 (m, 4H) ; 2,41 (m, 1H) ; de 3,19 à 3,50 (m, 4H) ; 3,79 (m, 2H) ; 4,65 (m, 1H) ; 7,19 (t large, J = 9,0 Hz, 2H) ; 7,71 (dd large, J = 6,0 et 9,0 Hz, 2H) ; 8,17 (s , 1H) ; de 10,1 à 10,3 (m très étalé, 1H) ; 10,5 (s, 1H). [α]²⁰_{D} = +15.3 +/- 0.6° (c 0.5, MeOH).

### Exemple 94

### Chlorhydrate de 3-[4-(4-chloro-phényl)-pyrazol-1-yl]-1-aza-bicyclo[2.2.2]octane

Une solution de 600 mg de 4-(4-chloro-phényl)-1H-pyrazole et de 420 mg de tert-butylate de potassium dans 20 cm³ de diméthylformamide, sous atmosphère inerte, est agitée 2 h à une température voisine de 20°C puis elle est additionnée d'une solution de 1,1 g d'ester 1-aza-bicyclo[2.2.2]oct-3-yl d'acide toluène-4-sulfonique dans 20 cm³ de diméthylformamide. Après 15 h de chauffage à 100°C, le milieu réactionnel est jeté dans de l'eau, et le mélange est extrait par deux fois de l'acétate d'éthyle. Les phases organiques réunies sont lavées successivement par deux fois de l'eau et une solution aqueuse saturée de chlorure de sodium, séchées sur sulfate de magnésium et évaporées sous pression réduite (2,7 kPa). L'huile orangée obtenue (1 g) est purifiée par chromatographie-flash sur alumine CTB1 sous pression d'argon (50 kPa) [éluant : acétate d'éthyle puis acétate d'éthyle / méthanol (95 / 5 en volumes)]. Après concentration des fractions sous pression réduite (2,7 kPa), on obtient une huile orangée (840 mg) qui est purifiée par chromatographie-flash sur silice sous pression d'argon (50 kPa) [éluant : acétate d'éthyle, acétate d'éthyle / méthanol (80 / 20 en volumes) puis dichlorométhane / méthanol ( 80 / 20 en volumes)]. La concentration des fractions appropriées sous pression réduite (2,7 kPa) fournit un solide crème ( 250mg) qui est dissous dans de l'acétate d'éthyle. La solution est additionnée de 0,85 cm³ d'éther chlorhydrique 1 N puis elle est évaporée à sec sous pression réduite (2,7 kPa). Le résidu est trituré dans de l'éther diisopropylique et le solide résultant est filtré puis séché sous pression réduite (2,7 kPa) pour donner 239 mg de chlorhydrate de 3-[4-(4-chloro-phényl)-pyrazol-1-yl]-1-aza-bicyclo[2.2.2]octane sous la forme d'un solide crème. Spectre de masse (EI) : 287(+)/..=M(+)/...(1 Cl) ; 36(+)/38(+)=HCl(+)=Salification via HCl. Spectre RMN 1H (400MHz) - δ en ppm - dans le DMSO-d6 : 1,71 (m, 2H) ; 1,98 (m, 2H) ; 2,41 (m, 1H) ; de 3,20 à 3,46 (m, 4H) ; 3,78 (m, 1H) ; 3,92 (m, 1H) ; 4,89 (m, 1H) ; 7,44 (d large, J = 8,5 Hz, 2H) ; 7,64 (d large, J = 8,5 Hz, 2H) ; 8,05 (s, 1H) ; 8,46 (s, 1H) ; de 10,05 à 10,35 (m très étalé, 1H).

Le 4-(4-chloro-phényl)-1H-pyrazole peut être préparé de la manière suivante :

A une solution sous atmosphère d'argon et sous agitation de 2,8 g de 4-(4-chloro-phényl)-1-(toluène-4-sulfonyl)-1H-pyrazole dans 30 cm³ de tétrahydrofurane, on ajoute 21 cm³ d'une solution 1 N de fluorure de tétrabutyl-ammonium dans le tétrahydrofiuane. Après δ h de chauffage au reflux du solvant, le milieu réactionnel est évaporé sous pression réduite (2,7 kPa) et le résidu est additionné d'acétate d'éthyle. La phase organique est lavée successivement par deux fois de l'eau et une solution aqueuse saturée de chlorure de sodium ; elle est séchée sur sulfate de magnésium et évaporée sous pression réduite (2,7 kPa). Le solide jaune obtenu (2,1 g) est purifié par chromatographie-flash sur silice sous pression d'argon (50 kPa) [éluant : cyclohexane / acétate d'éthyle (50 / 50 en volumes)]. Après concentration des fractions sous pression réduite (2,7 kPa), on obtient 1,2 g de 4-(4-chloro-phényl)-1H-pyrazole sous la forme d'un solide blanc. Spectre de masse (IE) : m/z=178 (M⁺) pic de base, m/z=151 [(M - HCN)⁺], m/z=116 [(m/z=151 - Cl)⁺], m/z=89 [(m/z=116 - HCN)⁺].

Le 4-(4-ehloro-phényl)-1-(toluène-4-sulfonyl)-1H-pyrazole peut être préparé de la manière suivante :

A une solution sous atmosphère d'argon et sous agitation de 5 g de 4-iodo-1-(toluène-4-sulfonyl)-1H-pyrazole dans 60 cm³ de toluène additionnés de 15 cm³ d'éthanol, on ajoute 6,1 g d'acide 4-chloro-phényl boronique, 20 cm³ d'une solution aqueuse de carbonate de potassium 2 N, et 2,1 g de tétrakis(triphénylphosphine)palladium. Après 3 h de chauffage à 100°C et 16 h à une température voisine de 20°C, le milieu réactionnel est évaporé sous pression réduite (2,7 kPa). Le résidu est additionné d'acétate d'éthyle, d'eau et de noir de charbon, et il est filtré sur supercel. Le filtrat est décanté, puis la phase organique est lavée successivement par deux fois de l'eau et une solution aqueuse saturée de chlorure de sodium ; elle est séchée sur sulfate de magnésium et évaporée sous pression réduite (2,7 kPa). L'huile obtenue (10,7 g) est purifiée par chromatographie-flash sur silice sous pression d'argon (50 kPa) [éluant : cyclohexane / acétate d'éthyle (90 / 10 en volumes)]. Après concentration des fractions sous pression réduite (2,7 kPa), le solide résultant est trituré dans de l'éther diisopropylique. On obtient, après filtration et séchage sous pression réduite (2,7 kPa), 2,8 g de 4-(4-chloro-phényl-1-[toluène-4-sulfonyl)-1H-pyrazole sous forme d'un solide orangé. Spectre RMN 1H: (300MHz ) - δ en ppm - dans le DMSO-d6 : 2,41 (s, 3H) ; de 7,44 à 7,53 (m, 4H) ; 7,80 (d large, J = 9,0 Hz, 2H) ; 7,92 (d large, J = 9,0 Hz, 2H) ; 8,42 (s , 1H) ; 9,04 (s, 1H) .

### Exemple 95

### Chlorhydrate de 3-[4-(4-chloro-phényl)-pyrazol-1-ylméthyl]-1-aza-bicyclo[2.2.2]octane

A une suspension de 345 mg d'hydrure de sodium (à 75 % en masse dans l'huile de vaseline) dans 15 cm³ de diméthylformamide on ajoute progressivement, sous atmosphère d'argon et à une température voisine de 20°C, une solution de 640 mg de 4-(4-chloro-phényl)-1H-pyrazole dans 25 cm² de diméthylformamide. Après 45 min d'agitation à une température voisine de 50°C, on ajoute par petites portions 1,4 g de chlorhydrate de 3-chlorométhyl-1-aza-bicyclo[2.2.2]octane puis chauffe pendant 15 heures à une température voisine de 100°C. Le mélange est refroidi à une température voisine de 20°C puis il est jeté dans de l'eau. La phase aqueuse est extraite avec deux fois de l'acétate d'éthyle. Les phases organiques réunies sont lavées avec deux fois de l'eau et de la saumure, puis séchées sur sulfate de magnésium, filtrées et concentrées à sec sous pression réduite (2,7 kPa). L'huile orangée obtenue (1,3 g) est purifiée par chromatographie-flash sur alumine CTB1 sous pression d'argon (50 kPa) [éluant : acétate d'éthyle puis acétate d'éthyle / méthanol (99 / 1 puis 97 / 3 en volumes)]. Après concentration des fractions sous pression réduite (2,7 kPa), on obtient une huile (520 mg) qui est purifiée par chromatographie-flash sur silice sous pression d'argon (50 kPa) [éluant : dichlorométhane / méthanol ammoniacal 7 N ( 97 / 3 en volumes)]. La concentration des fractions appropriées sous pression réduite (2,7 kPa) fournit une huile jaune pâle (330 mg) qui est dissoute dans de l'acétate d'éthyle. La solution est additionnée de 0,985 cm³ d'éther chlorhydrique 1 N puis elle est évaporée à sec sous pression réduite (2,7 kPa). Le résidu est trituré dans de l'éther diisopropylique et le solide résultant est filtré puis séché sous pression réduite (2,7 kPa) pour donner 353 mg de chlorhydrate de 3-[4-(4-chloro-phényl)-pyrazol-1-ylméthyl]-1-aza-bicyclo[2.2.2]octane sous la forme d'un solide blanc. Spectre de masse (EI) : 301(+)/...=M(+)/...(1Cl). Spectre RMN 1H (300MHz ) - δ en ppm - dans le DMSO-d6 : de 1,65 à 2,13 (m, 5H) ; .2,58 (m, 1H) ; 2,93 (m, 1H) ; de 3,09 à 3,63 (m, 5H) ; de 4,22 à 4,34 (m, 2H) ; 7,42 (d large, J = 9,0 Hz, 2H) ; 7,62 (d large, J = 9,0 Hz, 2H) ; 7,95 (s, 1H) ; 8,28 (s, 1H) ; de 9,90 à 10,2 (m très étalé, 1H).

### Exemple 96

### 3-[4-(3-Chloro-4-méthoxy-phényl)-pyrazol-1-ylméthyl]-1-aza-bicyclo[2.2.2]octane

A une suspension de 530 mg d'hydrure de sodium (à 75 % en masse dans l'huile de vaseline) dans 15 cm³ de diméthylformamide on ajoute progressivement, sous atmosphère d'argon et à une température voisine de 20°C, une solution de 1,1 g de 4-(3-chloro-4-méthoxy-phényl)-1H-pyrazole dans 25 cm³ de diméthylformamide.

Après 1 h d'agitation à une température voisine de 50°C, on ajoute par petites portions 2,07 g de chlorhydrate de 3-chlorométhyl-l-aza-bicyclo[2.2.2]octane puis chauffe pendant 15 heures à une température voisine de 100°C. Le mélange est refroidi à une température voisine de 20°C puis il est jeté dans de l'eau. La phase aqueuse est extraite avec deux fois de l'acétate d'éthyle. Les phases organiques réunies sont lavées avec deux fois de l'eau et de la saumure, puis séchées sur sulfate de magnésium, filtrées et concentrées à sec sous pression réduite (2,7 kPa). L'huile orangée obtenue (1,8 g) est purifiée par chromatographie-flash sur alumine CTB1 sous pression d'argon (50 kPa) [éluant : acétate d'éthyle puis acétate d'éthyle / méthanol (98 / 2 puis 95 / 5 en volumes)]. Après concentration des fractions sous pression réduite (2,7 kPa), on obtient une huile (550 mg) qui est purifiée par chromatographie-flash sur silice sous pression d'argon (50 kPa) [éluant : dichlorométhane / méthanol ammoniacal 7 N ( 98 / 2 en volumes)]. La concentration des fractions appropriées sous pression réduite (2,7 kPa) fournit une huile jaune pâle ( 400 mg) qui est à nouveau purifiée par chromatographie-flash sur alumine CTB1 sous pression d'argon (50 kPa) [éluant : acétate d'éthyle / méthanol (95 / 5 en volumes) puis dichlorométhane / méthanol (80 / 20 en volumes)]. Après concentration des fractions sous pression réduite (2,7 kPa) on obtient 140 mg de 3-[4-(3-chloro-4-méthoxy-phényl)-pyrazol-1-ylméthyl]-1-aza-bicyclo[2.2.2]octane sous la forme d'un solide blanc. Spectre de masse (CI) : 332(+)/...=(M+H)(+)/... (1 Cl) ; 349(+)=(M+NH4)(+)/.... Spectre RMN 1H (400MHz) - δ en ppm - dans le DMSO-d6 : de 1,28 à 1,57 (m, 4H) ; 1,77 (m, 1H) ; 2,15 (m, 1H) ; 2,36 (m, 1H) ; de 2,58 à 2,88 (m, 5H) ; 3,85 (s, 3H) ; 4,10 (d, J = 8,0 Hz, 2H) ; 7,12 (d, J = 8,0 Hz, 1H) ; 7,50 (dd, J = 2,0 et 8,0 Hz, 1H) ; 7,64 (d, J = 2,0 Hz, 1H) ; 7,85 (s, 1H) ; 8,08 (s, 1H).

Le 4-(3-chloro-4-méthoxy-phényl)-1H-pyrazole peut être préparé de la manière suivante :

A une solution sous atmosphère d'argon et sous agitation de 5,2 g de 4-(3-chloro-4-méthoxy-phényl)-1-(toluène-4-sulfonyl)-1H-pyrazole dans 50 cm³ de tétrahydrofurane, on ajoute 35 cm³ d'une solution 1 N de fluorure de tétrabutyl-ammonium dans le tétrahydrofurane. Après 6 h de chauffage au reflux du solvant, le milieu réactionnel est évaporé sous pression réduite (2,7 kPa) et le résidu est additionné d'acétate d'éthyle. La phase organique est lavée successivement par deux fois de l'eau et une solution aqueuse saturée de chlorure de sodium ; elle est séchée sur sulfate de magnésium et évaporée sous pression réduite (2,7 kPa). Le solide orangé obtenu (5,1 g) est purifié par chromatographie-flash sur silice sous pression d'argon (50 kPa) [éluant : cyclohexane / acétate d'éthyle (50 / 50 en volumes)]. Après concentration des fractions sous pression réduite (2,7 kPa), on obtient 1,84 g de 4-(3-chloro-4-méthoxy-phényl-1H-pyrazole sous la forme d'un solide jaune pâle. Spectre de masse (EI) : 208(+)/...=M(+)/...(1 Cl) ; 193(+)/... =M(-)/...-CH3.

Le 4-(3-chloro-4-méthoxy-phényl)-1-(toluène-4-sulfonyl)-1H-pyrazole peut être préparé de la manière suivante :

A une solution sous atmosphère d'argon et sous agitation de 5,6 g de 4-iodo-1-(toluène-4-sulfonyl)-1H-pyrazole dans 60 cm³ de toluène additionnés de 15 cm³ d'éthanol, on ajoute 9 g d'acide 4-chloro-3-méthoxy-phényl boronique, 24 cm³ d'une solution aqueuse de carbonate de potassium 2 N, et 2,45 g de tétrakis(triphénylphosphine)palladium. Après 3 h de chauffage à 100°C et 16 h à une température voisine de 20°C, le milieu réactionnel est évaporé sous pression réduite (2,7 kPa). Le résidu est additionné d'acétate d'éthyle, d'eau et de noir de charbon, et il est filtré sur supercel. Le filtrat est décanté, puis la phase organique est lavée successivement par deux fois de l'eau et une solution aqueuse saturée de chlorure de sodium ; elle est séchée sur sulfate de magnésium et évaporée sous pression réduite (2,7 kPa). Le solide obtenu est trituré dans de l'acétate d'éthyle, filtré et séché sous pression réduite (2,7 kPa) pour donner 5,2 g de 4-(3-chloro-4-méthoxy-phényl)-1-(toluène-4-sulfonyl)-1H-pyrazole sous la forme d'un solide beige utilisé sans autre purification à l'étape suivante. Spectre RMN 1H (300MHz) - δ en ppm - dans le DMSO-d6 pour 70% du mélange : 2,40 (m, 3H) ; 3,88 (s large, 3H) ; 7,19 (d, J = 8,5 Hz, 1H) ; 7,50 (d large, J = 8,5 Hz, 2H) ; 7,71 (dd, J = 2,0 et 8,5 Hz, 1H) ; de 7,88 à 7,93 (m, 3H) ; 8,41 (s, 1H) ; 8,99 (s, 1H) (pureté évaluée à 70% en R.M.N. ¹H + acide boronique de départ).

### Exemple 97

### Dichlorhydrate de 4-[1-aza-bicyclo[2.2.2]oct-3-ylméthyl)-1H-pyrazol-4-yl]-2-chloro-phénol

Une solution sous atmosphère d'argon et sous agitation de 470 mg de 3-[4-(3-chloro-4-méthoxy-phényl)-pyrazol-1-ylméthyl]-1-aza-bicyclo[2.2.2]octane dans 20 cm³ de dichlorométhane est refroidie dans un bain glacé puis elle est additionnée de 12 cm³ d'une solution de tribromure de bore 1 N dans le dichlorométhane. Après avoir laissé remonter le milieu réactionnel à une température voisine de 20°C, on poursuit la réaction 15 h à cette température puis jette le mélange dans de l'eau additionnée de dichlorométhane. La solution est amenée à pH voisin de 8 par addition de soude 1 N. Le précipité formé est filtré, repris dans de l'éthanol à chaud. Après filtration de la suspension à chaud, le filtrat est additionné de d'eau et de noir de charbon, filtré sur papier Wattman^{®} et évaporé à sec sous pression réduite (2,7 kPa). Le solide beige résultant (330 mg) est dissous dans 10 cm³ d'éthanol et 10 cm³ d'acide chlorhydrique 12 N. Après 12 h de chauffage au reflux du solvant, le mélange est refroidi à une température voisine de 20°C, évaporé à sec sous pression réduite (2,7 kPa). Le solide obtenu est, à trois reprises, successivement dissous dans de l'éthanol et évaporé à sec sous pression réduite (2,7 kPa), puis il est trituré dans de l'éher diisopropylique, filtré et séché sous vide (2,7 kPa) pour donner 370 mg de dichlorhydrate de 4-[1-(1-aza-bicyclo[2.2.2]oct-3-ylméthyl)-1H-pyrazol-4-yl]-2-chloro-phénol sous forme d'un solide crème. Spectre de masse (ESP) : 318(+)/... =(M+H)(+). Spectre RMN 1H (300MHz ) - δ en ppm - dans le DMSO-d6 : de 1,66 à 1,92 (m, 4H) ; 2,07 (m, 1H) ; 2,60 (m, 1H) ; 2,94 (m, 1H) ; de 3,10 à 3,39 (m, 5H) ; de 4,15 à 4,32 (m, 2H) ; 7,00 (d, J = 8,5 Hz, 1H) ; 7,34 (dd, J = 2,5 et 8,5 Hz, 1H) ; 7,56 (d, J = 2,5 Hz, 1H) ; 7,85 (s, 1H) ; 8,16 (s, 1H) ; de 9,80 à 10,3 (m très étalé, 2H).

### Exemple 98

### 4-[1-(1-Aza-bicyclo[2.2.2]oct-3-yl)-1H-pyrazol-4-yl]-2-chloro-phénol

Une solution sous atmosphère d'argon et sous agitation de 750 mg de 3-[4-(3-chloro-4-méthoxy-phényl)-pyrazol-1-yl]-1-aza-bicyclo[2.2.2]octane dans 25 cm³ de dichlorométhane est refroidie dans un bain glacé puis elle est additionnée de 11 cm³ d'une solution de tribromure de bore 1 N dans le dichlorométhane. Après 30 min de réaction à une température voisine de 0°C, 3 h de chauffage à 40°C puis 15 h à une température voisine de 20°C, le mélange réactionnel est jeté dans de l'eau additionnée de dichlorométhane. La suspension résultante est filtrée et le solide est repris dans de l'eau. Le mélange est ajusté à pH 8 puis extrait par de l'acétate d'éthyle. La phase organique est lavée successivement par deux fois de l'eau et une solution aqueuse saturée de chlorure de sodium, séchée sur sulfate de magnésium et évaporée sous pression réduite (2,7 kPa). Le solide beige obtenu (800 mg) est purifié par chromatographie-flash sur silice sous pression d'argon (50 kPa) [éluant : dichlorométhane / méthanol (80 / 20 en volumes)]. La concentration des fractions appropriées sous pression réduite (2,7 kPa) fournit un résidu qui est trituré dans de l'éther diisopropylique, filtré et séché sous pression réduite (2,7 kPa) pour donner 240 mg de 4-[1-(1-aza-bicyclo[2.2.2]oct-3-yl)-1H-pyrazol-4-yl]-2-chloro-phénol sous la forme d'un solide blanc. Spectre de masse (EI) : 303(+)/...=M(+)/....(1 Cl). Spectre RMN 1H (400MHz) - δ en ppm - dans le DMSO-d6 : 1,32 (m, 1H) ; 1,54 (m, 1H) ; 1,70 (m, 2H) ; 2,02 (m, 1H) 2,77 (m, 3H) ; 2,98 (m, 1H) ; de 3,20 à 3,50 (m, 2H) ; 4,42 (m, 1H) ; 6,98 (d, J = 9,0 Hz, 1H) ; 7,40 (dd, J = 2,0 et 9,0 Hz, 1H) ; 7,62. (d, J = 2,0 Hz, 1H) ; 7,87 (s, 1H) ; 8,28 (s, 1H) ; 10,05 (m étalé, 1H).

Le 3-[4-(3-chloro-4-méthoxy-phényl)-1-pyrazol-1-yl]-1-aza-bicyclo[2.2.2]octane peut être préparé de la manière suivante :

Une solution de 1,35 g de 4-(3-chloro-4-méthoxy-phényl)-1H-pyrazole et de 800 mg de tert-butylate de potassium dans 20 cm³ de diméthylformamide, sous atmosphère inerte, est agitée 1 h à une température voisine de 20°C puis elle est additionnée d'une solution de 2 g d'ester 1-aza-bicyclo[2.2.2]oct-3-yl d'acide toluène-4-sulfonique dans 20 cm³ de diméthylformamide. Après 18 h de chauffage à 100°C, le milieu réactionnel est jeté dans de l'eau, et le mélange est extrait par deux fois de l'acétate d'éthyle. Les phases organiques réunies sont lavées successivement par deux fois de l'eau et une solution aqueuse saturée de chlorure de sodium, séchées sur sulfate de magnésium et évaporées sous pression réduite (2,7 kPa). L'huile orangée obtenue (2 g) est purifiée par chromatographie-flash sur silice sous pression d'argon (50 kPa) [éluant : acétate d'éthyle, acétate d'éthyle / méthanol (80 / 20 en volumes) puis dichlorométhane / méthanol (80 / 20 en volumes)]. Après concentration des fractions sous pression réduite (2,7 kPa), on obtient 480 mg de 3-[4-(3-chloro-4-méthoxy-phényl)-pyrazol-1-yl]-1-aza-bicyclo[2.2.2]octane sous forme d'une huile orangée. Spectre de masse (IE) :m/z=317 (M⁺·), m/z=234 [(M - C₅H₉N)⁺·], m/z=109 [C₇H₁₁N⁺·], m/z=97 [C₆H₁₁N⁺·] pic de base.

### Exemple 99 et 100

### (-)-4-[1-(1-Aza-bicyclo[2.2.2]oct-3-yl)-1H-pyrazol-4-yl]-2-chloro-phénol et (+)-4-[1-(1-aza-bicyclo[2.2.2]oct-3-yl)-1H-pyrazol-4-yl]-2-chloro-phénol

Une solution éthanolique contenant 0,173 g de 4-[1-(1-aza-bicyclo[2.2.2]oct-3-yl)-1H-pyrazol-4-yl]-2-chloro-phénol est introduite sur colonne de 8 cm de diamètre contenant 1180 g de phase stationnaire chirale Chiralpak AD^{™}. L'élution est réalisée à l'aide d'un mélange d'heptane, d'éthanol, de méthanol et de triéthylamine (80 / 10 / 10 / 0,1 en volumes), le débit de la phase mobile étant de 120 ml / min. L'énantiomère lévogyre est élué en première position, la solution est évaporée à sec sous pression réduite (2,7 kPa) pour donner 0.093 g de (-)-4-[1-(1-Aza-bicyclo[2.2.2]oct-3-yl)-1H-pyrazol-4-yl]-2-chloro-phénol sous forme d'huile.

Spectre de masse (EI) : 303(+)/...=M(+)/...(1 Cl). Spectre RMN 1H (400MHz) - δ en ppm - dans le DMSO-d6 : 1,31 (m, 1H) ; 1,53 (m, 1H) ; 1,68 (m, 2H) ; 2,09 (m, 1H) ; de 2,67 à 2,80 (m, 3H) ; 2,97 (m, 1H) ; de 3,18 à 3,48 (m, 2H) ; 4,39 (m, 1H) ; 6,95 (d, J = 8,5 Hz, 1H) ; 7,38 (dd, J = 2,5 et 8,5 Hz, 1H) ; 7,60 (d, J = 2,5 Hz, 1H) ; 7,85 (s, 1H) ; 8,23 (s, 1H) ; de 9,98 à 10,15 (m très étalé, 1H). [α]²⁰_{D} = -4,1 +/- 0,6° (c 0.5, MeOH).

La solution contenant l'énantiomère dextrogyre élué en deuxième position est évaporée à sec sous pression réduite (2,7 kPa) pour donner 0.102 g de (+)-4-[1-(1-Aza-bicyclo[2.2.2]oct-3-yl)-1H-pyrazol-4-yl]-2-chloro-phénol sous forme d'huile.

Spectre de masse (EI) : 303(+)/...=M(+)/...(1 Cl). Spectre RMN 1H (400MHz) - δ en ppm - dans le DMSO-d6 : 1,35 (m, 1H) ; 1,54 (m, 1H) ; 1,70 (m, 2H) ; 2,12 (m, 1H) ; de 2,70 à 2,83 (m, 3H) ; 3,00 (m, 1H) ; de 3,21 à 3,51 (m, 2H) ; 4,43 (m, 1H) ; 6,95 (d, J = 8,5 Hz, 1H) ; 7,38 (dd, J = 2,5 et 8,5 Hz, 1H) ; 7,61 (d, J = 2,5 Hz, 1H) ; 7,87 (s, 1H) ; 8,27 (s, 1H) ; 10,05 (m large, 1H). [α]²⁰_{D} = +5,3 +/- 0,4° (c 0.5, MeOH).

### Exemple 101 et 102

### (+)-1-(1-Aza-bicyclo[2.2.2]oct-3-yl)-4-pyridin-2-yl-1H-pyrazol-3-ol et (-)-1-(1-aza-bicyclo[2.2.2]oct-3-yl)-4-pyridin-2-yl-1H-pyrazol-3-ol

Une solution éthanolique contenant 0,3 g de chlorhydrate de (+/-)-1-(1-aza-bicyclo[2.2.2]oct-3-yl)-4-pyridin-2-yl-1H-pyrazol-3-ol est introduite sur une colonne de 8 cm de diamètre contenant 1180 g de phase stationnaire chirale Chiralpak AD^{™} 20 µM. L'élution est réalisée à l'aide d'un mélange d'heptane, d'éthanol, de méthanol et de triéthylamine (70 / 15 / 15 / 0,2 en volumes), le débit de la phase mobile étant de 120 ml/mn. L'énantiomère lévogyre est élué en première position, la solution est évaporée à sec sous pression réduite (2,7 kPa) pour donner 0,109 g de (-)-1-(1-aza-bicyclo[2.2.2]oct-3-yl)-4-pyridin-2-yl-1H-pyrazol-3-ol sous forme d'huile.

Spectre de masse (IE) : m/z=270 (M⁺·), m/z=187 [(M - C₅H₉N)⁺] pic de base. Spectre RMN 1H (400MHz ) - δ en ppm - dans le DMSO-d6 (référencé à 2,50 ppm) :1,31 (m, 1H) ; de 1,55 à 1,70 (m, 3H) ; 2,07 (m, 1H) ; de 2,65 à 2,75 (m, 2H) ; 2,90 (m, 1H) ; de 3,14 à 3,39 (m partiellement masqué, 3H) ; 4,21 (m, 1H) ; 7,14 (m, 1H) ; 7,72 (d large, J = 7,5 Hz, 1H) ; 7,78 (m, 1H) ; 8,26 (s, 1H) ; 8,45 (d large, J = 5,0 Hz, 1H) ; de 10,9 à 11,1 (m étalé, 1H). [α]²⁰_{D} = -40.7 +/- 0.8° (c 0.5, diméthylformamide).

La solution contenant l'énantiomère dextrogyre élue en deuxième position est évaporée à sec sous pression réduite (2,7 kPa) pour donner 0.113 g de (+)-1-(1-aza-bicyclo[2.2.2]oct-3-yl)-4-pyridin-2-yl-1H-pyrazol-3-ol sous forme d'huile.

Spectre de masse (IE) : m/z=270 (M⁺·), m/z=187 [(M - C₅H₉N)⁺] pic de base. Spectre RMN 1H (400MHz ) - δ en ppm - dans le DMSO-d6 (référencé à 2,50 ppm) :1,32 (m, 1H) ; de 1,56 à 1,68 (m, 3H) ; 2,08 (m, 1H) ; de 2,66 à 2,76 (m, 2H) ; 2,92 (m, 1H) ; de 3,14 à 3,42 (m partiellement masqué, 3H) ; 4,22 (m, 1H) ; 7,14 (m, 1H) ; 7,71 (d large, J = 7,5 Hz, 1H) ; 7,78 (m, 1H) ; 8,27 (s, 1H) ; 8,47 (d large, J = 5,0 Hz, 1H) ; de 10,8 à 11,15 (m étalé, 1H). [α]²⁰_{D} = +35.4 +/-0.8° (c 0.5, diméthylformamide).

Le chlorhydrate de (+/-)-1-(1-aza-bicyclo[2.2.2]oct-3-yl)-4-pyridin-2-yl-1H-pyrazol-3-ol peut être préparé de la manière suivante :

Une solution de 330 mg de 3-[3-(cyclohex-2-enyloxy)-4-pyridin-2-yl-pyrazol-1-yl]-1-aza-bicyclo[2.2.2]octane dans 7 cm³ de dioxane est additionnée de 7 cm³ de dioxane chlorhydrique 4 N. Après 15 h d'agitation à une température voisine de 20°C, l'insoluble formé est filtré, rincé par de l'oxyde de diisopropyle et séché sous pression réduite (2,7 kPa) pour donner 300 mg de chlorhydrate de (+/-)-1-(1-aza-bicyclo[2.2.2]oct-3-yl)-4-pyridin-2 yl-1H-pyrazol-3-ol sous la forme d'un solide blanc. Spectre de masse (IE) : m/z=270 (M⁺·), m/z=187 [(M - C₅H₉N)⁺] pic de base, m/z=36 (HCl⁺·).

Le 3-[3-(cyclohex-2-enyloxy)-4-pyridin-2-yl-pyrazol-1-yl]-1-aza-bicyclo[2.2.2]octane peut être préparé de la manière suivante :

Une solution de 2,5 g de 2-[3-(cyclohex-2-enyloxy)-1H-pyrazol-4-yl]-pyridine et de 1,4 g de tert-butylate de potassium dans 20 cm³ de diméthylformamide, sous atmosphère inerte, est agitée 1 h à 50°C puis elle est additionnée d'une solution de 4 g d'ester 1-aza-bicyclo[2.2.2]oct-3-yl d'acide toluène-4-sulfonique dans 20 cm³ de diméthylformamide. Après 15 h de chauffage à 100°C, le milieu réactionnel est jeté dans de l'eau, et le mélange est extrait par deux fois de l'acétate d'éthyle. Les phases organiques réunies sont lavées successivement par deux fois de l'eau et une solution aqueuse saturée de chlorure de sodium, séchées sur sulfate de magnésium et évaporées sous pression réduite (2,7 kPa). L'huile orangée obtenue (5,3 g) est purifiée par chromatographie-flash sur alumine CTB1 sous pression d'argon (50 kPa) [éluant : cyclohexane / acétate d'éthyle (60 / 40 en volumes), acétate d'éthyle puis acétate d'éthyle / méthanol (95 / 5 en volumes)]. Après concentration des fractions sous pression réduite (2,7 kPa), on obtient une huile qui est purifiée par chromatographie-flash sur silice sous pression d'argon (50 kPa) [éluant : dichlorométhane / méthanol (95/5 puis 70 / 30 en volumes)]. La concentration des fractions appropriées sous pression réduite (2,7 kPa) fournit 370 mg de 3-[3-(cyclohex-2-enyloxy)-4-pyridin-2-yl-pyrnol-1-yl]-1-aza-bicyclo[2.2.2]octane sous la forme d'une huile jaune pâle. Spectre RMN 1H (400MHz) - δ en ppm - dans le DMSO-d6 (référencé à 2,50 ppm) :1,35 (m, 1H) ; de 1,59 à 1,70 (m, 4H) ; 1,78 (m, 1H) ; de 1,86 à 2,17 (m, 5H) ; de 2,67 à 2,78 (m, 3H) ; 2,96 (m, 1H) ; 3,19 (m, 1H) ; de 3,27 à 3,37 (m masqué, 1H) ; 4,30 (m, 1H) ; 5,20 (m, 1H) ; de 5,95 à 6,04 (m, 2H) ; 7,10 (m, 1H) ; 7,74 (m, 2H) ; 8,18 (s, 1H) ; 8,47 (d large, J = 5,0 Hz, 1H) .

### Exemple 103 et 104

### (+)-1-(1-Aza-bicyclo[2.2.2]oct-3-yl)-4-phenyl-1H-pyrazol-3-ylamine et (-)-1-(1-aza bicyclo[2.2.2]oct-3-yl)-4-phenyl-1H-pyrazol-3-ylamine

Une solution éthanolique contenant 0,13 g de (+/-)-1-(1-aza-bicyclo[2.2.2]oct-3-yl)-4-phenyl-1H-pyrazol-3-ylamine est introduite sur une colonne de 8 cm de diamètre contenant 1180 g de phase stationnaire chirale Chiralpak AD^{™} 20 µM. L'élution est réalisée à l'aide d'un mélange d'heptane, d'éthanol et de triéthylamine (50 / 50 / 0,1 en volumes), le débit de la phase mobile étant de 120 ml / min.

L'énantiomère lévogyre est élué en première position, la solution est évaporée à sec sous pression réduite (2,7 kPa) pour donner 0,035 g de (-)-1-(1-aza-bicyclo[2.2.2]oct-3-yl)-4-phenyl-1H-pyrazol-3-ylamine sous forme d'huile.

Spectre de masse (IE) : m/z=268 (M⁺) pic de base, m/z=185 [(M - C₅H₉N⁺], m/z=109 (C₇H₁₁N⁺·). Spectre RMN 1H (300MHz ) - δ en ppm - dans le DMSO-d6 (référencé à 2,50 ppm) :1,37 (m, 1H) ; de 1,61 à 1,74 (m, 3H) ; 2,11 (m, 1H) ; de 2,71 à 2,85 (m, 3H) ; 3,00 (m, 1H) ; de 3,12à 3,53 (m partiellement masqué, 2H) ; 4,21 (m, 1H) ; 4,65 (m étalé, 2H) ; 7,13 (tt, J = 1,5 et 7,5 Hz, 1H) ; 7,32 (t large, J = 7,5 Hz, 2H) ; 7,52 (d large, J = 7,5 Hz, 2H) ; 7,90 (s, 1H) . [α]²⁰_{D} = - (diméthylformamide).

La solution contenant l'énantiomère dextrogyre élué en deuxième position est évaporée à sec sous pression réduite (2,7 kPa) pour donner 0,039 g de (+)-1-(1-aza-bicyclo[2.2.2]oct-3-yl)-4-phenyl-1H-pyrazol-3-ylamine sous forme d'huile.

Spectre de masse (IE) : m/z=268 (M⁺·), m/z=185 [(M - C₅H₉N)⁺], m/z=109 (C₇H₁₁N⁺·) pic de base. Spectre RMN 1H (300MHz ) - δ en ppm - dans le DMSO-d6 (référencé à 2,50 ppm) : 1,34 (m, 1H) ; de 1,60 à 1,73 (m, 3H) ; 2,09 (m, 1H) ; de 2,68 à 2,82 (m, 3H) ; de 2,90 à 3,53 (m partiellement masqué, 3H) ; 4,19 (m, 1H) ; 4,63 (m étalé, 2H) ; 7,12 (t large, J = 7,5 Hz, 1H) ; 7,32 (t large, J = 7,5 Hz, 2H) ; 7,51 (d large, J = 7,5 Hz, 2H) ; 7,89 (s, 1H) . [α]²⁰_{D} = + (diméthylformamide).

La (+/-)-1-(1-aza-bicyclo[2.2.2]oct-3-yl)-4-phenyl-1H-pyrazol-3-ylamine peut être préparée de la manière suivante :

Une solution de 280 mg de diallyl-[1-(1-aza-bicyclo[2.2.2]oct-3-yl)-4-phenyl-1H-pyrazol-3-yl]-amine dans 10 cm³ de dichlorométhane sous argon et sous agitation est additionnée de 1,1 g d'acide 1,3-diméthylbarbiturique et de 50 mg de tétrakis(triphénylphosphine)palladium. Après 15 h de chauffage au reflux du milieu réactionnel le mélange est évaporé à sec sous pression réduite (2,7 kPa). Le résidu est repris par de l'acide chlorhydrique 1 N et la solution est lavée par 2 fois de l'acétate d'éthyle. La phase aqueuse résultante est alcalinisée par de la soude 1 N et extraite par 2 fois de l'acétate d'éthyle. Les phases organiques réunies sont lavées par une solution aqueuse saturée de chlorure de sodium, séchées sur sulfate de magnésium et évaporées sous pression réduite (2,7 kPa). Le résidu est repris par du dichlorométhane et la solution est traitée par du noir de charbon, filtrée sur supercel et évaporée sous pression réduite (2,7 kPa) pour donner 130 mg de (+/-)-1-(1-aza-bicyclo[2.2.2]oct-3-yl)-4-phenyl-1H-pyrazol-3-ylamine sous la forme d'une huile orange. Spectre RMN 1H (300MHz ) - δ en ppm - dans le DMSO-d6 (référencé à 2,50 ppm) : de 1,20 à 1,38 (m, 1H) ; de 1,58 à 1,71 (m, 3H) ; 2,07 (m, 1H) ; de 2,62 à 2,79 (m, 3H) ; de 2,88 à 3,63 (m partiellement masqué, 3H) ; 4,16 (m, 1H) ; 4,63 (s large, 2H) ; 7,13 (t large, J = 7,5 Hz, 1H) ; 7,32 (t large, J = 7,5 Hz, 2H) ; 7,52 (d large, J = 7,5 Hz, 2H) ; 7,89 (s, 1H) .

La diallyl-[1-(1-aza-bicyclo[2.2.2]oct-3-yl)-4-phenyl-1H-pyrazol-3-yl]-amine peut être préparée de la manière suivante :

Une solution de 1,3 g de diallyl-(4-phenyl-1H-pyrazol-3-yl)-amine et de 730 mg de tert-butylate de potassium dans 20 cm³ de diméthylformamide, sous atmosphère d'argon, est agitée 45 min à 45°C puis elle est additionnée d'une solution de 2,3 g d'ester 1-aza-bicyclo[2.2.2]oct-3-yl d'acide toluène-4-sulfonique dans 15 cm³ de diméthylformamide. Après 15 h de chauffage à 100°C, le milieu réactionnel est jeté dans de l'eau, et le mélange est extrait par deux fois de l'acétate d'éthyle. Les phases organiques réunies sont lavées successivement par deux fois de l'eau et une solution aqueuse saturée de chlorure de sodium, séchées sur sulfate de magnésium et évaporées sous pression réduite (2,7 kPa). L'huile orangée obtenue (1,8 g) est purifiée par chromatographie-flash sur alumine CTB sous pression d'argon (50 kPa) [éluant : cyclohexane / acétate d'éthyle (60 / 40 en volumes)]. Après concentration des fractions sous pression réduite (2,7 kPa), on obtient une huile qui est purifiée par chromatographie-flash sur silice sous pression d'argon (50 kPa) [éluant : dichlorométhane / méthanol (50 / 50 en volumes)]. La concentration des fractions appropriées sous pression réduite (2,7 kPa) fournit 280 mg de diallyl-[1-(1-aza-bicyclo[2.2.2]oct-3-yl)-4-phenyl-1H-pyrazol-3-yl]-amine contenant 20 % (estimé en RMN 1H) de diallyl-(4-phenyl-1H-pyrazol-3-yl)-amine sous la forme d'une huile jaune pâle. Spectre RMN 1H (300MHz ) - δ en ppm - dans le DMSO-d6 (référencé à 2,50 ppm) :1,29 (m, 1H) ; de 1,53 à 1,69 (m, 3H) ; 2,03 (m, 1H) ; de 2,62 à 2,80 (m, 3H) ; 2,97 (m, 1H) ; 3,17 (m, 1H) ; 2,39 (m, 1H) ; de 3,52 à 3,52 (m, 4H) ; 4,24 (m, 1H) ; de 4,98 à 5,17 (m, 4H) ; de 5,75 à 5,91 (m, 2H) ; 7,17 (tt, J = 1,5 et 7,5 Hz, 1H) ; 7,33 (t large, J = 7,5 Hz, 2H) ; 7,61 (d large, J = 7,5 Hz, 2H) ; 7,93 (s, 1H).

La diallyl-(4-phenyl-1H-pyrazol-3-yl)-amine peut être préparée de la manière suivante :

A une solution sous atmosphère d'argon et sous agitation de 2,9 g de diallyl-[4-phenyl-1-(2-tnmethylsilanyl-ethoxymethyl)-1H-pyrazol-3-yl]-amine dans 25 cm³ de tétrahydrofurane, on ajoute 22 cm³ d'une solution 1 N de fluorure de tétrabutyl-ammonium dans le tétrahydrofurane. Après 18 h de chauffage au reflux du solvant, on ajoute 6,3 cm³ de solution 1 N de fluorure de tétrabutyl-ammonium et poursuit le chauffage pendant 4 h. Après évaporation à sec du milieu réactionnel sous pression réduite (2,7 kPa), le résidu est additionné d'acétate d'éthyle et la phase organique est lavée successivement par deux fois de l'eau et une solution aqueuse saturée de chlorure de sodium ; elle est séchée sur sulfate de magnésium et évaporée sous pression réduite (2,7 kPa). L'huile brune obtenue (1,8 g) est purifiée par chromatographie-flash sur silice sous pression d'argon (50 kPa) [éluant : cyclohexane / acétate d'éthyle (70 / 30 en volumes)]. Après concentration des fractions sous pression réduite (2,7 kPa), on obtient 1,3 g de diallyl-(4-phenyl-1H-pyrazol-3-yl)-amine sous la forme d'une huile orange. Spectre de masse (IE) : m/z=239 (M⁺·) pic de base, m/z=198 [(M - C₃H₅)⁺], m/z=41 (C₃H₅⁺).

La diallyl-[4-phenyl-1-(2-trimethylsilanyl-ethoxymethyl)-1H-pyrazol-3-yl]-amine peut être préparée de la manière suivante :

A une solution sous atmosphère d'argon et sous agitation de 2,9 g de 4-phenyl-1-(2-trimethylsilanyl-ethoxymethyl)-1H-pyrazol-3-ylamine dans 60 cm³ d'acétonitrile, on ajoute 8,17 g de carbonate de césium et 4,35 cm³ de bromure d'allyle. Après 15 h de chauffage au reflux du solvant, le milieu réactionnel est évaporé à sec sous pression réduite (2,7 kPa) et le résidu est repris par de l'acétate d'éthyle. La solution organique est lavée successivement par deux fois de l'eau et une solution aqueuse saturée de chlorure de sodium ; elle est séchée sur sulfate de magnésium et évaporée sous pression réduite (2,7 kPa). L'huile orangée obtenue (4 g) est purifiée par chromatographie-flash sur silice sous pression d'argon (50 kPa) [éluant : cyclohexane / acétate d'éthyle (93 / 7 en volumes)]. Après concentration des fractions sous pression réduite (2,7 kPa), on obtient 2,9 g de diallyl-[4-phenyl-1-(2-trimethylsilanyl-ethoxymethyl)-1H pyrazol-3-yl]-amine sous la forme d'une huile orange. Spectre RMN 1H (300MHz) - δ en ppm - dans le DMSO-d6 (référencé à 2,50 ppm): -0,03 (s, 9H) ; 0,83 (m, 2H) ; de 3,52 à 3,63 (m, 6H) ; de 5,04 à 5,15 (m, 4H) ; 5,24 (s, 2H) ; de 5,74 à 5,90 (m, 2H) ; 7,21 (tt, J = 1,5 et 7,5 Hz, 1H) ; 7,36 (t large, J = 7,5 Hz, 2H) ; 7,58 (d large, J = 7,5 Hz, 2H) ; 7,96 (s, 1H).

La 4-phenyl-1-(2-trimethylsilanyl-ethoxymethyl)-1H-pyrazol-3-ylamine peut être préparée de la manière suivante :

Un mélange de 50 cm³ d'éthanol et 50 cm³ d'eau est additionné de 3,7 g de fer, 1,8 g de chlorure d'ammonium puis d'une solution de 3,5 g de 3-nitro-4-phenyl-1-(2-trimethylsilanyl-ethoxymethyl)-1H-pyrazole dans 50 cm³ d'éthanol. Après 8 h de chauffage au reflux du solvant et sous agitation et 15 h à une température voisine de 20°C, le milieu réactionnel est filtré sur supercel et le filtrat est évaporé à sec sous pression réduite (2,7 kPa). Le résidu est repris par de l'acétate d'éthyle et la solution organique est lavée successivement par deux fois de l'eau et une solution aqueuse saturée de chlorure de sodium puis elle est séchée sur sulfate de magnésium et évaporée sous pression réduite (2,7 kPa) pour donner 2,9 g de 4-phenyl-1-(2-trimethylsilanyl-ethoxymethyl)-1H-pyrazol-3-ylamine sous la forme d'une huile orangée. Spectre RMN 1H (400MHz ) - δ en ppm - dans le DMSO-d6 (référencé à 2,50 ppm) : -0,01 (s, 9H) ; 0,86 (m, 2H) ; 3,56 (m, 2H) ; 4,75 (s large, 2H) ; 5,17 (s, 2H) ; 7,18 (t large, J = 7,5 Hz, 1H) ; 7,35 (t large, J = 7,5 Hz, 2H) ; 7,50 (d large, J = 7,5 Hz, 2H) ; 7,88 (s, 1H).

Le 3-nitro-4-phenyl-1-(2-trimethylsilanyl-ethoxymethyl)-1H-pyrazole peut être préparé de la manière suivante :

A une solution sous atmosphère d'argon et sous agitation de 7,8 g de 4-iodo-3-nitro-1-(2-trimethylsilanyl-ethoxymethyl)-1H-pyrazole dans 120 cm³ de toluène additionnés de 30 cm³ d'éthanol, on ajoute 8,8 g d'acide phényl boronique, 36 cm³ d'une solution aqueuse de carbonate de potassium 2 N, et 3,6 g de tétrakis(triphénylphosphine)palladium. Après 15 h de chauffage à 100°C, le milieu réactionnel est refroidi à une température voisine de 20°C puis il est évaporé sous pression réduite (2,7 kPa). Le résidu est repris par de l'acétae d'éthyle et la solution organique est lavée successivement par deux fois de l'eau et une solution aqueuse saturée de chlorure de sodium ; elle est séchée sur sulfate de magnésium et évaporée sous pression réduite (2,7 kPa) pour donner 14,3 g d'une huile brune qui purifiée par chromatographie-flash sur silice sous pression d'argon (50 kPa) [éluant : cyclohexane / acétate d'éthyle (90 / 10 puis 70 / 30 en volumes)]. La concentration des fractions appropriées sous pression réduite (2,7 kPa) fournit 4,5 g de 3-nitro-4-phenyl-1-(2-trimethylsilanyl-ethoxymethyl)-1H-pyrazole sous la forme d'une huile jaune. Spectre de masse (IE) : m/z=319 (M⁺·), m/z=246 [(M - C₃H₉Si)⁺] pic de base, m/z=73 (C₃H₉Si⁺).

Le 4-iodo-3-nitro-1-(2-trimethylsilanyl-ethoxymethyl)-1H-pyrazole peut être préparé de la manière suivante :

A une suspension de 1,7 g d'hydrure de sodium (à 75 % dans l'huile de vaseline) dans 120 cm³ de diméthylformamide sous atmosphère d'argon et sous agitation, sont ajoutés par portion 10 g de 3-nitro-4-phenyl-1H-pyrazole. Le mélange est agité 45 min à une température voisine de 20°C, puis il est additionné lentement de 14 cm³ de chlorure de 2-trimethylsilanyl-ethoxymethyle. Après 15 h d'agitation à une température voisine de 20°C, le milieu réactionnel est jeté dans 500 cm³ d'eau et le mélange est extrait par trois fois 500 cm³ d'acétate d'éthyle. Les phases organiques sont réunies, lavées successivement par deux fois de l'eau et une solution aqueuse saturée de chlorure de sodium, séchées sur du sulfate de magnésium, filtrées et évaporées sous pression réduite (2,7 kPa) pour donner une huile orange (22 g) qui est purifiée par chromatographie-flash sur silice sous pression d'argon (50 kPa) [éluant : cyclohexane / acétate d'éthyle (85 / 15 en volumes)]. Après concentration des fractions sous pression réduite, on obtient 7,8 g de 4-iodo-3-nitro-1-(2-trimethylsilanyl-ethoxymethyl)-1H-pyrazole sous forme d'une huile jaune pâle. Spectre RMN 1H (300MHz) - δ en ppm - dans le DMSO-d6 (référencé à 2,50 ppm): -0,03 (s, 9H) ; 0,87 (m, 2H) ; 3,61 (m, 2H) ; 5,51 (s, 2H) ; 8,52 (s, 1H).

### Exemples 105 et 106

### Chlorhydrates de 1-(1-aza-bicyclo[2.2.2]oct-3-yl)-4-(1H-indol-4-yl)-1H-pyrazole, énantiomères A et B

On opère comme à l'exemple 38 mais avec 0,78 g de 1-(1-aza-bicyclo[2.2.2]oct-3-yl)-4-iodo-1H-pyrazole, 0,51 g d'ester pinacolique de l'acide 1H-indol-4-yl boronique, 0,475 g de carbonate de sodium, 0,13 g de dichloro-1,1'-bis(diphénylphosphinoferrocène)palladium dans 35 cm³ de dioxane et 5 cm³ d'eau. Après purification par chromatographie sous une pression d'azote de 50 kPa sur une cartouche d'alumine basique (Merck) en éluant avec un mélange de cyclohexane et d'acétate d'éthyle (50 / 50 puis 25 / 75 puis 10 / 90 en volumes) puis par un mélange d'acétate d'éthyle et de méthanol (95 / 5 en volumes). Les fractions 49 à 120 sont réunies, concentrées à sec sous pression réduite (3 kPa). Les fractions 192 à 205 sont réunies, lavées par 5 cm³ et concentrées à sec sous pression réduite (3 kPa). Les 2 lots, sous forme de poudre orange, sont joints (266 mg) et purifiés par HPLC pour séparation des énantiomères sur colonne Chiralcel OD 20µm avec comme éluant un mélange d'heptane, de méthanol, d'éthanol, de triéthylamine (60 / 10 / 30 / 0,2 en volumes) ; chaque énantiomère est ensuite repris par 10 cm³ de dichlorométhane et 5 cm³ d'eau. La phase organique est lavée par une solution aqueuse saturée de chlorure de sodium, séchée sur du sulfate de magnésium, filtrée et concentré à sec sous pression réduite (3kPa). Chaque poudre blanche ainsi obtenue est ensuite reprise par 20 cm³ d'éthanol absolu puis portée à reflux du solvant pendant 20 minutes. Chaque solution est filtrée, lavée par 5 cm³ d'éthanol absolu, additonnée de 1 cm³ d'une solution 1 N d'éther de diéthyle chlorhydrique. Les solutions sont concentrées à sec sous pression réduite, reprises par 5 cm³ d'éthanol et à nouveau concentrées à sec sous pression ; les résidus sont précipités dans 5 cm³ d'éther de diisopropyle puis filtrés sur verre fritté. On obtient ainsi 45 mg de chlorhydrate de (+)-1-(1-aza-bicyclo[2.2.2]oct-3-yl)-4-(1H-indol-4-yl)-1H-pyrazole, énantiomère A sous forme d'une poudre beige ([α]²⁰_{D} = +21,0°, solvant : diméthylsulfoxyde, concentration : 0,5) et 39 mg de chlorhydrate de (-)-1-(1-aza-bicyclo[2.2.2]oct-3-yl)-4-(1H-indol-4-yl)-1H-pyrazole, énantiomère B sous forme d'une poudre beige ([α]²⁰_{D} = -22,7°, solvant : diméthylsulfoxyde, concentration : 0,5).

Les compositions pharmaceutiques selon l'invention sont constituées par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 5 mg et 1000 mg par jour par voie orale pour un adulte avec des doses unitaires allant de 1 mg à 250 mg de substance active.

D'une façon générale, le médecin déterminera la posologie appropriée en fonction de l'âge, du poids et de tous les autres facteurs propres au sujet à traiter.

Les composés de formule (I) selon l'invention seront utiles comme médicament dans le traitement des maladies dues à un dysfonctionnement des récepteurs nicotiniques alpha 7 ou répondant favorablement à une modulation de ceux-ci ; dans le traitement, la prévention, le diagnostic et/ou le suivi de l'évolution de troubles ou de maladies psychiatriques ou neurologiques du système nerveux central impliquant des altérations des fonctions cognitives ou du traitement de l'information sensorielle.

Plus particulièrement les maladies ou les troubles traités concernent les capacités cognitives, d'attention, les facultés de concentration, d'apprentissage et/ou de mémorisation, la maladie d'Alzheimer et les troubles cognitifs associés, les démences séniles, les démences vasculaires, les altérations cognitives légères, les déficits mnésiques liés à l'age, les altérations cognitives liées aux infections bactériennes ou virales, les déficits de l'attention liés à l'hyperactivité, la schizophrénie, le traitement des syndromes inflammatoires, la colite ulcéreuse, la maladie de Crohn, le syndrome du colon irritable, les arthrites, le traitement des douleurs aiguës ou chroniques, les fibromyalgies, le traitement des dégénérescences neuronales aiguës consécutives à un traumatisme, aux accidents vasculaires cérébraux, à l'ischémie ou à l'hypoxie cérébrale, le traitement des dégénérescences neuronales chroniques observées au cours de la maladie de Parkinson, de la chorée de Huntington, de l'atrophie multisystématisée, de la paralysie supranucléaire progressive, de la sclérose latérale amyotrophique, le traitement de l'épilepsie, le traitement des dépressions, de l'anxiété, des psychoses maniaco-depressives, des troubles obsessifs compulsifs, des phobies, des syndromes de stress post-traumatiques, des attaques de panique, du syndrome de Gilles de la Tourettes, de l'anorexie et de la boulimie, les troubles du sommeil.

Les composés de formule (I) peuvent également être utilisées pour instaurer une réduction de la consommation de substances addictives, pour aider au maintien de l'abstinence vis à vis de celles-ci ou pour en atténuer les symptômes du sevrage.

Les composés de formule (I) peuvent également être utilisées comme agent diagnostic.

Les exemples suivants illustrent des compositions selon l'invention :

### EXEMPLE A

On prépare, selon la technique habituelle, des gélules dosées à 50 mg de produit actif ayant la composition suivante :

| | |
|---|---|
| - Composé de formule (I) | 50 mg |
| - Cellulose | 18 mg |
| - Lactose | 55 mg |
| - Silice colloïdale | 1 mg |
| - Carboxyméthylamidon sodique | 10 mg |
| - Talc | 10 mg |
| - Stéarate de magnésium | 1 mg |

### EXEMPLE B

On prépare selon la technique habituelle des comprimés dosés à 50 mg de produit actif ayant la composition suivante :

| | |
|---|---|
| - Composé de formule (I) | 50 mg |
| - Lactose | 104 mg |
| - Cellulose | 40 mg |
| - Polyvidone | 10 mg |
| - Carboxyméthylamidon sodique | 22 mg |
| - Talc | 10 mg |
| - Stéarate de magnésium | 2 mg |
| - Silice colloïdale | 2 mg |

- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :

| | |
|---|---|
| - Composé de formule (I) | 10 mg |
| - Acide benzoïque | 80 mg |
| - Alcool benzylique | 0,06 ml |
| - Benzoate de sodium | 80 mg |
| - Ethanol à 95 % | 0,4 ml |
| - Hydroxyde de sodium | 24 mg |
| - Propylène glycol | 1,6 ml |
| - Eau q.s.p. | 4 ml |

## Revendications

1. Composé de formule (I) dans laquelle 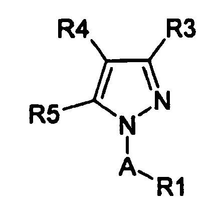 A est, si il est présent, un radical alkyle (C1-C6), un radical alkényle (C3-C6), un radical alkynyle (C3-C6), un radical cycloalkyle (C3-C7), un radical cycloalkényle (C5-C7), ces radicaux sont éventuellement substitués par 1 ou plusieurs substituants choisis parmi alkyle (C1-C5), alkényle (C2-C5), alkynyle (C2-C5), cycloalkyle (C3-C7), cycloalkényle (C5-C7), arylalkyle, hétéroarylalkyle, aryle, hétéroaryle, halogène, R1 est un groupement NR6R7, azacycloalkyle (C4-C7), azacycloalkényle (C5-C7) azabicycloalkyle (C5-C9), azabicycloalkényle (C5-C9), ces groupements sont éventuellement substitués par 1 ou plusieurs substituants choisis parmi alkyle (C1-C5), cycloalkyle (C3-C5), halogène,
A-R1 est tel que l'azote de R1 et l'azote 1 du pyrazole sont obligatoirement séparés par au moins deux atomes de carbone,
R3 est un radical H, halogène, OH, SH, NH2, ORc, SRc, SORa, S02Ra, NHCHO, NRaRb, NHC(O)Ra, NHC(S)Ra, NHSO2Ra,
R4 est un radical aryle ou hétéroaryle étant éventuellement substitué par 1 ou plusieurs substituants choisis parmi halogène, CN, NO₂, NHz, OH, SH, COOH, CHO, C(O)NH2, C(S)NH2, S02H, SO2NH2, NHCHO, C(O)Ra, C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra, aryle, hétéroaryle, hétérocycloalkyle (C4-C7), polyfluoroalkyle, trifluorométhylsulfanyle, trifluorométhoxy, (1-6C) alkyle linéaire ou ramifié, alkényle (C2-C6), alkynyle (C2-C6), ces substituants étant éventuellement substitués par un ou plusieurs alkyle, halogène, OH, méthoxy,
R5 est un radical H, halogène, CF3, CHF2, CH2F, alkyle (C1-C6) linéaire ou ramifié, cycloalkyl (C3-C7),
Ra est (1-6C) alkyle linéaire ou ramifié, alkényle, alkynyl , cycloalkyle (C3-C7), cycloalkényle (C5-C7), hétérocycloalkyle (C4-C7), arylalkyle, hétéroarylalkyle, aryle, hétéroaryle, polyfluoroalkyle,
Rb est indépendamment de Ra un hydrogène, (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Ra et Rb peuvent former un cycle saturé ou insaturé à 5, 6 ou 7 chaînons ayant ou non un hétéroatome tel que O, S, N, ce cycle étant éventuellement substitué par un ou plusieurs radicaux alkyle, halogène,
Rc est un radical, alkyle linéaire ou ramifié (C1-C6), alkényle (C3-C6), alkynyle (C3-C6), cycloalkyle (C3-C7), cycloalkényle (C5-C7), hétérocycloalkyle (C4-C7), (hétéro)arylalkyle, (hétéro)aryle, (poly)fluoroalkyle, C(O)R8, C(S)R8, SO2R8,
R6 et R7 sont indépendamment l'un de l'autre un hydrogène, alkyle (C1-C6), alkényle (C3-C6), alkynyle (C3-C6), cycloalkyle (C3-C7), cycloalkényle (C5-C7), hétérocycloalkyle (C4-C7), un arylalkyle, hétéroarylalkyle,
R6 et R7 peuvent former un cycle saturé ou insaturé à 5, 6 ou 7 chaînons ayant ou non un hétéroatome tel que O, S, N et qui est éventuellement substitué par un ou plusieurs alkyles, halogènes,
R8 est un radical Ra ou NRaRb,
leurs racémiques, énantiomères, diastéréoisomères et leurs mélanges, leurs tautomères et leurs sels pharmaceutiquement acceptables à l'exception du 3-(3-pyridinyl)-1H-pyrazole-1-butanamine, du 4-(3-pyridinyl)-1H-pyrazole-1-butanamine et du N-(diéthyl)-4-phényl-1H-pyrazole-1-éthylamine.

2. Composé de formule (I) selon la revendication 1, dans laquelle : 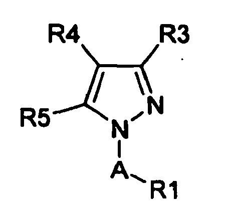 A est, si il est présent, un radical alkyle (C1-C6), un radical alkényle (C3-C6), un radical alkynyle (C3-C6), un radical cycloalkyle (C3-C7), un radical cycloalkényle (C5-C7), ces radicaux sont éventuellement substitués par 1 ou plusieurs substituants choisis parmi alkyle (C1-C5), alkényle (C2-C5), alkynyle (C2-C5), cycloalkyle (C3-C7), cycloalkényle (C5-C7), arylalkyle, hétéroarylalkyle, aryle, hétéroaryle, halogène, R1 est un groupement NR6R7, azacycloalkyle (C4-C7), azacycloalkényle (C5-C7) azabicycloalkyle (C5-C9), azabicycloalkényle (C5-C9), ces groupements sont éventuellement substitués par 1 ou plusieurs substituants choisis parmi alkyle (C1-C5), cycloalkyle (C3-C5), halogène,
A-R1 est tel que l'azote de R1 et l'azote 1 du pyrazole sont obligatoirement séparés par au moins deux atomes de carbone,
R3 est un radical OH, NH2, OMe, H
R4 est un radical aryle ou hétéroaryle étant éventuellement substitué par 1 ou plusieurs substituants choisis parmi halogène, CN, NO₂, NH₂, OH, SH, COOH, CHO, C(O)NH2, C(S)NH2, SO2H, SO2NH2, NHCHO, C(O)Ra, C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra, aryle, hétéroaryle, hétérocycloalkyle (C4-C7), polyfluoroalkyle, trifluorométhylsulfanyle, trifluorométhoxy, (1-6C) alkyle linéaire ou ramifié, alkényle (C2-C6), alkynyle (C2-C6), ces substituants étant éventuellement substitués par un ou plusieurs alkyle, halogène, OH, méthoxy,
R5 est un radical hydrogène ou Me,
Ra est (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Rb est indépendamment de Ra un hydrogène, (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Ra et Rb peuvent former un cycle saturé ou insaturé à 5, 6 ou 7 chaînons ayant ou non un hétéroatome tel que O, S, N, ce cycle étant éventuellement substitué par un ou plusieurs radicaux alkyle, halogène,
Rc est un radical, alkyle linéaire ou ramifié (C1-C6), alkényle (C3-C6), alkynyle (C3-C6), cycloalkyle (C3-C7), cycloalkényle (C5-C7), hétérocycloalkyle (C4-C7), (hétéro)arylalkyle, (hétéro)aryle, (poly)fluoroalkyle, C(O)R8, C(S)R8, SO2R8,
R6 et R7 sont indépendamment l'un de l'autre un hydrogène, alkyle (C1-C6), alkényle (C3-C6), alkynyle (C3-C6), cycloalkyle (C3-C7), cycloalkényle (C5-C7), hétérocycloalkyle (C4-C7), un arylalkyle, hétéroarylalkyle, R6 et R7 peuvent former un cycle saturé ou insaturé à 5, 6 ou 7 chaînons ayant ou non un hétéroatome tel que O, S, N et qui est éventuellement substitué par un ou plusieurs alkyles, halogènes,
R8 est un radical Ra ou NRaRb,
leurs racémiques, énantiomères, diastéréoisomères et leurs mélanges, leurs tautomères et leurs sels pharmaceutiquement acceptables à l'exception du 3-(3-pyridinyl)-1H-pyrazole-1-butanamine, du 4-(3-pyridinyl)-1H-pyrazole-1-butanamine et du N-(diéthyl)-4-phényl-1H-pyrazole-1-éthylamine.

3. Composé de formule (I) selon la revendication 1, dans laquelle : 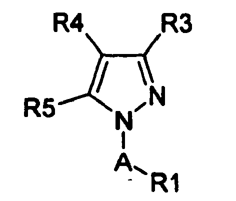 A est, si il est présent, un radical alkyle (C1-C6), un radical alkényle (C3-C6), un radical alkynyle (C3-C6), un radical cycloalkyle (C3-C7), un radical cycloalkényle (C5-C7), ces radicaux sont éventuellement substitués par 1 ou plusieurs substituants choisis parmi alkyle (C1-C5), alkényle (C2-C5), alkynyle (C2-C5), cycloalkyle (C3-C7), cycloalkényle (C5-C7), arylalkyle, hétéroarylalkyle, aryle, hétéroaryle, halogène, R1 est un groupement NR6R7, azacycloalkyle (C4-C7), azacycloalkényle (C5-C7) azabicycloalkyle (C5-C9), azabicycloalkényle (C5-C9), ces groupements sont éventuellement substitués par 1 ou plusieurs substituants choisis parmi alkyle (C1-C5), cycloalkyle (C3-C5), halogène,
A-R1 est tel que l'azote de R1 et l'azote 1 du pyrazole sont obligatoirement séparés par au moins deux atomes de carbone,
R3 est un radical OH, NH2, OMe, H
R4 est un radical aryle ou hétéroaryle étant éventuellement substitué par 1 ou plusieurs substituants choisis parmi halogène, CN, NO₂, NH₂, OH, SH, COOH, CHO, C(O)NH2, C(S)NH2, SO2H, S02NH2, NHCHO, C(O)Ra, C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra, aryle, hétéroaryle, hétérocycloalkyle (C4-C7), polyfluoroalkyle, trifluorométhylsulfanyle, trifluorométhoxy, (1-6C) alkyle linéaire ou ramifié, alkényle (C2-C6), alkynyle (C2-C6), ces substituants étant éventuellement substitués par un ou plusieurs alkyle, halogène, OH, méthoxy,
R5 est un hydrogène,
Ra est (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Rb est indépendamment de Ra un hydrogène, (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Ra et Rb peuvent former un cycle saturé ou insaturé à 5, 6 ou 7 chaînons ayant ou non un hétéroatome tel que O, S, N, ce cycle étant éventuellement substitué par un ou plusieurs radicaux alkyle, halogène,
Rc est un radical, alkyle linéaire ou ramifié (C1-C6), alkényle (C3-C6), alkynyle (C3-C6), cycloalkyle (C3-C7), cycloalkényle (C5-C7), hétérocycloalkyle (C4-C7), (hétéro)arylalkyle, (hétéro)aryle, (poly)fluoroalkyle, C(O)R8, C(S)R8, SO2R8,
R6 et R7 sont indépendamment l'un de l'autre un hydrogène, alkyle (C1-C6), alkényle (C3-C6), alkynyle (C3-C6), cycloalkyle (C3-C7), cycloalkényle (C5-C7), hétérocycloalkyle (C4-C7), un arylalkyle, hétéroarylalkyle,
R6 et R7 peuvent former un cycle saturé ou insaturé à 5, 6 ou 7 chaînons ayant ou non un hétéroatome tel que O, S, N et qui est éventuellement substitué par un ou plusieurs alkyles, halogènes,
R8 est un radical Ra ou NRaRb,
leurs racémiques, énantiomères, diastéréoisomères et leurs mélanges, leurs tautomères et leurs sels pharmaceutiquement acceptables à l'exception du 3-(3-pyridinyl)-1H-pyrazole-1-butanamine, du 4-(3-pyridinyl)-1H-pyrazole-1-butanamine et du N-(diéthyl)-4-phényl-1H-pyrazole-1-éthylamine.

4. Composé selon la revendication 1 **caractérisé par le fait qu'**il est choisi parmi :
1-[2-(3-méthoxy-4-phényl-pyrazol-1-yl)-éthyl]-pipéridine
1-(1-aza-bicyclo[2.2.2]oct-3-ylméthyl)-4-phényl-1H-pyrazol-1-ol
3-(3-benzyloxy-4-phényl-pyrazol-1-ylméthyl)-1-aza-bicyclo[2.2.2]octane
3-(3-méthoxy-4-phényl.pyrazol-1-ylméthyl)-1-aza-bicyclo[2.2.2]octane
1-(1-aza-bicyclo[2.2.2]oct-3-yl)-4-phényl-1H-pyrazol-3-ol
1-(2-perhydro-azépin-1-yl)-éthyl)-4-phényl-1H-pyrazol-3-ol
1-[2-(2-méthyl-pipéridin-1-yl)-éthyl]-4-phényl-1H-pyrazol-3-ol
1-[2-(4-fluoro-pipéridin-1-yl)-éthyl]-4-phényl-1H-pyrazol-3-ol
1-[2-(3-méthyl-pipéridin-1-yl)-éthyl]-4-phényl-1H-pyrazol-3-ol
1-[2-(3,6-dihydro-2H-pyridin-1-yl)-éthyl]-4-phényl-1H-pyrazol-3-ol
1-[2-(7-aza-bicyclo[2.2.1]hept-7-yl)-éthyl]-4-phényl-1H-pyrazol-3-ol
1-[2-(2-aza-bicyclo[2.2.2]oct-2-yl)-éthyl]-4-phényl-1H-pyrazol-3-ol
1-[2-(2-aza-bicyclo[2.2.1]hept-2-yl)-éthyl]-4-phényl-1H-pyrazol-3-ol
1-[2-diméthylamino-éthyl]-4-phényl-1H-pyrazol-3-ol
1-[3-diméthylamino-propyl]-4-phényl-1H-pyrazol-3-ol
1-[2-((2S,6R)-2,6-diméthyl-pipéridin-1-yl)-éthyl]-4-phényl-1H-pyrazol-3-ol
1-[2-diéthylamino-éthyl]-4-phényl-1H-pyrazol-3-ol
1-(2-diisopropylamino-éthyl)-4-phényl-1H-pyrazol-3-ol
4-phényl-1-(2-pyrrolidin-1-yl-éthyl)-1H-pyrazol-3-ol
3-(3-méthoxy-4-phényl-pyrazol-1-yl)-1-aza-bicyclo[2.2.2]octane
1-[2-(3-difluorométhoxy-4-phényl-pyrazol-1-yl)-éthyl]-pipéridine
4-phényl-1-(2-pipéridin-1-yl-éthyl)-1H-pyrazol-3-ylamine
4-phényl-1-(2-pipéridin-1-yl-éthyl)-1H-pyrazol-3-ylamine
N-[4-phényl-1-(2-pipéridin-1-yl-éthyl)-1H-pyrazol-3-yl]-acétamide
N-[4-phényl-1-(2-pipéridin-1-yl-éthyl)-1H-pyrazol-3-yl]-méthanesulfonamide
1-(2-diméthylamino-propyl)-4-phényl-1H-pyrazol-3-ol
1-(1-méthyl-pipéridin-3-ylméthyl)-4-phényl-1H-pyrazol-3-ol
5-méthyl-4-phényl-1-(2-pipéridin-1-yl-éthyl)-1H-pyrazol-3-ol
4-(3-amino-phényl)-1-(2-diméthylamino-éthyl)-1H-pyrazol-3-ol
N-{3-[3-hydroxy-1-(2-diméthylamino-éthyl)-1H-pyrazol-4-yl]₋phényl}-acétamide
4-(4-amino-phényl)-1-(2-diméthylamino-éthyl)-1H-pyrazol-3-ol
1-(2-diméthylamino-éthyl)-4-(4'-fluoro-biphényl-3-yl)-1H-pyrazol-3-ol
4-biphényl-3-yl-1-(2-diméthylamino-éthyl)-1H-pyrazol-3-ol
1-(2-diméthylamino-éthyl)-4-(4'-fluoro-biphényl-4-yl)-1H-pyrazol-3-ol
1-(2-pipéridin-1-yl-éthyl)-4-pyridin-2-yl-1H-pyrazol-3-ol
1-(2-pipéridin-1-yl-éthyl)-4-pyridin-4-yl-1H-pyrazol-3-ol
4-(4-fluoro-phényl)-1-(2-pipéridin-1-yl-éthyl)-1H-pyrazol-3-ol
4-(4-trifluorométhoxy-phényl)-1-(2-pipéridin-1-yl-éthyl)-1H-pyrazol-3-ol
4-phényl-1-(2-pipéridin-l-yl-propyl)-1H-pyrazol-3-ol
3-(4-phényl-pyrazol-1-yl-méthyl)-1-aza-bicyclo[2.2.2]octane
4-(5-chloro-thiophèn-2-yl)-1-(2-pipéridin-1-yl-éthyl)-1 H-pyrazol-3-ol
4-(3-méthoxy-phényl)-1-(2-pipéridin-1-yl-éthyl)-1H-pyrazol-3-ol
4-(2-méthoxy-phényl)-1-(2-pipéridin-1-yl-éthyl)-1H-pyrazol-3-ol
4-(3-hydroxy-phényl)-1-(2-pipéridin-1-yl-éthyl)-1H-prazol-3-ol
4-(4-hydroxy-phényl)-1-(2-pipéridin-1-yl-éthyl)-1H-pyrazol-3-ol
4-(4-méthoxy-phényl)-1-(2-pipéridin-1-yl-éthyl)-1H-pyrazol-3-ol
4-(3-fluoro-phényl)-1-(2-pipéridin-1-yl-éthyl)-1H-pyrazol-3 -ol
1-(2-pipéridin-1-yl-éthyl)-4-(3-trifluorométhyl-phényl)-1H-pyrazol-3-ol
1-(2-pipéridin-1-yl-éthyl)-4-pyridin-3-yl-1H-pyrazol-3-ol
4-(4-chloro-phényl)-1-(2-pipéridin-1-yl-éthyl)-1H-pyrazol-3-ol
4-(3-chloro-phényl)-1-(2-pipéridin-1-yl-éthyl)-1H-pyrazol-3-ol
4-(2-fluoro-phényl)-1-(2-pipéridin-1-yl-éthyl)-1H-pyrazol-3 -ol :
4-(2-chloro-phényl)-1-(2-pipéridin-1-yl-éthyl)-1H-pyrazol-3-ol
1-(1-aza-bicyclo[2.2.2]oct-3-yl)-4-(4-chloro-phényl)-1H-pyrazol-3-ol
1-(1-aza-bicyclo[2.2.2]oct-3-yl)-4-(3-chloro-phényl)-1H-pyrazol-3-ol
1-(1-aza-bicyclo[2.2.2]oct-3-yl)-4-(3-fluoro-phényl)-1H-pyrazol-3-ol
1-(1-méthyl-pyrrolidin-3-yl)-4-phényl-1H-pyrazol-3-ol
1-[2-(1-méthyl-prrolidin-2-yl)-éthyl]-4-phényl-1H-pyrazol-3-ol
1-(pyrrolidin-3-yl)-4-phényl-1H-pyrazol-3-ol
1-[(1-méthyl-pyrrolidin-2-(S)-yl)-méthyl]-4-phényl-1H-pyrazol-3-ol
4-phényl-1-pyrrolidin-3-yl-méthyl-1H-pyrazol-3-ol
1-((2R) 1-méthyl-pyrrolidin-2-ylméthyl)-4-phényl-1H-pyrazol-3-ol
4-phényl-1 -(piperidin-3-yl)-1 H-pyrazol-3-ol
1-(1-méthyl-pipéridin-2-ylméthyl)-4-phényl-1H-pyrazol-3-ol
1-(1-méthyl-azepan-3-yl)-4-phényl-1H-pyrazol-3-ol
4-phényl-1-(2-pipéridin-1-yl-éthyl)-1H-pyrazol-3-ol
4-(thiophèn-2-yl)-1-(2-pipéridin-1-yl-éthyl)-1H-pyrazol-3-ol
4-(3,4-dichloro-phényl)-1-(2-pipéridin-1-yl-éthyl)-1H-pyrazol-3-ol
4-(4-Bromo-phényl)-1-(2-pipéridin-1-yl-éthyl)-1H-pyrazol-3-ol
4-(1H-indol-5-yl)-1-(2-pipéridin-1-yl-éthyl)-1H-pyrazol-3-ol
4-(5-Bromo-thiophèn-2-yl)-1-(2-pipéridin-1-yl-éthyl)-1H-pyrazol-3-ol
2-[1-(2-Pipéridin-1-yl-éthyl)-1H-pyrazol-4-yl] benzamide
4-(2-hydroxy-phényl)-1-(2-pipéridin-1-yl-éthyl)-1H-pyrazole
4-(1H-Indol-5-yl)-1-(2-pipéridin-1-yl-éthyl)-1H-pyrazole
4-(4-méthyl-phényl)-1-(2-pipéridin-1-yl-éthyl)-1H-pyrazol-3-ol
1-(1-Aza-bicyclo[2.2.2]oct-3-yl)-4-(1H-indol-5-yl)-1H-pyrazole
(+)1-(1-Aza-bicyclo[2.2.2]oct-3-yl)-4-(1H-indol-5-yl)-1H-pyrazole
(-)1-(1-Aza-bicyclo[2.2.2]oct-3-yl)-4-(1H-indol-5-yl)-1H-pyrazole
1-(1-aza-bicyclo[2.2.2]oct-3-yl)-4-(5-chloro-thiophèn-2-yl)-1H-pyrazol-3-ol,
1-(1-aza-bicyclo[2.2.2]oct-3-yl)-4-(5-chloro-thiophèn-2-yl)-1H-pyrazol-3-ol,
1-(1-aza-bicyclo[2.2.2]oct-2-ylméthyl)-4-phényl-1H-pyrazol-3-ol
3-[4-(3,5-difluoro-phényl)-pyrazol-1-yl]-1-aza-bicyclo[2.2.2]octane :
4-benzo[b]thiophèn-2-yl-1-(2-pipéridin-1-yl-éthyl)-1H-pyrazol-3-ol
1-(2-pipéridin-1-yl-éthyl)-4-thiophèn-3-yl-1H-pyrazol-3-ol
4-[3-hydroxy-1-(2-pipéridin-1-yl-éthyl)-1H-pyrazol-4-yl]-benzamide
3-[3-hydroxy-1-(2-pipéridin-1-yl-éthyl)-1H-pyrazol-4-yl]-benzamide
(-)-1-(1-aza-bicyclo[2.2.2]oct-3-yl)-4-phényl-1H-pyrazol-3-ol
(+)-1-(1-aza-bicyclo[2.2.2]oct-3-yl)-4-phényl-1H-pyrazol-3-ol
(-)-1-(1-aza-bicyclo[2.2.2]oct-3-ylméthyl)-4-phényl-1H-pyrazol-1-ol
(+)-1-(1-aza-bicyclo[2.2.2]oct-3-ylméthyl)-4-phényl-1H-pyrazol-1-ol
1-(1-aza-bicyclo[2.2.2]oct-3-ylméthyl)-4-(4-chloro-phényl)-1H-pyrazol-3-ol
(-)-1-(1-aza-bicyclo[2.2.2]oct-3-yl)-4-(4-chloro-phényl)-1H-pyrazol-3-ol
(+)-1-(1-aza-bicyclo[2.2.2]oct-3-yl)-4-(4-chloro-phényl)-1H-pyrazol-3-ol
(-)-1-(1-aza-bicyclo[2.2.2]oct-3-yl)-4-(4-fluoro-phényl)-1H-pyrazol-3-ol
(+)-1-(1-aza-bicyclo[2.2.2]oct-3-yl)-4-(4-fluoro-phényl)-1H-pyrazol-3-ol
3-[4-(4-chloro-phényl)-pyrazol-1-yl]-1-aza-bicyclo[2.2.2]octane
3-[4-(4-chloro-phényl)-pyrazol-1-ylméthyl]-1-aza-bicyclo[2.2.2]octane
3-[4-(3-Chloro-4-méthoxy-phényl)-pyrazol-1-ylméthyl]-1-aza-bicyclo[2.2.2]octane
4-[1-(1-aza-bicyclo[2.2.2]oct-3-ylméthyl)-1H-pyrazol-4-yl]-2-chloro-phénol
4-[1-(1-Aza-bicyclo [2.2.2]oct-3-yl)-1H-pyrazol-4-yl]-2-chloro-phénol
(-)-4-[1-(1-Aza-bicyclo[2.2.2]oct-3-yl)-1H-pyrazol-4-yl]-2-chloro-phénol
(+)-4-[1-(1-Aza-bicyclo[2.2.2]oct-3-yl)-1H-pyrazol-4-yl]-2-chloro-phénol
(+)-1-(1-Aza-bicyclo[2.2.2]oct-3-yl)-4-pyridin-2-yl-1H-pyrazol-3-ol
(-)-1-(1-aza-bicyclo[2.2.2]oct-3-yl)-4-pyridin-2-yl-1H-pyrazol-3-ol
(+)-1-(1-Aza-bicyclo[2.2.2]oct-3-yl)-4-phenyl-1H-pyrazol-3-ylamine
(-)-1-(1-aza-bicyclo[2.2.2]oct-3-yl)-4-phenyl-1H-pyrazol-3-ylamine
(+)-1-(1-aza-bicyclo[2.2.2]oct-3-yl)-4-(1H-indol-4-yl)-1H-pyrazole,
(-)-1-(1-aza-bicyclo[2.2.2]oct-3-yl)-4-(1H-indol-4-yl)-1H-pyrazole,
leurs racémiques, énantiomères, diastéréoisomères et leurs mélanges, leurs tautomères et leurs sels pharmaceutiquement acceptables.

5. Procédé de préparation d'un composé de formule (I) tel que défini dans la revendication 1 et pour lequel R3 est OH à partir de composé (II) 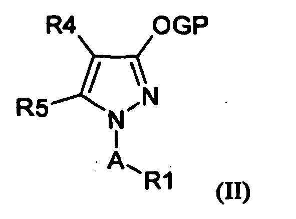 dans lequel GP est un groupement protecteur de la fonction hydroxyle **caractérisé en ce que** le composé (II) est déprotégé puis transformé éventuellement en sel pharmaceutiquement acceptable.

6. Procédé de préparation d'un composé de formule (I) tel que défini dans la revendication 1 et pour lequel R3 est ORc, H, NH2 par alkylation de pyrazoles de formule (III) 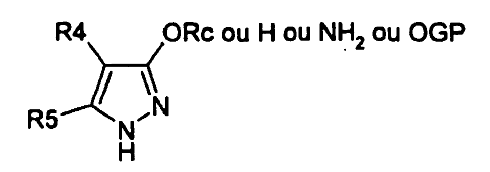 dans lequel GP est un groupement protecteur de la fonction OH avec un composé de formule (IV) R1-A-X dans lequel X est Cl, Br, I, OTs, OMs, OTf en milieu basique dans un solvant aprotique et déprotège éventuellement et transforme éventuellement le produit obtenu en sel pharmaceutiquement acceptable.

7. Composition pharmaceutique comprenant dans un milieu pharmaceutiquement acceptable, au moins un composé défini selon l'une quelconque des revendications 1 à 4.

8. Composé de formule (I) dans laquelle 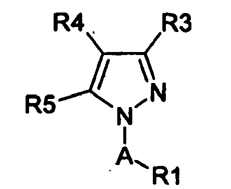 A est, si il est présent, un radical alkyle (C1-C6), un radical alkényle (C3-C6), un radical alkynyle (C3-C6), un radical cycloalkyle (C3-C7), un radical cycloalkényle (C5-C7), ces radicaux sont éventuellement substitués par 1 ou plusieurs substituants choisis parmi alkyle (C1-C5), alkényle (C2-C5), alkynyle (C2-C5), cycloalkyle (C3-C7), cycloalkényle (C5-C7), arylalkyle, hétéroarylalkyle, aryle, hétéroaryle, halogène,
R1 est un groupement NR6R7, azacycloalkyle (C4-C7), azacycloalkényle (C5-C7) azabicycloalkyle (C5-C9), azabicycloalkényle (C5-C9), ces groupements sont éventuellement substitués par 1 ou plusieurs substituants choisis parmi alkyle (C1-C5), cycloalkyle (C3-C5), halogène,
A-R1 est tel que l'azote de R1 et l'azote 1 du pyrazole sont obligatoirement séparés par au moins deux atomes de carbone,
R3 est un radical H, halogène, OH, SH, NH2, ORc, SRc, SORa, SO2Ra, NHCHO, NRaRb, NHC(O)Ra, NHC(S)Ra, NHSO2Ra ,
R4 est un radical aryle ou hétéroaryle étant éventuellement substitué par 1 ou plusieurs substituants choisis parmi halogène, CN, NO₂, NH₂, OH, SH, COOH, CHO, C(O)NH2, C(S)NH2, SO2H, SO2NH2, NHCHO, C(O)Ra, C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra, aryle hétéroaryle, hétérocycloalkyle (C4-C7), polyfluoroalkyle, trifluorométhylsulfanyle, trifluorométhoxy, (1-6C) alkyle linéaire ou ramifié, alkényle (C2-C6), alkynyle (C2-C6), ces substituants étant éventuellement substitués par un ou plusieurs alkyle, halogène, OH, méthoxy,
R5 est un radical H, halogène, CF3, CHF2, CH2F, alkyle (C1-C6) linéaire ou ramifié, cycloalkyl (C3-C7),
Ra est (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Rb est indépendamment de Ra un hydrogène, (1-6C) alkyle linéaire ou ramifié, alkényle, alkynyle, cycloalkyle (C3-C7), cycloalkényle (C5-C7), hétérocycloalkyle (C4-C7), arylalkyle, hétéroarylalkyle, aryle, hétéroaryle, polyfluoroalkyle,
Ra et Rb peuvent former un cycle saturé ou insaturé à 5, 6 ou 7 chaînons ayant ou non un hétéroatome tel que O, S, N, ce cycle étant éventuellement substitué par un ou plusieurs radicaux alkyle, halogène,
Rc est un radical, alkyle linéaire ou ramifié (C1-C6), alkényle (C3-C6), alkynyle (C3-C6), cycloalkyle (C3-C7), cycloalkényle (C5-C7), hétérocycloalkyle (C4-C7),
(hétéro)arylalkyle, (hétéro)aryle, (poly)fluoroalkyle, C(O)R8, C(S)R8, SO2R8,
R6 et R7 sont indépendamment l'un de l'autre un hydrogène, alkyle (C1-C6), alkényle (C3-C6), alkynyle (C3-C6), cycloalkyle (C3-C7), cycloalkényle (C5-C7), hétérocycloalkyle (C4-C7), un arylalkyle, hétéroarylalkyle,
R6 et R7 peuvent former un cycle saturé ou insaturé à 5, 6 ou 7 chaînons ayant ou non un hétéroatome tel que O, S, N et qui est éventuellement substitué par un ou plusieurs alkyles, halogènes,
R8 est un radical Ra ou NRaRb
à l'exception du N-(diéthyl)-4-phényl-1H-pyrazole-1-éthylamine,
leurs racémiques, énantiomères, diastéréoisomères et leurs mélanges, leurs tautomères et leurs sels pharmaceutiquement acceptables pour la préparation d'un médicament.

9. Composé selon la revendication 8, de formule (I) dans laquelle : 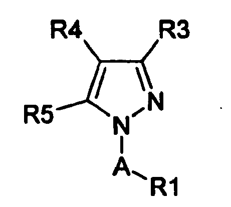 A est, si il est présent, un radical alkyle (C1-C6), un radical alkényle (C3-C6), un radical alkynyle (C3-C6), un radical cycloalkyle (C3-C7), un radical cycloalkényle (C5-C7), ces radicaux sont éventuellement substitués par 1 ou plusieurs substituants choisis parmi alkyle (C1-C5), alkényle (C2-C5), alkynyle (C2-C5), cycloalkyle (C3-C7), cycloalkényle (C5-C7), arylalkyle, hétéroarylalkyle, aryle, hétéroaryle, halogène,
R1 est un groupement NR6R7, azacycloalkyle (C4-C7), azacycloalkényle (C5-C7) azabicycloalkyle (C5-C9), azabicycloalkényle (C5-C9), ces groupements sont éventuellement substitués par 1 ou plusieurs substituants choisis parmi alkyle (C1-C5), cycloalkyle (C3-C5), halogène,
A-R1 est tel que l'azote de R1 et l'azote 1 du pyrazole sont obligatoirement séparés par au moins deux atomes de carbone,
R3 est un radical OH, NH2, OMe, H
R4 est un radical aryle ou hétéroaryle étant éventuellement substitué par 1 ou plusieurs substituants choisis parmi halogène, CN, NO₂, NH₂, OH, SH, COOH, CHO, C(O)NH2, C(S)NH2, SO2H, SO2NH2, NHCHO, C(O)Ra, C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra, aryle, hétéroaryle, hétérocycloalkyle (C4-C7), polyfluoroalkyle, trifluorométhylsulfanyle, trifluorométhoxy, (1-6C) alkyle linéaire ou ramifié, alkényle (C2-C6), alkynyle (C2-C6), ces substituants étant éventuellement substitués par un ou plusieurs alkyle, halogène, OH, méthoxy,
R5 est un radical hydrogène ou Me,
Ra est (1-6C) alkyle linéaire ou ramifié, alkényle, alkynyle, cycloalkyle (C3-C7), cycloalkényle (C5-C7), hétérocycloalkyle (C4-C7), arylalkyle, hétéroarylalkyle, aryle, hétéroaryle, polyfluoroalkyle,
Rb est indépendamment de Ra un hydrogène, (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Ra et Rb peuvent former un cycle saturé ou insaturé à 5, 6 ou 7 chaînons ayant ou non un hétéroatome tel que O, S, N, ce cycle étant éventuellement substitué par un ou plusieurs radicaux alkyle, halogène,
Rc est un radical, alkyle linéaire ou ramifié (C1-C6), alkényle (C3-C6), alkynyle (C3-C6), cycloalkyle (C3-C7), cycloalkényle (C5-C7), hétérocycloalkyle (C4-C7), (hétéro)arylalkyle, (hétéro)aryle, (poly)fluoroalkyle, C(O)R8, C(S)R8, SO2R8,
R6 et R7 sont indépendamment l'un de l'autre un hydrogène, alkyle (C1-C6), alkényle (C3-C6), alkynyle (C3-C6), cycloalkyle (C3-C7), cycloalkényle (C5-C7), hétérocycloalkyle (C4-C7), un arylalkyle, hétéroarylalkyle,
R6 et R7 peuvent former un cycle saturé ou insaturé à 5, 6 ou 7 chaînons ayant ou non un hétéroatome tel que O, S, N et qui est éventuellement substitué par un ou plusieurs alkyles, halogènes,
R8 est un radical Ra ou NRaRb,
à l'exception du N-(diéthyl)-4-phényl-1H-pyrazole-1-éthylamine,
leurs racémiques, énantiomères, diastéréoisomères et leurs mélanges, leurs tautomères et leurs sels pharmaceutiquement acceptables pour la préparation d'un médicament.

10. Composé selon la revendication 8, de formule (I) dans laquelle : 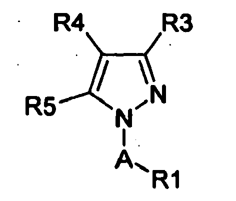 A est, si il est présent, un radical alkyle (C1-C6), un radical alkényle (C3-C6), un radical alkynyle (C3-C6), un radical cycloalkyle (C3-C7), un radical cycloalkényle (C5-C7), ces radicaux sont éventuellement substitués par 1 ou plusieurs substituants choisis parmi alkyle (C1-C5), alkényle (C2-C5), alkynyle (C2-C5), cycloalkyle (C3-C7), cycloalkényle (C5-C7), arylalkyle, hétéroarylalkyle, aryle, hétéroaryle, halogène,
R1 est un groupement NR6R7, azacycloalkyle (C4-C7), azacycloalkényle (C5-C7) azabicycloalkyle (C5-C9), azabicycloalkényle (C5-C9), ces groupements sont éventuellement substitués par 1 ou plusieurs substituants choisis parmi alkyle (C1-C5), cycloalkyle (C3-C5), halogène,
A-R1 est tel que l'azote de R1 et l'azote 1 du pyrazole sont obligatoirement séparés par au moins deux atomes de carbone,
R3 est un radical OH, NH2, OMe, H
R4 est un radical aryle ou hétéroaryle étant éventuellement substitué par 1 ou plusieurs substituants choisis parmi halogène, CN, NO₂, NH₂, OH, SH, COOH, CHO, C(O)NH2, C(S)NH2, SO2H, SO2NH2, NHCHO, C(O)Ra, C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra, aryle, hétéroaryle, hétérocycloalkyle (C4-C7), polyfluoroalkyle, trifluomméthylsulfanyle, trifluorométhoxy, (1-6C) alkyle linéaire ou ramifié, alkényle (C2-C6), alkynyle (C2-C6), ces substituants étant éventuellement substitués par un ou plusieurs alkyle, halogène, OH, méthoxy,
R5 est un hydrogène,
Ra est (1-6C) alkyle linéaire ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Rb est indépendamment de Ra un hydrogène, (1-6C) alkyle linéaire, ou ramifié, alkényle, alkynyle , cycloalkyle (C3-C7), cycloalkényle (C5-C7), hétérocycloalkyle (C4-C7), arylalkyle, hétéroarylalkyle, aryle, hétéroaryle, polyfluoroalkyle,
Ra et Rb peuvent former un cycle saturé ou insaturé à 5, 6 ou 7 chaînons ayant ou non un hétéroatome tel que O, S, N, ce cycle étant éventuellement substitué par un ou plusieurs radicaux alkyle, halogène,
Rc est un radical, alkyle linéaire ou ramifié (C1-C6), alkényle (C3-C6), alkynyle (C3-C6), cycloalkyle (C3-C7), cycloalkényle (C5-C7), hétérocycloalkyle (C4-C7), (hétéro)arylalkyle, (hétéro)aryle, (poly)fluoroalkyle, C(O)R8, C(S)R8, SO2R8,
R6 et R7 sont indépendamment l'un de l'autre un hydrogène, alkyle (C1-C6), alkényle (C3-C6), alkynyle (C3-C6), cycloalkyle (C3-C7), cycloalkényle (C5-C7), hétérocycloalkyle (C4-C7), un arylalkyle, hétéroarylalkyle,
R6 et R7 peuvent former un cycle saturé ou insaturé à 5, 6 ou 7 chaînons ayant ou non un hétéroatome tel que O, S, N et qui est éventuellement substitué par un ou plusieurs alkyles, halogènes,
R8 est un radical Ra ou NRaRb,
à l'exception du N-(diéthyl)4-phényl-1H-pyrazole-1-éthylamine,
leurs racémiques, énantiomères, diastéréoisomères et leurs mélanges, leurs tautomères et leurs sels pharmaceutiquement acceptables pour la préparation d'un médicament.

11. Utilisation d'au moins un composé défini selon l'une quelconque des revendications 1 à 4 pour la préparation d'un médicament destiné au traitement des maladies dues à un dysfonctionnement des récepteurs nicotiniques α7 ou répondant favorablement à une modulation de ceux-ci.

12. Utilisation d'au moins un composé défini selon l'une quelconque des revendications 1 à 4 pour la préparation d'un médicament destiné au traitement, la prévention, le diagnostic et/ou le suivi de l'évolution de troubles ou de maladies psychiatriques ou neurologiques du système nerveux central impliquant des altérations des fonctions cognitives ou du traitement de l'information sensorielle.

13. Utilisation selon la revendication 12 **caractérisée par le fait que** la maladie ou le trouble concerne les capacités cognitives, d'attention, les facultés de concentration, d'apprentissage et/ou de mémorisation.

14. Utilisation selon la revendication 12 **caractérisée par le fait que** la maladie est la maladie d'Alzheimer et les troubles cognitifs associés.

15. Utilisation selon la revendication 12 **caractérisée par le fait que** la maladie est la schizophrénie.

16. Utilisation d'au moins un composé selon l'une quelconque des revendications 1 à 4 pour la préparation d'un médicament destiné à instaurer une réduction de la consommation de substances addictives, pour aider au maintien de l'abstinence vis à vis de celles-ci ou pour en atténuer les symptômes du sevrage et pour son application thérapeutique dans le traitement des dégénéresences neuronales aigues ou chroniques.

## Claims

1. A compound of formula (I) in which 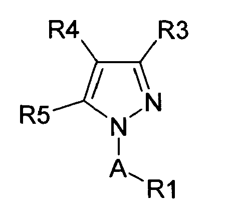 A is, if it is present, a (C1-C6) alkyl radical, a (C3-C6) alkenyl radical, a (C3-C6) alkynyl radical, a (C3-C7) cycloalkyl radical or a (C5-C7) cycloalkenyl radical; these radicals are optionally substituted with one or more substituents chosen from (C1-C5) alkyl, (C2-C5) alkenyl, (C2-C5) alkynyl, (C3-C7) cycloalkyl, (C5-C7) cycloalkenyl, arylalkyl, heteroarylalkyl, aryl, heteroaryl and halogen,
R1 is an NR6R7, (C4-C7) azacycloalkyl, (C5-C7) azacycloalkenyl, (C5-C9) azabicycloalkyl or (C5-C9) azabicycloalkenyl group; these groups are optionally substituted with one or more substituents chosen from (C1-C5) alkyl, (C3-C5) cycloalkyl and halogen,
A-R1 is such that the nitrogen of R1 and the nitrogen in the 1-position of the pyrazole are necessarily separated by at least two carbon atoms,
R3 is an H, halogen, OH, SH, NH₂, ORc, SRc, SORa, SO₂Ra, NHCHO, NRaRb, NHC(O)Ra, NHC(S)Ra or NHSO₂Ra radical,
R4 is an aryl or heteroaryl radical being optionally substituted with one or more substituents chosen from halogen, CN, NO₂, NH₂, OH, SH, COOH, CHO, C(O)NH₂, C(S)NH₂, SO₂H, SO₂NH₂, NHCHO, C (O) Ra, C(O)ORa, C(O)NRaRb, C(S)NRaRb, S(O)Ra, SO₂Ra, SO₂NRaRb, ORc, SRc, O-C (O) Ra, -O-C (S) Ra, NRaRb, NHC(O)Ra, NHC (S) Ra, NHCONH₂, NHCONRaRb, NHSO₂Ra, aryl, heteroaryl, (C4-C7) heterocycloalkyl, polyfluoroalkyl, trifluoromethyl-sulfanyl, trifluoromethoxy, linear or branched (C1-C6) alkyl, (C2-C6) alkenyl and (C2-C6) alkynyl, these substituents being optionally substituted with one or more alkyl, halogen, OH, methoxy,
R5 is an H, halogen, CF₃, CHF₂, CH₂F, linear or branched (C1-C6) alkyl or (C3-C7) cycloalkyl radical,
Ra is linear or branched (C1-C6) alkyl, alkenyl, alkynyl, (C3-C7) cycloalkyl, (C5-C7) cycloalkenyl, (C4-C7) heterocycloalkyl, arylalkyl, heteroarylalkyl, aryl, heteroaryl or polyfluoroalkyl,
Rb is, independently of Ra, a hydrogen, linear or branched (C1-C6) alkyl, alkenyl, alkynyl, (C3-C7) cycloalkyl, (C5-C7) cycloalkenyl, (C4-C7) heterocycloalkyl, arylalkyl, heteroarylalkyl, aryl, heteroaryl or polyfluoroalkyl,
Ra and Rb may form a saturated or unsaturated ring containing 5, 6 or 7 ring members, which may or may not have a hetero atom such as O, S or N, this ring being optionally substituted with one or more alkyl, halogen radicals,
Rc is a linear or branched (C1-C6) alkyl, (C3-C6) alkenyl, (C3-C6) alkynyl, (C3-C7) cycloalkyl, (C5-C7) cycloalkenyl, (C4-C7) heterocycloalkyl, (hetero)arylalkyl, (hetero)aryl, (poly)fluoroalkyl, C(O)R8, C(S)R8 or SO₂R8 radical,
R6 and R7 are, independently of one another, a hydrogen, (C1-C6) alkyl, (C3-C6) alkenyl, (C3-C6) alkynyl, (C3-C7) cycloalkyl, (C5-C7) cycloalkenyl, (C4-C7) heterocycloalkyl, an arylalkyl or heteroarylalkyl,
R6 and R7 may form a saturated or unsaturated ring with 5, 6 or 7 ring members, which may or may not have a hetero atom such as O, S or N and which is optionally substituted with one or more alkyls, halogens,
R8 is an Ra or NRaRb radical,
their racemates, enantiomers and diastereoisomers and their mixtures, their tautomers and their pharmaceutically acceptable salts, with the exception of 3-(3-pyridinyl)-1H-pyrazole-1-butanamine, of 4-(3-pyridinyl)-1H-pyrazole-1-butanamine and of N-(diethyl)-4-phenyl-1H-pyrazole-1-ethylamine.

2. A compound of formula (I) as claimed in claim 1, in which: 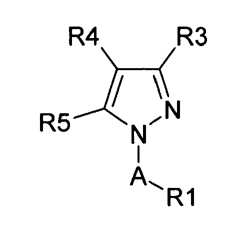 A is, if it is present, a (C1-C6) alkyl radical, a (C3-C6) alkenyl radical, a (C3-C6) alkynyl radical, a (C3-C7) cycloalkyl radical or a (C5-C7) cycloalkenyl radical; these radicals are optionally substituted with one or more substituents chosen from (C1-C5) alkyl, (C2-C5) alkenyl, (C2-C5) alkynyl, (C3-C7) cycloalkyl, (C5-C7) cycloalkenyl, arylalkyl, heteroarylalkyl, aryl, heteroaryl and halogen,
R1 is an NR6R7, (C4-C7) azacycloalkyl, (C5-C7) azacycloalkenyl, (C5-C9) azabicycloalkyl or (C5-C9) azabicycloalkenyl group; these groups are optionally substituted with one or more substituents chosen from (C1-C5) alkyl, (C3-C5) cycloalkyl and halogen,
A-R1 is such that the nitrogen of R1 and the nitrogen in the 1-position of the pyrazole are necessarily separated by at least two carbon atoms,
R3 is an OH, NH₂, OMe or H radical,
R4 is an aryl or heteroaryl radical being optionally substituted with one or more substituents chosen from halogen, CN, NO₂, NH₂, OH, SH, COOH, CHO, C(O)NH₂, C(S)NH₂, SO₂H, SO₂NH₂, NHCHO, C(O)Ra, C(O)ORa, C(O)NRaRb, C(S)NRaRb, S (O) Ra, SO₂Ra, SO₂NRaRb, ORc, SRc, O-C(O)Ra, -O-C(S)Ra, NRaRb, NHC(O)Ra, NHC(S)Ra, NHCONH₂, NHCONRaRb, NHSO₂Ra, aryl, heteroaryl, (C4-C7) heterocycloalkyl, polyfluoroalkyl, trifluoromethyl-sulfanyl, trifluoromethoxy, linear or branched (C1-C6) alkyl, (C2-C6) alkenyl and (C2-C6) alkynyl, these substituents being optionally substituted with one or more alkyl, halogen, OH, methoxy,
R5 is a hydrogen or Me radical,
Ra is linear or branched (C1-C6) alkyl, alkenyl, alkynyl, (C3-C7) cycloalkyl, (C5-C7) cycloalkenyl, (C4-C7) heterocycloalkyl, arylalkyl, heteroarylalkyl, aryl, heteroaryl or polyfluoroalkyl,
Rb is, independently of Ra, a hydrogen, linear or branched (C1-C6) alkyl, alkenyl, alkynyl, (C3-C7) cycloalkyl, (C5-C7) cycloalkenyl, (C4-C7) heterocycloalkyl, arylalkyl, heteroarylalkyl, aryl, heteroaryl or polyfluoroalkyl,
Ra and Rb may form a saturated or unsaturated ring containing 5, 6 or 7 ring members, which may or may not have a hetero atom such as O, S or N, this ring being optionally substituted with one or more alkyl, halogen radicals,
Rc is a linear or branched (C1-C6) alkyl, (C3-C6) alkenyl, (C3-C6) alkynyl, (C3-C7) cycloalkyl, (C5-C7) cycloalkenyl, (C4-C7) heterocycloalkyl, (hetero)-arylalkyl, (hetero)aryl, (poly)fluoroalkyl, C(O)R8, C(S)R8 or SO₂R8 radical,
R6 and R7 are, independently of one another, a hydrogen, (C1-C6) alkyl, (C3-C6) alkenyl, (C3-C6) alkynyl, (C3-C7) cycloalkyl, (C5-C7) cycloalkenyl, (C4-C7) heterocycloalkyl, an arylalkyl or heteroarylalkyl,
R6 and R7 may form a saturated or unsaturated ring with 5, 6 or 7 ring members, which may or may not have a hetero atom such as O, S or N and which is optionally substituted with one or more alkyls, halogens,
R8 is an Ra or NRaRb radical,
their racemates, enantiomers and diastereoisomers and their mixtures, their tautomers and their pharmaceutically acceptable salts, with the exception of 3-(3-pyridinyl)-1H-pyrazole-1-butanamine, of 4-(3-pyridinyl)-1H-pyrazole-1-butanamine and of N-(diethyl)-4-phenyl-1H-pyrazole-1-ethylamine.

3. A compound of formula (I) as claimed in claim 1, in which: 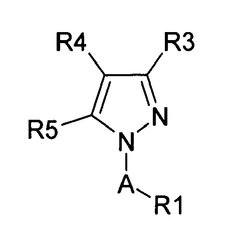 A is, if it is present, a (C1-C6) alkyl radical, a (C3-C6) alkenyl radical, a (C3-C6) alkynyl radical, a (C3-C7) cycloalkyl radical or a (C5-C7) cycloalkenyl radical; these radicals are optionally substituted with one or more substituents chosen from (C1-C5) alkyl, (C2-C5) alkenyl, (C2-C5) alkynyl, (C3-C7) cycloalkyl, (C5-C7) cycloalkenyl, arylalkyl, heteroarylalkyl, aryl, heteroaryl and halogen,
R1 is an NR6R7, (C4-C7) azacycloalkyl, (C5-C7) azacycloalkenyl, (C5-C9) azabicycloalkyl or (C5-C9) azabicycloalkenyl group; these groups are optionally substituted with one or more substituents chosen from (C1-C5) alkyl, (C3-C5) cycloalkyl and halogen,
A-R1 is such that the nitrogen of R1 and the nitrogen in the 1-position of the pyrazole are necessarily separated by at least two carbon atoms,
R3 is an OH, NH₂, OMe or H radical,
R4 is an aryl or heteroaryl radical being optionally substituted with one or more substituents chosen from halogen, CN, NO₂, NH₂, OH, SH, COOH, CHO, C(O)NH₂, C(S)NH2, SO₂H, SO₂NH₂, NHCHO, C(O)Ra, C(O)ORa, C(O)NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH₂, NHCONRaRb, NHSO₂Ra, aryl, heteroaryl, (C4-C7) heterocycloalkyl, polyfluoroalkyl, trifluoromethyl-sulfanyl, trifluoromethoxy, linear or branched (C1-C6) alkyl, (C2-C6) alkenyl and (C2-C6) alkynyl, these substituents being optionally substituted with one or more alkyl, halogen, OH, methoxy,
R5 is a hydrogen,
Ra is linear or branched (C1-C6) alkyl, alkenyl, alkynyl, (C3-C7) cycloalkyl, (C5-C7) cycloalkenyl, (C4-C7) heterocycloalkyl, arylalkyl, heteroarylalkyl, aryl, heteroaryl or polyfluoroalkyl,
Rb is, independently of Ra, a hydrogen, linear or branched (C1-C6) alkyl, alkenyl, alkynyl, (C3-C7) cycloalkyl, (C5-C7) cycloalkenyl, (C4-C7) heterocycloalkyl, arylalkyl, heteroarylalkyl, aryl, heteroaryl or polyfluoroalkyl,
Ra and Rb may form a saturated or unsaturated ring containing 5, 6 or 7 ring members, which may or may not have a hetero atom such as O, S or N, this ring being optionally substituted with one or more alkyl, halogen radicals,
Rc is a linear or branched (C1-C6) alkyl, (C3-C6) alkenyl, (C3-C6) alkynyl, (C3-C7) cycloalkyl, (C5-C7) cycloalkenyl, (C4-C7) heterocycloalkyl, (hetero)arylalkyl, (hetero)aryl, (poly)fluoroalkyl, C(O)R8, C(S)R8 or SO₂R8 radical,
R6 and R7 are, independently of one another, a hydrogen, (C1-C6) alkyl, (C3-C6) alkenyl, (C3-C6) alkynyl, (C3-C7) cycloalkyl, (C5-C7) cycloalkenyl, (C4-C7) heterocycloalkyl, an arylalkyl or heteroarylalkyl,
R6 and R7 may form a saturated or unsaturated ring with 5, 6 or 7 ring members, which may or may not have a hetero atom such as O, S or N and which is optionally substituted with one or more alkyls, halogens,
R8 is an Ra or NRaRb radical,
their racemates, enantiomers and diastereoisomers and their mixtures, their tautomers and their pharmaceutically acceptable salts, with the exception of 3-(3-pyridinyl)-1H-pyrazole-1-butanamine, of 4-(3-pyridinyl)-1H-pyrazole-1-butanamine and of N-(diethyl)-4-phenyl-1H-pyrazole-1-ethylamine.

4. The compound as claimed in claim 1, **characterized in that** it is chosen from:
1-[2-(3-Methoxy-4-phenylpyrazol-1-yl)ethyl]piperidine
1-(1-Azabicyclo[2.2.2]oct-3-ylmethyl)-4-phenyl-1H-pyrazol-1-ol
3-(3-Benzyloxy-4-phenylpyrazol-1-ylmethyl)-1-azabicyclo[2.2.2]octane
3-(3-Methoxy-4-phenylpyrazol-1-ylmethyl)-1-azabicyclo[2.2.2]octane
1-(1-Azabicyclo[2.2.2]oct-3-yl)-4-phenyl-1H-pyrazol-3-ol
1-(2-Perhydroazepin-1-ylethyl)-4-phenyl-1H-pyrazol-3-ol
1-[2-(2-Methylpiperidin-1-yl)ethyl]-4-phenyl-1H-pyrazol-3-ol
1-[2-(4-Fluoropiperidin-1-yl)ethyl]-4-phenyl-1H-pyrazol-3-ol
1-[2-(3-Methylpiperidin-1-yl)ethyl]-4-phenyl-1H-pyrazol-3-ol
1-[2-(3,6-Dihydro-2H-pyridin-1-yl)ethyl]-4-phenyl-1H-pyrazol-3-ol
1-[2-(7-Azabicyclo[2.2.1]hept-7-yl)ethyl]-4-phenyl-1H-pyrazol-3-ol
1-[2-(2-Azabicyclo[2.2.2]oct-2-yl)ethyl]-4-phenyl-1H-pyrazol-3-ol
1-[2-(2-Azabicyclo[2.2.1]hept-2-yl)ethyl]-4-phenyl-1H-pyrazol-3-ol
1-[2-Dimethylaminoethyl]-4-phenyl-1H-pyrazol-3-ol
1-[3-Dimethylaminopropyl]-4-phenyl-1H-pyrazol-3-ol
1-[2-((2S,6R)-2,6-Dimethylpiperidin-1-yl)ethyl]-4-phenyl-1H-pyrazol-3-ol
1-[2-Diethylaminoethyl]-4-phenyl-1H-pyrazol-3-ol
1-(2-Diisopropylaminoethyl)-4-phenyl-1H-pyrazol-3-ol
4-Phenyl-1-(2-pyrrolidin-1-ylethyl)-1H-pyrazol-3-ol
3-(3-Methoxy-4-phenylpyrazol-1-yl)-1-azabicyclo-[2.2.2]octane
1-[2-(3-Difluoromethoxy-4-phenylpyrazol-1-yl)ethyl]-piperidine
4-Phenyl-1-(2-piperidin-1-ylethyl)-1H-pyrazol-3-ylamine
4-Phenyl-1-(2-piperidin-1-ylethyl)-1H-pyrazol-3-ylamine
N-[4-Phenyl-1-(2-piperidin-1-ylethyl)-1H-pyrazol-3-yl]acetamide
N-[4-Phenyl-1-(2-piperidin-1-ylethyl)-1H-pyrazol-3-yl]methanesulfonamide
1-(2-Dimethylaminopropyl)-4-phenyl-1H-pyrazol-3-ol
1-(1-Methylpiperidin-3-ylmethyl)-4-phenyl-1H-pyrazol-3-ol
5-Methyl-4-phenyl-1-(2-piperidin-1-ylethyl)-1H-pyrazol-3-ol
4-(3-Aminophenyl)-1-(2-dimethylaminoethyl)-1H-pyrazol-3-ol
N-{3-[3-Hydroxy-1-(2-dimethylaminoethyl)-1H-pyrazol-4-yl]phenyl}acetamide
4-(4-Aminophenyl)-1-(2-dimethylaminoethyl)-1H-pyrazol-3-ol
1-(2-Dimethylaminoethyl)-4-(4'-fluorobiphenyl-3-yl)-1H-pyrazol-3-ol
4-Biphenyl-3-yl-1-(2-dimethylaminoethyl)-1H-pyrazol-3-ol
1-(2-Dimethylaminoethyl)-4-(4'-fluorobiphenyl-4-yl)-1H-pyrazol-3-ol
1-(2-Piperidin-1-ylethyl)-4-pyridin-2-yl-1H-pyrazol-3-ol
1-(2-Piperidin-1-ylethyl)-4-pyridin-4-yl-1H-pyrazol-3-ol
4-(4-Fluorophenyl)-1-(2-piperidin-1-ylethyl)-1H-pyrazol-3-ol
4-(4-Trifluoromethoxyphenyl)-1-(2-piperidin-1-ylethyl)-1H-pyrazol-3-ol
4-Phenyl-1-(2-piperidin-1-ylpropyl)-1H-pyrazol-3-ol
3-(4-Phenylpyrazol-1-ylmethyl)-1-azabicyclo[2.2.2]-octane
4-(5-Chlorothiophen-2-yl)-1-(2-piperidin-1-ylethyl)-1H-pyrazol-3-ol
4-(3-Methoxyphenyl)-1-(2-piperidin-1-ylethyl)-1H-pyrazol-3-ol
4-(2-Methoxyphenyl)-1-(2-piperidin-1-ylethyl)-1H-pyrazole-3-ol
4-(3-Hydroxyphenyl)-1-(2-piperidin-1-ylethyl)-1H-pyrazol-3-ol
4-(4-Hydroxyphenyl)-1-(2-piperidin-1-ylethyl)-1H-pyrazol-3-ol
4-(4-Methoxyphenyl)-1-(2-piperidin-1-ylethyl)-1H-pyrazol-3-ol
4-(3-Fluorophenyl)-1-(2-piperidin-1-ylethyl)-1H-pyrazol-3-ol
1-(2-Piperidin-1-ylethyl)-4-(3-trifluoromethylphenyl)-1H-pyrazol-3-ol
1-(2-Piperidin-1-ylethyl)-4-pyridin-3-yl-1H-pyrazol-3-ol
4-(4-Chlorophenyl)-1-(2-piperidin-1-ylethyl)-1H-pyrazol-3-ol
4-(3-Chlorophenyl)-1-(2-piperidin-1-ylethyl)-1H-pyrazol-3-ol
4-(2-Fluorophenyl)-1-(2-piperidin-1-ylethyl)-1H-pyrazol-3-ol
4-(2-Chlorophenyl)-1-(2-piperidin-1-ylethyl)-1H-pyrazol-3-ol
1-(1-Azabicyclo[2.2.2]oct-3-yl)-4-(4-chlorophenyl)-1H-pyrazol-3-ol
1-(1-Azabicyclo[2.2.2] oct-3-yl)-4- (3-chlorophenyl)-1H-pyrazol-3-ol
1-(1-Azabicyclo[2.2.2]oct-3-yl)-4-(3-fluorophenyl)-1H-pyrazol-3-ol
1-(1-Methylpyrrolidin-3-yl)-4-phenyl-1H-pyrazol-3-ol
1-[2-(1-Methylpyrrolidin-2-yl)ethyl]-4-phenyl-1H-pyrazol-3-ol
1-(Pyrrolidin-3-yl)-4-phenyl-1H-pyrazol-3-ol
1-[(1-Methylpyrrolidin-2-(S)-yl)methyl]-4-phenyl-1H-pyrazol-3-ol
4-Phenyl-1-pyrrolidin-3-ylmethyl-1H-pyrazol-3-ol
1-((2R)-1-Methylpyrrolidin-2-ylmethyl)-4-phenyl-1H-pyrazol-3-ol
4-Phenyl-1-(piperidin-3-yl)-1H-pyrazol-3-ol
1-(1-Methylpiperidin-2-ylmethyl)-4-phenyl-1H-pyrazol-3-ol
1-(1-Methylazepan-3-yl)-4-phenyl-1H-pyrazol-3-ol
4-Phenyl-1-(2-piperidin-1-ylethyl)-1H-pyrazol-3-ol
4-(Thiophen-2-yl)-1-(2-piperidin-1-ylethyl)-1H-pyrazol-3-ol
4-(3,4-Dichlorophenyl)-1-(2-piperidin-1-ylethyl)-1H-pyrazol-3-ol
4-(4-Bromophenyl)-1-(2-piperidin-1-ylethyl)-1H-pyrazol-3-ol
4-(1H-Indol-5-yl)-1-(2-piperidin-1-ylethyl)-1H-pyrazol-3-ol
4-(5-Bromothiophen-2-yl)-1-(2-piperidin-1-ylethyl)-1H-pyrazol-3-ol
2-[1-(2-Piperidin-1-ylethyl)-1H-pyrazol-4-yl]benzamide
4-(2-Hydroxyphenyl)-1-(2-piperidin-1-ylethyl)-1H-pyrazole
4-(1H-Indol-5-yl)-1-(2-piperidin-1-ylethyl)-1H-pyrazole
4-(4-Methylphenyl)-1-(2-piperidin-1-ylethyl)-1H-pyrazol-3-ol
1-(1-Azabicyclo[2.2.2] oct-3-yl)-4- (1H-indol-5-yl)-1H-pyrazole
(+)-1-(Azabicyclo[2.2.2]oct-3-yl)-4-(1H-indol-5-yl)-1H-pyrazole
(-)-1-(Azabicyclo[2.2.2]oct-3-yl)-4-(1H-indol-5-yl)-1H-pyrazole
1-(1-Azabicyclo[2.2.2]oct-3-yl)-4-(5-chlorothiophen-2-yl)-1H-pyrazol-3-ol
1-(1-Azabicyclo[2.2.2]oct-3-yl)-4-(5-chlorothiophen-2-yl)-1H-pyrazol-3-ol
1-(1-Azabicyclo[2.2.2]oct-2-ylmethyl)-4-phenyl-1H-pyrazol-3-ol
3-[4-(3,5-Difluorophenyl)pyrazol-1-yl]-1-azabicyclo[2.2.2]octane
4-Benzo[b]thiophen-2-yl-1-(2-piperidin-1-ylethyl)-1H-pyrazol-3-ol
1-(2-Piperidin-1-ylethyl)-4-thiophen-3-yl-1H-pyrazol-3-ol
4-[3-Hydroxy-1-(2-piperidin-1-ylethyl)-1H-pyrazol-4-yl]benzamide
3-[3-Hydroxy-1-(2-piperidin-1-ylethyl)-1H-pyrazol-4-yl]benzamide
(-)-1-(1-Azabicyclo[2.2.2]oct-3-yl)-4-phenyl-1H-pyrazol-3-ol
(+)-1-(1-Azabicyclo[2.2.2]oct-3-yl)-4-phenyl-1H-pyrazol-3-ol
(-)-1-(1-Azabicyclo[2.2.2]oct-3-ylmethyl)-4-phenyl-1H-pyrazol-1-ol
(+)-1-(1-Azabicyclo[2.2.2]oct-3-ylmethyl)-4-phenyl-1H-pyrazol-l-ol
1-(1-Azabicyclo[2.2.2]oct-3-ylmethyl)-4-(4-chlorophenyl)-1H-pyrazol-3-ol
(-)-1-(1-Azabicyclo[2.2.2]oct-3-yl)-4-(4-chlorophenyl)-1H-pyrazol-3-ol
(+)-1-(1-Azabicyclo[2.2.2]oct-3-yl)-4-(4-chlorophenyl)-1H-pyrazol-3-ol
(-)-1-(1-Azabicyclo[2.2.2]oct-3-yl)-4-(4-fluorophenyl)-1H-pyrazol-3-ol
(+)-1-(1-Azabicyclo[2.2.2]oct-3-yl)-4-(4-fluorophenyl)-1H-pyrazol-3-ol
3-[4-(4-Chlorophenyl)pyrazol-1-yl]-1-azabicyclo-[2.2.2]octane
3-[4-(4-Chlorophenyl)pyrazol-1-ylmethyl]-1-azabicyclo[2.2.2]octane
3-[4-(3-Chloro-4-methoxyphenyl)pyrazol-1-ylmethyl]-1-azabicyclo[2.2.2]octane
4-[1-(1-Azabicyclo[2.2.2]oct-3-ylmethyl)-1H-pyrazol-4-yl]-2-chlorophenol
4-[1-(1-Azabicyclo[2.2.2]oct-3-yl)-1H-pyrazol-4-yl]-2-chlorophenol
(-)-4-[1-(1-Azabicyclo[2.2.2]oct-3-yl)-1H-pyrazol-4-yl]-2-chlorophenol
(+)-4-[1-(1-Azabicyclo[2.2.2]oct-3-yl)-1H-pyrazol-4-yl]-2-chlorophenol
(+)-1-(1-Azabicyclo[2.2.2]oct-3-yl)-4-pyridin-2-yl-1H-pyrazol-3-ol
(-)-1-(1-Azabicyclo[2.2.2]oct-3-yl)-4-pyridin-2-yl-1H-pyrazol-3-ol
(+)-1-(1-Azabicyclo[2.2.2]oct-3-yl)-4-phenyl-1H-pyrazol-3-ylamine
(-)-1-(1-Azabicyclo[2.2.2]oct-3-yl)-4-phenyl-1H-pyrazol-3-ylamine
(+)-1-(1-Azabicyclo[2.2.2]oct-3-yl)-4-(1H-indol-4-yl)-1H-pyrazole
(-)-1-(1-Azabicyclo[2.2.2]oct-3-yl)-4-(1H-indol-4-yl)-1H-pyrazole,
their racemates, enantiomers and diastereoisomers and their mixtures, their tautomers and their pharmaceutically acceptable salts.

5. A process for preparing a compound of formula (I) as defined in claim 1 and for which R3 is OH from compound (II) 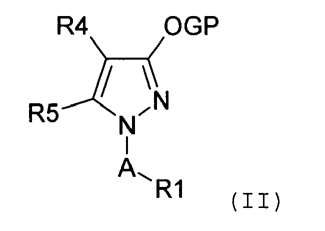 in which GP is a hydroxyl function-protecting group, **characterized in that** the compound (II) is deprotected and then optionally converted into a pharmaceutically acceptable salt.

6. A process for preparing a compound of formula (I) as defined in claim 1 and for which R3 is ORc, H or NH₂, by alkylation of pyrazoles of formula (III) 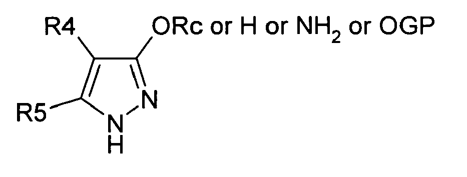 in which GP is an OH function-protecting group, with a compound of formula (IV) Rl-A-X in which X is Cl, Br, I, OTs, OMs or OTf, in basic medium in an aprotic solvent, and the product obtained is optionally deprotected and optionally converted into a pharmaceutically acceptable salt.

7. A pharmaceutical composition comprising, in a pharmaceutically acceptable medium, at least one compound defined in any one of claims 1 to 4.

8. A compound of formula (I) in which 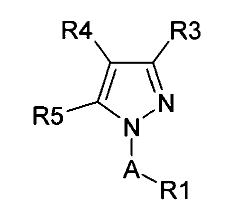 A is, if it is present, a (Cl-C6) alkyl radical, a (C3-C6) alkenyl radical, a (C3-C6) alkynyl radical, a (C3-C7) cycloalkyl radical or a (C5-C7) cycloalkenyl radical; these radicals are optionally substituted with one or more substituents chosen from (C1-C5) alkyl, (C2-C5) alkenyl, (C2-C5) alkynyl, (C3-C7) cycloalkyl, (C5-C7) cycloalkenyl, arylalkyl, heteroarylalkyl, aryl, heteroaryl and halogen,
R1 is an NR6R7, (C4-C7) azacycloalkyl, (C5-C7) azacycloalkenyl, (C5-C9) azabicycloalkyl or (C5-C9) azabicycloalkenyl group; these groups are optionally substituted with one or more substituents chosen from (C1-C5) alkyl, (C3-C5) cycloalkyl and halogen,
A-R1 is such that the nitrogen of R1 and the nitrogen in the 1-position of the pyrazole are necessarily separated by at least two carbon atoms,
R3 is an H, halogen, OH, SH, NH₂, ORc, SRc, SORa, SO₂Ra NHCHO, NRaRb, NHC(O)Ra, NHC(S)Ra or NHSO₂R radical,
R4 is an aryl or heteroaryl radical being optionally substituted with one or more substituents chosen from halogen, CN, NO₂, NH₂, OH, SH, COOH, CHO, C(O)NH₂, C(S)NH₂, SO₂H, SO₂NH₂, NHCHO, C(O)Ra, C(O)ORa, C(O)NRaRb, C (S) NRaRb, S(O)Ra, SO₂Ra,SO₂NRaRb, ORc, SRc, O-C (O) Ra, -O-C (S) Ra, NRaRb, NHC (O) Ra, NHC (S) Ra, NHCONH₂, NHCONRaRb, NHSO₂Ra, aryl, heteroaryl, (C4-C7) heterocycloalkyl, polyfluoroalkyl, trifluoromethyl-sulfanyl, trifluoromethoxy, linear or branched (C1-C6) alkyl, (C2-C6) alkenyl and (C2-C6) alkynyl, these substituents being optionally substituted with one or more alkyl, halogen, OH, methoxy,
R5 is an H, halogen, CF₃, CHF₂, CH₂F, linear or branched (C1-C6) alkyl or (C3-C7) cycloalkyl radical,
Ra is linear or branched (C1-C6) alkyl, alkenyl, alkynyl, (C3-C7) cycloalkyl, (C5-C7) cycloalkenyl, (C4-C7) heterocycloalkyl, arylalkyl, heteroarylalkyl, aryl, heteroaryl or polyfluoroalkyl,
Rb is, independently of Ra, a hydrogen, linear or branched (C1-C6) alkyl, alkenyl, alkynyl, (C3-C7) cycloalkyl, (C5-C7) cycloalkenyl, (C4-C7) heterocycloalkyl, arylalkyl, heteroarylalkyl, aryl, heteroaryl or polyfluoroalkyl,
Ra and Rb may form a saturated or unsaturated ring containing 5, 6 or 7 ring members, which may or may not have a hetero atom such as O, S or N, this ring being optionally substituted with one or more alkyl, halogen radicals,
Rc is a linear or branched (C1-C6) alkyl, (C3-C6) alkenyl, (C3-C6) alkynyl, (C3-C7) cycloalkyl, (C5-C7) cycloalkenyl, (C4-C7) heterocycloalkyl, (hetero)-arylalkyl, (hetero)aryl, (poly)fluoroalkyl, C(O)R8, C(S)R8 or SO₂R8 radical,
R6 and R7 are, independently of one another, a hydrogen, (C1-C6) alkyl, (C3-C6) alkenyl, (C3-C6) alkynyl, (C3-C7) cycloalkyl, (C5-C7) cycloalkenyl, (C4-C7) heterocycloalkyl, an arylalkyl or heteroarylalkyl,
R6 and R7 may form a saturated or unsaturated ring with 5, 6 or 7 ring members, which may or may not have a hetero atom such as O, S or N and which is optionally substituted with one or more alkyls, halogens,
R8 is an Ra or NRaRb radical,
with the exception of N-(diethyl)-4-phenyl-1H-pyrazole-1-ethylamine,
their racemates, enantiomers and diastereoisomers, and their mixtures, their tautomers and their pharmaceutically acceptable salts, for preparing a medicinal product.

9. A compound as claimed in claim 8, of formula (I) in which: 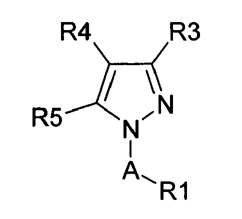 A is, if it is present, a (C1-C6) alkyl radical, a (C3-C6) alkenyl radical, a (C3-C6) alkynyl radical, a (C3-C7) cycloalkyl radical or a (C5-C7) cycloalkenyl radical; these radicals are optionally substituted with one or more substituents chosen from (C1-C5) alkyl, (C2-C5) alkenyl, (C2-C5) alkynyl, (C3-C7) cycloalkyl, (C5-C7) cycloalkenyl, arylalkyl, heteroarylalkyl, aryl, heteroaryl and halogen,
R1 is an NR6R7, (C4-C7) azacycloalkyl, (C5-C7) azacycloalkenyl, (C5-C9) azabicycloalkyl or (C5-C9) azabicycloalkenyl group; these groups are optionally substituted with one or more substituents chosen from (C1-C5) alkyl, (C3-C5) cycloalkyl and halogen,
A-R1 is such that the nitrogen of R1 and the nitrogen in the 1-position of the pyrazole are necessarily separated by at least two carbon atoms,
R3 is an OH, NH₂, OMe or H radical,
R4 is an aryl or heteroaryl radical being optionally substituted with one or more substituents chosen from halogen, CN, NO₂, NH₂, OH, SH, COOH, CHO, C (O) NH₂, C(S)NH₂, SO₂H, SO₂NH₂, NHCHO, C(O)Ra, C (O) ORa, C(O)NRaRb, C (S) NRaRb, S(O)Ra, SO₂Ra, SO₂NRaRb, ORc, SRc, O-C (O) Ra, -O-C(S)Ra, NRaRb, NHC (O) Ra, NHC (S) Ra, NHCONH₂, NHCONRaRb, NHSO₂Ra, aryl, heteroaryl, (C4-C7) heterocycloalkyl, polyfluoroalkyl, trifluoromethyl-sulfanyl, trifluoromethoxy, linear or branched (C1-C6) alkyl, (C2-C6) alkenyl and (C2-C6) alkynyl, these substituents being optionally substituted with one or more alkyl, halogen, OH, methoxy,
R5 is a hydrogen or Me radical,
Ra is linear or branched (C1-C6) alkyl, alkenyl, alkynyl, (C3-C7) cycloalkyl, (C5-C7) cycloalkenyl, (C4-C7) heterocycloalkyl, arylalkyl, heteroarylalkyl, aryl, heteroaryl or polyfluoroalkyl,
Rb is, independently of Ra, a hydrogen, linear or branched (C1-C6) alkyl, alkenyl, alkynyl, (C3-C7) cycloalkyl, (C5-C7) cycloalkenyl, (C4-C7) heterocycloalkyl, arylalkyl, heteroarylalkyl, aryl, heteroaryl or polyfluoroalkyl,
Ra and Rb may form a saturated or unsaturated ring containing 5, 6 or 7 ring members, which may or may not have a hetero atom such as O, S or N, this ring being optionally substituted with one or more alkyl, halogen radicals,
Rc is a linear or branched (C1-C6) alkyl, (C3-C6) alkenyl, (C3-C6) alkynyl, (C3-C7) cycloalkyl, (C5-C7) cycloalkenyl, (C4-C7) heterocycloalkyl, (hetero)-arylalkyl, (hetero)aryl, (poly)fluoroalkyl, C(O)R8, C(S)R8 or SO₂R8 radical,
R6 and R7 are, independently of one another, a hydrogen, (C1-C6) alkyl, (C3-C6) alkenyl, (C3-C6) alkynyl, (C3-C7) cycloalkyl, (C5-C7) cycloalkenyl, (C4-C7) heterocycloalkyl, an arylalkyl or heteroarylalkyl,
R6 and R7 may form a saturated or unsaturated ring with 5, 6 or 7 ring members, which may or may not have a hetero atom such as O, S or N and which is optionally substituted with one or more alkyls, halogens,
R8 is an Ra or NRaRb radical,
with the exception of N-(diethyl)-4-phenyl-1H-pyrazole-1-ethylamine,
their racemates, enantiomers and diastereoisomers and their mixtures, their tautomers and their pharmaceutically acceptable salts, for preparing a medicinal product.

10. A compound as claimed in claim 8, of formula (I)
in which: 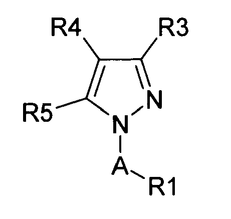 A is, if it is present, a (C1-C6) alkyl radical, a (C3-C6) alkenyl radical, a (C3-C6) alkynyl radical, a (C3-C7) cycloalkyl radical or a (C5-C7) cycloalkenyl radical; these radicals are optionally substituted with one or more substituents chosen from (C1-C5) alkyl, (C2-C5) alkenyl, (C2-C5) alkynyl, (C3-C7) cycloalkyl, (C5-C7) cycloalkenyl, arylalkyl, heteroarylalkyl, aryl, heteroaryl and halogen,
R1 is an NR6R7, (C4-C7) azacycloalkyl, (C5-C7) azacycloalkenyl, (C5-C9) azabicycloalkyl or (C5-C9) azabicycloalkenyl group; these groups are optionally substituted with one or more substituents chosen from (C1-C5) alkyl, (C3-C5) cycloalkyl and halogen,
A-R1 is such that the nitrogen of R1 and the nitrogen in the 1-position of the pyrazole are necessarily separated by at least two carbon atoms,
R3 is an OH, NH₂, OMe or H radical,
R4 is an aryl or heteroaryl radical being optionally substituted with one or more substituents chosen from halogen, CN, NO₂, NH₂, OH, SH, COOH, CHO, C(O)NH₂, C(S)NH₂, SO₂H, SO₂NH₂, NHCHO, C (0) Ra, C (O) ORa, C(O)NRaRb, C(S)NRaRb, S(O)Ra, SO₂Ra, SO₂NRaRb, ORc, SRc, O-C (O) Ra, -O-C(S)Ra, NRaRb, NHC(O)Ra, NHC(S)Ra, NHCONH₂, NHCONRaRb, NHSO₂Ra, aryl, heteroaryl, (C4-C7) heterocycloalkyl, polyfluoroalkyl, trifluoromethyl-sulfanyl, trifluoromethoxy, linear or branched (C1-C6) alkyl, (C2-C6) alkenyl and (C2-C6) alkynyl, these substituents being optionally substituted with one or more alkyl, halogen, OH, methoxy,
R5 is a hydrogen,
Ra is linear or branched (C1-C6) alkyl, alkenyl, alkynyl, (C3-C7) cycloalkyl, (C5-C7) cycloalkenyl, (C4-C7) heterocycloalkyl, arylalkyl, heteroarylalkyl, aryl, heteroaryl or polyfluoroalkyl,
Rb is, independently of Ra, a hydrogen, linear or branched (C1-C6) alkyl, alkenyl, alkynyl, (C3-C7) cycloalkyl, (C5-C7) cycloalkenyl, (C4-C7) heterocycloalkyl, arylalkyl, heteroarylalkyl, aryl, heteroaryl or polyfluoroalkyl,
Ra and Rb may form a saturated or unsaturated ring containing 5, 6 or 7 ring members, which may or may not have a hetero atom such as O, S or N, this ring being optionally substituted with one or more alkyl, halogen radicals,
Rc is a linear or branched (C1-C6) alkyl, (C3-C6) alkenyl, (C3-C6) alkynyl, (C3-C7) cycloalkyl, (C5-C7) cycloalkenyl, (C4-C7) heterocycloalkyl, (hetero)arylalkyl, (hetero)aryl, (poly)fluoroalkyl, C(O)R8, C(S)R8 or SO₂R8 radical,
R6 and R7 are, independently of one another, a hydrogen, (C1-C6) alkyl, (C3-C6) alkenyl, (C3-C6) alkynyl, (C3-C7) cycloalkyl, (C5-C7) cycloalkenyl, (C4-C7) heterocycloalkyl, an arylalkyl or heteroarylalkyl,
R6 and R7 may form a saturated or unsaturated ring with 5, 6 or 7 ring members, which may or may not have a hetero atom such as O, S or N and which is optionally substituted with one or more alkyls, halogens,
R8 is an Ra or NRaRb radical,
with the exception of N-(diethyl)-4-phenyl-1H-pyrazole-1-ethylamine,
their racemates, enantiomers and diastereoisomers and their mixtures, their tautomers and their pharmaceutically acceptable salts, for preparing a medicinal product.

11. The use of at least one compound defined as claimed in any one of claims 1 to 4, for preparing a medicinal product for use in the treatment of diseases due to a dysfunction of α7 nicotinic receptors or responding favorably to a modulation thereof.

12. The use of at least one compound defined as claimed in any one of claims 1 to 4, for preparing a medicinal product for use in the treatment, prevention, diagnosis , monitoring of the evolution of psychiatric disorders or diseases or neurological disorders or diseases of the central nervous system involving an impairment of cognitive functions or of the processing of sensory information.

13. The use as claimed in claim 12, **characterized in that** the disease or the disorder concerns cognitive abilities and attention capacity, and the ability to concentrate, to learn and/or to memorize.

14. The use as claimed in claim 12, **characterized in that** the disease is Alzheimer's disease and related cognitive disorders.

15. The use as claimed in claim 12, **characterized in that** the disease is schizophrenia.

16. The use of at least one compound as claimed in any one of claims 1 to 4, for preparing a medicinal product intended to establish a decrease in the consumption of addictive substances, for helping to maintain an abstinence with respect to said substances or for reducing the symptoms of withdrawal therefrom, and for its therapeutic application in the treatment of acute or chronic neuron degeneration.

## Patentansprüche

1. Verbindung der Formel (I) mit den folgenden Bedeutungen: 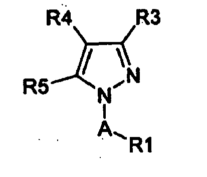 A, falls vorhanden, ist ein (C1-C6)-Alkylrest, ein (C3-C6)-Alkenylrest, ein (C3-C6)-Alkinylrest, ein (C3-C7)-Cycloalkylrest, ein (C5-C7)-Cycloalkenylrest, wobei diese Reste gegebenenfalls durch 1 oder mehrere Substituenten aus der Reihe (C1-C5) -Alkyl, (C2-C5)-Alkenyl, (C2-C5)-Alkinyl, (C3-C7)-Cycloalkyl, (C5-C7)-Cycloalkenyl, Arylalkyl, Heteroarylalkyl, Aryl, Heteroaryl, Halogen substituiert sind,
R1 ist eine NR6R7-, eine (C4-C7)-Azacycloalkyl-, eine (C5-C7)-Azacycloalkenyl-, eine (C5-C9)-Azabicycloalkyl-, eine (C5-C9)-Azabicycloalkenyl-Gruppe, wobei diese Gruppen gegebenenfalls durch 1 oder mehrere Sustituenten aus der Reihe (C1-C5)-Alkyl, (C3-C5)-Cycloalkyl, Halogen substituiert sind,
A-R1 ist derart, daß der Stickstoff von R1 und der Stickstoff 1 des Pyrazols immer durch mindestens zwei Kohlenstoffatome getrennt sind,
R3 ist ein H-, Halogen-, OH-, SH-, NH2-, ORc-, SRc-, SORa-, SO2Ra-, NHCHO-, NRaRb-, NHC(O)Ra-, NHC(S)Ra-, NHSO2Ra-Rest,
R4 ist ein Aryl- oder ein Heteroarylrest, der gegebenenfalls durch 1 oder mehrere Substituenten aus der Reihe Halogen, CN, NO₂, NH₂, OH, SH, COOH, CHO, C(O)NH2, C(S)NH2, SO2H, SO2NH2, NHCHO, C (O) Ra, C (O) ORa, C (O) NRaRb, C(S)NRaRb, S(O)Ra, SO₂Ra, SO₂NRaRb, ORc, SRc, O-C (O) Ra, -O-C (S) Ra, NRaRb, NHC(O)Ra, NHC(S)Ra, NHCONH2, NHCONRaRb, NHSO₂Ra, Aryl, Heteroaryl, (C4-C7)-Heterocycloalkyl, Polyfluoralkyl, Trifluormethylsulfanyl, Trifluormethoxy, geradkettiges oder verzweigtes (C1-C6)-Alkyl, (C2-C6)-Alkenyl, (C2-C6)-Alkinyl substituiert ist, wobei diese Substituenten gegebenfalls durch einen oder mehrere Reste Alkyl, Halogen, OH, Methoxy substituiert sind,
R5 ist ein H-, Halogen-, CF3-, CHF2-, CH2F-, geradkettiger oder verzweigter (C1-C6)-Alkyl-, (C3-C7)-Cycloalkylrest,
Ra ist geradkettiges oder verzweigtes (C1-C6)-Alkyl, Alkenyl, Alkinyl, (C3-C7)-Cycloalkyl, (C5-C7)-Cycloalkenyl, (C4-C7)-Heterocycloalkyl, Arylalkyl, Heteroarylalkyl, Aryl, Heteroaryl, Polyfluoralkyl,
Rb ist unabhängig von Ra Wasserstoff, geradkettiges oder verzweigtes (C1-C6)-Alkyl, Alkenyl, Alkinyl, (C3-C7)-Cycloalkyl, (C5-C7)-Cycloalkenyl, (C4-C7)-Heterocycloalkyl, Arylalkyl, Heteroarylalkyl, Aryl, Heteroaryl, Polyfluoralkyl,
Ra und Rb können einen gesättigten oder ungesättigten Ring mit 5, 6 oder 7 Ringgliedern mit oder ohne Heteroatom wie O, S, N bilden, wobei dieser Ring gegebenenfalls durch einen oder mehrere Alkyl- oder Halogenreste substituiert ist,
Rc ist ein geradkettiger oder verzweigter (C1-C6)-Alkyl-, (C3-C6)-Alkenyl-, (C3-C6)-Alkinyl-, (C3-C7)-Cycloalkyl-, (C5-C7)-Cycloalkenyl-, (C4-C7)-Heterocycloalkyl-, (Hetero)arylalkyl-, (Hetero)aryl-, (Poly)fluoralkyl-, C(O)R8-, C(S)R8-, SO2R8-Rest,
R6 und R7 bedeuten unabhängig voneinander Wasserstoff, (C1-C6)-Alkyl, (C3-C6)-Alkenyl, (C3-C6)-Alkinyl, (C3-C7)-Cycloalkyl, (C5-C7)-Cycloalkenyl, (C4-C7)-Heterocycloalkyl, ein Arylalkyl, Heteroarylalkyl,
R6 und R7 können einen gesättigten oder ungesättigten Ring mit 5, 6 oder 7 Ringgliedern mit oder ohne Heteroatom wie O, S, N bilden, wobei dieser Ring gegebenenfalls durch ein oder mehrere Alkyl- oder Halogenreste substituiert ist,
R8 ist ein Ra- oder NRaRb-Rest,
ihre racemischen Mischungen, Enantiomere, Diastereoisomere und ihre Mischungen, ihre Tautomere und ihre pharmazeutisch annehmbaren Salze mit Ausnahme von 3-(3-Pyridinyl)-1H-pyrazol-1-butanamin, von 4-(3-Pyridinyl)-1H-pyrazol-1-butanamin und von N-(Diethyl)-4-phenyl-1H-pyrazol-1-ethylamin.

2. Verbindung der Formel (I) nach Anspruch 1, mit den folgenden Bedeutungen: 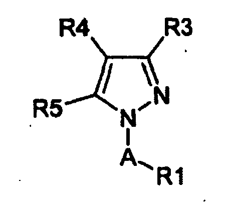 A, falls vorhanden, ist ein (C1-C6)-Alkylrest, ein (C3-C6)-Alkenylrest, ein (C3-C6)-Alkinylrest, ein (C3-C7)-Cycloalkylrest, ein (C5-C7)-Cycloalkenylrest, wobei diese Reste gegebenenfalls durch 1 oder mehrere Substituenten aus der Reihe (C1-C5)-Alkyl, (C2-C5)-Alkenyl, (C2-C5)-Alkinyl, (C3-C7)-Cycloalkyl, (C5-C7)-Cycloalkenyl, Arylalkyl, Heteroarylalkyl, Aryl, Heteroaryl, Halogen substituiert sind,
R1 ist eine NR6R7-, eine (C4-C7)-Azacycloalkyl-, eine (C5-C7)-Azacycloalkenyl-, eine (C5-C9)-Azabicycloalkyl-, eine (C5-C9)-Azabicycloalkenyl-Gruppe, wobei diese Gruppen gegebenenfalls durch 1 oder mehrere Sustituenten aus der Reihe (C1-C5)-Alkyl, (C3-C5)-Cycloalkyl, Halogen substituiert sind,
A-R1 ist derart, daß der Stickstoff von R1 und der Stickstoff 1 des Pyrazols immer durch mindestens zwei Kohlenstoffatome getrennt sind,
R3 ist ein OH-, NH2-, OMe-, H-Rest,
R4 ist ein Aryl- oder ein Heteroarylrest, der gegebenenfalls durch 1 oder mehrere Substituenten aus der Reihe Halogen, CN, NO₂, NH₂, OH, SH, COOH, CHO, C(O)NH2, C(S)NH2, SO2H, SO2NH2, NHCHO, C (O) Ra, C(O)ORa, C (O) NRaRb, C(S)NRaRb, S (O) Ra, SO₂Ra, SO₂NRaRb, ORc, SRc, O-C (O) Ra, -O-C (S) Ra, NRaRb, NHC(O)Ra, NHC(S)Ra, NHCONH2, NHCONRaRb, NHSO₂Ra, Aryl, Heteroaryl, (C4-C7)-Heterocycloalkyl, Polyfluoralkyl, Trifluormethylsulfanyl, Trifluormethoxy, geradkettiges oder verzweigtes (C1-C6)-Alkyl, (C2-C6)-Alkenyl, (C2-C6)-Alkinyl substituiert ist, wobei diese Substituenten gegebenfalls durch einen oder mehrere Reste Alkyl, Halogen, OH, Methoxy substituiert sind,
R5 ist ein Wasserstoff- oder Me-Rest,
Ra ist geradkettiges oder verzweigtes (C1-C6)-Alkyl, Alkenyl, Alkinyl, (C3-C7)-Cycloalkyl, (C5-C7)-Cycloalkenyl, (C4-C7)-Heterocycloalkyl, Arylalkyl, Heteroarylalkyl, Aryl, Heteroaryl, Polyfluoralkyl,
Rb ist unabhängig von Ra Wasserstoff, geradkettiges oder verzweigtes (C1-C6)-Alkyl, Alkenyl, Alkinyl, (C3-C7)-Cycloalkyl, (C5-C7)-Cycloalkenyl, (C4-C7)-Heterocycloalkyl, Arylalkyl, Heteroarylalkyl, Aryl, Heteroaryl, Polyfluoralkyl,
Ra und Rb können einen gesättigten oder ungesättigten Ring mit 5, 6 oder 7 Ringgliedern mit oder ohne Heteroatom wie O, S, N bilden, wobei dieser Ring gegebenenfalls durch einen oder mehrere Alkyl- oder Halogenreste substituiert ist,
Rc ist ein geradkettiger oder verzweigter (C1-C6)-Alkyl-, (C3-C6)-Alkenyl-, (C3-C6)-Alkinyl-, (C3-C7)-Cycloalkyl-, (C5-C7)-Cycloalkenyl-, (C4-C7)-Heterocycloalkyl-, (Hetero)arylalkyl-, (Hetero)aryl-, (Poly)fluoralkyl-, C(O)R8-, C(S)R8-, SO2R8-Rest,
R6 und R7 bedeuten unabhängig voneinander Wasserstoff, (C1-C6)-Alkyl, (C3-C6)-Alkenyl, (C3-C6)-Alkinyl, (C3-C7)-Cycloalkyl, (C5-C7)-Cycloalkenyl, (C4-C7)-Heterocycloalkyl, Arylalkyl, Heteroarylalkyl,
R6 und R7 können einen gesättigten oder ungesättigten Ring mit 5, 6 oder 7 Ringgliedern mit oder ohne Heteroatom wie O, S, N bilden, wobei dieser Ring gegebenenfalls durch ein oder mehrere Alkyl- oder Halogenreste substituiert ist,
R8 ist ein Ra- oder NRaRb-Rest,
ihre racemischen Mischungen, Enantiomere, Diastereoisomere und ihre Mischungen, ihre Tautomere und ihre pharmazeutisch annehmbaren Salze mit Ausnahme von 3-(3-Pyridinyl)-1H-pyrazol-1-butanamin, von 4-(3-Pyridinyl)-1H-pyrazol-1-butanamin und von N-(Diethyl)-4-phenyl-1H-pyrazol-1-ethylamin.

3. Verbindung der Formel (I) nach Anspruch 1, mit den folgenden Bedeutungen: 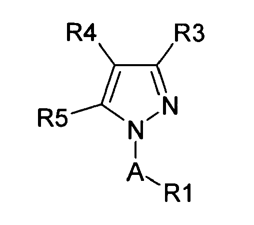 A, falls vorhanden, ist ein (C1-C6)-Alkylrest, ein (C3-C6)-Alkenylrest, ein (C3-C6)-Alkinylrest, ein (C3-C7)-Cycloalkylrest, ein (C5-C7)-Cycloalkenylrest, wobei diese Reste gegebenenfalls durch 1 oder mehrere Substituenten aus der Reihe (C1-C5) -Alkyl, (C2-C5)-Alkenyl, (C2-C5)-Alkinyl, (C3-C7)-Cycloalkyl, (C5-C7)-Cycloalkenyl, Arylalkyl, Heteroarylalkyl, Aryl, Heteroaryl, Halogen substituiert sind,
R1 ist eine NR6R7-, eine (C4-C7)-Azacycloalkyl-, eine (C5-C7)-Azacycloalkenyl-, eine (C5-C9)-Azabicycloalkyl-, eine (C5-C9)-Azabicycloalkenyl-Gruppe, wobei diese Gruppen gegebenenfalls durch 1 oder mehrere Sustituenten aus der Reihe (C1-C5)-Alkyl, (C3-C5)-Cycloalkyl, Halogen substituiert sind,
A-R1 ist derart, daß der Stickstoff von R1 und der Stickstoff 1 des Pyrazols immer durch mindestens zwei Kohlenstoffatome getrennt sind,
R3 ist ein OH-, NH2-, OMe-, H-Rest,
R4 ist ein Aryl- oder ein Heteroarylrest, der gegebenenfalls durch 1 oder mehrere Substituenten aus der Reihe Halogen, CN, NO₂, NH₂, OH, SH, COOH, CHO, C(O)NH2, C(S)NH2, SO2H, SO2NH2, NHCHO, C (O) Ra, C (O) ORa, C (O) NRaRb, C(S)NRaRb, S(O)Ra, SO₂Ra, SO₂NRaRb, ORc, SRc, O-C(O)Ra, -O-C(S)Ra, NRaRb, NHC(O)Ra, NHC(S)Ra, NHCONH2, NHCONRaRb, NHSO₂Ra, Aryl, Heteroaryl, (C4-C7)-Heterocycloalkyl, Polyfluoralkyl, Trifluormethylsulfanyl, Trifluormethoxy, geradkettiges oder verzweigtes (C1-C6)-Alkyl, (C2-C6)-Alkenyl, (C2-C6)-Alkinyl substituiert ist, wobei diese Substituenten gegebenfalls durch einen oder mehrere Reste Alkyl, Halogen, OH, Methoxy substituiert sind,
R5 ist Wasserstoff,
Ra ist geradkettiges oder verzweigtes (C1-C6)-Alkyl, Alkenyl, Alkinyl, (C3-C7)-Cycloalkyl, (C5-C7)-Cycloalkenyl, (C4-C7)-Heterocycloalkyl, Arylalkyl, Heteroarylalkyl, Aryl, Heteroaryl, Polyfluoralkyl,
Rb ist unabhängig von Ra Wasserstoff, geradkettiges oder verzweigtes (C1-C6)-Alkyl, Alkenyl, Alkinyl, (C3-C7)-Cycloalkyl, (C5-C7)-Cycloalkenyl, (C4-C7)-Heterocycloalkyl, Arylalkyl, Heteroarylalkyl, Aryl, Heteroaryl, Polyfluoralkyl,
Ra und Rb können einen gesättigten oder ungesättigten Ring mit 5, 6 oder 7 Ringgliedern mit oder ohne Heteroatom wie O, S, N bilden, wobei dieser Ring gegebenenfalls durch einen oder mehrere Alkyl- oder Halogenreste substituiert ist,
Rc ist ein geradkettiger oder verzweigter (C1-C6)-Alkyl-, (C3-C6)-Alkenyl-, (C3-C6)-Alkinyl-, (C3-C7)-Cycloalkyl-, (C5-C7)-Cycloalkenyl-, (C4-C7)-Heterocycloalkyl-, (Hetero)arylalkyl-, (Hetero)-aryl-, (Poly)fluoralkyl-, C(O)R8-, C(S)R8-, SO2R8-Rest,
R6 und R7 bedeuten unabhängig voneinander Wasserstoff, (C1-C6)-Alkyl, (C3-C6)-Alkenyl, (C3-C6)-Alkinyl, (C3-C7)-Cycloalkyl, (C5-C7)-Cycloalkenyl, (C4-C7)-Heterocycloalkyl, Arylalkyl, Heteroarylalkyl,
R6 und R7 können einen gesättigten oder ungesättigten Ring mit 5, 6 oder 7 Ringgliedern mit oder ohne Heteroatom wie O, S, N bilden, wobei dieser Ring gegebenenfalls durch einen oder mehrere Alkyl- oder Halogenreste substituiert ist,
R8 ist ein Ra- oder NRaRb-Rest,
ihre racemischen Mischungen, Enantiomere, Diastereoisomere und ihre Mischungen, ihre Tautomere und ihre pharmazeutisch annehmbaren Salze mit Ausnahme von 3-(3-Pyridinyl)-1H-pyrazol-1-butanamin, von 4-(3-Pyridinyl)-1H-pyrazol-1-butanamin und von N-(Diethyl)-4-phenyl-1H-pyrazol-1-ethylamin.

4. Verbindung nach Anspruch 1, **dadurch gekennzeichnet, daß** sie aus der folgenden Reihe stammt:
1-[2-(3-Methoxy-4-phenylpyrazol-1-yl)-ethyl]-piperidin
1-(1-Azabicyclo[2.2.2]oct-3-ylmethyl)-4-phenyl-1H-pyrazol-1-ol
3-(3-Benzyloxy-4-phenylpyrazol-1-ylmethyl)-1-azabicyclo[2.2.2]octan
3-(3-Methoxy-4-phenylpyrazol-1-ylmethyl)-1-azabicyclo[2.2.2]octan
1-(1-Azabicyclo[2.2.2]oct-3-yl)-4-phenyl-1H-pyrazol-3-ol
1-(2-Perhydroazepin-1-ylethyl)-4-phenyl-1H-pyrazol-3-ol
1-[2-(2-Methylpiperidin-1-yl)ethyl]-4-phenyl-1H-pyrazol-3-ol
1-[2-(4-Fluorpiperidin-1-yl)ethyl]-4-phenyl-1H-pyrazol-3-ol
1-[2-(3-Methylpiperidin-1-yl)ethyl]-4-phenyl-1H-pyrazol-3-ol
1-[2-(3,6-Dihydro-2H-pyridin-1-yl)ethyl]-4-phenyl-1H-pyrazol-3-ol
1-[2-(7-Azabicyclo[2.2.1]hept-7-yl)ethyl]-4-phenyl-1H-pyrazol-3-ol
1-[2-(2-Azabicyclo[2.2.2]oct-2-yl)ethyl]-4-phenyl-1H-pyrazol-3-ol
1-[2-(2-Azabicyclo[2.2.1]hept-2-yl)ethyl]-4-phenyl-1H-pyrazol-3-ol
1-[2-Dimethylaminoethyl]-4-phenyl-1H-pyrazol-3-ol
1-[3-Dimethylaminopropyl]-4-phenyl-1H-pyrazol-3-ol
1-[2-((2S,6R)-2,6-Dimethylpiperidin-1-yl)ethyl]-4-phenyl-1H-pyrazol-3-ol
1-[2-Diethylaminoethyl]-4-phenyl-1H-pyrazol-3-ol
1-(2-Diisopropylaminoethyl)-4-phenyl-1H-pyrazol-3-ol
4-Phenyl-1-(2-pyrrolidin-1-ylethyl)-1H-pyrazol-3-ol
3-(3-Methoxy-4-phenylpyrazol-1-yl)-1-azabicyclo-[2.2.2]octan
1-[2-(3-Difluormethoxy-4-phenylpyrazol-1-yl)ethyl]piperidin
4-Phenyl-1-(2-piperidin-1-ylethyl)-1H-pyrazol-3-ylamin
4-Phenyl-1-(2-piperidin-1-ylethyl)-1H-pyrazol-3-ylamin
N-[4-Phenyl-1-(2-piperidin-1-ylethyl)-1H-pyrazol-3-yl]acetamid
N-[4-Phenyl-1-(2-piperidin-1-ylethyl)-1H-pyrazol-3-yl]methanesulfonamid
1-(2-Dimethylaminopropyl)-4-phenyl-1H-pyrazol-3-ol
1-(1-Methylpiperidin-3-ylmethyl)-4-phenyl-1H-pyrazol-3-ol
5-Methyl-4-phenyl-1-(2-piperidin-1-ylethyl)-1H-pyrazol-3-ol
4-(3-Aminophenyl)-1-(2-dimethylaminoethyl)-1H-pyrazol-3-ol
N-{3-[3-Hydroxy-1-(2-dimethylaminoethyl)-1H-pyrazol-4-yl]phenyl}acetamid
4-(4-Aminophenyl)-1-(2-dimethylaminoethyl)-1H-pyrazol-3-ol
1-(2-Dimethylaminoethyl)-4-(4'-fluorbiphenyl-3-yl)-1H-pyrazol-3-ol
4-Biphenyl-3-yl-1-(2-dimethylaminoethyl)-1H-pyrazol-3-ol
1-(2-Dimethylaminoethyl)-4-(4'-fluorbiphenyl-4-yl)-1H-pyrazol-3-ol
1-(2-Piperidin-1-ylethyl)-4-pyridin-2-yl-1H-pyrazol-3-ol
1-(2-Piperidin-1-ylethyl)-4-pyridin-4-yl-1H-pyrazol-3-ol
4-(4-Fluorphenyl)-1-(2-piperidin-1-ylethyl)-1H-pyrazol-3-ol
4-(4-Trifluormethoxyphenyl)-1-(2-piperidin-1-ylethyl)-1H-pyrazol-3-ol
4-Phenyl-1-(2-piperidin-1-ylpropyl)-1H-pyrazol-3-ol
3-(4-Phenylpyrazol-1-ylmethyl)-1-azabicyclo[2.2.2] octan
4-(5-Chlorthiophen-2-yl)-1-(2-piperidin-1-ylethyl)-1H-pyrazol-3-ol
4-(3-Methoxyphenyl)-1-(2-piperidin-1-ylethyl)-1H-pyrazol-3-ol
4-(2-Methoxyphenyl)-1-(2-piperidin-1-ylethyl)-1H-pyrazol-3-ol
4-(3-Hydroxyphenyl)-1-(2-piperidin-1-ylethyl)-1H-pyrazol-3-ol
4-(4-Hydroxyphenyl)-1-(2-piperidin-1-ylethyl)-1H-pyrazol-3-ol
4-(4-Methoxyphenyl)-1-(2-piperidin-1-ylethyl)-1H-pyrazol-3-ol
4-(3-Fluorphenyl)-1-(2-piperidin-1-ylethyl)-1H-pyrazol-3-ol
1-(2-Piperidin-1-ylethyl)-4-(3-trifluormethylphenyl)-1H-pyrazol-3-ol
1-(2-Piperidin-1-ylethyl)-4-pyridin-3-yl-1H-pyrazol-3-ol
4-(4-Chlorphenyl)-1-(2-piperidin-1-ylethyl)-1H-pyrazol-3-ol
4-(3-Chlorphenyl)-1-(2-piperidin-1-ylethyl)-1H-pyrazol-3-ol
4-(2-Fluorphenyl)-1-(2-piperidin-1-ylethyl)-1H-pyrazol-3-ol
4-(2-Chlorphenyl)-1-(2-piperidin-1-ylethyl)-1H-pyrazol-3-ol
1-(1-Azabicyclo[2.2.2]oct-3-yl)-4-(4-chlorphenyl)-1H-pyrazol-3-ol
1-(1-Azabicyclo[2.2.2]oct-3-yl)-4-(3-chlorphenyl)-1H-pyrazol-3-ol
1-(1-Azabicyclo[2.2.2]oct-3-yl)-4-(3-fluorphenyl)-1H-pyrazol-3-ol
1-(1-Methylpyrrolidin-3-yl)-4-phenyl-1H-pyrazol-3-ol
1-[2-(1-Methylpyrrolidin-2-yl)ethyl]-4-phenyl-1H-pyrazol-3-ol
1-(Pyrrolidin-3-yl)-4-phenyl-1H-pyrazol-3-ol
1-[(1-Methylpyrrolidin-2-(S)-yl)methyl]-4-phenyl-1H-pyrazol-3-ol
4-Phenyl-1-pyrrolidin-3-ylmethyl-1H-pyrazol-3-ol
1-((2R)-1-Methylpyrrolidin-2-ylmethyl)-4-phenyl-1H-pyrazol-3-ol
4-Phenyl-1-(piperidin-3-yl)-1H-pyrazol-3-ol
1-(1-Methylpiperidin-2-ylmethyl)-4-phenyl-1H-pyrazol-3-ol
1-(1-Methylazepan-3-yl)-4-phenyl-1H-pyrazol-3-ol
4-Phenyl-1-(2-piperidin-1-ylethyl)-1H-pyrazol-3-ol
4-(Thiophen-2-yl)-1-(2-piperidin-1-ylethyl)-1H-pyrazol-3-ol
4-(3,4-Dichlorphenyl)-1-(2-piperidin-1-ylethyl)-1H-pyrazol-3-ol
4-(4-Bromphenyl)-1-(2-piperidin-1-ylethyl)-1H-pyrazol-3-ol
4-(1H-Indol-5-yl)-1-(2-piperidin-1-ylethyl)-1H-pyrazol-3-ol
4-(5-Bromthiophen-2-yl)-1-(2-piperidin-1-ylethyl)-1H-pyrazol-3-ol
2-[1-(2-Piperidin-1-ylethyl)-1H-pyrazol-4-yl]benzamid
4-(2-Hydroxyphenyl)-1-(2-piperidin-1-ylethyl)-1H-pyrazol
4-(1H-Indol-5-yl)-1-(2-piperidin-1-ylethyl)-1H-pyrazol
4-(4-Methylphenyl)-1-(2-piperidin-1-ylethyl)-1H-pyrazol-3-ol
1-(1-Azabicyclo[2.2.2]oct-3-yl)-4-(1H-indol-5-yl)-1H-pyrazol
(+)-1-(1-Azabicyclo[2.2.2]oct-3-yl)-4-(1H-indol-5-yl)-1H-pyrazol
(-)-1-(1-Azabicyclo[2.2.2]oct-3-yl)-4-(1H-indol-5-yl)-1H-pyrazol
1-(1-Azabicyclo[2.2.2]oct-3-yl)-4-(5-chlorthiophen-2-yl)-1H-pyrazol-3-ol
1-(1-Azabicyclo[2.2.2]oct-3-yl)-4-(5-chlorthiophen-2-yl)-1H-pyrazol-3-ol
1-(1-Azabicyclo[2.2.2]oct-2-ylmethyl)-4-phenyl-1H-pyrazol-3-ol
3-[4-(3,5-Difluorphenyl)pyrazol-1-yl]-1-azabicyclo[2.2.2]octan
4-Benzo[b]thiophen-2-yl-1-(2-piperidin-1-ylethyl)-1H-pyrazol-3-ol
1-(2-Piperidin-1-ylethyl)-4-thiophen-3-yl-1H-pyrazol-3-ol
4-[3-Hydroxy-1-(2-piperidin-1-ylethyl)-1H-pyrazol-4-yl]benzamid
3-[3-Hydroxy-1-(2-piperidin-1-ylethyl)-1H-pyrazol-4-yl]benzamid
(-)-1-(1-Azabicyclo[2.2.2]oct-3-yl)-4-phenyl-1H-pyrazol-3-ol
(+)-1-(1-Azabicyclo[2.2.2]oct-3-yl)-4-phenyl-1H-pyrazol-3-ol
(-)-1-(1-Azabicyclo[2.2.2]oct-3-ylmethyl)-4-phenyl-1H-pyrazol-1-ol
(+)-1-(1-Azabicyclo[2.2.2]oct-3-ylmethyl)-4-phenyl-1H-pyrazol-1-ol
1-(1-Azabicyclo[2.2.2]oct-3-ylmethyl)-4-(4-chlorphenyl)-1H-pyrazol-3-ol
(-)-1-(1-Azabicyclo[2.2.2]oct-3-yl)-4-(4-chlorphenyl)-1H-pyrazol-3-ol
(+)-1-(1-Azabicyclo[2.2.2]oct-3-yl)-4-(4-chlorphenyl)-1H-pyrazol-3-ol
(-)-1-(1-Azabicyclo[2.2.2]oct-3-yl)-4-(4-fluorphenyl)-1H-pyrazol-3-ol
(+)-1-(1-Azabicyclo[2.2.2]oct-3-yl)-4-(4-fluorphenyl)-1H-pyrazol-3-ol
3-[4-(4-Chlorphenyl)pyrazol-1-yl]-1-azabicyclo-[2.2.2]octan
3-[4-(4-Chlorphenyl)pyrazol-1-ylmethyl]-1-azabicyclo[2.2.2]octan
3-[4-(3-Chlor-4-methoxyphenyl)pyrazol-1-ylmethyl]-1-azabicyclo[2.2.2]octan
4-[1-(1-Azabicyclo[2.2.2]oct-3-ylmethyl)-1H-pyrazol-4-yl]-2-chlorphenol
4-[1-(1-Azabicyclo[2.2.2]oct-3-yl)-1H-pyrazol-4-yl]-2-chlorphenol
(-)-4-[1-(1-Azabicyclo[2.2.2]oct-3-yl)-1H-pyrazol-4-yl]-2-chlorphenol
(+)-4-[1-(1-Azabicyclo[2.2.2]oct-3-yl)-1H-pyrazol-4-yl]-2-chlorphenol
(+)-1-(1-Azabicyclo[2.2.2]oct-3-yl)-4-pyridin-2-yl-1H-pyrazol-3-ol
(-)-1-(1-Azabicyclo[2.2.2]oct-3-yl)-4-pyridin-2-yl-1H-pyrazol-3-ol
(+)-1-(1-Azabicyclo[2.2.2]oct-3-yl)-4-phenyl-1H-pyrazol-3-ylamin
(-)-1-(1-Azabicyclo[2.2.2]oct-3-yl)-4-phenyl-1H-pyrazol-3-ylamin
(+)-1-(1-Azabicyclo[2.2.2]oct-3-yl)-4-(1H-indol-4-yl)-1H-pyrazol
(-)-1-(1-Azabicyclo[2.2.2]oct-3-yl)-4-(1H-indol-4-yl)-1H-pyrazol,
ihre racemischen Mischungen, Enantiomere, Diastereoisomere und ihre Mischungen, ihre Tautomere und ihre pharmazeutisch annehmbaren Salze.

5. Verfahren zur Herstellung einer Verbindung der Formel (I) nach Anspruch 1, bei der R3 OH bedeutet, ausgehend von der Verbindung (II) 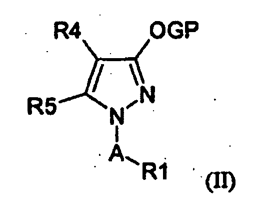 in der GP eine Schutzgruppe der Hydroxylfunktion bedeutet, **dadurch gekennzeichnet, daß** die Verbindung (II) entschützt und anschließend gegebenenfalls in ein pharmazeutisch annehmbares Salz überführt wird.

6. Verfahren zur Herstellung einer Verbindung der Formel (I) nach Anspruch 1, bei der R3 ORc, H, NH2 bedeutet, durch Alkylierung der Pyrazole der Formel (III) 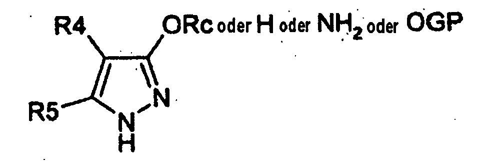 in der GP eine Schutzgruppe der OH-Funktion ist, mit einer Verbindung der Formel (IV) R1-A-X, in der X Cl, Br, I, OTs, OMs, OTf bedeutet, unter basischen Bedingungen in einem aprotischen Lösungsmittel und gegebenenfalls Entschützen und gegebenenfalls Überführen des erhaltenen Produkts in ein pharmazeutisch annehmbares Salz.

7. Pharmazeutische Zusammensetzung, enthaltend mindestens eine Verbindung nach einem der Ansprüche 1-4 in einem pharmazeutisch annehmbaren Medium.

8. Verbindung der Formel (I), mit den folgenden Bedeutungen: 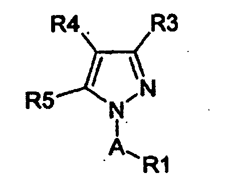 A, falls vorhanden, ist ein (C1-C6) -Alkylrest, ein (C3-C6)-Alkenylrest, ein (C3-C6)-Alkinylrest, ein (C3-C7)-Cycloalkylrest, ein (C5-C7)-Cycloalkenylrest, wobei diese Reste gegebenenfalls durch 1 oder mehrere Substituenten aus der Reihe (C1-C5)-Alkyl, (C2-C5)-Alkenyl, (C2-C5)-Alkinyl, (C3-C7)-Cycloalkyl, (C5-C7)-Cycloalkenyl, Arylalkyl, Heteroarylalkyl, Aryl, Heteroaryl, Halogen substituiert sind,
R1 ist eine NR6R7-, eine (C4-C7)-Azacycloalkyl-, eine (C5-C7)-Azacycloalkenyl-, eine (C5-C9)-Azabicycloalkyl-, eine (C5-C9)-Azabicycloalkenyl-Gruppe, wobei diese Gruppen gegebenenfalls durch 1 oder mehrere Sustituenten aus der Reihe (C1-C5)-Alkyl, (C3-C5)-Cycloalkyl, Halogen substituiert sind,
A-R1 ist derart, daß der Stickstoff von R1 und der Stickstoff 1 des Pyrazols immer durch mindestens zwei Kohlenstoffatome getrennt sind,
R3 ist ein H-, Halogen-, OH-, SH-, NH2-, ORc-, SRc-, SORa-, SO2Ra-, NHCHO-, NRaRb-, NHC(O)Ra-, NHC(S)Ra-, NHSO2Ra-Rest,
R4 ist ein Aryl- oder ein Heteroarylrest, der gegebenenfalls durch 1 oder mehrere Substituenten aus der Reihe Halogen, CN, NO₂, NH₂, OH, SH, COOH, CHO, C(O)NH2, C(S)NH2, SO2H, SO2NH2, NHCHO, C(O)Ra, C(O)ORa, C (O) NRaRb, C(S)NRaRb, S (O) Ra, SO₂Ra, SO₂NRaRb, ORc, SRc, O-C(O)Ra, -O-C (S) Ra, NRaRb, NHC(O)Ra, NHC(S)Ra, NHCONH2, NHCONRaRb, NHSO₂Ra, Aryl, Heteroaryl, (C4-C7)-Heterocycloalkyl, Polyfluoralkyl, Trifluormethylsulfanyl, Trifluormethoxy, geradkettiges oder verzweigtes (C1-C6)-Alkyl, (C2-C6)-Alkenyl, (C2-C6)-Alkinyl substituiert ist, wobei diese Substituenten gegebenfalls durch einen oder mehrere Reste Alkyl, Halogen, OH, Methoxy substituiert sind,
R5 ist ein H-, Halogen-, CF3-, CHF2-, CH2F-, geradkettiger oder verzweigter (C1-C6)-Alkyl-, (C3-C7)-Cycloalkyl-Rest,
Ra ist geradkettiges oder verzweigtes (C1-C6)-Alkyl, Alkenyl, Alkinyl, (C3-C7)-Cycloalkyl, (C5-C7)-Cycloalkenyl, (C4-C7)-Heterocycloalkyl, Arylalkyl, Heteroarylalkyl, Aryl, Heteroaryl, Polyfluoralkyl,
Rb ist unabhängig von Ra Wasserstoff, geradkettiges oder verzweigtes (C1-C6)-Alkyl, Alkenyl, Alkinyl, (C3-C7)-Cycloalkyl, (C5-C7)-Cycloalkenyl, (C4-C7)-Heterocycloalkyl, Arylalkyl, Heteroarylalkyl, Aryl, Heteroaryl, Polyfluoralkyl,
Ra und Rb können einen gesättigten oder ungesättigten Ring mit 5, 6 oder 7 Ringgliedern mit oder ohne Heteroatom wie O, S, N bilden, wobei dieser Ring gegebenenfalls durch einen oder mehrere Alkyl- oder Halogenreste substituiert ist,
Rc ist ein geradkettiger oder verzweigter (C1-C6)-Alkyl-, (C3-C6)-Alkenyl-, (C3-C6)-Alkinyl-, (C3-C7)-Cycloalkyl-, (C5-C7)-Cycloalkenyl-, (C4-C7)-Heterocycloalkyl-, (Hetero)arylalkyl-, (Hetero)aryl-, (Poly)fluoralkyl-, C(O)R8-, C(S)R8-, SO2R8-Rest,
R6 und R7 bedeuten unabhängig voneinander Wasserstoff, (C1-C6)-Alkyl, (C3-C6)-Alkenyl, (C3-C6)-Alkinyl, (C3-C7)-Cycloalkyl, (C5-C7)-Cycloalkenyl, (C4-C7)-Heterocycloalkyl, Arylalkyl, Heteroarylalkyl,
R6 und R7 können einen gesättigten oder ungesättigten Ring mit 5, 6 oder 7 Ringgliedern mit oder ohne Heteroatom wie O, S, N bilden, wobei dieser Ring gegebenenfalls durch einen oder mehrere Alkyl- oder Halogenreste substituiert ist,
R8 ist ein Ra- oder NRaRb-Rest,
mit Ausnahme von N-(Diethyl)-4-phenyl-1H-pyrazol-1-ethylamin,
ihre racemischen Mischungen, Enantiomere, Diastereoisomere und ihre Mischungen, ihre Tautomere und ihre pharmazeutisch annehmbaren Salze für die Herstellung eines Arzneimittels.

9. Verbindung nach Anspruch 8, der Formel (I), mit den folgenden Bedeutungen: 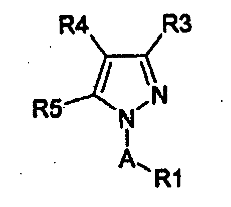 A, falls vorhanden, ist ein (C1-C6)-Alkylrest, ein (C3-C6)-Alkenylrest, ein (C3-C6)-Alkinylrest, ein (C3-C7)-Cycloalkylrest, ein (C5-C7)-Cycloalkenylrest, wobei diese Reste gegebenenfalls durch 1 oder mehrere Substituenten aus der Reihe (C1-C5)-Alkyl, (C2-C5)-Alkenyl, (C2-C5)-Alkinyl, (C3-C7)-Cycloalkyl, (C5-C7)-Cycloalkenyl, Arylalkyl, Heteroarylalkyl, Aryl, Heteroaryl, Halogen substituiert sind,
R1 ist eine NR6R7-, eine (C4-C7)-Azacycloalkyl-, eine (C5-C7)-Azacycloalkenyl-, eine (C5-C9)-Azabicycloalkyl-, eine (C5-C9)-Azabicycloalkenyl-Gruppe, wobei diese Gruppen gegebenenfalls durch 1 oder mehrere Sustituenten aus der Reihe (C1-C5)-Alkyl, (C3-C5)-Cycloalkyl, Halogen substituiert sind,
A-R1 ist derart, daß der Stickstoff von R1 und der Stickstoff 1 des Pyrazols immer durch mindestens zwei Kohlenstoffatome getrennt sind,
R3 ist ein OH-, NH2-, OMe-, H-Rest,
R4 ist ein Aryl- oder ein Heteroarylrest, der gegebenenfalls durch 1 oder mehrere Substituenten aus der Reihe Halogen, CN, NO₂, NH₂, OH, SH, COOH, CHO, C(O)NH2, C(S)NH2, SO2H, SO2NH2, NHCHO, C(O)Ra, C (O) ORa, C (O) NRaRb, C(S)NRaRb, S (O) Ra, SO₂Ra, SO₂NRaRb, ORc, SRc, O-C (O) Ra, -O-C (S) Ra, NRaRb, NHC(O)Ra, NHC(S)Ra, NHCONH2, NHCONRaRb, NHSO₂Ra, Aryl, Heteroaryl, (C4-C7)-Heterocycloalkyl, Polyfluoralkyl, Trifluormethylsulfanyl, Trifluormethoxy, geradkettiges oder verzweigtes (C1-C6)-Alkyl, (C2-C6)-Alkenyl, (C2-C6)-Alkinyl substituiert ist, wobei diese Substituenten gegebenfalls durch einen oder mehrere Reste Alkyl, Halogen, OH, Methoxy substituiert sind,
R5 ist ein Wasserstoff- oder Me-Rest,
Ra ist geradkettiges oder verzweigtes (C1-C6)-Alkyl, Alkenyl, Alkinyl, (C3-C7)-Cycloalkyl, (C5-C7)-Cycloalkenyl, (C4-C7)-Heterocycloalkyl, Arylalkyl, Heteroarylalkyl, Aryl, Heteroaryl, Polyfluoralkyl,
Rb ist unabhängig von Ra Wasserstoff, geradkettiges oder verzweigtes (C1-C6)-Alkyl, Alkenyl, Alkinyl, (C3-C7)-Cycloalkyl, (C5-C7)-Cycloalkenyl, (C4-C7)-Heterocycloalkyl, Arylalkyl, Heteroarylalkyl, Aryl, Heteroaryl, Polyfluoralkyl,
Ra und Rb können einen gesättigten oder ungesättigten Ring mit 5, 6 oder 7 Ringgliedern mit oder ohne Heteroatom wie O, S, N bilden, wobei dieser Ring gegebenenfalls durch einen oder mehrere Alkyl- oder Halogenreste substituiert ist,
Rc ist ein geradkettiger oder verzweigter (C1-C6)-Alkyl-, (C3-C6)-Alkenyl-, (C3-C6)-Alkinyl-, (C3-C7)-Cycloalkyl-, (C5-C7)-Cycloalkenyl-, (C4-C7)-Heterocycloalkyl-, (Hetero)arylalkyl-, (Hetero)aryl-, (Poly)fluoralkyl-, C(O)R8-, C(S)R8-, SO2R8-Rest,
R6 und R7 bedeuten unabhängig voneinander Wasserstoff, (C1-C6)-Alkyl, (C3-C6)-Alkenyl, (C3-C6)-Alkinyl, (C3-C7)-Cycloalkyl, (C5-C7)-Cycloalkenyl, (C4-C7)-Heterocycloalkyl, Arylalkyl, Heteroarylalkyl,
R6 und R7 können einen gesättigten oder ungesättigten Ring mit 5, 6 oder 7 Ringgliedern mit oder ohne Heteroatom wie O, S, N bilden, wobei dieser Ring gegebenenfalls durch einen oder mehrere Alkyl- oder Halogenreste substituiert ist,
R8 ist ein Ra- oder NRaRb-Rest,
mit Ausnahme von N-(Diethyl)-4-phenyl-1H-pyrazol-1-ethylamin,
ihre racemischen Mischungen, Enantiomere, Diastereoisomere und ihre Mischungen, ihre Tautomere und ihre pharmazeutisch annehmbaren Salze für die Herstellung eines Arzneimittels.

10. Verbindung nach Anspruch 8, der Formel (I), mit den folgenden Bedeutungen: 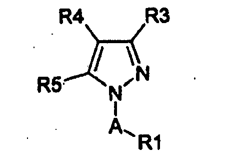 A, falls vorhanden, ist ein (C1-C6)-Alkylrest, ein (C3-C6)-Alkenylrest, ein (C3-C6)-Alkinylrest, ein (C3-C7)-Cycloalkylrest, ein (C5-C7)-Cycloalkenylrest, wobei diese Reste gegebenenfalls durch 1 oder mehrere Substituenten aus der Reihe (C1-C5)-Alkyl, (C2-C5)-Alkenyl, (C2-C5)-Alkinyl, (C3-C7)-Cycloalkyl, (C5-C7)-Cycloalkenyl, Arylalkyl, Heteroarylalkyl, Aryl, Heteroaryl, Halogen substituiert sind,
R1 ist eine NR6R7-, eine (C4-C7)-Azacycloalkyl-, eine (C5-C7)-Azacycloalkenyl-, eine (C5-C9)-Azabicycloalkyl-, eine (C5-C9)-Azabicycloalkenyl-Gruppe, wobei diese Gruppen gegebenenfalls durch 1 oder mehrere Sustituenten aus der Reihe (C1-C5)-Alkyl, (C3-C5)-Cycloalkyl, Halogen substituiert sind,
A-R1 ist derart, daß der Stickstoff von R1 und der Stickstoff 1 des Pyrazols immer durch mindestens zwei Kohlenstoffatome getrennt sind,
R3 ist ein OH-, NH2-, OMe-, H-Rest,
R4 ist ein Aryl- oder ein Heteroarylrest, der gegebenenfalls durch 1 oder mehrere Substituenten aus der Reihe Halogen, CN, NO₂, NH₂, OH, SH, COOH, CHO, C(O)NH2, C(S)NH2, SO2H, SO2NH2, NHCHO, C (O) Ra, C (O) ORa, C (O) NRaRb, C(S)NRaRb, S (O) Ra, SO₂Ra, SO₂NRaRb, ORc, SRc, O-C (O) Ra, -O-C (S) Ra, NRaRb, NHC(O)Ra, NHC(S)Ra, NHCONH2, NHCONRaRb, NHSO₂Ra, Aryl, Heteroaryl, (C4-C7)-Heterocycloalkyl, Polyfluoralkyl, Trifluormethylsulfanyl, Trifluormethoxy, geradkettiges oder verzweigtes (C1-C6)-Alkyl, (C2-C6)-Alkenyl, (C2-C6)-Alkinyl substituiert ist, wobei diese Substituenten gegebenfalls durch einen oder mehrere Reste Alkyl, Halogen, OH, Methoxy substituiert sind,
R5 ist Wasserstoff,
Ra ist geradkettiges oder verzweigtes (C1-C6)-Alkyl, Alkenyl, Alkinyl, (C3-C7)-Cycloalkyl, (C5-C7)-Cycloalkenyl, (C4-C7)-Heterocycloalkyl, Arylalkyl, Heteroarylalkyl, Aryl, Heteroaryl, Polyfluoralkyl,
Rb ist unabhängig von Ra Wasserstoff, geradkettiges oder verzweigtes (C1-C6)-Alkyl, Alkenyl, Alkinyl, (C3-C7)-Cycloalkyl, (C5-C7)-Cycloalkenyl, (C4-C7)-Heterocycloalkyl, Arylalkyl, Heteroarylalkyl, Aryl, Heteroaryl, Polyfluoralkyl,
Ra und Rb können einen gesättigten oder ungesättigten Ring mit 5, 6 oder 7 Ringgliedern mit oder ohne Heteroatom wie O, S, N bilden, wobei dieser Ring gegebenenfalls durch einen oder mehrere Alkyl- oder Halogenreste substituiert ist,
Rc ist ein geradkettiger oder verzweigter (C1-C6)-Alkyl-, (C3-C6)-Alkenyl-, (C3-C6)-Alkinyl-, (C3-C7)-Cycloalkyl-, (C5-C7)-Cycloalkenyl-, (C4-C7)-Heterocycloalkyl-, (Hetero)arylalkyl-, (Hetero)aryl-, (Poly)fluoralkyl-, C(O)R8-, C(S)R8-, SO2R8-Rest,
R6 und R7 bedeuten unabhängig voneinander Wasserstoff, (C1-C6)-Alkyl, (C3-C6)-Alkenyl, (C3-C6)-Alkinyl, (C3-C7)-Cycloalkyl, (C5-C7)-Cycloalkenyl, (C4-C7)-Heterocycloalkyl, Arylalkyl, Heteroarylalkyl,
R6 und R7 können einen gesättigten oder ungesättigten Ring mit 5, 6 oder 7 Ringgliedern mit oder ohne Heteroatom wie O, S, N bilden, wobei dieser Ring gegebenenfalls durch einen oder mehrere Alkyl- oder Halogenreste substituiert ist,
R8 ist ein Ra- oder NRaRb-Rest,
mit Ausnahme von N-(Diethyl)-4-phenyl-1H-pyrazol-1-ethylamin,
ihre racemischen Mischungen, Enantiomere, Diastereoisomere und ihre Mischungen, ihre Tautomere und ihre pharmazeutisch annehmbaren Salze für die Herstellung eines Arzneimittels.

11. Verwendung von mindestens einer Verbindung nach einem der Ansprüche 1-4 zur Herstellung eines Arzneimittels für die Behandlung von Krankheiten, die auf eine Unterfunktion der α7-nikotinergen Rezeptoren zurückzuführen sind oder die günstig auf eine Modulation derselben ansprechen.

12. Verwendung von mindestens einer Verbindung nach einem der Ansprüche 1-4 zur Herstellung eines Arzneimittels für die Behandlung, die Vorbeugung, die Diagnose und/oder die Nachsorge der Entstehung von psychiatrischen oder neurologischen Störungen oder Erkrankungen des Zentralnervensystems, an denen Veränderungen der kognitiven Funktionen oder der Behandlung der sensorischen Informationen beteiligt sind.

13. Verwendung nach Anspruch 12, **dadurch gekennzeichnet, daß** die Krankheit oder die Störung die kognitiven Funktionen, das Vermögen der Aufmerksamkeit, die Konzentrationsfähigkeit, die Lernfähigkeit und/oder die Erinnerungsfähigkeit betreffen.

14. Verwendung nach Anpruch 12, **dadurch gekennzeichnet, daß** es sich bei der Krankheit um Morbus Alzheimer und die damit assoziierten kognitiven Störungen handelt.

15. Verwendung nach Anspruch 12, **dadurch gekennzeichnet, daß** es sich bei der Krankheit um Schizophrenie handelt.

16. Verwendung von mindestens einer Verbindung nach einem der Ansprüche 1-4 für die Herstellung eines Arzneimittels zur Einführung einer Reduktion der Aufnahme von Suchtmitteln, zur Unterstützung der Aufrechterhaltung der Abstinenz gegenüber diesen Suchtmitteln oder zur Linderung der Entzugssymptome und für seine therapeutische Anwendung in der Behandlung von akuten oder chronischen Nervendegenerationserscheinungen.
